# EUROPEAN PATENT APPLICATION

(11) **EP 4 122 927 A1**
(43) Date of publication of application: **25.01.2023**
(21) Application number: 21770539.1
(22) Date of filing: 16.03.2021
(51) Int. Cl.: C07D 401/14, C07D 403/14, C07D 403/04, A61K 31/506, A61K 31/444, A61P 35/00, A61P 37/00

(54) **SUBSTITUTED PYRIMIDINE OR PYRIDINE AMINE DERIVATIVE, COMPOSITION THEREOF, AND MEDICAL USE THEREOF**

(30) Priority: 16.03.2020 CN 202010183457; 16.11.2020 CN 202011279917
(71) Applicant: Shanghai Haiyan Pharmaceutical Technology Co., Ltd., Shanghai 201203 (CN); Yangtze River Pharmaceutical Group Co., Ltd., Taizhou, Jiangsu 225321 (CN)
(72) Inventor: LIU, Lifeng, Shanghai 201203 (CN); ZHAO, Zhiming, Shanghai 201203 (CN); XU, Feng, Shanghai 201203 (CN); WANG, Hailong, Shanghai 201203 (CN); XI, Baoxin, Shanghai 201203 (CN); TONG, Zhongan, Shanghai 201203 (CN); GUAN, Huiping, Shanghai 201203 (CN)
(74) Representative: DREISS Patentanwälte PartG mbB
(86) International application number: PCT/CN2021/081126
(87) International publication number: WO 2021/185256

(57) **Abstract**

Provided is a substituted pyrimidine or pyridine amine derivative represented by formula (I), and a pharmaceutically acceptable salt, solvate, stereoisomer, prodrug, and pharmaceutical composition thereof, as well as medical application thereof. The derivative has significant adenosine A_{2A} receptor and/or adenosine A_{2B} receptor antagonistic activities.

## Description

### TECHNICAL FIELD

The present disclosure relates to the technical field of medicines, and particularly relates to a substituted pyrimidine or pyridine amine derivative, or a pharmaceutically acceptable salt, solvate, stereoisomer, or prodrug, a pharmaceutical composition thereof, as well as medical use thereof.

### BACKGROUND

Adenosine is an endogenous nucleoside present throughout human cells, consists of an adenine and a ribose, and is widely distributed in and outside cells. Adenosine is involved in a variety of physiological and biochemical functions in the body. For example, adenosine directly enters cardiac muscles to be phosphorylated to form adenosine triphosphate (ATP), which is involved in myocardial energy metabolism. In the central nervous system (CNS), adenosine controls neurotransmitter release and postsynaptic neuronal responses to regulate movements, protect neurons, and affect important life processes such as sleeping and awakening. In a pathological state, the concentration of extracellular adenosine will significantly increase in the case of tumor or hypoxia. Adenosine can play an important role in tumor immunosuppression by promoting tumor angiogenesis, proliferation, progression, and tumor metastasis.

Adenosine receptors (ARs) belong to the family of guanosine-binding protein coupled receptors (GPCRs), and the endogenous ligand thereof is adenosine. Currently known adenosine receptors include four subtypes, i.e., A1, A2a, A2b, and A3. Binding of adenosine to the A1 or A3 receptor can inhibit production of cyclic adenosine monophosphate (cAMP), and binding of adenosine to the A2a or A2b receptor can activate adenosine-activating enzyme so as to up-regulate the level of cAMPs, thereby playing a role in further physiological regulation.

The A1 receptor and the A3 receptor are mainly expressed in CNS, and the A2a receptor and the A2b receptor are expressed in both of CNS and the peripheral system. In a tumor microenvironment, the A2a receptor and the A2b receptor are widely expressed in immune cells and have strong immunosuppressive activity. The increase in the concentration of extracellular adenosine is one of the important mechanisms of action of tumor cell immune escape, and the concentration of extracellular adenosine is determined by the ATP level as well as the expression of CD39 and CD73. The increase in the concentration of extracellular adenosine is associated with release of abundant ATPs in response to apoptosis or hypoxia in the tumor microenvironment, and the concentration will be 10 to 20 times that in normal tissues. Binding of adenosine to an adenosine receptor in the tumor microenvironment can inhibit an anti-tumor response such as inhibiting the functions of CD8+ T cells, improving the functions of immunosuppression regulatory T cells, and inhibiting the functions of dendritic cells serving as antigen-presenting cells. The latest studies show that the binding of adenosine to the A2a receptor can further inhibit the tumor-killing effect of natural killer cells. Further studies show that an A2a adenosine receptor agonist can improve the activity and the tumor-killing capability of dendritic antigen-presenting cells, T cells, and natural killer cells, inhibit the functions of regulatory T cells (T-regs), myeloid-derived suppressor cells (MDSCs), and tumor-associated macrophages (TAMs), eliminate tumor immune tolerance, and promote a tumor immune response, thereby inhibiting tumor growth and prolonging the lifetime of a mouse. In addition, it has been reported that the A2b receptor can promote tumor metastasis in mouse models of melanoma and triple negative breast cancer, which means that an A2b receptor agonist can be used as an effective target for cancer treatment. Therefore, blocking the activation of adenosine signaling pathway to reduce or release immunosuppression and improve the anti-tumor functions of immune cells, especially T cells, is considered as one of the effective cancer treatment methods. An A2a/A2b dual-receptor agonist was used to block the activation of these two receptors, that is, mechanically, different immune cell populations are regulated to comprehensively block an immunosuppressive action of adenosine in a microenvironment, which has far-reaching clinical application value for tumor treatment.

At present, administration of an adenosine receptor agonist alone or administration of an adenosine receptor agonist together with other chemotherapeutic drugs/immunoregulatory drugs has become the clinical study hotspot.

### SUMMARY

An objective of the present disclosure is to provide a substituted pyrimidine or pyridine amine derivative with higher activity, higher selectivity, and lower toxicity. In a first aspect, the present disclosure provides a compound represented by Formula (I), or a pharmaceutically acceptable salt, stereoisomer, solvate or prodrug thereof: 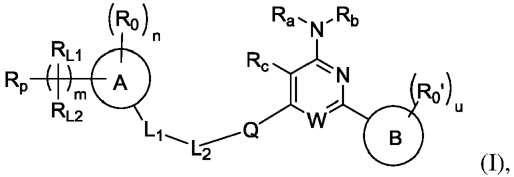
where the ring A is five- to ten-membered heteroaryl, phenyl, or pyridonyl;
the ring B is phenyl or five- to ten-membered heteroaryl;
Q is five- or six-membered heteroaryl, phenyl, C₃₋₆ cycloalkyl, four- to eight-membered heterocycloalkyl, six- to twelve-membered fused heterocycloalkyl, seven- to eleven-membered benzoheterocycloalkyl, seven- to eleven-membered heteroaryl-fused heterocycloalkyl, seven- to eleven-membered spirocyclyl, or seven- to eleven-membered heterospirocyclyl, wherein the five- or six-membered heteroaryl, the phenyl, the C₃₋₆ cycloalkyl, the four- to eight-membered heterocycloalkyl, the six- to twelve-membered fused heterocycloalkyl, the seven- to eleven-membered benzoheterocycloalkyl, the seven- to eleven-membered heteroaryl-fused heterocycloalkyl, the seven- to eleven-membered spirocyclyl, and the seven- to eleven-membered heterospirocyclyl are unsubstituted or substituted with one, two, or three substituents each independently selected from the group consisting of deuterium, halogen, cyano, hydroxyl, carboxyl, oxo, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxy, NRₐ₀R_{b0}, -C(O)(C₁₋₃ alkyl), -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
L₁ and L₂ are each independently a bond, NR₁', CR₂'R₃', O, S, or C(O), provided that L₁ and L₂ are not both O or S;
R₁', R₂', and R₃' are each independently hydrogen, deuterium, cyano, hydroxyl, halogen (preferably fluorine or chlorine), or C₁₋₆ alkyl; or R₁' is linked to R₂' to form a three-to eight-membered heterocycloalkyl ring, or R₂' is linked to R₃' to form a three- to eight-membered heterocycloalkyl ring, wherein the three- to eight-membered heterocycloalkyl ring is unsubstituted or substituted with one, two, or three substituents each independently selected from the group consisting of deuterium, halogen, cyano, hydroxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
W is N or CR_{w};
R_{w} is cyano, hydroxyl, halogen (preferably fluorine or chlorine), halogenated C₁₋₈ alkyl (preferably halogenated C₁₋₆ alkyl, and more preferably halogenated C₁₋₃ alkyl), halogenated C₁₋₈ alkoxy (preferably halogenated C₁₋₆ alkoxy, and more preferably halogenated C₁₋₃ alkoxy), or C₁₋₈ alkoxy (preferably C₁₋₆ alkoxy, and more preferably C₁₋₃ alkoxy);
R_{c}, Rₐ, and R_{b} are defined as follows:
   (i) R_{c} is fluorine, chlorine, cyano, C₁₋₃ alkoxy, or halogenated C₁₋₃ alkoxy;
      Rₐ and R_{b} are each independently hydrogen, deuterium, C₁₋₈ alkyl (preferably C₁₋₆ alkyl, and more preferably C₁₋₃ alkyl), C₁₋₈ alkoxy (preferably C₁₋₆ alkoxy, and more preferably C₁₋₃ alkoxy), -C(O)(C₁₋₈ alkyl) (preferably -C(O)(C₁₋₆ alkyl), and more preferably -C(O)(C₁₋₃ alkyl)), -(CH₂)ₜ(C₃₋₈ cycloalkyl) (preferably -(CH₂)ₜ(C₃₋₆ cycloalkyl)), -(CH₂)ₜ-(three- to eight-membered heterocycloalkyl), or a structure represented by Formula 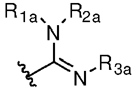 (a): Formula (a) , wherein the C₁₋₈ alkyl, the -C(O)(C₁₋₈ alkyl), and the -(CH₂)ₜ-(three-to eight-membered heterocycloalkyl) are unsubstituted or substituted with one, two, or three substituents each independently selected from the group consisting of deuterium, halogen, cyano, hydroxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl; or
      Rₐ and R_{b} together with the nitrogen atom linked thereto form a five- or six-membered saturated or partially unsaturated heteromonocyclic ring, wherein the five- or six-membered saturated or partially unsaturated heteromonocyclic ring is unsubstituted or substituted with one, two, or three substituents each independently selected from the group consisting of deuterium, halogen, cyano, hydroxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl,
      where R₁ₐ is hydrogen or C₁₋₃ alkyl; and R₂ₐ and R₃ₐ are each independently hydrogen or C₁₋₃ alkyl, or R₂ₐ is linked to R₃ₐ to form a five- to eight-membered heterocycloalkenyl ring or a five- or six-membered heteroaryl ring, wherein the five- to eight-membered heterocycloalkenyl ring contains two, three, or four nitrogen atoms and zero, one, or two oxygen atoms, the five- or six-membered heteroaryl ring contains two, three, or four nitrogen atoms and zero or one oxygen atom, and the five- to eight-membered heterocycloalkenyl ring and the five- or six-membered heteroaryl ring are unsubstituted or substituted with one, two, or three substituents each independently selected from the group consisting of halogen, cyano, hydroxyl, C₁₋₃ alkyl, C₁₋₃ alkoxy, halogenated C₁₋₃ alkyl, and halogenated C₁₋₃ alkoxy; or
   (ii) R_{c} is linked to Rₐ to form a fused five- or six-membered saturated or partially unsaturated heteromonocyclic ring, or a fused five- or six-membered heteroaryl ring, wherein the fused five- or six-membered saturated or partially unsaturated heteromonocyclic ring and the fused five- or six-membered heteroaryl ring are unsubstituted or substituted with one, two, or three substituents each independently selected from the group consisting of deuterium, halogen, cyano, hydroxyl, oxo, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
      R_{b} is hydrogen, deuterium, C₁₋₈ alkyl (preferably C₁₋₆ alkyl, and more preferably C₁₋₃ alkyl), -C(O)(C₁₋₈ alkyl) (preferably -C(O)(C₁₋₆ alkyl), and more preferably -C(O)(C₁₋₃ alkyl)), -(CH₂)ₜ(C₃₋₈ cycloalkyl) (preferably -(CH₂)ₜ(C₃₋₆ cycloalkyl)), -(CH₂)ₜ-(three- to eight-membered heterocycloalkyl) or a structure represented by Formula (a), wherein the C₁₋₈ alkyl, the -C(O)(C₁₋₈ alkyl), and the -(CH₂)ₜ-(three- to eight-membered heterocycloalkyl) are unsubstituted or substituted with one, two, or three substituents each independently selected from the group consisting of deuterium, halogen, cyano, hydroxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
      t is 0, 1, 2, or 3;
      R_{L1} and R_{L2} are each independently hydrogen, hydroxyl, halogen, C₁₋₃ alkyl, C₁₋₃ alkoxy, or halogenated C₁₋₃ alkyl; or R_{L1} and R_{L2} together with the carbon atom linked thereto form a three- to seven-membered saturated or partially unsaturated monocyclic ring or a three- to seven-membered saturated or partially unsaturated heteromonocyclic ring, wherein the three- to seven-membered saturated or partially unsaturated monocyclic ring and the three- to seven-membered saturated or partially unsaturated heteromonocyclic ring are unsubstituted or substituted with one, two, or three substituents each independently selected from the group consisting of deuterium, halogen, cyano, hydroxyl, carboxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
      m is 0, 1, 2, or 3;
      Rₚ is hydrogen, hydroxyl, carboxyl, cyano, C₁₋₈ alkyl (preferably C₁₋₆ alkyl, and more preferably C₁₋₃ alkyl), halogenated C₁₋₈ alkyl (preferably halogenated C₁₋₆ alkyl, and more preferably halogenated C₁₋₃ alkyl), halogenated C₁₋₈ alkoxy (preferably halogenated C₁₋₆ alkoxy, and more preferably halogenated C₁₋₃ alkoxy), C₁₋₈ alkoxy (preferably C₁₋₆ alkoxy, and more preferably C₁₋₃ alkoxy), C₃₋₈ cycloalkyl (preferably C₃₋₆ cycloalkyl), -SO₂(C₁₋₈ alkyl) (preferably -SO₂(C₁₋₆ alkyl), and more preferably -SO₂(C₁₋₃ alkyl)), -SO₂NRₐ₀R_{b0}, -(PO)(C₁₋₃ alkyl)₂, -NHSO₂(C₁₋₃ alkyl), -C(O)NRₐ₀R_{b0}, -C(O)NRₐ₁R_{b1}, three- to six-membered heterocycloalkyl, -X-(CRₚ₁Rₚ₂)_{q}-(three- to six-membered heterocycloalkyl), eight- to ten-membered heterocycloalkyl, seven- to eleven-membered heterospirocyclyl, five- or six-membered heteroaryl, eight- to ten-membered heteroaryl, NRₐ₁R_{b1}, or NRₐ'R_{b}', wherein the C₃₋₈ cycloalkyl, the three- to six-membered heterocycloalkyl, the eight- to ten-membered heterocycloalkyl, the seven- to eleven-membered heterospirocyclyl, the five- or six-membered heteroaryl, and the eight- to ten-membered heteroaryl are unsubstituted or substituted with one, two, or three substituents each independently selected from the group consisting of deuterium, halogen, cyano, hydroxyl, carboxyl, oxo, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, NRₐ₀R_{b0}, -C(O)(C₁₋₃ alkyl), -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
      X is O or NRₚ₃;
      Rₚ₁ and Rₚ₂ are each independently hydrogen, hydroxyl, halogen, or C₁₋₃ alkyl;
      Rₚ₃ is hydrogen or C₁₋₃ alkyl;
      q is 0, 1, 2, or 3;
      Rₐ' and R_{b}' together with the nitrogen atom linked thereto form a four- to eight-membered saturated heteromonocyclic ring, an eight- to ten-membered saturated heterobicyclic ring, or a seven- to eleven-membered heterospiro ring, wherein the four- to eight-membered saturated heteromonocyclic ring, the eight- to ten-membered saturated heterobicyclic ring, and the seven- to eleven-membered heterospiro ring are unsubstituted or substituted with one, two, or three substituents each independently selected from the group consisting of halogen, cyano, hydroxyl, carboxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -(CH₂)ₜ₁-NRₐ₀R_{b0}, -(CH₂)ₜ₁-SO₂(C₁₋₃ alkyl), -(CH₂)ₜ₁-S(O)(C₁₋₃ alkyl), -(CH₂)ₜ₁-C(O)NRₐ₀R_{b0}, -(CH₂)ₜ₁-C(O)O(C₁₋₃ alkyl), -(CH₂)ₜ₁-OC(O)(C₁₋₃ alkyl), -(CH₂)ₜ₁-(C₃₋₆ cycloalkyl), C₃₋₆ cycloalkyloxy, and -(CH₂)ₜ₁-(three- to six-membered heterocycloalkyl);
      tl is independently selected from 0, 1, 2, or 3;
      (R₀)ₙ refers to n substituents R₀ replacing n hydrogen atoms on the ring A, where n is 0, 1, 2 or 3, and the n substituents R₀ are identical or different from each other and are each independently halogen, cyano, hydroxyl, carboxyl, C₁₋₃ alkyl, C₁₋₃ alkoxy, hydroxyl-substituted C₁₋₃ alkyl, C₁₋₃ alkoxy-substituted C₁₋₃ alkyl, C₂₋₄ alkenyl, C₂₋₄ alkynyl, halogenated C₁₋₃ alkyl, halogenated C₁₋₃ alkoxy, -SO₂(C₁₋₃ alkyl), -SO₂NRₐ₀R_{b0}, -N(Rₐ₀)SO₂(C₁₋₃ alkyl), -P(O)Rₐ₀R_{b0}, -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, or three- to six-membered heterocycloalkyl;
      (R₀')ᵤ refers to u substituents R₀' replacing u hydrogen atoms on the ring B, wherein u is 0, 1, 2, 3, 4 or 5, and the u substituents R₀' are identical or different from each other and are each independently hydrogen, cyano, acetyl, hydroxyl, carboxyl, halogen (preferably fluorine or chlorine), NRₐ₀R_{b0}, C₁₋₈ alkyl (preferably C₁₋₆ alkyl, and more preferably C₁₋₃ alkyl), C₁₋₈ alkoxy (preferably C₁₋₆ alkoxy, and more preferably C₁₋₃ alkoxy), C₃₋₈ cycloalkyl (preferably C₃₋₆ cycloalkyl), three- to six-membered heterocycloalkyl, five- or six-membered heteroaryl, or C₆₋₁₀ aryl, wherein the C₁₋₈ alkyl, the C₁₋₈ alkoxy, the C₃₋₈ cycloalkyl, the three- to six-membered heterocycloalkyl, the five- or six-membered heteroaryl, and the C₆₋₁₀ aryl are unsubstituted or substituted with one, two, or three substituents each independently selected from the group consisting of deuterium, halogen, cyano, hydroxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, C₃₋₆ cycloalkyl, C₃₋₆ cycloalkyloxy, three- to six-membered heterocycloalkyl, phenyl, and five- or six-membered heteroaryl;
      Rₐ₀ and R_{b0} are each independently hydrogen or C₁₋₃ alkyl; or Rₐ₀ and R_{b0} together with the nitrogen atom linked thereto form a four- to six-membered saturated heteromonocyclic ring, wherein the four- to six-membered saturated heteromonocyclic ring is optionally substituted with one, two, or three substituents each independently selected from the group consisting of deuterium, halogen, cyano, hydroxyl, carboxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, and three- to six-membered heterocycloalkyl;
      Rₐ₁ and R_{b1} are each independently hydrogen, C₁₋₃ alkyl, C₃₋₆ cycloalkyl, three- to six-membered heterocycloalkyl or -(CR^{a}R^{b})ₛ-R^{c}, wherein the three- to six-membered heterocycloalkyl is unsubstituted or substituted with one, two, or three substituents each independently selected from the group consisting of deuterium, halogen, cyano, hydroxyl, carboxyl, oxo, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)(C₁₋₃ alkyl), -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
      R^{a} and R^{b} are each independently hydrogen, hydroxyl, halogen, C₁₋₃ alkyl, C₁₋₃ alkoxy, or halogenated C₁₋₃ alkyl; or R^{a} and R^{b} together with the carbon atom linked thereto form a three- to seven-membered saturated or partially unsaturated monocyclic ring or a three- to seven-membered saturated or partially unsaturated heteromonocyclic ring, wherein the three- to seven-membered saturated or partially unsaturated monocyclic ring and the three- to seven-membered saturated or partially unsaturated heteromonocyclic ring are unsubstituted or substituted with one, two, or three substituents each independently selected from the group consisting of deuterium, halogen, cyano, hydroxyl, carboxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
      R^{c} is hydrogen, hydroxyl, carboxyl, C₁₋₈ alkyl (preferably C₁₋₆ alkyl, and more preferably C₁₋₃ alkyl), halogenated C₁₋₈ alkyl (preferably halogenated C₁₋₆ alkyl, and more preferably halogenated C₁₋₃ alkyl), halogenated C₁₋₈ alkoxy (preferably halogenated C₁₋₆ alkoxy, and more preferably halogenated C₁₋₃ alkoxy), C₁₋₈ alkoxy (preferably C₁₋₆ alkoxy, and more preferably C₁₋₃ alkoxy), or C₃₋₈ cycloalkyl (preferably C₃₋₆ cycloalkyl); and
      s is 1, 2, or 3.

More preferably, in the compound represented by Formula (I), the ring A is five- to ten-membered heteroaryl, phenyl, or pyridonyl;
the ring B is phenyl or five- to ten-membered heteroaryl;
Q is five- or six-membered heteroaryl, phenyl, C₃₋₆ cycloalkyl, four- to eight-membered heterocycloalkyl, six- to twelve-membered fused heterocycloalkyl, seven- to eleven-membered benzoheterocycloalkyl, seven- to eleven-membered heteroaryl-fused heterocycloalkyl, seven- to eleven-membered spirocyclyl, or seven- to eleven-membered heterospirocyclyl, wherein the five- or six-membered heteroaryl, the phenyl, the C₃₋₆ cycloalkyl, the four- to eight-membered heterocycloalkyl, the six- to twelve-membered fused heterocycloalkyl, the seven- to eleven-membered benzoheterocycloalkyl, the seven- to eleven-membered heteroaryl-fused heterocycloalkyl, the seven- to eleven-membered spirocyclyl, and the seven- to eleven-membered heterospirocyclyl are unsubstituted or substituted with one, two, or three substituents each independently selected from the group consisting of deuterium, halogen, cyano, hydroxyl, carboxyl, oxo, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, NRₐ₀R_{b0}, -C(O)(C₁₋₃ alkyl), -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
L₁ and L₂ are each independently a bond, NR₁', CR₂'R₃', O, S, or C(O), provided that L₁ and L₂ are not both O or S;
R₁', R₂', and R₃' are each independently hydrogen, deuterium, cyano, hydroxyl, halogen (preferably fluorine or chlorine), or C₁₋₆ alkyl; or R₁' is linked to R₂' to form a three-to eight-membered heterocycloalkyl ring, or R₂' is linked to R₃' to form a three- to eight-membered heterocycloalkyl ring, wherein the three- to eight-membered heterocycloalkyl ring is unsubstituted or substituted with one, two, or three substituents each independently selected from the group consisting of deuterium, halogen, cyano, hydroxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
W is N or CR_{w};
R_{w} is cyano, hydroxyl, halogen (preferably fluorine or chlorine), halogenated C₁₋₈ alkyl (preferably halogenated C₁₋₆ alkyl, and more preferably halogenated C₁₋₃ alkyl), halogenated C₁₋₈ alkoxy (preferably halogenated C₁₋₆ alkoxy, and more preferably halogenated C₁₋₃ alkoxy), or C₁₋₈ alkoxy (preferably C₁₋₆ alkoxy, and more preferably C₁₋₃ alkoxy);
R_{c}, Rₐ, and R_{b} are defined as follows:
   (i) R_{c} is fluorine, chlorine, cyano, C₁₋₃ alkoxy, or halogenated C₁₋₃ alkoxy;
      Rₐ and R_{b} are each independently hydrogen, deuterium, C₁₋₈ alkyl (preferably C₁₋₆ alkyl, and more preferably C₁₋₃ alkyl), -C(O)(C₁₋₈ alkyl) (preferably -C(O)(C₁₋₆ alkyl), and more preferably -C(O)(C₁₋₃ alkyl)), -(CH₂)ₜ(C₃₋₈ cycloalkyl) (preferably -(CH₂)ₜ(C₃₋₆ cycloalkyl)), -(CH₂)ₜ-(three- to eight-membered heterocycloalkyl), or a structure represented 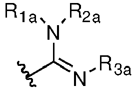 by Formula (a): Formula (a) , wherein the C₁₋₈ alkyl, the -C(O)(C₁₋₈ alkyl), and the -(CH₂)ₜ-(three- to eight-membered heterocycloalkyl) are unsubstituted or substituted with one, two, or three substituents each independently selected from the group consisting of deuterium, halogen, cyano, hydroxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl; or
      Rₐ and R_{b} together with the nitrogen atom linked thereto form a five- or six-membered saturated or partially unsaturated heteromonocyclic ring, wherein the five- or six-membered saturated or partially unsaturated heteromonocyclic ring is unsubstituted or substituted with one, two, or three substituents each independently selected from the group consisting of deuterium, halogen, cyano, hydroxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl,
      where R₁ₐ is hydrogen or C₁₋₃ alkyl; R₂ₐ and R₃ₐ are each independently hydrogen or C₁₋₃ alkyl, or R₂ₐ is linked to R₃ₐ to form a five- to eight-membered heterocycloalkenyl ring or a five- or six-membered heteroaryl ring, wherein the five- to eight-membered heterocycloalkenyl ring contains two, three, or four nitrogen atoms and zero, one, or two oxygen atoms, the five- or six-membered heteroaryl ring contains two, three, or four nitrogen atoms and zero or one oxygen atom, and the five- to eight-membered heterocycloalkenyl ring and the five- or six-membered heteroaryl ring are unsubstituted or substituted with one, two, or three substituents each independently selected from the group consisting of halogen, cyano, hydroxyl, C₁₋₃ alkyl, C₁₋₃ alkoxy, halogenated C₁₋₃ alkyl, and halogenated C₁₋₃ alkoxy;
   (ii) R_{c} is linked to Rₐ to form a fused five- or six-membered saturated or partially unsaturated heteromonocyclic ring, or a fused five- or six-membered heteroaryl ring, wherein the fused five- or six-membered saturated or partially unsaturated heteromonocyclic ring and the fused five- or six-membered heteroaryl ring are unsubstituted or substituted with one, two, or three substituents each independently selected from the group consisting of deuterium, halogen, cyano, hydroxyl, oxo, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
      R_{b} is hydrogen, deuterium, C₁₋₈ alkyl (preferably C₁₋₆ alkyl, and more preferably C₁₋₃ alkyl), -C(O)(C₁₋₈ alkyl) (preferably -C(O)(C₁₋₆ alkyl), and more preferably -C(O)(C₁₋₃ alkyl)), -(CH₂)ₜ(C₃₋₈ cycloalkyl) (preferably -(CH₂)ₜ(C₃₋₆ cycloalkyl)), -(CH₂)ₜ-(three- to eight-membered heterocycloalkyl), or a structure represented by Formula (a), wherein the C₁₋₈ alkyl, the -C(O)(C₁₋₈ alkyl), and the -(CH₂)ₜ-(three- to eight-membered heterocycloalkyl) are unsubstituted or substituted with one, two, or three substituents each independently selected from the group consisting of deuterium, halogen, cyano, hydroxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
      t is 0, 1, 2, or 3;
      R_{L1} and R_{L2} are each independently hydrogen, hydroxyl, halogen, C₁₋₃ alkyl, C₁₋₃ alkoxy, or halogenated C₁₋₃ alkyl; or R_{L1} and R_{L2} together with the carbon atom linked thereto form a three- to seven-membered saturated or partially unsaturated monocyclic ring or a three- to seven-membered saturated or partially unsaturated heteromonocyclic ring, wherein the three- to seven-membered saturated or partially unsaturated monocyclic ring and the three- to seven-membered saturated or partially unsaturated heteromonocyclic ring are unsubstituted or substituted with one, two, or three substituents each independently selected from the group consisting of deuterium, halogen, cyano, hydroxyl, carboxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
      m is 0, 1, 2, or 3;
      Rₚ is hydrogen, hydroxyl, carboxyl, C₁₋₈ alkyl (preferably C₁₋₆ alkyl, and more preferably C₁₋₃ alkyl), halogenated C₁₋₈ alkyl (preferably halogenated C₁₋₆ alkyl, and more preferably halogenated C₁₋₃ alkyl), halogenated C₁₋₈ alkoxy (preferably halogenated C₁₋₆ alkoxy, and more preferably halogenated C₁₋₃ alkoxy), C₁₋₈ alkoxy (preferably C₁₋₆ alkoxy, and more preferably C₁₋₃ alkoxy), C₃₋₈ cycloalkyl (preferably C₃₋₆ cycloalkyl), -SO₂(C₁₋₈ alkyl) (preferably -SO₂(C₁₋₆ alkyl), and more preferably -SO₂(C₁₋₃ alkyl)), -SO₂NRₐ₀R_{b0}, -C(O)NRₐ₀R_{b0}, -C(O)NRₐ₁R_{b1}, three- to six-membered heterocycloalkyl, -X-(CRₚ₁Rₚ₂)_{q}-(three- to six-membered heterocycloalkyl), eight- to ten-membered heterocycloalkyl, seven- to eleven-membered heterospirocyclyl, five- or six-membered heteroaryl, eight- to ten-membered heteroaryl, NRₐ₁R_{b1}, or NRₐ'R_{b}', wherein the C₃₋₈ cycloalkyl, the three- to six-membered heterocycloalkyl, the eight- to ten-membered heterocycloalkyl, the seven- to eleven-membered heterospirocyclyl, the five- or six-membered heteroaryl, and the eight- to ten-membered heteroaryl are unsubstituted or substituted with one, two, or three substituents each independently selected from the group consisting of deuterium, halogen, cyano, hydroxyl, carboxyl, oxo, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, NRₐ₀R_{b0}, -C(O)(C₁₋₃ alkyl), -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
      X is O or NRₚ₃;
      Rₚ₁ and Rₚ₂ are each independently hydrogen, hydroxyl, halogen, or C₁₋₃ alkyl;
      Rₚ₃ is hydrogen or C₁₋₃ alkyl;
      q is 0, 1, 2, or 3;
      Rₐ' and R_{b}' together with the nitrogen atom linked thereto form a four- to eight-membered saturated heteromonocyclic ring, an eight- to ten-membered saturated heterobicyclic ring, or a seven- to eleven-membered heterospiro ring, wherein the four- to eight-membered saturated heteromonocyclic ring, the eight- to ten-membered saturated heterobicyclic ring, and the seven- to eleven-membered heterospiro ring are unsubstituted or substituted with one, two, or three substituents each independently selected from the group consisting of halogen, cyano, hydroxyl, carboxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -(CH₂)ₜ₁-NRₐ₀R_{b0}, -(CH₂)ₜ₁-SO₂(C₁₋₃ alkyl), -(CH₂)ₜ₁-S(O)(C₁₋₃ alkyl), -(CH₂)ₜ₁-C(O)NRₐ₀R_{b0}, -(CH₂)ₜ₁-C(O)O(C₁₋₃ alkyl), -(CH₂)ₜ₁-OC(O)(C₁₋₃ alkyl), -(CH₂)ₜ₁-(C₃₋₆ cycloalkyl), C₃₋₆ cycloalkyloxy, and -(CH₂)ₜ₁-(three- to six-membered heterocycloalkyl);
      tl is independently 0, 1, 2, or 3;
      (R₀)ₙ refers to that hydrogen on the ring A is substituted with n R₀, n is 0, 1, 2 or 3, and all R₀ are identical or different from each other, and are each independently halogen, cyano, hydroxyl, carboxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -SO₂NRₐ₀R_{b0}, -N(Rₐ₀)SO₂(C₁₋₃ alkyl), -P(O)Rₐ₀R_{b0}, -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, or three- to six-membered heterocycloalkyl;
      (R₀')ᵤ refers to u substituents R₀' replacing u hydrogen atoms on the ring B, wherein u is 0, 1, 2, 3, 4 or 5, the u substituents R₀' are identical or different from each other and are each independently hydrogen, cyano, acetyl, hydroxyl, carboxyl, halogen (preferably fluorine or chlorine), NRₐ₀R_{b0}, C₁₋₈ alkyl (preferably C₁₋₆ alkyl, and more preferably C₁₋₃ alkyl), C₁₋₈ alkoxy (preferably C₁₋₆ alkoxy, and more preferably C₁₋₃ alkoxy), C₃₋₈ cycloalkyl (preferably C₃₋₆ cycloalkyl), three- to six-membered heterocycloalkyl, five- or six-membered heteroaryl, or C₆₋₁₀ aryl, wherein the C₁₋₈ alkyl, the C₁₋₈ alkoxy, the C₃₋₈ cycloalkyl, the three-to six-membered heterocycloalkyl, the five- or six-membered heteroaryl, and the C₆₋₁₀ aryl are unsubstituted or substituted with one, two, or three substituents each independently selected from the group consisting of deuterium, halogen, cyano, hydroxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, C₃₋₆ cycloalkyl, C₃₋₆ cycloalkyloxy, three- to six-membered heterocycloalkyl, phenyl, and five- or six-membered heteroaryl;
      Rₐ₀ and R_{b0} are each independently hydrogen or C₁₋₃ alkyl; or Rₐ₀ and R_{b0} together with the nitrogen atom linked thereto form a four- to six-membered saturated heteromonocyclic ring, wherein the four- to six-membered saturated heteromonocyclic ring is optionally substituted with one, two, or three substituents each independently selected from the group consisting of deuterium, halogen, cyano, hydroxyl, carboxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, and three- to six-membered heterocycloalkyl;
      Rₐ₁ and R_{b1} are each independently hydrogen, C₁₋₃ alkyl, C₃₋₆ cycloalkyl, three- to six-membered heterocycloalkyl, or -(CR^{a}R^{b})ₛ-R^{c}, wherein the three- to six-membered heterocycloalkyl is unsubstituted or substituted with one, two, or three substituents each independently selected from the group consisting of deuterium, halogen, cyano, hydroxyl, carboxyl, oxo, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)(C₁₋₃ alkyl), -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
      R^{a} and R^{b} are each independently hydrogen, hydroxyl, halogen, C₁₋₃ alkyl, C₁₋₃ alkoxy, or halogenated C₁₋₃ alkyl; or R^{a} and R^{b} together with the carbon atom linked thereto form a three- to seven-membered saturated or partially unsaturated monocyclic ring or a three- to seven-membered saturated or partially unsaturated heteromonocyclic ring, wherein the three- to seven-membered saturated or partially unsaturated monocyclic ring and the three- to seven-membered saturated or partially unsaturated heteromonocyclic ring are unsubstituted or substituted with one, two, or three substituents each independently selected from the group consisting of deuterium, halogen, cyano, hydroxyl, carboxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
      R^{c} is hydrogen, hydroxyl, carboxyl, C₁₋₈ alkyl (preferably C₁₋₆ alkyl, and more preferably C₁₋₃ alkyl), halogenated C₁₋₈ alkyl (preferably halogenated C₁₋₆ alkyl, and more preferably halogenated C₁₋₃ alkyl), halogenated C₁₋₈ alkoxy (preferably halogenated C₁₋₆ alkoxy, and more preferably halogenated C₁₋₃ alkoxy), C₁₋₈ alkoxy (preferably C₁₋₆ alkoxy, and more preferably C₁₋₃ alkoxy), or C₃₋₈ cycloalkyl (preferably C₃₋₆ cycloalkyl); and
      s is 1, 2, or 3.

More preferably, the ring A is five- or six-membered heteroaryl, eight- to ten-membered heteroaryl, phenyl, or pyridonyl.

More preferably, the ring A is five- or six-membered heteroaryl, phenyl, or pyridonyl.

More preferably, the ring A is selected from the group consisting of thienyl, furyl, thiazolyl, isothiazolyl, imidazolyl, oxazolyl, pyrrolyl, pyrazolyl, triazolyl, 1,2,3-triazolyl, 1,2,4-triazolyl, 1,2,5-triazolyl, 1,3,4-triazolyl, tetrazolyl, isoxazolyl, oxadiazolyl, 1,2,3-oxadiazolyl, 1,2,4-oxadiazolyl, 1,2,5-oxadiazolyl, 1,3,4-oxadiazolyl, thiadiazolyl, pyridyl, pyridazinyl, pyrimidinyl, pyrazinyl, triazinyl, tetrazinyl, phenyl, and pyridonyl.

More preferably, the ring A is phenyl, pyridyl, pyrazolyl, pyrimidinyl, 1,2,3-triazolyl, 1,2,4-triazolyl, or pyridonyl.

More preferably, the ring A is selected from the group consisting of: 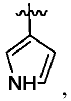 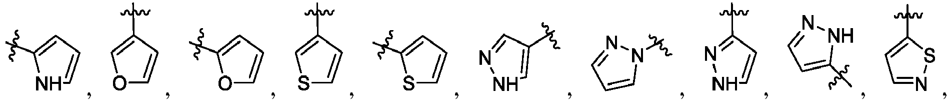 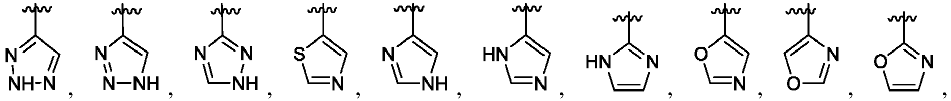 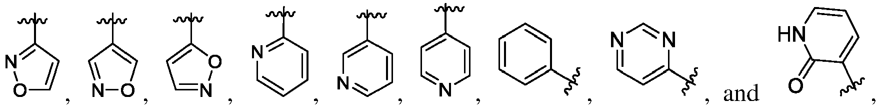 where " 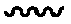 " refers to a linkage between the ring A and L1.

More preferably, the ring A is selected from the group consisting of: 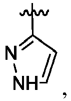 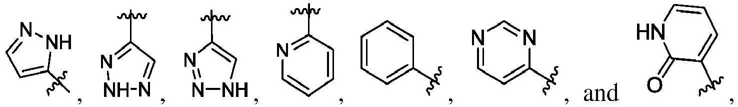 where " 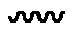 " refers to a linkage between the ring A and L1.

More preferably, the ring B is phenyl, five- or six-membered heteroaryl, or eight-to ten-membered heteroaryl.

More preferably, the ring B is phenyl.

More preferably, L₁ and L₂ are respectively a bond and CR₂'R₃'.

More preferably, L₁ is a bond; and L₂ is CR₂'R₃'.

More preferably, L₁ and L₂ are respectively a bond and CH₂.

More preferably, L₁ is a bond; and L₂ is CH₂.

More preferably, W is N.

More preferably, R_{c} is fluorine, chlorine, or C₁₋₃ alkoxy. More preferably, R_{c} is fluorine, chlorine, cyano, hydroxyl, methyl, ethyl, n-propyl, isopropyl, methoxy, ethoxy, n-propoxy, or isopropoxy.

More preferably, R_{c} is fluorine, chlorine, or methoxy.

More preferably, R_{c} is fluorine or chlorine. More preferably, Q is five- or six-membered heteroaryl, phenyl, C₃₋₆ cycloalkyl, four- to six-membered heterocycloalkyl, eight- to ten-membered fused heterocycloalkyl, eight- to ten-membered benzoheterocycloalkyl, eight- to ten-membered heteroaryl-fused heterocycloalkyl, seven- to elven-membered spirocyclyl, or seven- to eleven-membered heterospirocyclyl.

More preferably, Q is five- or six-membered heteroaryl that is selected from the following structures:

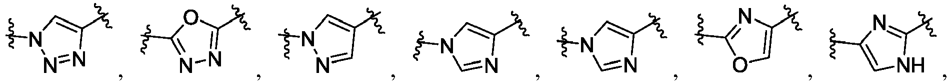

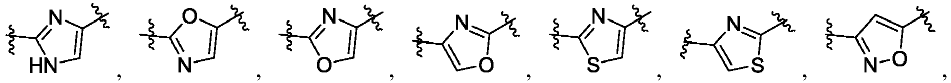

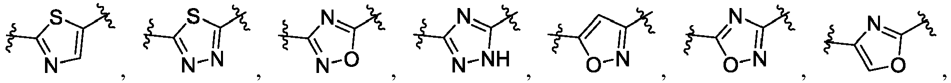

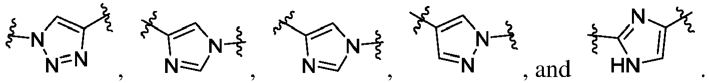

More preferably, Q is three- to six-membered heterocycloalkyl that is selected from the following structures:

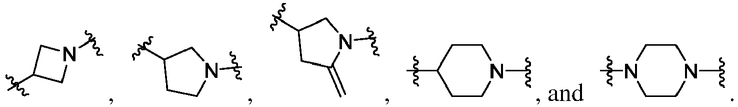

More preferably, Q is five- or six-membered heteroaryl.

More preferably, Q is pyrazolyl or 1,2,3-triazolyl.

More preferably, Q is selected from the following structures:

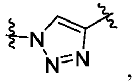

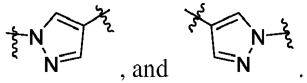

More preferably, Rₐ and R_{b} are each independently hydrogen, deuterium, C₁₋₃ alkyl, -C(O)(C₁₋₃ alkyl), -(CH₂)ₜ(C₃₋₆ cycloalkyl), -(CH₂)ₜ-(four- to six-membered heterocycloalkyl) or a structure represented by Formula (a), or Rₐ and R_{b} together with the nitrogen atom linked thereto form a five- or six-membered saturated heteromonocyclic ring, wherein the C₁₋₃ alkyl, the -C(O)(C₁₋₃ alkyl), the -(CH₂)ₜ-(four- to six-membered heterocycloalkyl), and the five- or six-membered saturated heteromonocyclic ring are unsubstituted or substituted with one, two, or three substituents each independently selected from the group consisting of deuterium, halogen, cyano, hydroxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, and four- to six-membered heterocycloalkyl, wherein the four- to six-membered heterocycloalkyl is selected from the group consisting of azetidinyl, oxetanyl, tetrahydrofuranyl, tetrahydrothienyl, tetrahydropyrrolyl, oxazolidinyl, dioxolanyl, piperidinyl, piperazinyl, morpholinyl, dioxanyl, thiomorpholinyl, thiomorpholine-1,1-dioxide, and tetrahydropyranyl; the C₃₋₆ cycloalkyl is selected from the group consisting of cyclopropyl, cyclobutyl, cyclopentyl, and cyclohexyl; and the five- or six-membered saturated heteromonocyclic ring is selected from the group consisting of a tetrahydrofuran ring, a tetrahydrothiophene ring, a tetrahydropyrrole ring, a piperidine ring, oxazolidine, a piperazine ring, dioxolane, dioxane, a morpholine ring, a thiomorpholine ring, and a tetrahydropyran ring.

More preferably, Rₐ and R_{b} are hydrogen or deuterium.

More preferably, Rₐ is hydrogen; and R_{b} is hydrogen.

More preferably, Rₐ is hydrogen; and R_{b} is a structure represented by Formula (a).

More preferably, R₂ₐ is linked to R₃ₐ to form a five- to eight-membered heterocycloalkenyl ring or a five- or six-membered heteroaryl ring, wherein the five- to eight-membered heterocycloalkenyl ring is selected from the group consisting of a 4,5-dihydro-1H-imidazole ring, a 1,4,5,6-tetrahydropyrimidine ring, a 3,4,7,8-tetrahydro-2H-1,4,6-oxadiazine ring, a 1,6-dihydropyrimidine ring, a 4,5,6,7-tetrahydro-1H-1,3-diazepine ring, and a 2,5,6,7-tetrahydro-1,3,5-oxadiazepine ring; and the five- or six-membered heteroaryl ring is selected from the group consisting of a 1H-imidazole ring, a 4H-1,2,4-triazole ring, a 1H-1,2,4-triazole ring, a pyrimidine ring, a 1,2,4-triazine ring, a 1,3,5-triazine ring, and a 1,2,4-triazine ring.

More preferably, the structure represented by Formula (a) is selected from the group consisting of:

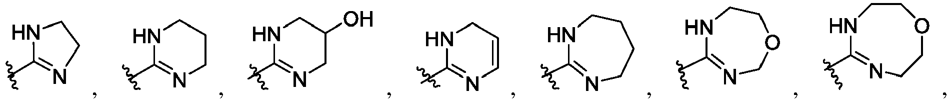

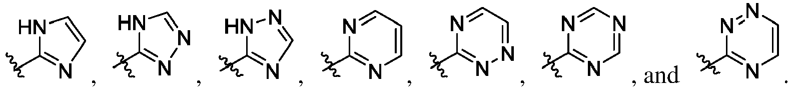

More preferably, R_{c} is linked to Rₐ to form a fused five- or six-membered saturated heteromonocyclic ring, or a fused five- or six-membered heteroaryl ring, wherein the fused five- or six-membered saturated heteromonocyclic ring and the fused five- or six-membered heteroaryl ring are unsubstituted or substituted with one, two, or three substituents each independently selected from the group consisting of deuterium, halogen, cyano, hydroxyl, oxo, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, four- to six-membered heterocycloalkyl, phenyl, and five- or six-membered heteroaryl, wherein the fused five- or six-membered saturated heteromonocyclic ring is selected from the group consisting of a tetrahydropyrrole ring, a piperidine ring, oxazolidine, a piperazine ring, a morpholine ring, a morpholin-3-one ring, a piperazin-2-one ring, and a piperidin-2-one ring; and the fused five- or six-membered heteroaryl ring is selected from the group consisting of a thiophene ring, a furan ring, a thiazole ring, an imidazole ring, an oxazole ring, a pyrrole ring, a pyrazole ring, a triazole ring, a 1,2,3-triazole ring, a 1,2,4-triazole ring, a 1,2,5-triazole ring, a 1,3,4-triazole ring, a tetrazole ring, an isoxazole ring, an oxadiazole ring, a 1,2,3-oxadiazole ring, a 1,2,4-oxadiazole ring, a 1,2,5-oxadiazole ring, a 1,3,4-oxadiazole ring, a thiadiazole ring, a pyridine ring, a pyridazine ring, a pyrimidine ring, and a pyrazine ring.

More preferably, R_{b} is hydrogen; and R_{c} is linked to Rₐ to form a fused five- or six-membered saturated heteromonocyclic ring.

More preferably, R_{b} is hydrogen; and R_{c} is linked to Rₐ to form

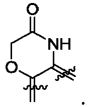

More preferably, (R₀)ₙ refers to n substituents R₀ repalcing n hydrogen atoms on the ring A, wherein n is 0 or 1, and R₀ is halogen (preferably fluorine or chlorine), hydroxyl, C₁₋₃ alkyl, C₁₋₃ alkoxy, hydroxyl-substituted C₁₋₃ alkyl, C₁₋₃ alkoxy-substituted C₁₋₃ alkyl (preferably methoxy-substituted C₁₋₃ alkyl), or C₃₋₆ cycloalkyl (preferably cyclopropyl).

More preferably, (R₀)ₙ refers to n substituents R₀ replacing n hydrogen atoms on the ring A, wherein n is 0 or 1, and R₀ is halogen (preferably fluorine or chlorine), C₁₋₃ alkyl, C₁₋₃ alkoxy, hydroxyl-substituted C₁₋₃ alkyl, C₁₋₃ alkoxy-substituted C₁₋₃ alkyl (preferably methoxy-substituted C₁₋₃ alkyl), or C₃₋₆ cycloalkyl (preferably cyclopropyl).

More preferably, R_{L1} and R_{L2} are each independently hydrogen, hydroxyl, halogen (preferably fluorine or chlorine, and more preferably fluorine), C₁₋₃ alkyl (preferably methyl or ethyl), C₁₋₃ alkoxy (preferably methoxyl), or halogenated C₁₋₃ alkyl (preferably trifluoromethyl).

More preferably, m is 0, 1, or 2.

More preferably, Rₚ is hydrogen, hydroxyl, carboxyl, cyano, C₁₋₆ alkyl (preferably C₁₋₃ alkyl, and more preferably methyl or ethyl), halogenated C₁₋₆ alkyl (preferably halogenated C₁₋₃ alkyl, and more preferably trifluoromethyl or difluoromethyl), C₁₋₆ alkoxy (preferably C₁₋₃ alkoxy, and more preferably methoxyl), C₃₋₆ cycloalkyl (preferably cyclopropyl, cyclobutyl or cyclopentyl), -SO₂(C₁₋₆ alkyl) (preferably -SO₂(C₁₋₃ alkyl), and more preferably -SO₂CH₃), -(PO)(C₁₋₃ alkyl)₂ (preferably -(PO)(CH₃)₂), -NHSO₂(C₁₋₃ alkyl) (preferably -NHSO₂CH₃), -C(O)NRₐ₀R_{b0} (preferably -C(O)NH₂), three- to six-membered heterocycloalkyl (preferably four- to six-membered heterocycloalkyl, and more preferably oxetanyl, tetrahydrofuranyl, tetrahydropyranyl, azetidinyl, morpholinyl, piperazinyl or azetidine-2-one) or NRₐ₁R_{b1} (preferably -NH₂), wherein the C₃₋₆ cycloalkyl (preferably cyclopropyl, cyclobutyl, or cyclopentyl) and the three- to six-membered heterocycloalkyl (preferably four- to six-membered heterocycloalkyl, and more preferably oxetanyl, tetrahydrofuranyl, tetrahydropyranyl, azetidinyl, morpholinyl, piperazinyl, or azetidine-2-one) are unsubstituted or substituted with one or two substituents each independently selected from the group consisting of halogen (preferably fluorine or chlorine, and more preferably fluorine), hydroxyl, and C₁₋₃ alkyl (preferably methyl).

More preferably, Rₚ-(CR_{L1}R_{L2})ₘ- is Rₚ‴-(CH₂)ₘ-, or a structure represented by Formula (b), Formula (c), Formula (d), Formula (e), Formula (f), or Formula (g): 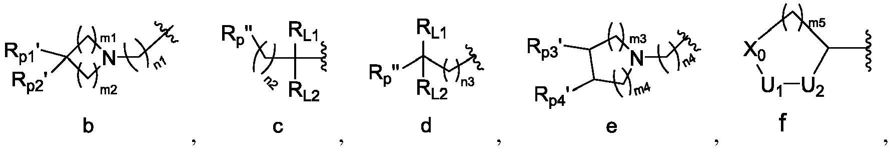 and 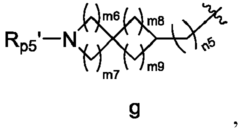
where, Rₚ₁' is hydrogen, halogen, cyano, hydroxyl, carboxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -(CH₂)ₜ₁-NRₐ₀R_{b0}, -(CH₂)ₜ₁-SO₂(C₁₋₃ alkyl), -(CH₂)ₜ₁-S(O)(C₁₋₃ alkyl), -(CH₂)ₜ₁-C(O)NRₐ₀R_{b0}, -(CH₂)ₜ₁-C(O)O(C₁₋₃ alkyl), -(CH₂)ₜ₁-OC(O)(C₁₋₃ alkyl), -(CH₂)ₜ₁-(C₃₋₆ cycloalkyl), C₃₋₆ cycloalkyloxy, or -(CH₂)ₜ₁-(four- to six-membered heterocycloalkyl); Rₚ₂' is hydrogen or C₁₋₃ alkyl; or Rₚ₁' and Rₚ₂' together with the carbon atom linked thereto form a four- to six-membered saturated heteromonocyclic ring, wherein the four- to six-membered saturated heteromonocyclic ring is unsubstituted or substituted with one, two, or three substituents each independently selected from the group consisting of halogen, cyano, hydroxyl, carboxyl, oxo, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -C(O)(C₁₋₃ alkyl), -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), NRₐ₀R_{b0}, C₃₋₆ cycloalkyl, C₃₋₆ cycloalkyloxy, three- to six-membered heterocycloalkyl, phenyl, and five- or six-membered heteroaryl;
Rₚ₃' and Rₚ₄' together with the carbon atoms linked thereto form a fused four- to six-membered saturated heteromonocyclic ring, wherein the fused four- to six-membered saturated heteromonocyclic ring is unsubstituted or substituted with one, two, or three substituents each independently selected from the group consisting of halogen, cyano, hydroxyl, carboxyl, oxo, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -C(O)(C₁₋₃ alkyl), -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), NRₐ₀R_{b0}, C₃₋₆ cycloalkyl, C₃₋₆ cycloalkyloxy, three- to six-membered heterocycloalkyl, phenyl, and five- or six-membered heteroaryl;
Rₚ" is hydrogen, hydroxyl, carboxyl, C₁₋₈ alkyl (preferably C₁₋₆ alkyl, and more preferably C₁₋₃ alkyl), halogenated C₁₋₈ alkyl (preferably halogenated C₁₋₆ alkyl, and more preferably halogenated C₁₋₃ alkyl), halogenated C₁₋₈ alkoxy (preferably halogenated C₁₋₆ alkoxy, and more preferably halogenated C₁₋₃ alkoxy), C₁₋₈ alkoxy (preferably C₁₋₆ alkoxy, and more preferably C₁₋₃ alkoxy), C₃₋₈ cycloalkyl (preferably C₃₋₆ cycloalkyl), -SO₂(C₁₋₈ alkyl) (preferably -SO₂(C₁₋₆ alkyl), and more preferably -SO₂(C₁₋₃ alkyl)), -SO₂NRₐ₀R_{b0}, -C(O)NRₐ₀R_{b0}, -C(O)NRₐ₁R_{b1}, four- to six-membered heterocycloalkyl, or -X-(CRₚ₁Rₚ₂)_{q}-(four- to six-membered heterocycloalkyl);
U₁ is CR_{U11}R_{U12}; U₂ is CR_{U21}R_{U22}; and X₀ is O, NR¹, or CR²R³;
Run and R_{U12} are each independently hydrogen; or Run and R_{U12} together with the carbon atom linked thereto form a three- to seven-membered saturated or partially unsaturated heteromonocyclic ring or a three- to seven-membered saturated or partially unsaturated monocyclic ring, wherein the three- to seven-membered saturated or partially unsaturated heteromonocyclic ring and the three- to seven-membered saturated or partially unsaturated monocyclic ring are unsubstituted or substituted with one, two, or three substituents each independently selected from the group consisting of deuterium, halogen, cyano, hydroxyl, carboxyl, C₁₋₃ alkyl, C₁₋₃ alkoxy, NRₐ₀R_{b0}, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)(C₁₋₃ alkyl), -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
R_{U21} and R_{U22} are each independently hydrogen; or R_{U21} and R_{U22} together with the carbon atom linked thereto form a three- to seven-membered saturated or partially unsaturated heteromonocyclic ring or a three- to seven-membered saturated or partially unsaturated monocyclic ring, wherein the three- to seven-membered saturated or partially unsaturated heteromonocyclic ring and the three- to seven-membered saturated or partially unsaturated monocyclic ring are unsubstituted or substituted with one, two, or three substituents each independently selected from the group consisting of deuterium, halogen, cyano, hydroxyl, carboxyl, C₁₋₃ alkyl, C₁₋₃ alkoxy, NRₐ₀R_{b0}, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)(C₁₋₃ alkyl), -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
R¹ is hydrogen, C₁₋₃ alkyl, -SO₂(C₁₋₃ alkyl), -S(O)(C₁₋₃ alkyl), -C(O)(C₁₋₃ alkyl), -C(O)NRₐ₀R_{b0}, -C(O)O(C₁₋₃ alkyl), C₃₋₆ cycloalkyl, three- to six-membered heterocycloalkyl, phenyl, or five- or six-membered heteroaryl;
R² and R³ are each independently hydrogen, halogen, cyano, hydroxyl, carboxyl, C₁₋₃ alkyl, C₁₋₃ alkoxy, NRₐ₀R_{b0}, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)(C₁₋₃ alkyl), -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, or five- or six-membered heteroaryl; or R² and R³ together with the carbon atom linked thereto form a three- to seven-membered saturated or partially unsaturated heteromonocyclic ring or a three- to seven-membered saturated or partially unsaturated monocyclic ring, wherein the three- to seven-membered saturated or partially unsaturated heteromonocyclic ring and the three- to seven-membered saturated or partially unsaturated monocyclic ring are unsubstituted or substituted with one, two, or three substituents each independently selected from the group consisting of deuterium, halogen, cyano, hydroxyl, carboxyl, C₁₋₃ alkyl, C₁₋₃ alkoxy, NRₐ₀R_{b0}, halogenated C₁₋₃ alkyl, halogenated C₁₋₃ alkoxy, -SO₂(C₁₋₃ alkyl), -S(O)(C₁₋₃ alkyl), -C(O)NRₐ₀R_{b0}, -C(O)(C₁₋₃ alkyl), -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
Rₚ‴ is hydrogen, hydroxyl, carboxyl, C₁₋₈ alkyl (preferably C₁₋₆ alkyl, and more preferably C₁₋₃ alkyl), halogenated C₁₋₈ alkyl (preferably halogenated C₁₋₆ alkyl, and more preferably halogenated C₁₋₃ alkyl), halogenated C₁₋₈ alkoxy (preferably halogenated C₁₋₆ alkoxy, and more preferably halogenated C₁₋₃ alkoxy), C₁₋₈ alkoxy (preferably C₁₋₆ alkoxy, and more preferably C₁₋₃ alkoxy), C₃₋₈ cycloalkyl (preferably C₃₋₆ cycloalkyl), -SO₂(C₁₋₈ alkyl) (preferably -SO₂(C₁₋₆ alkyl), and more preferably -SO₂(C₁₋₃ alkyl)), -SO₂NRₐ₀R_{b0}, -C(O)NRₐ₀R_{b0}, -C(O)NRₐ₁R_{b1}, three- to six-membered heterocycloalkyl, -X-(CRₚ₁Rₚ₂)_{q}-(three- to six-membered heterocycloalkyl), five- or six-membered heteroaryl, eight- to ten-membered heteroaryl, or NRₐ₁R_{b1}, wherein the three- to six-membered heterocycloalkyl, the five- or six-membered heteroaryl, and the eight- to ten-membered heteroaryl are unsubstituted or substituted with one, two, or three substituents each independently selected from the group consisting of deuterium, halogen, cyano, hydroxyl, carboxyl, oxo, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, -C(O)(C₁₋₃ alkyl), -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), NRₐ₀R_{b0}, C₃₋₆ cycloalkyl, C₃₋₆ cycloalkyloxy, three- to six-membered heterocycloalkyl, phenyl, and five- or six-membered heteroaryl;
Rₚ₅' is hydrogen, C₁₋₃ alkyl, -SO₂(C₁₋₃ alkyl), -S(O)(C₁₋₃ alkyl), -C(O)(C₁₋₃ alkyl), -C(O)NRₐ₀R_{b0}, -C(O)O(C₁₋₃ alkyl), C₃₋₆ cycloalkyl, three- to six-membered heterocycloalkyl, phenyl, or five- or six-membered heteroaryl;
n1, n2, n3, n4, and n5 are each independently 0, 1, or 2;
m1 and m2 are each independently 1, 2, or 3;
m3, m4, and m5 are each independently 0, 1, 2, or 3, provided that m3 and m4 are not both 0; and
m6, m7, m8, and m9 are each independently 1 or 2.

More preferably, the structure represented by Formula (b) is a structure represented by Formula (b-1), Formula (b-2), Formula (b-3), or Formula (b-4): 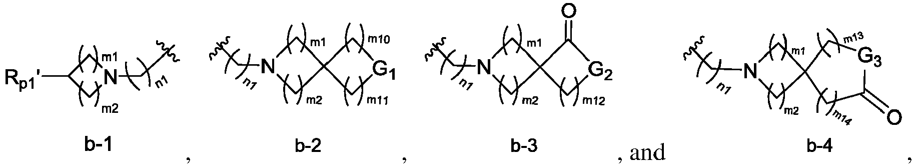
where, m10, mll, and m12 are each independently 1, 2, or 3;
m13 and ml4 are each independently 0, 1, or 2, provided that m13 and ml4 are not both 0;
G₁ is NR⁴ or O; G₂ is NR⁵ or O; and G₃ is NR⁶ or O; and
R⁴, R⁵, and R⁶ are each independently hydrogen, C₁₋₃ alkyl, -SO₂(C₁₋₃ alkyl), -S(O)(C₁₋₃ alkyl), -C(O)(C₁₋₃ alkyl), -C(O)NRₐ₀R_{b0}, -C(O)O(C₁₋₃ alkyl), C₃₋₆ cycloalkyl, three-to six-membered heterocycloalkyl, phenyl, or five- or six-membered heteroaryl.

More preferably, the structure represented by Formula (b) is selected from the group consisting of:

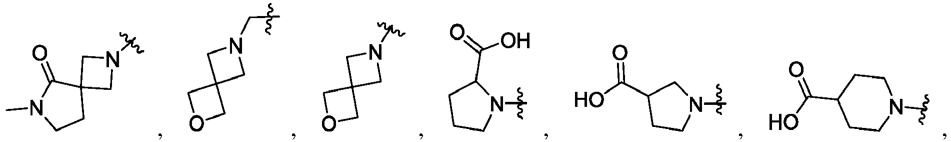

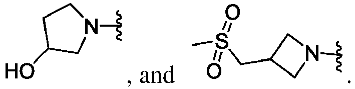

More preferably, the structure represented by Formula (c) or Formula (d) is selected from the group consisting of: 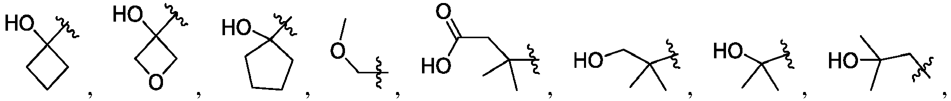 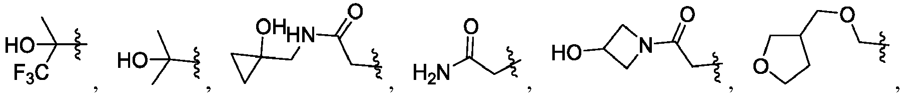 and

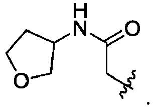

More preferably, the structure represented by Formula (e) is

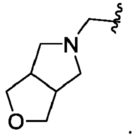

More preferably, the structure represented by Formula (f) is selected from the group consisting of:

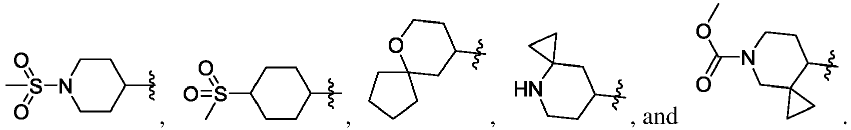

More preferably, the structure represented by Formula (g) is

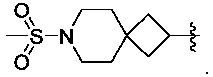

More preferably, Rₚ‴ is five- or six-membered heteroaryl or eight- to ten-membered heteroaryl that is selected from the group consisting of:

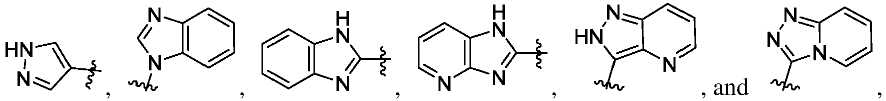

the above heteroaryl is optionally substituted with one, two, or three substituents each independently selected from the group consisting of deuterium, halogen, cyano, hydroxyl, carboxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, NRₐ₀R_{b0}, -C(O)(C₁₋₃ alkyl), -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl.

More preferably, Rₚ‴-(CH₂)ₘ- is selected from the group consisting of: methyl, ethyl, isopropyl, tert-butyl, trifluoromethyl, cyclopropyl, 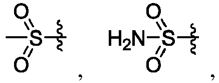 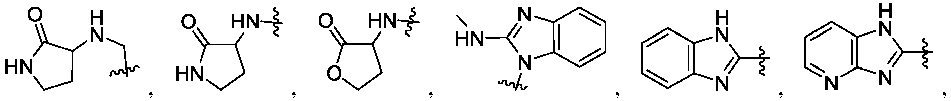 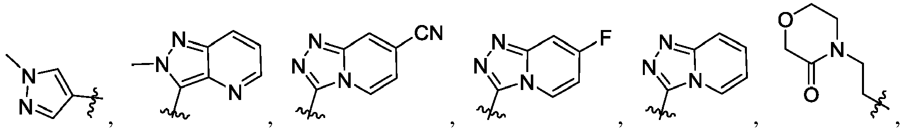 and

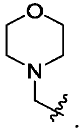

More preferably, (R₀')ᵤ refers to u substituents R₀' replacing u hydrogen atoms on the ring B, wherein u is 1 or 2, and the u substituents R₀' are identical or different from each other and are each independently hydrogen, cyano, halogen (preferably fluorine or chlorine), or C₁₋₈ alkyl (preferably C₁₋₆ alkyl, and more preferably C₁₋₃ alkyl).

More preferably, (R₀')ᵤ refers to u substituents R₀' replacing u hydrogen atoms on the ring B, wherein u is 2, and the u substituents R₀' are identical or different from each other and are each independently hydrogen, cyano, halogen (preferably fluorine or chlorine), or C₁₋₃ alkyl (preferably methyl).

More preferably, the ring B is phenyl; (R₀')ᵤ refers to u substituents R₀' replacing u hydrogen atoms on the ring B, wherein u is 1 or 2, and the u substituents R₀' are identical or different from each other and are each independently cyano or methyl.

More preferably,

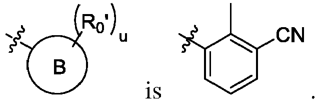

More preferably, the compound has a structure represented by Formula (II) or Formula (III): 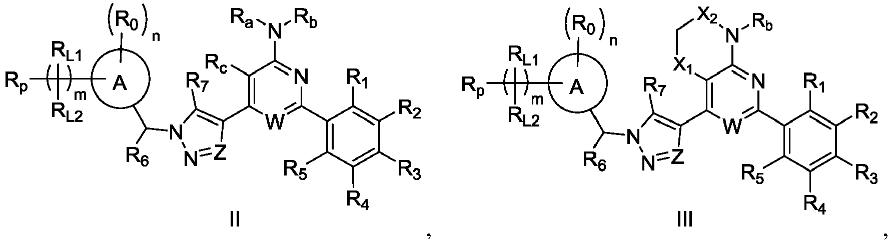
where, R_{c}, Rₐ, and R_{b} in Formula (II) are as defined in (i); and R_{b} in Formula (III) is as defined in (ii);
X₁ is O, NR₁₁, or CR₁₂R₁₃;
X₂ is C(O) or CR₂₁R₂₂;
R₁₁ is hydrogen or C₁₋₃ alkyl;
R₁₂, R₁₃, R₂₁, and R₂₂ are each independently hydrogen, halogen, C₁₋₃ alkyl, or C₁₋₃ alkoxy;
Z is N or CRz; and Rz is hydrogen, deuterium, or C₁₋₃ alkyl;
R₁, R₂, R₃, R₄, and R₅ are each independently hydrogen, cyano, acetyl, hydroxyl, carboxyl, halogen (preferably fluorine or chlorine), NRₐ₀R_{b0}, C₁₋₈ alkyl (preferably C₁₋₆ alkyl, and more preferably C₁₋₃ alkyl), C₁₋₈ alkoxy (preferably C₁₋₆ alkoxy, and more preferably C₁₋₃ alkoxy), C₃₋₈ cycloalkyl (preferably C₃₋₆ cycloalkyl), three- to six-membered heterocycloalkyl, five- or six-membered heteroaryl, or C₆₋₁₀ aryl, wherein the C₁₋₈ alkyl, the C₁₋₈ alkoxy, the C₃₋₈ cycloalkyl, the three- to six-membered heterocycloalkyl, the five- or six-membered heteroaryl, and the C₆₋₁₀ aryl are unsubstituted or substituted with one, two, or three substituents each independently selected from the group consisting of deuterium, halogen, cyano, hydroxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, C₃₋₆ cycloalkyl, C₃₋₆ cycloalkyloxy, three- to six-membered heterocycloalkyl, phenyl, and five- or six-membered heteroaryl; and
R₆ and R₇ are each independently hydrogen, deuterium, cyano, hydroxyl, halogen (preferably fluorine or chlorine), or C₁₋₆ alkyl.

More preferably, in Formula (II), R₁ is methyl; R₂ is cyano; and R₃, R₄, and R₅ are each independently hydrogen.

More preferably, 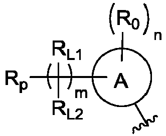 is: 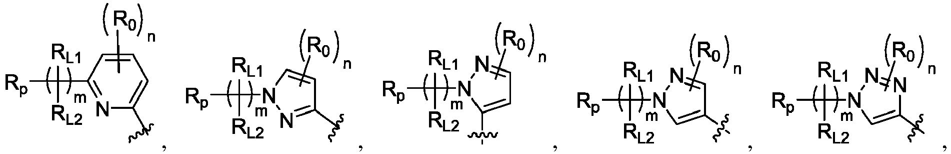 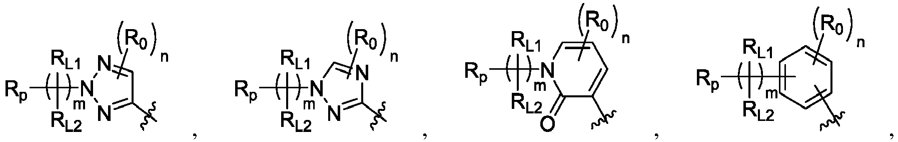 or

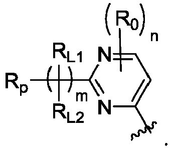

More preferably, 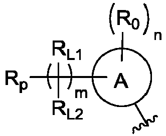 is:

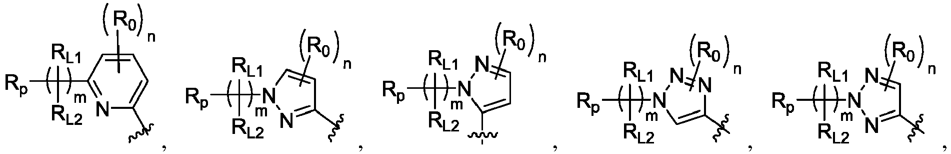

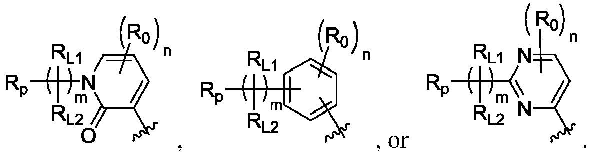

More preferably, X₁ is O.

More preferably, X₂ is C(O).

More preferably, Z is N.

More preferably, R₁, R₂, R₃, R₄, and R₅ are each independently hydrogen, cyano, halogen (preferably fluorine or chlorine), or C₁₋₈ alkyl (preferably C₁₋₆ alkyl, and more preferably C₁₋₃ alkyl).

More preferably, R₁ and R₂ are each independently hydrogen, cyano, halogen (preferably fluorine or chlorine), or C₁₋₃ alkyl (preferably methyl); and R₃, R₄, and R₅ are each independently hydrogen.

More preferably, R₁ is methyl; R₂ is cyano; and R₃, R₄, and R₅ are each independently hydrogen.

More preferably, R₆ and R₇ are each independently hydrogen.

More preferably, the four- to six-membered saturated heteromonocyclic ring is selected from the group consisting of azetidine, oxetane, a tetrahydrofuran ring, a tetrahydrothiophene ring, a tetrahydropyrrole ring, a piperidine ring, oxazolidine, a piperazine ring, dioxolane, dioxane, a morpholine ring, a thiomorpholine ring, a tetrahydropyran ring, a morpholin-3-one ring, a piperazin-2-one ring, and a piperidin-2-one ring.

More preferably, the three- to six-membered saturated monocyclic ring is selected from the group consisting of a cyclopropyl ring, a cyclobutyl ring, a cyclopentyl ring, and a cyclohexyl ring.

More preferably, the five- or six-membered heteroaryl is selected from the group consisting of thienyl, furyl, thiazolyl, imidazolyl, oxazolyl, pyrrolyl, pyrazolyl, triazolyl, 1,2,3-triazolyl, 1,2,4-triazolyl, 1,2,5-triazolyl, 1,3,4-triazolyl, tetrazolyl, isoxazolyl, oxadiazolyl, 1,2,3-oxadiazolyl, 1,2,4-oxadiazolyl, 1,2,5-oxadiazolyl, 1,3,4-oxadiazolyl, thiadiazolyl, pyridyl, pyridazinyl, pyrimidinyl, and pyrazinyl.

More preferably, the four- to six-membered heterocycloalkyl is selected from the group consisting of azetidinyl, oxetanyl, tetrahydrofuranyl, tetrahydrothienyl, tetrahydropyrrolyl, oxazolidinyl, dioxolanyl, piperidinyl, piperazinyl, morpholinyl, dioxanyl, thiomorpholinyl, thiomorpholine-1,1-dioxide, tetrahydropyranyl, pyrrolidine-2-one, dihydrofuran-2(3H)-one, morpholine-3-one, piperazine-2-one, and piperidine-2-one.

More preferably, the five- to eight-membered heterocycloalkenyl ring is selected from the group consisting of a 4,5-dihydro-1H-imidazole ring, a 1,4,5,6-tetrahydropyrimidine ring, a 3,4,7,8-tetrahydro-2H-1,4,6-oxadiazine ring, a 1,6-dihydropyrimidine ring, a 4,5,6,7-tetrahydro-1H-1,3-diazepine ring, and a 2,5,6,7-tetrahydro-1,3,5-oxadiazepine ring.

More preferably, the compound represented by Formula (I) is any one of the following compounds: 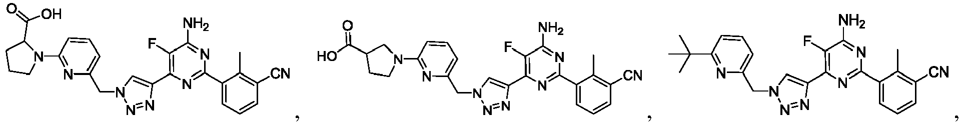 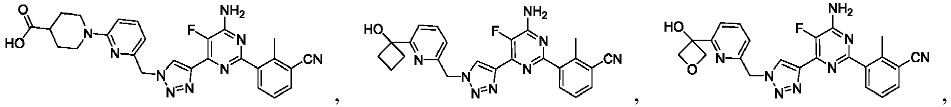 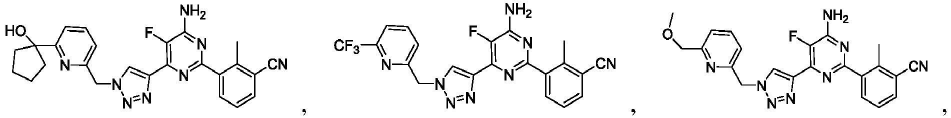 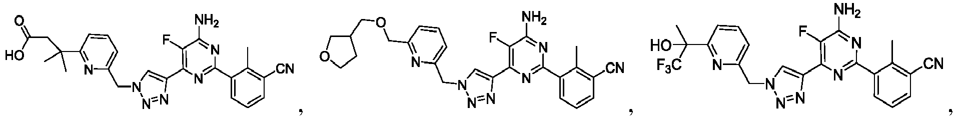 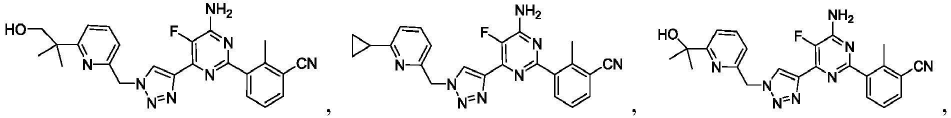 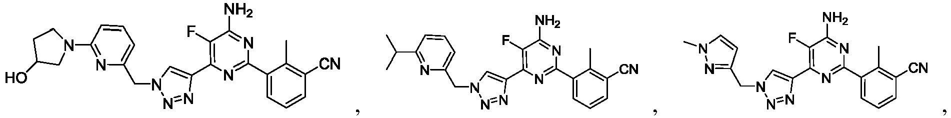 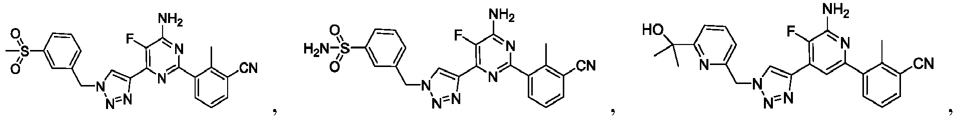 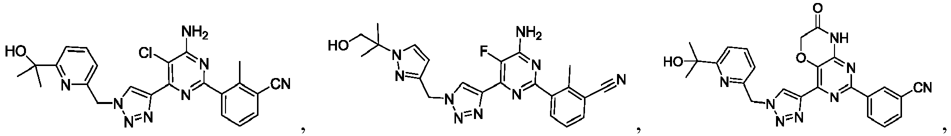 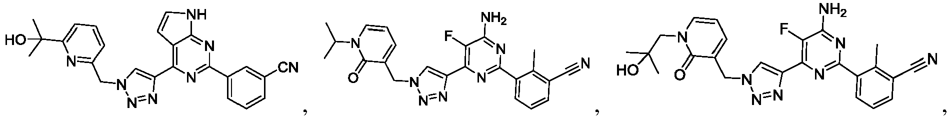 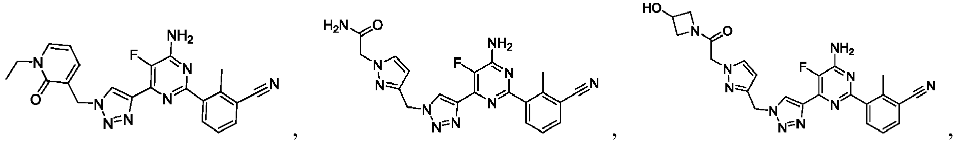 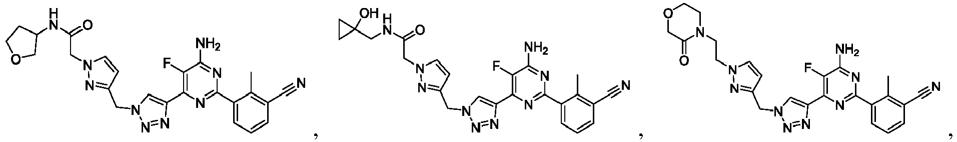 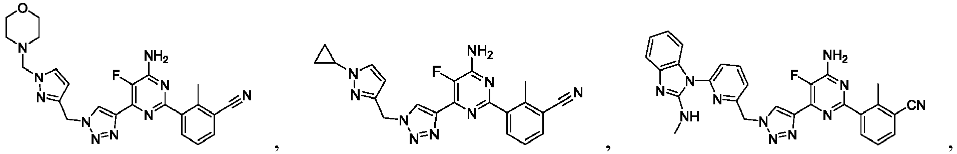 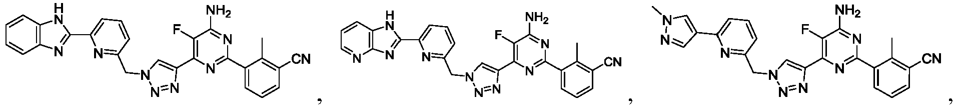 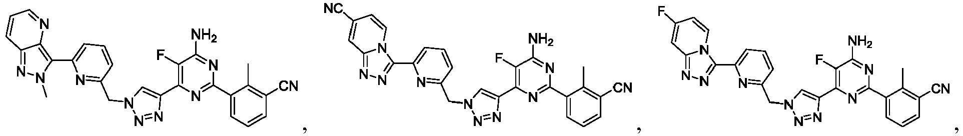 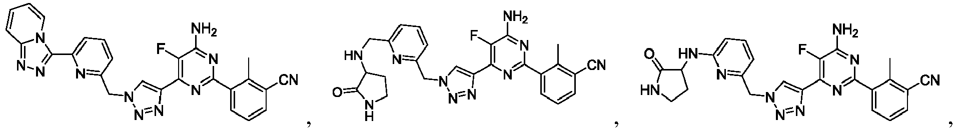 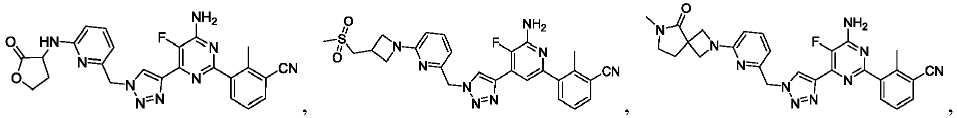 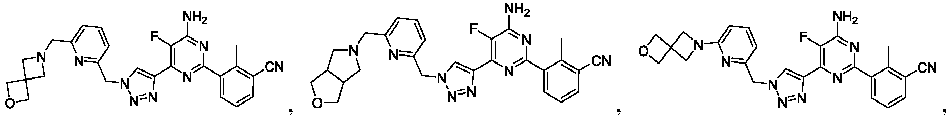 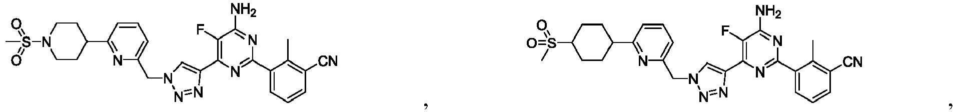 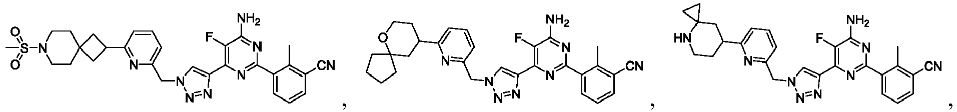 and

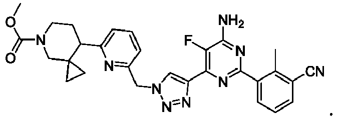

More preferably, the compound represented by Formula (I) is any one of the following compounds: 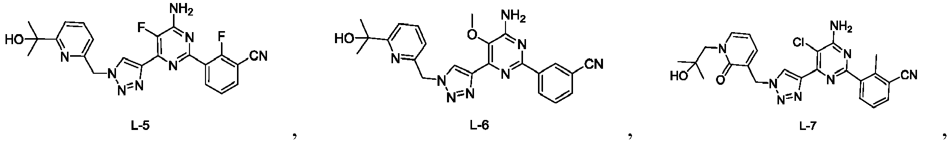 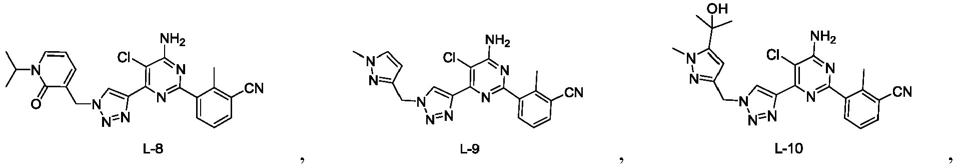 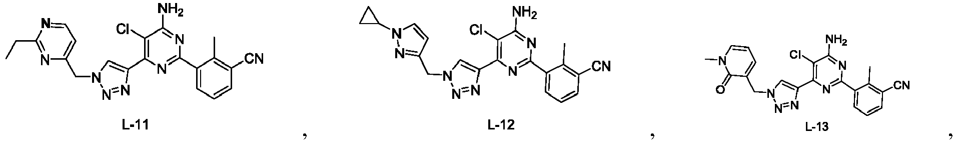 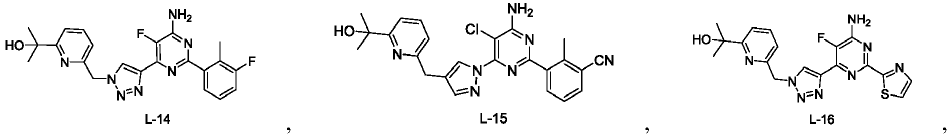 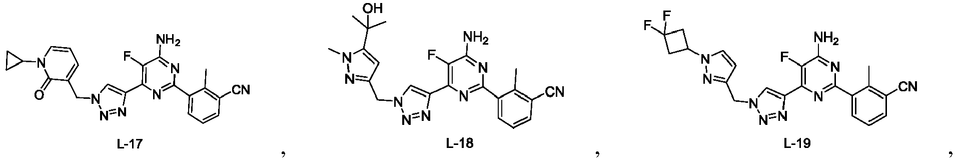 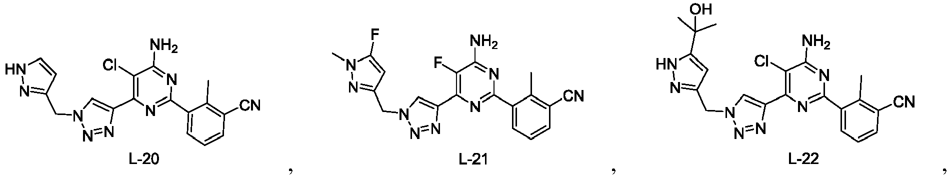 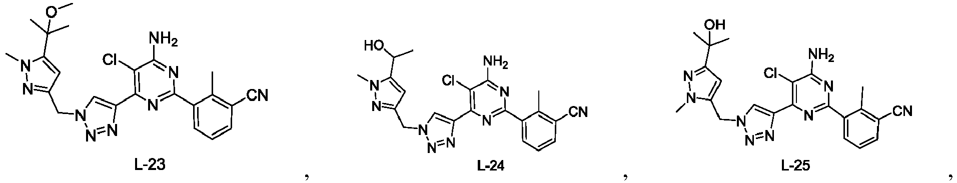 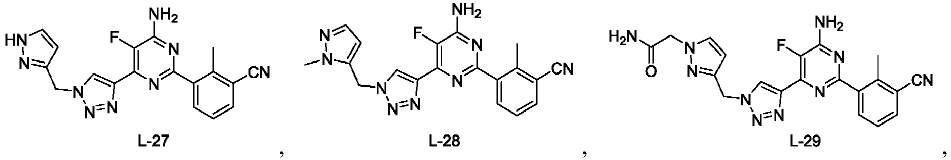 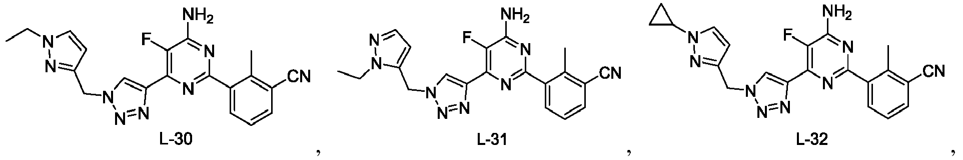 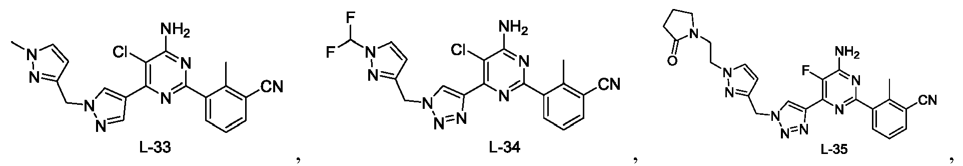 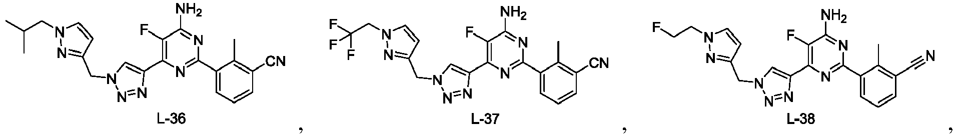 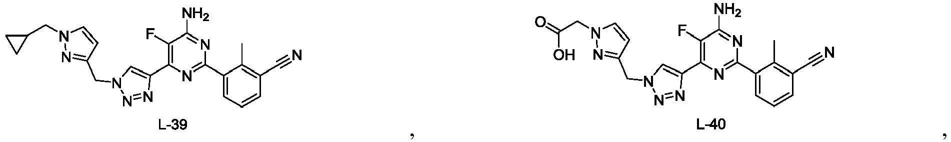 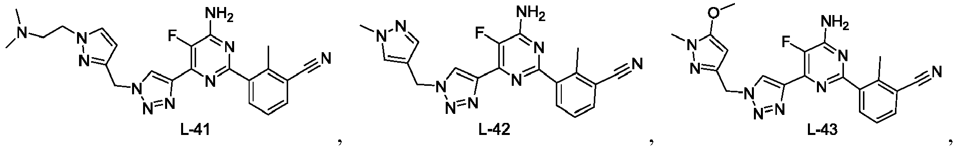 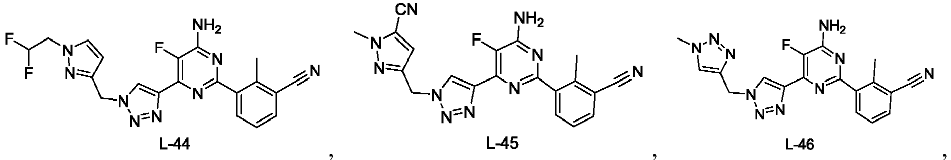 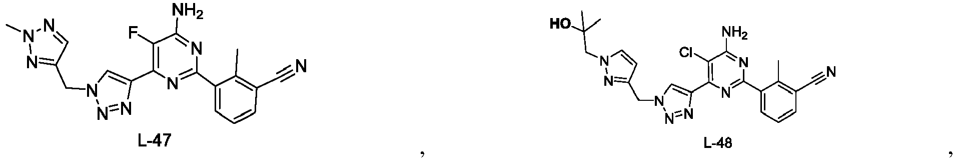 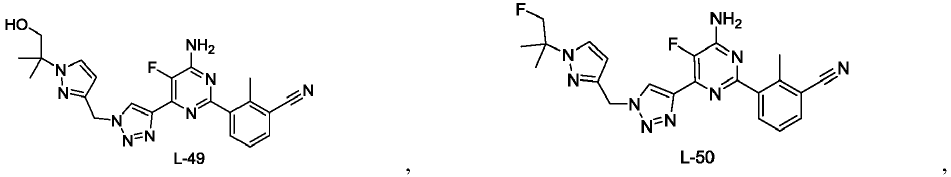 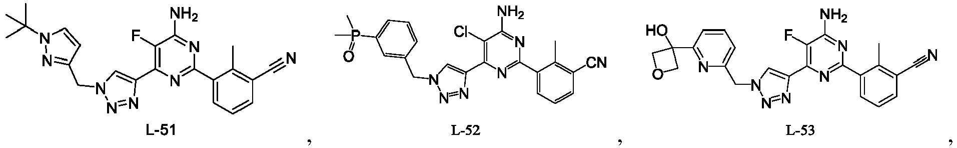 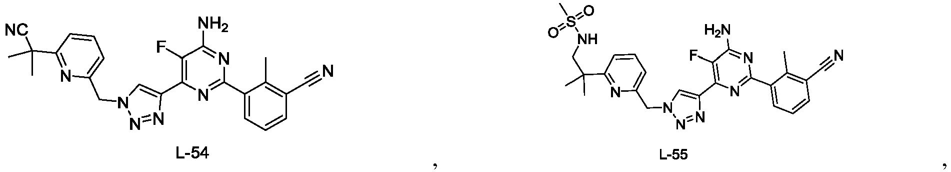 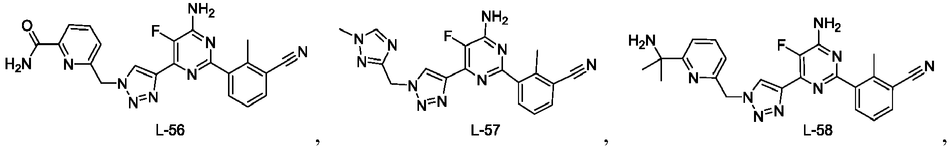 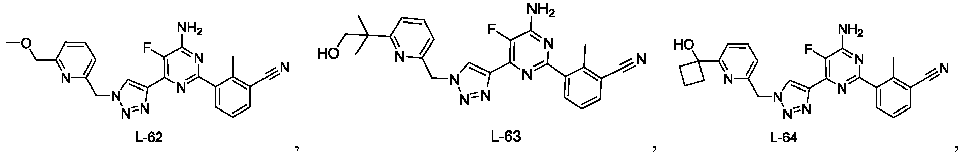 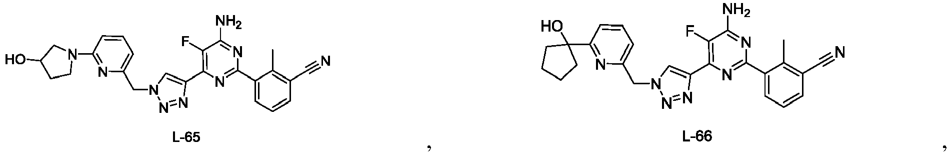 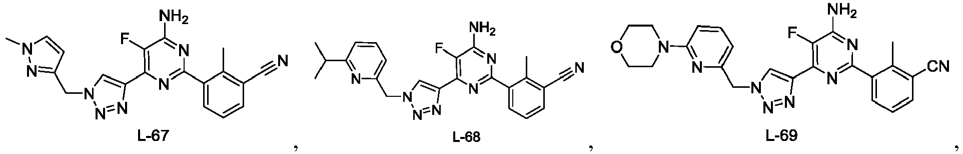 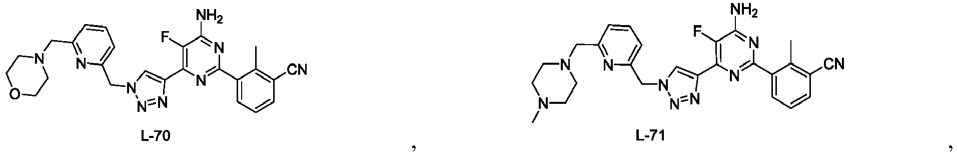 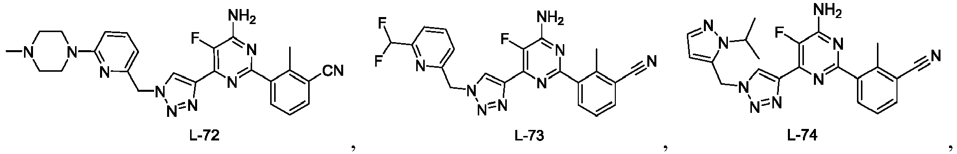 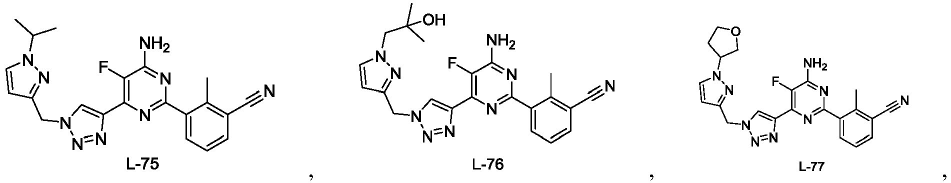 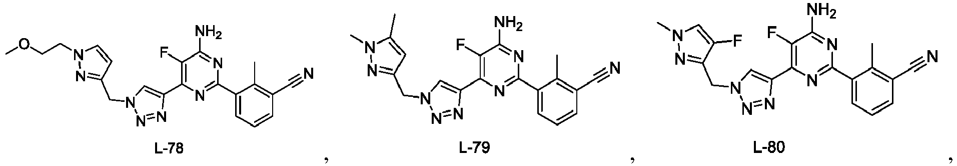 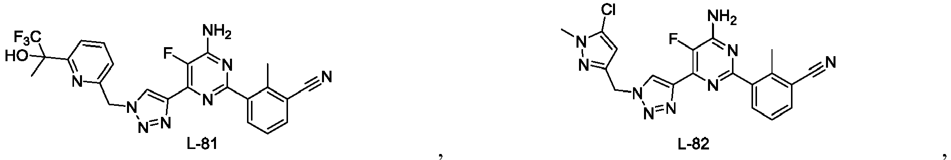 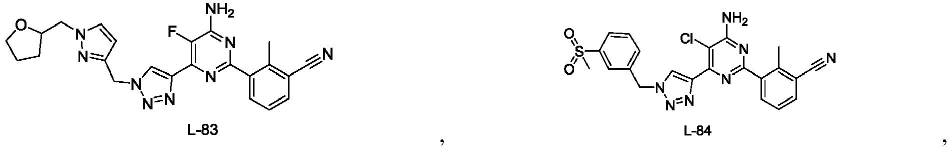 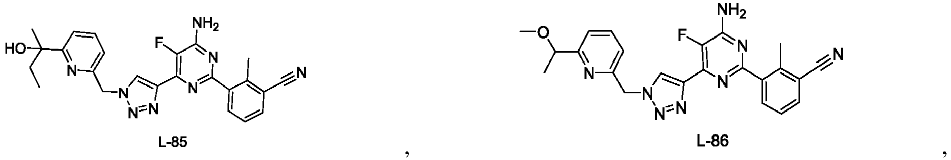 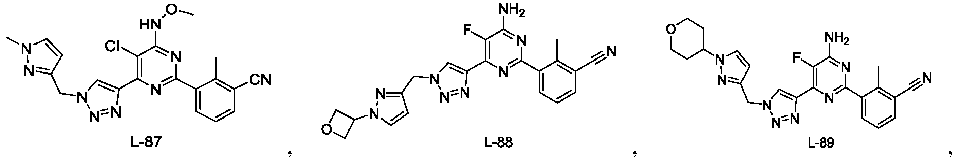 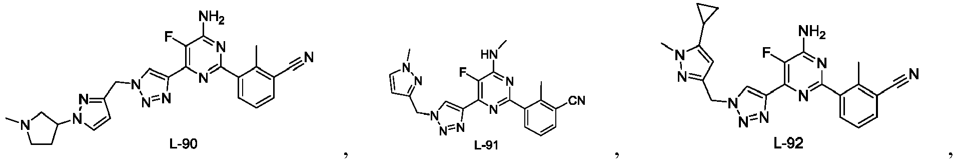 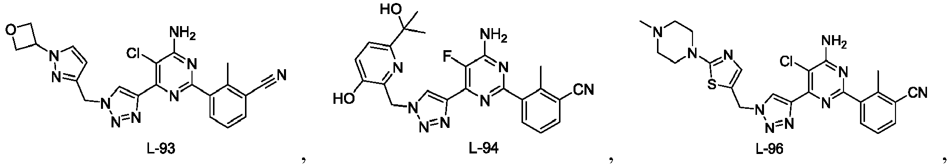 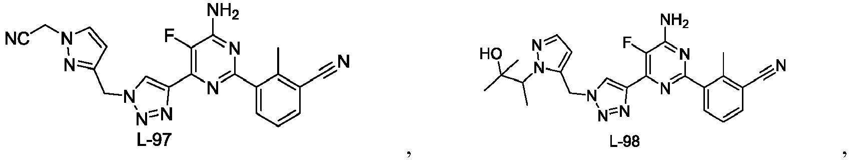 and

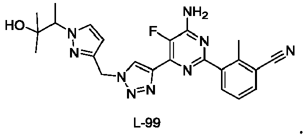

More preferably, the compound represented by Formula (I) is any one of the example compounds.

In a second aspect, the present disclosure provides a compound represented by Formula (IA), or a pharmaceutically acceptable salt, stereoisomer, solvate, or prodrug thereof: 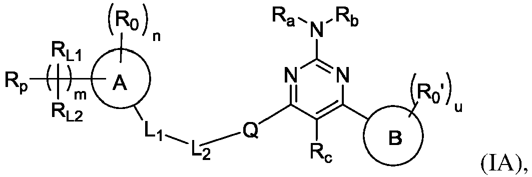 in Formula (IA), the ring A, the ring B, Q, L₁, L₂, R_{L1}, R_{L2}, R_{P}, m, (R₀)ₙ, (R₀')ᵤ are as described above; and R_{c}, Rₐ, and R_{b} are as defined in (i).

More preferably, the compound represented by Formula (IA) has a structure represented by Formula (IIA): 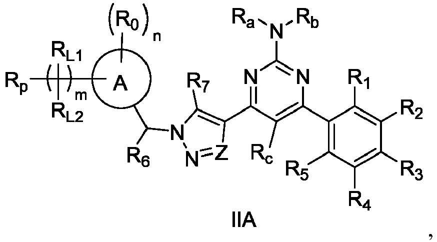
where, R_{c}, Rₐ, and R_{b} in Formula (IIA) are as defined in (i);
Z is N or CRz; and Rz is hydrogen, deuterium, or C₁₋₃ alkyl;
R₁, R₂, R₃, R₄, and R₅ are each independently hydrogen, cyano, acetyl, hydroxyl, carboxyl, halogen (preferably fluorine or chlorine), NRₐ₀R_{b0}, C₁₋₈ alkyl (preferably C₁₋₆ alkyl, and more preferably C₁₋₃ alkyl), C₁₋₈ alkoxy (preferably C₁₋₆ alkoxy, and more preferably C₁₋₃ alkoxy), C₃₋₈ cycloalkyl (preferably C₃₋₆ cycloalkyl), three- to six-membered heterocycloalkyl, five- or six-membered heteroaryl, or C₆₋₁₀ aryl, wherein the C₁₋₈ alkyl, the C₁₋₈ alkoxy, the C₃₋₈ cycloalkyl, the three- to six-membered heterocycloalkyl, the five- or six-membered heteroaryl, and the C₆₋₁₀ aryl are unsubstituted or substituted with one, two, or three substituents each independently selected from the group consisting of deuterium, halogen, cyano, hydroxyl, C₁₋₃ alkyl, C₁₋₃ alkoxy, C₂₋₄ alkenyl, C₂₋₄ alkynyl, halogenated C₁₋₃ alkyl, halogenated C₁₋₃ alkoxy, C₃₋₆ cycloalkyl, C₃₋₆ cycloalkyloxy, three- to six-membered heterocycloalkyl, phenyl, and five- or six-membered heteroaryl; and
R₆ and R₇ are each independently hydrogen, deuterium, cyano, hydroxyl, halogen (preferably fluorine and chlorine), or C₁₋₆ alkyl.

More preferably, 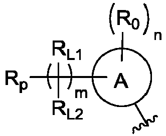 is: 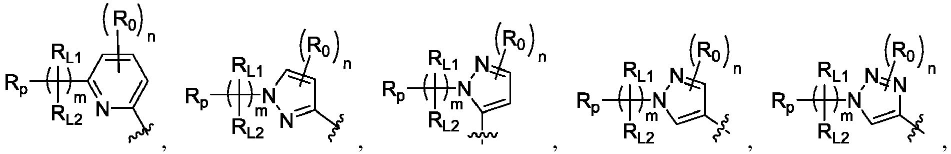 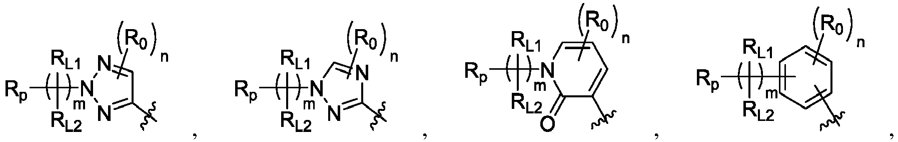 or

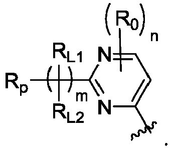

More preferably, 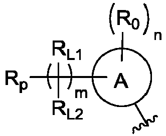 is:

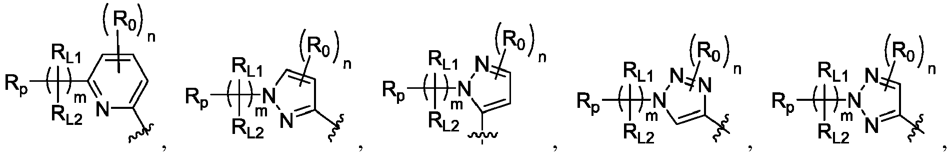

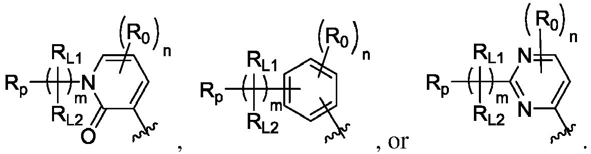

More preferably, Z is N.

More preferably, R₁, R₂, R₃, R₄, and R₅ are each independently hydrogen, cyano, halogen (preferably fluorine or chlorine), or C₁₋₈ alkyl (preferably C₁₋₆ alkyl, and more preferably C₁₋₃ alkyl).

More preferably, R₁ and R₂ are each independently hydrogen, cyano, halogen (preferably fluorine and chlorine), or C₁₋₃ alkyl (preferably methyl); and R₃, R₄, and R₅ are each independently hydrogen.

More preferably, R₁ is methyl; R₂ is cyano; and R₃, R₄, and R₅ are each independently hydrogen.

More preferably, R₆ and R₇ are each independently hydrogen.

More preferably, the compound represented by Formula (IA) is any one of the following compounds:

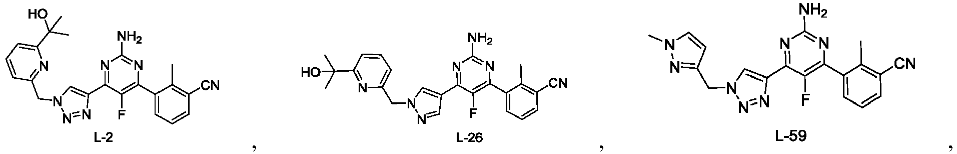

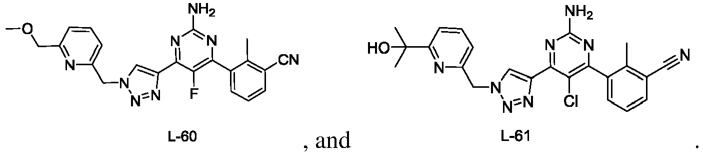

In a third aspect, the present disclosure provides a pharmaceutical composition, which includes the compound, or the pharmaceutically acceptable salt, stereoisomer, solvate, or prodrug thereof according to the first aspect or the second aspect of the present disclosure, and a pharmaceutically acceptable carrier.

In a fourth aspect, the present disclosure provides use of the compound, or the pharmaceutically acceptable salt, stereoisomer, solvate, or prodrug thereof according to the first aspect or the second aspect of the present disclosure, or the pharmaceutical composition according to the second aspect of the present disclosure in preparation of a drug for treating a disease mediated by an adenosine A_{2A} receptor and/or an adenosine A_{2B} receptor.

In a fifth aspect, the present disclosure provides a method for preventing and/or treating a disease mediated by an adenosine A_{2A} receptor and/or an adenosine A_{2B} receptor, the method including: administrating a therapeutically effective amount of the compound, or the pharmaceutically acceptable salt, stereoisomer, solvate, or prodrug thereof according to the first aspect or the second aspect of the present disclosure, or the pharmaceutical composition according to the third aspect of the present disclosure to a patient in need.

More preferably, the disease is mediated by the adenosine A_{2A} receptor.

More preferably, the disease is mediated by the adenosine A_{2B} receptor.

More preferably, the disease is mediated by both of the adenosine A_{2A} receptor and the adenosine A_{2B} receptor.

More preferably, the disease is cancer.

More preferably, the cancer is selected from the group consisting of prostate cancer, colon cancer, rectal cancer, pancreatic cancer, cervical cancer, stomach cancer, endometrial cancer, brain cancer, liver cancer, bladder cancer, ovarian cancer, testicular cancer, head cancer, neck cancer, melanoma, basal carcinoma, cancer of inner layer of mesothelium, leukemia, esophageal cancer, breast cancer, muscle cancer, connective tissue tumor, small cell lung cancer, non-small cell lung cancer, adrenal cancer, thyroid cancer, kidney cancer, and bone cancer; or the cancer is selected from the group consisting of malignant gliomas, mesothelioma, renal cell carcinoma, stomach cancer, sarcoma, choriocarcinoma, skin basal cell carcinoma, and testicular seminoma.

More preferably, the cancer is selected from the group consisting of melanoma, colorectal cancer, pancreatic cancer, breast cancer, prostate cancer, small cell lung cancer, non-small cell lung cancer, leukemia, brain tumor, lymphoma, ovarian cancer, Kaposi's sarcoma, renal cell cancer, head and neck cancer, and esophageal cancer.

More preferably, the disease is an immune-associated disease.

More preferably, the immune-associated disease is selected from the group consisting of rheumatoid arthritis, kidney failure, lupus, asthma, psoriasis, colitis, pancreatitis, allergies, fibrosis, anemic fibromyalgia, Alzheimer's disease, congestive heart failure, stroke, aortic valve stenosis, arteriosclerosis, osteoporosis, Parkinson's disease, infection, Crohn's disease, ulcerative colitis, allergic contact dermatitis and other eczema, systemic sclerosis, and multiple sclerosis.

It should be understood that within the scope of the present disclosure, the technical features described above and the technical features specifically described below (e.g., examples) can be combined with each other to form new or preferred technical solutions, which, due to space limitations, will not be described one by one herein.

### DESCRIPTION OF EMBODIMENTS

By extensive and in-depth researches, the inventors have unexpectedly discovered such substituted pyrimidine or pyridine amine derivatives, which have significant adenosine A_{2A} receptor and/or adenosine A_{2B} receptor activities. Therefore, this series of compounds is expected to be developed into drugs for preventing and treating adenosine A_{2A} receptor and/or adenosine A_{2B} receptor-mediated diseases. On this basis, the inventors have completed this invention.

### Term definition

In order to understand the technical content of the present disclosure more clearly, the terms used herein will be further described below.

"Alkyl" refers to straight-chain and branched saturated aliphatic hydrocarbyl groups. "C₁₋₈ alkyl" refers to alkyl having one to eight carbon atoms, C₁₋₆ alkyl is preferred, and C₁₋₃ alkyl is more preferred. Alkyl includes, but is not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, which are more preferred.

"Alkenyl" refers to straight-chain or branched unsaturated aliphatic hydrocarbyl groups having one or more carbon-carbon double bonds (C=C), "C₂₋₈ alkenyl" refers to alkenyl having two to eight carbon atoms, C₂₋₆ alkenyl is preferred, and C₂₋₄ alkenyl is more preferred, which are defined similarly. Alkenyl includes, but is not limited to, vinyl, propenyl, isopropenyl, n-butenyl, isobutenyl, pentenyl, and hexenyl.

"Alkynyl" refers to straight-chain and branched unsaturated aliphatic hydrocarbyl groups having one or more carbon-carbon triple bonds, "C₂₋₈ alkynyl" refers to alkynyl having two to eight carbon atoms, C₂₋₆ alkynyl is preferred, and C₂₋₄ alkynyl is more preferred, which are defined similarly. Alkynyl includes, but is not limited to, ethynyl, propynyl, n-butynyl, isobutynyl, pentynyl, and hexynyl.

"Cycloalkyl" and "cycloalkyl ring" are interchangeable, and both refer to saturated monocyclic, bicyclic or polycyclic hydrocarbyl groups, which can be fused with aryl or heteroaryl. Cycloalkyl rings can be optionally substituted. In some embodiments, a cycloalkyl ring contains one or more carbonyl, such as oxo groups. "C₃₋₈ cycloalkyl" refers to monocyclic cycloalkyl having three to eight carbon atoms. Cycloalkyl includes, but is not limited to, cyclopropyl, cyclobutyl, cyclopentyl, cyclohexyl, cycloheptyl, cyclooctyl, cyclobutanone, cyclopentanone, and cyclopentane-1,3-dione. C₃₋₆ cycloalkyl is preferred, which includes cyclopropyl, cyclobutyl, cyclopentyl, and cyclohexyl. "C₈₋₁₀ cycloalkyl" refers to fused bicyclic annular hydrocarbyl groups having eight to ten ring atoms, which includes, but is not limited to,

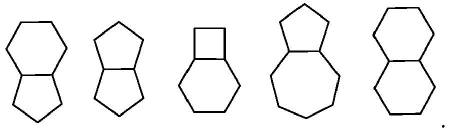

"Spirocyclyl" and "spiro ring" are interchangeable, and both refer to polycyclic annular hydrocarbyl groups having monocyclic rings sharing one common carbon atom (also called spiro atom). "Seven- to ten-membered spirocyclyl" refers to spiro rings having seven to eleven rings atoms. According to the number of rings, spiro rings are divided into spirobicyclic rings and spiropolycyclic rings, and spirobicyclic rings are preferred. Four-membered/five-membered, five-membered/five-member or five-membered/six-membered spirobicyclic rings are more preferred. For example:

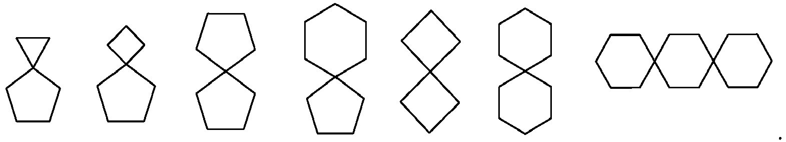

"Cycloalkenyl" and "cycloalkenyl ring" are interchangeable, and both refer to monocyclic, bicyclic, or polycyclic annular hydrocarbyl groups containing one or more carbon-carbon double bonds within a ring, which can be fused with aryl or heteroaryl. Cycloalkenyl rings can be optionally substituted. In some embodiments, a cycloalkenyl ring contains one or more carbonyl groups, such as oxo groups. "C₃₋₈ cycloalkenyl" refers to monocyclic cycloalkenyl having three to eight carbon atoms. C₃₋₆ cycloalkenyl is preferred. Cycloalkenyl includes, but is not limited to, cyclobutenyl, cyclopentenyl, cyclohexenyl, cyclohexadienyl, cycloheptatrienyl, cyclopentyl-2-en-1-one, cyclohexyl-2,5-dien-1-one, cyclohexyl-2-en-1-one, cyclohex-2-en-1,4-dione.

"Heterocycloalkyl" and "heterocycloalkyl ring" are interchangeable, and both refer to cycloalkyl containing at least one heteroatom selected from nitrogen, oxygen, or sulfur, which can be fused with aryl or heteroaryl. Heterocycloalkyl rings can be optionally substituted. In some embodiments, a heterocycloalkyl ring contains one or more carbonyl or thiocarbonyl groups, such as oxo and thio groups. "Three- to eight-membered heterocycloalkyl" refers to monocyclic annular hydrocarbyl groups having three to eight ring atoms of which one, two, or three ring atoms are heteroatoms selected from nitrogen, oxygen, and sulfur, and four- to eight-membered heterocycloalkyl is preferred. More preferred is three- to six-membered heterocycloalkyl, which has three to six ring atoms of which one or two ring atoms are heteroatoms selected from nitrogen, oxygen, and sulfur. More preferred is four- to six-membered heterocycloalkyl, which has four to six ring atoms of which one or two ring atoms are heteroatoms selected from nitrogen, oxygen, and sulfur. Monocyclic heterocycloalkyl includes, but is not limited to, aziridinyl, oxiranyl, azetidinyl, oxetanyl, tetrahydrofuranyl, tetrahydrothienyl, tetrahydropyrrolyl, oxazolidinyl, dioxolanyl, piperidinyl, piperazinyl, morpholinyl, dioxanyl, thiomorpholinyl, thiomorpholine-1,1-dioxide, tetrahydropyranyl, azetidine-2-one, oxetane-2-one, dihydrofuran-2(3H)-one, pyrrolidine-2-one, pyrrolidine-2,5-dione, dihydrofuran-2,5-dione, piperidine-2-one, tetrahydro-2H-pyran-2-one, piperazine-2-one, and morpholine-3-one. "Six- to twelve-membered heterocycloalkyl" and "six- to twelve-membered fused heterocycloalkyl" are interchangeable, and both refer to fused bicyclic annular hydrocarbyl groups having six to twelve ring atoms of which one, two, or three ring atoms are heteroatoms selected from nitrogen, oxygen, and sulfur. "Eight- to ten-membered heterocycloalkyl" and "eight- to ten-membered fused heterocycloalkyl" are interchangeable, and both refer to fused bicyclic annular hydrocarbyl groups having eight to ten ring atoms of which one, two, or three ring atoms are heteroatoms selected from nitrogen, oxygen, and sulfur. Bicyclic heterocycloalkyl includes, but is not limited to, hexahydro-1H-furo[3,4-c]pyrrole, octahydro-1H-cyclopenta[c]pyridine, hexahydro-1H-pyrrolo[2,1-c] [1,4]oxazine, octahydropyrrolo[2, 1-*a*]pyrazine, hexahydropyrrolo[1,2-*a*]pyrazin-4(1H)-one, and octahydrocyclopenta[c]pyrrole. In fused bicyclic heterocycloalkyl containing one or more nitrogen atoms, the linking point may be a carbon or nitrogen atom as long as the valence allows. A bicyclic heterocycloalkyl system may contain one or more heteroatoms within one or two rings.

"Heterospirocyclyl" and "heterospiro ring" are interchangeable, and both refer to monovalent non-aromatic ring systems having two monocyclic rings sharing one common carbon atom, which consists of carbon atoms and heteroatoms selected from nitrogen, oxygen, sulfur, and phosphorus, does not have a degree of unsaturation, and is linked to the parent nucleus through a single bond. The heterospiro ring is optionally substituted. In some embodiments, the heterospiro ring contains one or more carbonyl or thiocarbonyl groups, such as oxo and thio groups. "Seven- to eleven-membered heterospirocyclyl refers to heterospirocyclyl having seven to eleven ring atoms of which one, two, or three ring atoms are heteroatoms. Heterospirocyclyl includes, but is not limited to, 2,6-diazaspiro[3.4]octan-5-one, 2-oxa-6-azaspiro[3.3]heptanyl, 6-oxaspiro[3.3]heptan-2-yl, 7-methyl-7-azaspiro[3.5]nonan-2-yl, 7-methyl-2,7-diazaspiro[3.5]nonan-2-yl, and 9-methyl-9-phosphaspiro[5.5]undecan-3-yl.

"Heterocycloalkenyl" and "heterocycloalkenyl ring" are interchangeable, and both refer to heterocycloalkyl containing one or more carbon-carbon double bonds or carbon-nitrogen double bonds within a ring, which is not intended to include heteroaryl defined herein. These groups can be fused with aryl or heteroaryl. Heterocycloalkenyl rings are optionally substituted. In some embodiments, a heterocycloalkenyl ring contains one or more carbonyl or thiocarbonyl groups, such as oxo and thio groups. "Five- to eight-membered heterocycloalkenyl ring" refers to heterocycloalkenyl rings having five to eight ring atoms of which one, two, or three ring atoms are heteroatoms selected from nitrogen, oxygen, and sulfur. Five- or six-membered heterocycloalkenyl rings are preferred. Heterocycloalkenyl rings include, but are not limited to, a 4,5-dihydro-1H-imidazole ring, a 1,4,5,6-tetrahydropyrimidine ring, a 3,4,7,8-tetrahydro-2H-1,4,6-oxadiazine ring, a 1,6-dihydropyrimidine ring, a 4,5,6,7-tetrahydro-1H-1,3-diazepine ring, and a 2,5,6,7-tetrahydro-1,3,5-oxadiazepine ring.

"Aryl" and "aromatic ring" are interchangeable, and both refer to all-carbon monocyclic or fused polycyclic groups having a conjugated π-electron system (namely, rings sharing a common pair of adjacent carbon atoms), which can be fused with cycloalkyl rings, heterocycloalkyl rings, cycloalkenyl rings, heterocycloalkenyl rings, or heteroaryl. "C₆₋₁₀ aryl" refers to monocyclic or bicyclic aryl having six to ten carbon atoms, and aryl includes, but is not limited to, phenyl and naphthyl.

"Heteroaryl" and "heteroaryl ring" are interchangeable, and both refer to monocyclic, bicyclic, or polycyclic 4n+2 aromatic ring systems having ring carbon atoms and ring heteroatoms (e.g. having six or ten common π electrons that are in an annular arrangement), in which the heteroatoms are each independently selected from nitrogen, oxygen, and sulfur. In the present disclosure, heteroaryl also includes ring systems formed by fusing the above heteroaryl rings with one or more cycloalkyl rings, heterocycloalkyl rings, cycloalkenyl rings, heterocycloalkenyl rings, or aromatic rings. Heteroaryl rings can be optionally substituted. "Five- to ten-membered heteroaryl" refers to monocyclic or bicyclic heteroaryl having five to ten ring atoms of which one, two, three, or four ring atoms are heteroatoms. "Five- or six-membered heteroaryl" refers to monocyclic heteroaryl having five or six ring atoms of which one, two, three, or four ring atoms are heteroatoms, which includes, but is not limited to, thienyl, furyl, thiazolyl, isothiazolyl, imidazolyl, oxazolyl, pyrrolyl, pyrazolyl, triazolyl, 1,2,3-triazolyl, 1,2,4-triazolyl, 1,2,5-triazolyl, 1,3,4-triazolyl, tetrazolyl, isoxazolyl, oxadiazolyl, 1,2,3-oxadiazolyl, 1,2,4-oxadiazolyl, 1,2,5-oxadiazolyl, 1,3,4-oxadiazolyl, thiadiazolyl, pyridyl, pyridazinyl, pyrimidinyl, pyrazinyl, triazinyl, and tetrazinyl. "Eight- to ten-membered heteroaryl" refers to bicyclic heteroaryl having eight to ten ring atoms of which one, two, three, or four ring atoms are heteroatoms, which includes, but is not limited to, indolyl, isoindolyl, indazolyl, benzotriazolyl, benzothienyl, isobenzothienyl, benzofuranyl, benzisofuranyl, benzimidazolyl, benzoxazolyl, benzisoxazolyl, benzoxadiazolyl, benzothiazolyl, benzisothiazolyl, benzothiadiazolyl, indolazinyl, purinyl, pyrido[3,2-d]pyrimidinyl, pyrido[2,3-d]pyrimidinyl, pyrido[3,4-d]pyrimidinyl, pyrido[4,3-d]pyrimidinyl, 1,8-naphthyridinyl, 1,7-naphthyridinyl, 1,6-naphthyridinyl, 1,5-naphthyridinyl, pteridinyl, quinolinyl, isoquinolinyl, cinnolinyl, quinoxalinyl, phthalazinyl, and quinazolinyl. "Heteroatom" refers to hydrogen, oxygen, or sulfur. In heteroaryl containing one or more nitrogen atoms, the linking point may be a carbon or nitrogen atom as long as the valence allows. A bicyclic heteroaryl system may contain one or more heteroatoms within one or two rings.

"Fused" refers to a structure containing two or more rings sharing one or more bonds.

"Benzoheterocycloalkyl" refers to bicyclic, tricyclic, or polycyclic systems formed by fusing benzene rings with heterocycloalkyl rings, the heterocycloalkyl rings being as defined above. "Seven- to eleven-membered benzoheterocycloalkyl" refers to bicyclic annular groups having seven to eleven ring atoms of which one, two, three, or four ring atoms are heteroatoms selected from nitrogen, oxygen, and sulfur. Preferred is eight- to ten-membered benzoheterocycloalkyl, which has eight to ten ring atoms of which one, two, or three ring atoms are heteroatoms selected from nitrogen, oxygen, and sulfur. Benzoheterocycloalkyl includes, but is not limited to, indoline, benzo[d][1,3]dioxazole, 1,2,3,4-tetrahydroisoquinoline, and 3,4-dihydro-2H-benzo[b][1,4 ]oxazine.

"Heteroaryl-fused heterocycloalkyl" refers to bicyclic, tricyclic or polycyclic systems formed by fusing heteroaryl rings with heterocycloalkyl rings, the heterocycloalkyl rings being as defined as above. "Seven- to eleven-membered heteroaryl-fused heterocycloalkyl refers to a bicyclic annular group having seven to eleven ring atoms of which one, two, three, or four ring atoms are heteroatoms selected from nitrogen, oxygen, and sulfur. Preferred is eight to ten-membered heteroaryl-fused heterocycloalkyl, which has eight to ten ring atoms of which one, two, three, or four ring atoms are heteroatoms selected from nitrogen, oxygen, and sulfur. Heteroaryl-fused heterocycloalkyl includes, but is not limited to, 2,3-dihydro-1H-pyrrolo[2,3-b]pyridine, [1,3]dioxolane[4,5-b]pyridine, 2,3-dihydro-1H-pyrido[3,4-b][1,4]oxazine, 2,3,4,6-tetrahydropyrrolo[3,4-b][1,4]oxazine, 2,4,5,6-tetrahydrofuro[2,3-c]pyrazole, and 5,6,7,8-tetrahydropyrido[3,4-d]pyrimidine.

"Alkoxy" refers to -O-alkyl, wherein the alkyl is as defined above. C₁₋₈ alkoxy is preferred, C₁₋₆ alkoxy is more preferred, and C₁₋₃ alkoxy is the most preferred. Alkoxy includes, but is not limited to, methoxy, ethoxy, n-propoxy, isopropoxy, butoxy, tert-butoxy, isobutoxy, and pentoxy.

"Cycloalkyloxy" refers to -O-cycloalkyl, where the cycloalkyl is as defined above. C₃₋₈ aycloalkyloxy is preferred, and C₃₋₆ cycloalkyloxy is more preferred. Cycloalkyloxy includes, but is not limited to, cyclopropyloxy, cyclobutyloxy, cyclopentyloxy, and cyclohexyloxy.

"One bond" refers to a covalent bond through which two groups are linked.

"Halogen" refers to fluorine, chlorine, bromine, or iodine.

"Halogenated" refers to that one or more (e.g., one, two, three, four, or five) hydrogen atoms in a group is substituted by halogen.

For example, "halogenated C₁₋₈ alkyl" refers to alkyl substituted with one or more (e.g., one, two, three, four or five) halogen atoms, where the alkyl is as defined above. Halogenated C₁₋₆ alkyl is preferred, and halogenated C₁₋₃ alkyl is more preferred. Halogenated C₁₋₈ alkyl includes, but is not limited to, monochloromethyl, dichloromethyl, trichloromethyl, monochloroethyl, 1,2-dichloroethyl, trichloroethyl, monobromoethyl, monofluoromethyl, difluoromethyl, trifluoromethyl, monofluoroethyl, difluoroethyl, and trifluoroethyl.

For another example, "halogenated C₁₋₈ alkoxy" refers to alkoxy substituted with one or more (e.g., one, two, three, four or five) halogen atoms, where the alkoxy is as defined above. Halogenated C₁₋₆ alkoxy is preferred, and halogenated C₁₋₃ alkoxy is more preferred. Halogenated C₁₋₈ alkoxy includes, but is not limited to, trifluoromethoxy, trifluoroethoxy, monofluoromethoxy, monofluoroethoxy, difluoromethoxy, and difluoroethoxy.

For another example, "halogenated C₃₋₈ cycloalkyl" refers to cycloalkyl substituted with one or more (e.g., one, two, three, four or five) halogen atoms, where the cycloalkyl is as defined above. Halogenated C₃₋₆ cycloalkyl is preferred. Halogenated C₃₋₈ cycloalkyl includes, but is not limited to, trifluorocyclopropyl, monofluorocyclopropyl, monofluorocyclohexyl, difluorocyclopropyl, and difluorocyclohexyl.

"Deuterated C₁₋₈ alkyl" refers to alkyl substituted with one or more (e.g., one, two, three, four or five) deuterium atoms, where the alkyl is as defined above. Deuterated C₁₋₆ alkyl is preferred, and deuterated C₁₋₃ alkyl is more preferred. Deuterated C₁₋₈ alkyl includes, but is not limited to, mono-deuteromethyl, mono-deuteroethyl, di-deuteromethyl, di-deuteroethyl, tri-deuteromethyl, and tri-deuteroethyl.

"Amino" refers to NH₂, "cyano" refers to CN, "nitro" refers to NO₂, "benzyl" refers to -CH₂-phenyl, "oxo" refers to =O, "carboxyl" refers to -C(O)OH, "acetyl" refers to -C(O)CH₃, "hydroxymethyl" refers to -CH₂OH, "hydroxyethyl" refers to -CH₂CH₂OH or -CHOHCH₃, "hydroxyl" refers to -OH, "thiol" refers to SH, and cyclopropylidene has the following structure:

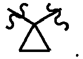

"Saturated or partially unsaturated monocyclic ring" refers to saturated or partially unsaturated all-carbon monocyclic systems, where the "partially unsaturated" refers to a ring moiety containing at least one double bond or triple bond, and is intended to cover rings having multiple unsaturated sites but is not intended to include the aryl or heteroaryl defined herein. In some embodiments, a saturated or partially unsaturated monocyclic ring contains one or more carbonyl groups, such as oxo groups. "Three- to seven-membered saturated or partially unsaturated monocyclic ring" has three to seven ring carbon atoms, saturated or partially unsaturated monocyclic rings having three to six ring carbon atoms are preferred, and saturated monocyclic rings having three to six annular carbon atoms are more preferred. Saturated or partially unsaturated monocyclic rings include, but are not limited to, a cyclopropyl ring, a cyclobutyl ring, a cyclopentyl ring, a cyclopentenyl ring, a cyclohexyl ring, a cyclohexenyl ring, a cyclohexadienyl ring, a cycloheptyl ring, a cycloheptatrienyl ring, a cyclopentanone ring, and a cyclopentane-1,3-dione ring.

"Saturated or partially unsaturated heteromonocyclic ring" refers to a saturated or partially unsaturated monocyclic ring in which one, two, or three ring carbon atoms are substituted by heteroatoms selected from nitrogen, oxygen, or S(O)t (where, t is an integer from 0 to 2), excluding rings having -O-O-, -O-S-, or -S-S, and other ring atoms are carbon. "Three- to seven-membered saturated or partially saturated heteromonocyclic ring" has three to seven ring atoms of which one, two, or three ring atoms are the above heteroatoms. Threeto six-membered saturated or partially unsaturated heteromonocyclic ring having three to six ring atoms of which one or two ring atoms are the above heteroatoms is preferred, five- or six-membered saturated or partially unsaturated heteromonocyclic ring having five or six ring atoms of which one or two ring atoms are the above heteroatoms is more preferred, and five- or six-membered saturated heteromonocyclic ring is the most preferred. Saturated heteromonocyclic rings include, but are not limited to, a propylene oxide ring, an azetidine ring, an oxetane ring, a tetrahydrofuran ring, a tetrahydrothiophene ring, a tetrahydropyrrole ring, a piperidine ring, a pyrroline ring, an oxazolidine ring, a piperazine ring, dioxolane, dioxane, a morpholine ring, a thiomorpholine ring, thiomorpholine-1,1-dioxide, a tetrahydropyran ring, an azetidin-2-one ring, an oxetan-2-one ring, a pyrrolidin-2-one ring, a pyrrolidin-2,5-dione ring, a piperidin-2-one ring, a dihydrofuran-2(3H)-one ring, a dihydrofuran-2,5-dione ring, a tetrahydro-2H-pyran-2-one ring, a piperazin-2-one ring, and a morpholin-3-one ring. Partially unsaturated heteromonocyclic rings include, but are not limited to, a 1,2-dihydro-azete ring, 2H-Oxete, a 2,5-dihydro-1H-pyrrole ring, a 2,5-dihydrofuran ring, a 2,3-dihydrofuran ring, a 2,3-dihydro-1H-pyrrole ring, a 3,4-dihydro-2H-pyran ring, a 1,2,3,4-tetrahydropyridine ring, a 3,6-dihydro-2H-pyran ring, a 1,2,3,6-tetrahydropyridine ring, 4,5-dihydro-1H-imidazole ring, a 1,4,5,6-tetrahydropyrimidine ring, a 3,4,7,8-tetrahydro-2H-1,4,6-oxadiazine ring, a 1,6-dihydropyrimidine ring, a 4,5,6,7-tetrahydro-1H-1,3-diazepine ring, and a 2,5,6,7-tetrahydro-1,3,5-oxadiazepine ring.

"Substituted" refers to that one or more hydrogen atoms (preferably one to five hydrogen atoms, and more preferably one to three hydrogen atoms) in a group are each independently substituted with a corresponding number of substituents. It is appreciated that the substituents are only in their possible chemical positions, and those skilled in the art can determine (either experimentally or theoretically) possible or impossible substitutions without involving undue effort. For example, amino or hydroxyl that has free hydrogen may be unstable when binding to carbon atoms with unsaturated (e.g., olefinic) bonds.

Unless otherwise defined, the "substituents each independently selected from ..." herein refers to that when more than one hydrogen atoms on a group are substituted by substituents, the substituents may be of the same type or of different types, and the selected substituents are independent of each other.

Unless otherwise defined, the "... are identical or different from each other and are each independently ..." refers to that when more than one same substituents are present in a general formula, the substituents may be identical or different from each other, and are independent of each other. For example, L is (CR₀₁R₀₂)ₛ, if s is 2, that is, L is (CR₀₁R₀₂)-(CR₀₁R₀₂), wherein the two substituents R₀₁ or R₀₂ may be identical or different from each other, and are independent of each other. For example, L may be C(CH₃)(CN)-C(CH₂CH₃)(OH), C(CH₃)(CN)-C(CH₃)(OH), or C(CN)(CH₂CH₃)-C(OH)(CH₂CH₃).

Unless otherwise defined, any one group herein may be substituted or unsubstituted. When the above group is substituted, substituents are preferably one to five of the following groups each independently selected from the group consisting of cyano, halogen (preferably fluorine or chlorine), C₁₋₈ alkyl (preferably C₁₋₆ alkyl, and more preferably C₁₋₃ alkyl), C₁₋₈ alkoxy (preferably C₁₋₆ alkoxy, and more preferably C₁₋₃ alkoxy), halogenated C₁₋₈ alkyl (preferably halogenated C₁₋₆ alkyl, and more preferably halogenated C₁₋₃ alkyl), C₃₋₈ cycloalkyl (preferably C₃₋₆ cycloalkyl), halogenated C₁₋₈ alkoxy (preferably halogenated C₁₋₆ alkoxy, and more preferably halogenated C₁₋₃ alkoxy), C₁₋₈ alkyl-substituted amino, halogenated C₁₋₈ alkyl-substituted amino, acetyl, hydroxyl, hydroxymethyl, hydroxyethyl, carboxyl, nitro, C₆₋₁₀ aryl (preferably phenyl), C₃₋₈ cycloalkyloxy (preferably C₃₋₆ cycloalkyloxy), C₂₋₈ alkenyl (preferably C₂₋₆ alkenyl, and more preferably C₂₋₄ alkenyl), C₂₋₈ alkynyl (preferably C₂₋₆ alkynyl, and more preferably C₂₋₄ alkynyl), -CONRₐ₀R_{b0}, -C(O)O(C₁₋₁₀ alkyl) (preferably -C(O)O(C₁₋₆ alkyl), and more preferably -C(O)O(C₁₋₃ alkyl)), -CHO, -OC(O)(C₁₋₁₀ alkyl) (preferably -OC(O)(C₁₋₆ alkyl), and more preferably -OC(O)(C₁₋₃ alkyl)), -SO₂(C₁₋₁₀ alkyl) (preferably -SO₂(C₁₋₆ alkyl), and more preferably -SO₂(C₁₋₃ alkyl)), -SO₂(C₆₋₁₀ aryl) (preferably -SO₂(C₆ aryl) such as -SO₂-phenyl), -CO(C₆₋₁₀ aryl) (preferably-CO(C₆ aryl) such as -CO-phenyl), four- to six-membered saturated or unsaturated heteromonocyclic ring, four- to six-membered saturated or unsaturated monocyclic ring, a five- or six-membered monocyclic heteroaryl ring, an eight- to ten-membered bicyclic heteroaryl ring, a spiro ring, a heterospiro ring, a bridged ring, or a bridged heterocyclic ring, wherein Rₐ₀ and R_{b0} are each independently hydrogen or C₁₋₃ alkyl.

The substituents described above can also be substituted with the groups described herein.

When the four- to six-membered saturated heteromonocyclic ring is substituted, substituents may be in their possible chemical positions, and examples of representative substitutions of a heteromonocyclic ring are as follows: 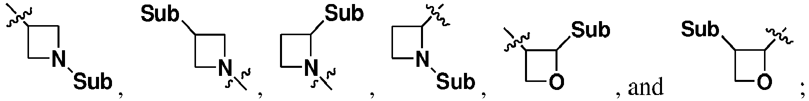 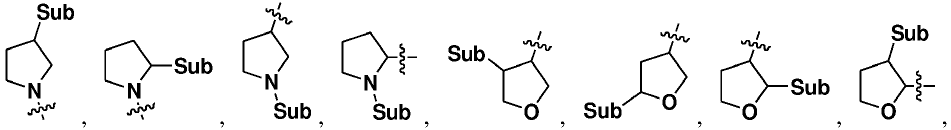 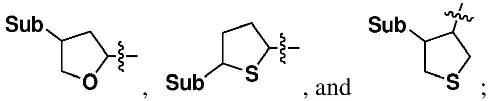 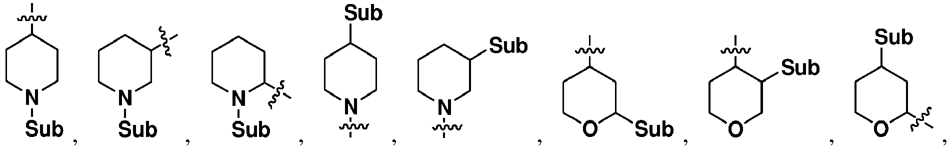 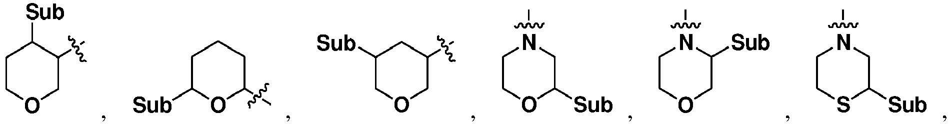 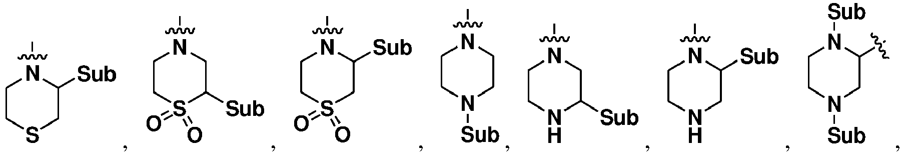 and 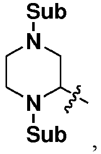 where, "Sub" represents the substituents described herein, and " 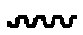 " represents the linkage with other atoms.

### Pharmaceutical composition

Usually, the compound, or the pharmaceutically acceptable salt, solvate, stereoisomer, or prodrug thereof of the present disclosure together with one or more pharmaceutical carriers can be prepared into suitable dosage forms for administration. These dosage forms are suitable for oral administration, rectal administration, topical administration, intraoral administration, and other parenteral administration (e.g., subcutaneous, intramuscular or intravenous administration). For example, dosage forms suitable for oral administration include capsules, tablets, granules, syrups, etc. The compound of the present disclosure contained in these formulations may be in the form of solid powder or particles; a solution or suspension in an aqueous or non-aqueous liquid; or a water-in-oil or oil-in-water emulsion, etc. The above dosage forms can be prepared from the active compound and one or more carriers or excipients by a universal pharmaceutical method. The above carriers need to be compatible with the active compound or other excipients. For solid formulations, commonly used nontoxic carriers include, but are not limited to, mannitol, lactose, starch, magnesium stearate, cellulose, glucose, and sucrose. Carriers used for liquid formulations include water, saline, dextrose water, ethylene glycol, and polyethylene glycol, etc. The active compound can form a solution or suspension with the above carriers.

"Pharmaceutically acceptable carrier" refers to nontoxic, inert, solid, or semi-solid materials or liquid filler, diluents, encapsulating materials or auxiliary formulations or any excipients, which are compatible with patients (preferably mammals, and more preferably humans), and are suitable for delivering the active reagent to a target without terminating the activity of the reagent.

"Active material of the present disclosure" or "active compound of the present disclosure" refers to the compound represented by Formula (I) or Formula (IA), or the pharmaceutically acceptable salt, solvate, stereoisomer, or prodrug thereof of the present disclosure, which has the adenosine A_{2A} receptor and/or adenosine A_{2B} receptor activities.

The compound of the present disclosure is prepared, quantified, and administrated in accordance with the codes of medical practice. "A therapeutically effective amount" of the compound administrated is determined based on factors such as a specific disorder to be treated, an individual to be treated, pathogenesis, a drug target, and an administration method.

"Therapeutically effective amount" refers to the quantity of the compound of the present disclosure that will initiate a biological or medical response in an individual, such as reducing or inhibiting the enzyme or protein activity or improving symptoms, alleviating a disorder, slowing or delaying the disease progression, or preventing a disease.

The therapeutically effective amount of the pharmaceutical composition of the present disclosure, or the compound, or the pharmaceutically acceptable salt, solvate, stereoisomer, or prodrug thereof of the present disclosure contained in the pharmaceutical composition is preferably 0.1 mg to 5 g/kg (body weight).

"Patient" refers to an animal, which is preferably a mammal, and more preferably human. The term "mammal" refers to warm-blood vertebrate mammals including cats, dogs, rabbits, bears, foxes, wolves, monkeys, deer, mice, pigs, and humans.

"Treatment" refers to alleviating, slowing down progression of, attenuating, preventing, or maintaining an existing disease or disorder (e.g., cancer). Treatment also includes curing one or more symptoms of a disease or disorder, preventing the development of the symptoms, or alleviating the symptoms to a certain extent.

The "pharmaceutically acceptable salt" includes pharmaceutically acceptable acid addition salts and pharmaceutically acceptable base addition salts.

"Pharmaceutically acceptable acid addition salt" refers to salts that can maintain biological effectiveness of free alkalis, do not have other side-effects, and are formed with inorganic acids or organic acids. Inorganic acid salts include, but are not limited to, hydrochloride, hydrobromide, sulfate, and phosphate; and organic acid salts include, but are not limited to, formate, acetate, propionate, glycolate, gluconate, lactate, oxalate, maleate, succinate, fumarate, tartrate, citrate, glutamate, aspartate, benzoate, mesylate, p-toluenesulfonate, and salicylate. These salts can be prepared by methods known in the art.

"Pharmaceutically acceptable base addition salt" includes, but is not limited to, inorganic alkali salts such as sodium salts, potassium salts, calcium salts, and magnesium salts, and organic alkali salts such as ammonium salts, triethylamine salts, lysine salts, and arginine salts. These salts can be prepared by methods known in the art.

"Solvate" mentioned herein refers to complexes formed by the compound of the present disclosure and solvents. They either react in solvents or are precipitated out or crystallized out of solvents. For example, a complex formed by the compound and water is called "hydrate". The solvate of the compound represented by Formula (I) falls within the scope of the present disclosure.

The compound represented by Formula (I) of the present disclosure may contain one or more chiral centers and may be present in different optically active forms. When the compound contains one chiral center, the compound includes an enantiomer. The present disclosure includes these two kinds of isomers and a mixture of isomers, such as a racemic mixture. An enantiomer can be separated by methods known in the art, such as crystallization and chiral chromatography. When the compound represented by Formula (I) contains more than one chiral center, a diastereomer may be present. The present disclosure includes separated optically pure specific isomers and a mixture of diastereomers. The diastereomer can be separated by methods known in the art, such as crystallization and preparative chromatography.

The present disclosure includes the prodrug of the above compound. Prodrug contains known amino-protecting groups and carboxyl-protecting groups, which will be hydrolyzed under physiological conditions or released by an enzyme reaction to obtain a parent compound. Prodrugs are specifically prepared with reference to Saulnier, M.G.; Frennesson, D.B.; Deshpande, M.S.; Hansel, S.B and Vysa, D.M. Bioorg. Med. Chem Lett. 1994, 4, 1985-1990; and Greenwald, R.B.; Choe, Y.H.; Conover, C.D.; Shum, K.; Wu, D.; Royzen, M.J. Med. Chem. 2000, 43, 475.

### Preparation methods

The present disclosure provides a preparation method of the compound represented by Formula (I). The compound represented by Formula (I) can be synthesized by a standard synthesis technique known to those skilled in the art or a combination of a method known in the art and the method of the present disclosure. Solvents, temperature, and other reaction conditions specified in the present disclosure can be modified according to techniques in the art. The reactions can be performed in order to obtain the compound of the present disclosure, or they can be used to synthesize fragments, which will be added subsequently by the method of the present disclosure and/or the method known in the art.

The compound of the present disclosure can be synthesized from suitable selectable starting materials by methods similar to the following methods or exemplary methods described in examples, or methods specified in relevant publication documents used by those skilled in the art. The starting materials used for synthesizing the compound of the present disclosure can be synthesized or purchased from the market. The compound of the present disclosure and other relevant compounds having different substituents can be synthesized from raw materials by techniques known to those skilled in the art. The general preparation method of the compound of the present disclosure may be derived from reactions known in the art, reaction reagents and conditions of which can be replaced with suitable reagents and conditions by those skilled in the art so as to introduce moieties in the molecule of the present disclosure.

Compared with the related art, the present disclosure mainly has the following advantages.

The present disclosure provides a series of substituted pyrimidine or pyridine amine derivatives with novel structures, which have high inhibitory activity to the adenosine A_{2A} receptor and/or the adenosine A_{2B} receptor, and have an IC₅₀ value of less than 100 nM (preferably less than 50 nM, and more preferably less than 10 nM). Therefore, the series of substituted pyrimidine or pyridine amine derivatives with novel structures can be used as drugs for treating cancer or other immune-associated diseases.

The present disclosure will be further described below with reference to specific examples. It should be understood that these examples are only used to explain the present disclosure but are not intended to limit the scope of the present disclosure. Experimental methods described in the following examples without specifying conditions follow conventional conditions specified in Sambrook et al. Molecular Cloning: A Laboratory Manual (New York: Cold Spring Harbor Laboratory Press, 1989) or conditions recommended by manufacturers. Unless otherwise specified, percentage and parts are calculated based on weight. Unless otherwise defined, the terms used herein have the same meanings as those familiar to those skilled in the art. In addition, any methods and materials similar or equivalent to those described herein can be used in the present disclosure.

### Reagents and instruments

¹HNMR: Bruker AVANCE-400 nuclear magnetic resonance (NMR) spectrometer, using tetramethylsilane (TMS) as the internal standard.

LC-MS: Agilent 1200 HPLC System/6140 MS liquid chromatography-mass spectrometry (manufacturer: Agilent), column: WatersX-Bridge, 150×4.6 mm, 3.5µm.

Preparative high-performance liquid chromatography (pre-HPLC): Waters PHW007, column: XBridge C18, 4.6×150 mm, 3.5 µm.

An ISCO Combiflash-Rf75 or Rf200 automated flash chromatography system was adopted, and Agela 4 g, 12 g, 20 g, 80 g, and 120 g disposable silica gel columns were adopted.

Known starting materials can be synthesized by methods known in the art, or purchased from ABCR GmbH & Co. KG, Acros Organics, Aldrich Chemical Company, Accela ChemBio Inc, Darui Chemical Products Company, etc.

DMF: dimethylformamide, DMSO: dimethyl sulfoxide, THF: tetrahydrofuran, CAN: acetonitrile, DIEA: N,N-diisopropylethylamine, EA: ethyl acetate, PE: petroleum ether, BINAP: (2R,3S)-2,2'-bis(diphenylphosphino)-1,1'-binaphthyl, NBS: N-bromosuccinimide, NCS: N-chlorosuccinimide, Pd₂(dba)₃: tris(dibenzylideneacetone)dipalladium, Pd(dppf)Cl₂: [1,1'-bis(diphenylphosphino)ferrocene]dichloropalladium, DPPA: diphenylphosphoryl azide, DBU: 1,8-diazabicycloundec-7-ene, TBAF: tetra-n-butylammonium fluoride, Na Ascorbate: sodium ascorbate, t-BuXPhosPd-G3: methanesulfonato(2-di-t-butylphosphino-2',4',6' -tri-i-propyl-1,1'-biphenyl)(2'-amino-1,1'-bi phenyl-2-yl)palladium (II), LAH: lithium aluminium hydride, TBSCl: tert-butyldimethylsilyl chloride, and TMSCl: trimethylsilyl chloride.

As used herein, the room temperature refers to 20 to 30°C.

### Preparation of an intermediate V1

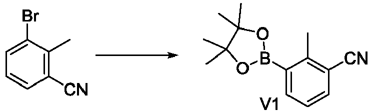

2-methyl-3-bromobenzonitrile (15 g, 76.51 mmol), bis(pinacolato)diboron (23.32 g, 91.82 mmol), potassium acetate (15.02 g, 153.03 mmol), Pd(dppf)Cl₂ (2.80 g), and DMSO (20 mL) were dissolved in dioxane (100 mL), the reaction solution was stirred under the protection of nitrogen gas at 100 °C for 3.5 h. After the reaction was completed, the reaction solution was evaporated under reduced pressure to remove the solvent so as to obtain a solid product, and the solid product was separated by column chromatography (EA/PE, 15 to 40%) to obtain a compound V1 (21.05 g, purity: 78.0%, yield: 100%). MS (ESI) 244.1 [M+H]⁺.

### Preparation of an intermediate V2

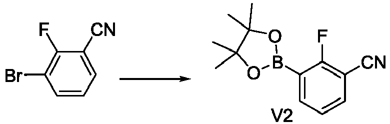

Referring to the preparation method of the intermediate V1 by replacing 2-methyl-3-bromobenzonitrile with 3-bromo-2-fluorobenzonitrile, a compound V2 (2.2 g, yield: 89.07%, purity: 94.8%) was obtained. MS (ESI) 166 [M-82+H]⁺.

### Preparation of an intermediate V3

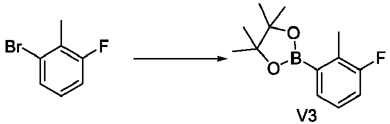

Referring to the preparation method of the intermediate V1 by replacing 2-methyl-3-bromobenzonitrile with 1-bromo-3-fluoro-2-methylbenzene, a compound V3 (1.92 g, yield: 81%, purity: 97%) was obtained. MS (ESI) 237 [M+H]⁺.

### Preparation of an intermediate V4

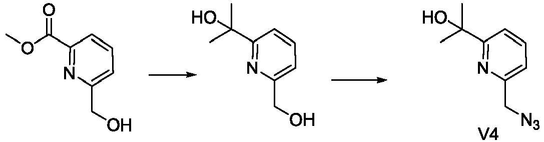

Step 1: methyl 6-(hydroxymethyl)picolinate (25 g, 149.70 mmol) serving as a raw material was added to anhydrous THF (400 mL), the reaction solution was cooled to 0°C, a THF solution of methylmagnesium bromide (1 M, 598.80 mL, 598.80 mmol) was dropwise added, and after the THF solution of methylmagnesium bromide was added, the reaction solution was naturally heated to the room temperature and stirred for 18 h. Then, the reaction solution was cooled to 0°C, a saturated ammonium chloride aqueous solution was added to neutralize the reaction solution, the mixture was extracted with ethyl acetate, obtained organic phases were combined, dried with anhydrous sodium sulfate, and filtered, and an obtained filtrate was evaporated under reduced pressure to dryness and purified by column chromatography (ethyl acetate/petroleum ether=0 to 35%) to obtain a colorless oily product: 2-(6-(hydroxymethyl)pyridin-2-yl)propan-2-ol (6.3 g, 25%). MS (ESI) 168.1 [M+H]⁺.

Step 2: the compound: 2-(6-(hydroxymethyl)pyridin-2-yl)propan-2-ol (400 mg, 2.39 mmol) was dissolved in DCM (20 mL), DPPA (790 mg, 2.87 mmol) and DBU (437 mg, 2.87 mmol) were added, and the reaction solution was stirred at the room temperature overnight until the reaction process monitored by LC-MS was completed. Then, the above reaction solution was added to water (100 mL), the mixture was extracted with dichloromethane (50 mL × 2), and a combined organic phase was dried with anhydrous sodium sulfate, concentrated under reduced pressure to dryness, and separated and purified by silica-gel column chromatography to obtain a compound V4 (350 mg, purity: 100%, yield: 76%). MS (ESI) 193 [M+H]⁺.

### Preparation of an intermediate V5

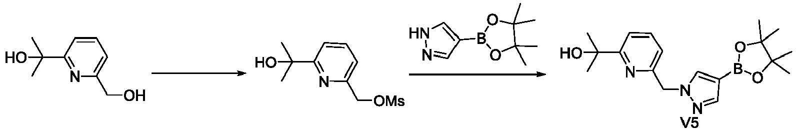

Step 1: 2-(6-(hydroxymethyl)pyridin-2-yl)propan-2-ol (1.5 g, 8.98 mmol) and triethylamine (1814 mg, 17.96 mmol) were dissolved in dichloromethane (15 mL), methylsulfonyl chloride (1536 mg, 13.47 mmol) dissolved in dichloromethane (20 mL) was slowly dropwise added under ice bath conditions, and after the methylsulfonyl chloride was added, the reaction solution was slowly heated to the room temperature and stirred for 1 h. Then, the reaction solution was extracted with dichloromethane (15 mL × 3), and a combined organic phase was washed with water (20 mL), dried with anhydrous sodium sulfate, and concentrated under reduced pressure to dryness to obtain a colorless oily liquid product: methyl (6-(2-hydroxypropan-2-yl)pyridin-2-yl)methanesulfonate (2.4 g, purity: 50%, yield: 54.5%). MS (ESI) 246 [M+H]⁺.

Step 2: the methyl (6-(2-hydroxypropan-2-yl)pyridin-2-yl)methanesulfonate (2.4 g, 4.9 mmol), 4-(4,4,5,5-tetramethyl-1,3,2-dioxaboroboran-2-yl)-1H-pyrazole (1426 mg, 7.35 mmol), and cesium carbonate (3195 mg, 9.8 mmol) were dissolved in acetonitrile (20 mL), and the reaction solution was stirred at 70°C for 4 h. Then, the reaction solution was filtered, an obtained residue was washed with dichloromethane, and an obtained filtrate was concentrated under reduced pressure to dryness and purified by column chromatography (methanol/dichloromethane= 0 to 5%) to obtain a colorless oily product V5 (1.5 g, purity: 75%, yield: 89.2%). MS (ESI) 344 [M+H]⁺.

### Preparation of an intermediate V6 and an intermediate V7

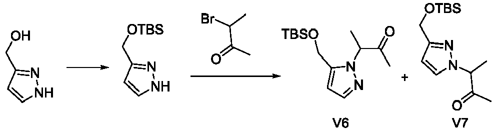

Referring to step 1 of Example 21 and step 2 of Example 10, an intermediate V6 and an intermediate V7 were prepared.

### Preparation of an intermediate V8

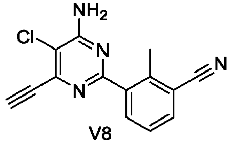

Referring to steps 1 to 4 of Example 1 by replacing 2,4,6-trichloro-5-fluoropyrimidine with tetrachloropyrimidine, an intermediate V8 was obtained. MS (ESI) 269.1 [M+H]⁺.

### Example 1: preparation of a compound L-1

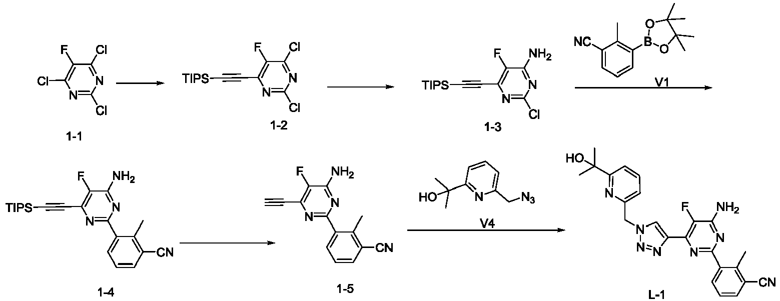

Step 1: 2,4,6-trichloro-5-fluoropyrimidine (2.0 g, 9.93 mmol), triisopropylsilylacetylene (1.8 g, 9.93 mmol), and triethylamine (3.01 g, 3.00 mmol) were dissolved in tetrahydrofuran (25 mL), the reaction solution was cooled to 0°C under the protection of argon gas, CuI (378 mg, 1.99 mmol) and Pd(PPh₃)₂Cl₂ (697 mg, 0.99 mmol) were added, and the reaction solution was heated to the room temperature and reacted for 3 h to obtain a compound 1-2 (3.44 g). MS (ESI) 347 [M+H]⁺.

Step 2: an ammonia water aqueous solution (10 mL, concentration: 80%) was added to the above tetrahydrofuran (25 mL) in which the compound 1-2 (3.44 g, 9.93 mmol) was dissolved, and the reaction solution was stirred at the room temperature for 2 d until the reaction process monitored by LC-MS was completed. Then, the above reaction solution was added to water (300 mL), the mixture was extracted with dichloromethane (100 mL × 2), and a combined organic phase was dried with anhydrous sodium sulfate, concentrated under reduced pressure to dryness, and separated and purified by silica-gel column chromatography to obtain a compound 1-3 (2.30 g, yield: 71%). MS (ESI) 328.1 [M+H]⁺.

Step 3: the compound 1-3 (500 mg, 1.52 mmol) and the compound V1 (408 mg, 1.68 mmol) were dissolved in a mixed solvent of acetonitrile (15 mL) and water (4 mL), Pd(PPh₃)₂Cl₂ (107 mg, 0.152 mmol) and KHCO₃ (611 mg, 6.10 mmol) were added, and the reaction solution was heated to 95°C under the protection of nitrogen gas and stirred overnight until the reaction process monitored by LC-MS was completed. Then, the reaction solution was added to water, the mixture was extracted with dichloromethane (50 mL × 2), and a combined organic phase was dried with anhydrous sodium sulfate, concentrated under reduced pressure to dryness, and separated and purified by silica-gel column chromatography to obtain a compound 1-4 (300 mg, yield: 48%). MS (ESI) 409.2 [M+H]⁺.

Step 4: the compound 1-4 (70 mg, 0.171 mmol) was added to tetrahydrofuran (2 mL), the reaction solution was cooled to 0°C, a tetrahydrofuran solution of TBAF (0.2 mL, 1.0 mol/L) was slowly added, and the reaction solution was stirred at the room temperature for 30 min to obtain a compound 1-5 (43 mg, yield: 100%). MS (ESI) 253.1 [M+H]⁺.

Step 5: the compound 1-5 (43 mg, 0.17 mmol) and the intermediate V4 (33 mg, 0.17 mmol) were added to a mixed solvent of tert-butanol (5 mL) and water (1.5 mL), Cu₂SO₄.5H₂O (12 mg, 0.051 mmol) and sodium ascorbate (20 mg, 0.102 mmol) were added under the protection of argon gas, and the reaction solution was heated to 40°C and reacted for 40 min until the reaction process monitored by LC-MS was completed. Then, the reaction solution was added to water, the mixture was extracted with dichloromethane (50 mL × 2), and a combined organic phase was dried with anhydrous sodium sulfate, concentrated under reduced pressure to dryness, and separated and purified by preparative high-performance liquid chromatography to obtain a white solid compound L-1 (4.39 mg, yield: 6%). ¹H NMR (400 MHz, DMSO-d6) δ 8.78 (s, 1H), 7.92 (d, J=7.8 Hz, 1H), 7.83 (dd, J=7.7, 1.2 Hz, 1H), 7.76 (t, J=7.8 Hz, 1H), 7.60-7.48 (m, 3H), 7.45 (t, J=7.5 Hz, 1H), 7.10 (d, J=6.9 Hz, 1H), 5.77 (s, 2H), 5.18 (s, 1H), 2.63 (s, 3H), 1.34 (s, 6H). MS (ESI) 445.2 [M+H]⁺.

### Example 2: preparation of a compound L-2

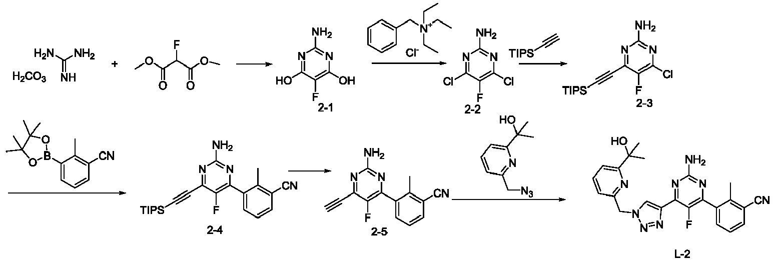

Step 1: sodium metal (1.4 g, 23.12 mmol) was slowly added to anhydrous ethanol (15 mL) in batches, the reaction solution was heated to 80°C under the protection of argon gas, reacted for 2 h, and cooled to the room temperature, a compound: guanidine carbonate (2.8 g, 23.12 mmol) was added, the reaction solution was heated to 80°C, reacted for 30 min, and cooled to the room temperature, dimethyl 2-fluoromalonate (5.0 g, 33.31 mmol) was added, and the reaction solution was heated to 80°C and stirred for 2 h until the reaction process monitored by LC-MS was completed. Then, the reaction solution was filtered, a solid was collected and dissolved in water, the pH of the solution was adjusted with concentrated hydrochloric acid to 5, a large amount of solid was precipitated out, followed by filteration and drying to obtain a compound 2-1 (2.8 g, yield: 83%). MS (ESI) 146.1 [M+H]⁺.

Step 2: the compound 2-1 (2.3 g, 15.85 mmol) was added to anhydrous acetonitrile (50 mL), the reaction solution was cooled to 0°C under the protection of argon gas, a compound: phosphorus oxychloride (17 mL) was added, phosphorus pentachloride (2.5 g, 12.01 mmol) and N-benzyl-N,N-diethylethylammonium chloride (11.0 g, 48.29 mmol) were added, and the reaction solution was heated to 80°C and stirred overnight until the reaction process monitored by LC-MS was completed. Then, the reaction solution was added to water, the pH of the mixture was adjusted with ammonia water to 8, the mixture was extracted with DCM (100 mL × 2), and obtained organic phases were combined, washed with 1 M hydrochloric acid, dried, and concentrated under reduced pressure to dryness to obtain a compound 2-2 (0.6 g, yield: 21%). MS (ESI) 182.1 [M+H]⁺.

Step 3: referring to step 1 of Example 1 by replacing the compound 1-1 with the compound 2-2, a compound 2-3 (0.8 g, yield: 81%) was obtained. MS (ESI) 328.1 [M+H]⁺.

Steps 4 to 6: referring to steps 3 to 5 of Example 1 by replacing the compound 1-3 with the compound 2-3, a white solid product L-2 (8.49 mg, yield: 10%) was obtained. MS (ESI) 445.2 [M+H]⁺. ¹H NMR (400 MHz, DMSO-d6) δ 8.76 (s, 1H), 7.98-7.83 (m, 2H), 7.73 (d, J=7.8 Hz, 1H), 7.58 (d, J=7.9 Hz, 1H), 7.51 (t, J=7.8 Hz, 1H), 7.37 (d, J=7.7 Hz, 1H), 6.87 (s, 2H), 5.88 (s, 2H), 2.62 (s, 3H), 2.40 (s, 3H), 1.63 (s, 6H).

### Example 3: preparation of a compound L-3

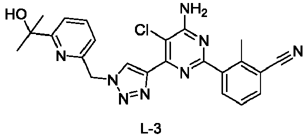

Referring to step 5 of Example 1, a white solid product L-3 (58.84 mg, yield: 43%) was prepared from the intermediate V8 and the intermediate V4. MS (ESI) 461.2 [M+H]⁺. ¹H NMR (400 MHz, DMSO-d6) δ 8.88 (s, 1H), 7.96 (d, J=7.8 Hz, 1H), 7.85 (d, J=7.6 Hz, 1H), 7.77 (t, J=7.8 Hz, 1H), 7.57 (d, J=7.8 Hz, 1H), 7.46 (t, J=7.7 Hz, 1H), 7.12 (d, J=7.4 Hz, 1H), 5.77 (s, 2H), 5.19 (s, 1H), 2.65 (s, 3H), 1.34 (s, 6H).

### Example 4: preparation of a compound L-4

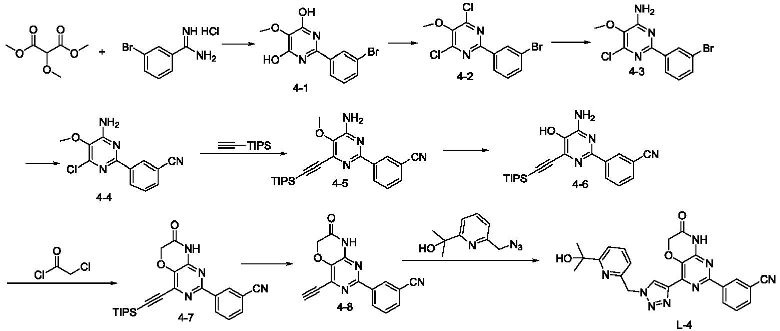

Step 1: sodium metal (1.52 g, 66.09 mmol) was added to methanol (60 mL) at 0°C, the reaction solution was naturally heated and stirred until the sodium metal was completely dissolved, 3-bromobenzamidine hydrochloride (5.0 g, 21.37 mmol) was added to the newly formed methanol solution of sodium methoxide, the reaction solution was stirred for 10 min and cooled to 0°C, a methanol solution (10 mL) of dimethyl methoxymaleate (3.46 g, 21.37 mmol) was slowly dropwise added to the reaction solution, and after the methanol solution of dimethyl methoxymaleate was added, the reaction solution was heated to reflux for 2 h. After the raw materials completely reacted, the reaction solution was evaporated under reduced pressure to dryness, neutralized with 1 M diluted hydrochloric acid until the pH was 7, and filtered to obtain a solid product, and the filter cake was washed with ethyl acetate and dried to obtain a solid compound 4-1 (3.7 g). MS (ESI) 297 [M+H]⁺.

Step 2: the compound 4-1 (500 mg, 1.77 mmol) was added to phosphorus oxychloride (6 mL), and the reaction solution was heated to 125°C and stirred for 16 h. After the raw materials completely reacted, the reaction solution was cooled to the room temperature, diluted with ethyl acetate, and added to an iced aqueous solution of sodium bicarbonate in batches while the iced aqueous solution of sodium bicarbonate was stirred, an organic phase was separated, the aqueous phase was extracted with ethyl acetate, obtained organic phases were combined, dried with anhydrous sodium sulfate, and filtered, an obtained filtrate was evaporated under reduced pressure to dryness, and an obtained crude product was subjected to column chromatography (ethyl acetate/petroleum ether=0 to 30%) to obtain a solid compound 4-2 (0.43 g). MS (ESI) 333 [M+H]⁺.

Step 3: referring to step 2 of Example 1 by replacing the compound 1-2 with the compound 4-2, a solid compound 4-3 (0.33 g) was obtained. MS (ESI) 316 [M+H]⁺.

Step 4: the compound 4-3 (341 mg, 1.09 mmol), zinc cyanide (76 mg, 0.65 mmol), and methanesulfonato(2-di-t-butylphosphino-2',4',6' -tri-i-propyl-1,1'-biphenyl)(2'-amino-1,1'-bi phenyl-2-yl)palladium (II) (87 mg, 0.11 mmol) were added to a mixed solvent of tetrahydrofuran (5 mL) and water (5 mL), the mixture reacted at 90°C for 2 h, and extracted with ethyl acetate, followed by drying with anhydrous sodium sulfate and filteration, an obtained filtrate was evaporated under reduced pressure to dryness, and an obtained crude product was subjected to column chromatography (ethyl acetate/petroleum ether=0 to 35%) to obtain a solid compound 4-4 (0.26 g). MS (ESI) 261 [M+H]⁺.

Step 5: referring to step 1 of Example 1 by replacing the compound 1-1 with the compound 4-4, a solid compound 4-5 (0.31 g) was obtained. MS (ESI) 407 [M+H]⁺.

Step 6: the compound 4-5 (320 mg, 0.79 mmol) was added to dry dichloromethane (20 mL), the reaction solution was cooled to 0°C, a dichloromethane solution of boron tribromide (1 M, 3.2 mL, 3.15 mmol) was dropwise added, and after the dichloromethane solution of boron tribromide was added, the reaction solution was naturally heated to the room temperature and stirred for 4 h. After the raw materials completely reacted, the reaction solution was added to a saturated sodium bicarbonate aqueous solution, and the mixture was extracted with dichloromethane, followed by drying with anhydrous sodium sulfate, filteration, and evaporation under reduced pressure to dryness to obtain a solid compound 4-6 (0.33 g). MS (ESI) 393 [M+H]⁺.

Step 7: the compound 4-6 (331 mg, 0.84 mmol), chloroacetyl chloride (113 mg, 1.01 mmol), and potassium carbonate (581 mg, 4.21 mmol) were added to dry tetrahydrofuran (6 mL), and the reaction solution was stirred at the room temperature for 2 h, heated to 70°C, and stirred for 18 h. After the raw materials completely reacted, the reaction solution was filtered to remove undissolved materials, and an obtained filtrate was concentrated and purified by column chromatography (ethyl acetate/petroleum ether=0 to 50%) to obtain a compound 4-7 (0.18 g). MS (ESI) 433 [M+H]⁺.

Step 8: the compound 4-7 (160 mg, 0.37 mmol) and a potassium fluoride aqueous solution (30%, 2 mL) were added to a mixed solvent of methanol and tetrahydrofuran (v/v=1: 1, 4 mL), and the reaction solution was stirred at 30°C for 2 days. Then, the reaction solution was extracted with ethyl acetate, an obtained organic phase was dried with anhydrous sodium sulfate and filtered, and an obtained filtrate was concentrated to obtain a compound 4-8 (132 mg) that was directly used for the next reaction without being purified. MS (ESI) 277 [M+H]⁺.

Step 9: referring to step 5 of Example 1 by replacing the compound 1-5 with the compound 4-8, a product L-4 (12.78 mg) was obtained. ¹H NMR (400 MHz, DMSO-d6) δ 11.87 (s, 1H), 8.95 (s, 1H), 8.60 (dd, J=7.5, 6.3 Hz, 2H), 7.95 (d, J=7.7 Hz, 1H), 7.75 (dt, J=13.7, 7.7 Hz, 2H), 7.58 (d, J=7.6 Hz, 1H), 7.10 (d, J=7.5 Hz, 1H), 5.81 (s, 2H), 5.20 (s, 1H), 4.86 (s, 2H), 1.37 (s, 6H). MS (ESI) 469.2 [M+H]⁺.

### Example 5: preparation of a compound L-5

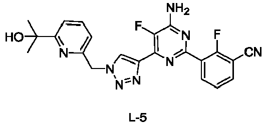

Referring to the preparation method of Example 1 by replacing the intermediate V1 with the intermediate V2, a white solid compound L-5 (28.08 mg, yield: 23.31%) was obtained. ¹H NMR (400 MHz, DMSO-d6) δ 8.79 (s, 1H), 8.26-8.21 (m, 1H), 8.02-7.98 (m, 1H), 7.78-7.74 (m, 1H), 7.60 (brs, 2H), 7.57 (d, J=8.0 Hz, 1H), 7.51-7.46 (m, 1H), 7.11 (d, J=7.6 Hz, 1H), 5.79 (s, 2H), 5.18 (s, 1H), 1.35 (s, 6H). MS (ESI) 449.2 [M+H]⁺.

### Example 6: preparation of a compound L-6

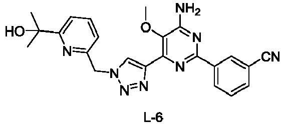

Referring to the preparation method of Example 1 by replacing the intermediate V1 with the intermediate V2, a white solid product L-6 (15.30 mg) was obtained. ¹H NMR (400 MHz, DMSO-d6) δ 8.88 (s, 1H), 8.62 (s, 1H), 8.58 (d, J=8.0 Hz, 1H), 7.90 (d, J=7.6 Hz, 1H), 7.77 (t, J=7.7 Hz, 1H), 7.67 (t, J=7.8 Hz, 1H), 7.58 (d, J=7.7 Hz, 1H), 7.20 (s, 2H), 7.11 (d, J=7.5 Hz, 1H), 5.79 (s, 2H), 5.19 (s, 1H), 3.72 (s, 3H), 1.38 (s, 6H). MS (ESI) 443.2 [M+H]⁺.

### Example 7: preparation of a compound L-7

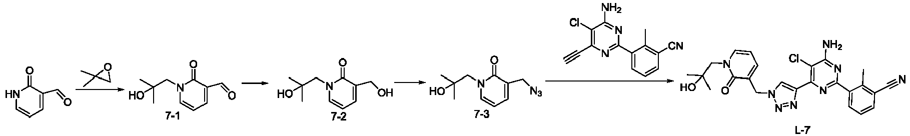

Step 1: 2-oxo-1,2-dihydropyridine-3-acetaldehyde (1.2 g, 9.76 mmol), 2,2-dimethyloxirane (1.8 mL, 120.26 mmol), and potassium carbonate (2.76 g, 20.0 mmol) were added to DMF (14 mL), and the reaction solution was stirred at 120°C for 3 h. Then, the reaction solution was filtered, and an obtained filtrate was concentrated and subjected to silica-gel column chromatography (EA/PE=0 to 100%) to obtain a solid compound 7-1 (1.03 g, yield: 54%). MS (ESI) 196.1 [M+H]⁺.

Step 2: the compound 7-1 (200 mg, 1.03 mmol) was added to tetrahydrofuran (10 mL), the reaction solution was cooled to 0°C, LAH (78 mg, 2.05 mmol) was added to the reaction solution, and the reaction solution was stirred at 0°C for 1 h. After the raw materials completely reacted, sodium sulfate decahydrate was added in batches until no bubbles were produced in the reaction solution, the reaction solution was stirred for 10 min and filtered, and an obtained filtrate was directly concentrated to obtain an oily compound 7-2 (183 mg, yield: 91%). MS (ESI) 198 [M+H]⁺.

Step 3: the compound 7-2 (183 mg, 0.93 mmol) was dissolved in dichloromethane (4 mL), DPPA (383 mg, 1.39 mmol) and DBU (212 mg, 1.39 mmol) were added in sequence, and the reaction solution was stirred at the room temperature for 18 h. After the raw materials completely reacted, the reaction solution was diluted with dichloromethane and washed twice with water, an obtained organic phase was dried with anhydrous sodium sulfate and filtered, and an obtained filtrate was concentrated to obtain an oily crude compound 7-3 (374 mg). MS (ESI) 223 [M+H]⁺.

Step 4: referring to step 5 of Example 1, a pale-yellow solid product L-7 (19.62 mg, yield: 11%) was prepared from the intermediate V8 and the compound 7-3. ¹H NMR (400 MHz, DMSO-d6) δ 8.70 (s, 1H), 7.94 (d, J=7.5 Hz, 1H), 7.84 (d, J=7.7 Hz, 1H), 7.62 (dd, J=6.8, 1.8 Hz, 1H), 7.46 (t, J=7.8 Hz, 1H), 7.41 (d, J=5.5 Hz, 1H), 6.22 (t, J=6.8 Hz, 1H), 5.45 (s, 2H), 4.72 (s, 1H), 3.90 (s, 2H), 2.62 (s, 3H), 1.01 (s, 6H). MS (ESI) 491.2 [M+H]⁺.

### Example 8: preparation of a compound L-8

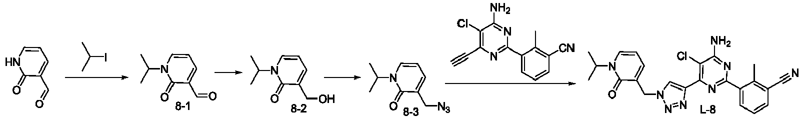

Referring to the preparation method of Example 7 by replacing 2,2-dimethyloxirane with 2-iodopropane and referring to step 5 of Example 1, a white solid product L-8 (14.43 mg, yield: 8.4%) was obtained. ¹H NMR (400 MHz, DMSO-d6) δ 8.75 (s, 1H), 7.95 (d, J=7.6 Hz, 1H), 7.84 (d, J=7.2 Hz, 1H), 7.76 (dd, J=7.0, 1.9 Hz, 1H), 7.46 (t, J=7.7 Hz, 1H), 7.30 (dd, J=6.8, 1.8 Hz, 1H), 6.28 (t, J=6.9 Hz, 1H), 5.45 (s, 2H), 5.04 (dt, J=13.6, 6.8 Hz, 1H), 2.63 (s, 3H), 1.25 (d, J=6.8 Hz, 6H). MS (ESI) 461.1 [M+H]⁺.

### Example 9: preparation of a compound L-9

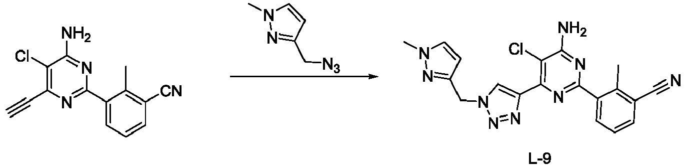

Referring to step 5 of Example 1, a pale-yellow solid product L-9 (23.77 mg, yield: 16%) was prepared from the intermediate V8 and 3-(azidomethyl)-1-methyl-1H-pyrazole. ¹H NMR (400 MHz, DMSO-d6) δ 8.73 (s, 1H), 7.95 (d, J=7.5 Hz, 1H), 7.84 (d, J=7.5 Hz, 1H), 7.63 (s, 1H), 7.46 (s, 1H), 6.24 (s, 1H), 5.59 (s, 2H), 3.77 (s, 3H), 2.63 (s, 3H). MS (ESI) 406.1 [M+H]⁺.

### Example 10: preparation of a compound L-10

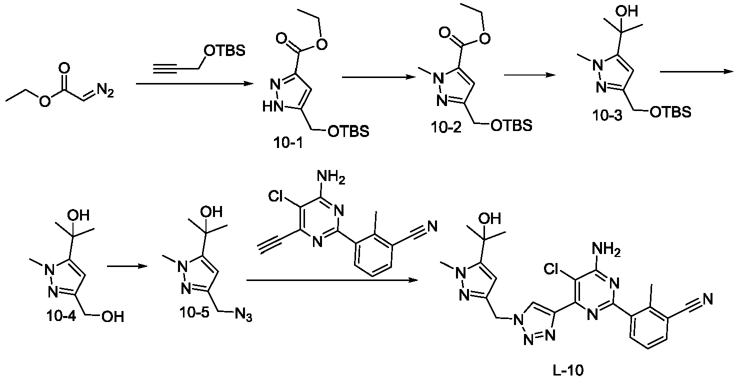

Step 1: a toluene solution (20 mL) of ethyl diazoacetate (3.04 g, 17.84 mmol) and tert-butyldimethyl(2-propynyloxy)silane (2.06 mL, 19.62 mmol) was heated to 140°C under microwave conditions and reacted for 30 min. Then, the reaction solution was concentrated and subjected to silica-gel column chromatography (EA/PE=0-35%) to obtain an oily compound 10-1 (2.7 g, yield: 36%). MS (ESI) 285 [M+H]⁺.

Step 2: the compound 10-1 (2.55 g, 9.11 mmol), iodomethane (3.88 g, 27.32 mmol), and potassium carbonate (3.77 g, 27.32 mmol) were added to DMF (20 mL), and the reaction solution was stirred at the room temperature for 3 h. Then, the reaction solution was added to water, the mixture was extracted with ethyl acetate, an obtained organic phase was dried with anhydrous sodium sulfate and filtered, and an obtained filtrate was concentrated and subjected to silica-gel column chromatography (EA/PE=0 to 15%) to obtain an oily compound 10-2 (0.81 g, yield: 30%). MS (ESI) 299 [M+H]⁺.

Step 3: the compound 10-2 (298 mg, 1.0 mmol) was added to tetrahydrofuran (10 mL), the reaction solution was cooled to 0°C, methyl magnesium iodide (3 M, 2 mL, 6.0 mmol) was slowly dropwise added to the reaction solution, and the reaction solution was heated to the room temperature and reacted for 1 h. Then, the reaction was quenched with a saturated ammonium chloride aqueous solution, the mixture was extracted with ethyl acetate, obtained organic phases were combined, dried with anhydrous sodium sulfate, and filtered, and an obtained filtrate was concentrated to obtain an oily compound 10-3 (262 mg, yield: 92%). MS (ESI) 285 [M+H]⁺.

Step 4: referring to step 3 of Example 21 by replacing the compound 21-2 with the compound 10-3, an oily compound 10-4 (512 mg) was obtained. MS (ESI) 171 [M+H]⁺.

Steps 5 and 6: referring to steps 3 and 4 of Example 7 by replacing the compound 7-2 with the compound 10-4, a white solid product L-10 (26 mg, yield: 9%) was obtained. ¹H NMR (400 MHz, DMSO-d6) δ 8.74 (s, 1H), 7.99-7.93 (m, 1H), 7.85 (dd, J=7.7, 1.2 Hz, 1H), 7.47 (t, J=7.8 Hz, 1H), 6.09 (s, 1H), 5.52 (s, 2H), 5.25 (s, 1H), 3.89 (s, 3H), 2.64 (s, 3H), 1.42 (s, 6H). MS (ESI) 464.1 [M+H]⁺.

### Example 11: preparation of a compound L-11

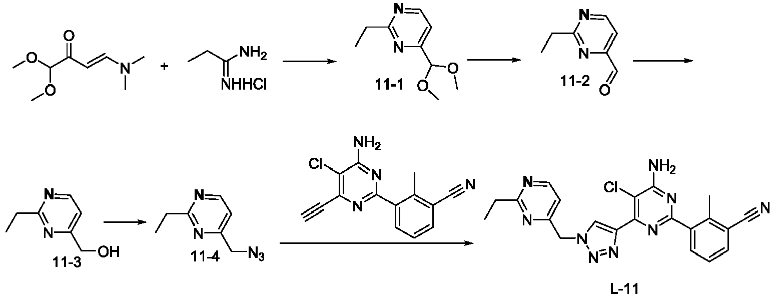

Step 1: referring to step 1 of Example 4, an oily compound 11-1 (2.1 g) was prepared from (E)-4-dimethylamino-1,1-dimethoxybut-3-en-2-one (3.16 g, 20 mmol) and procainamide hydrochloride (3.24 g, 30 mmol). MS (ESI) 183 [M+H]⁺.

Step 2: the compound 11-1 (1.3 g, 7.14 mmol) was added to hydrochloric acid (3 M, 12 mL), and the reaction solution was heated to 50°C and stirred for 18 h. After the raw materials completely reacted, the reaction solution was cooled to the room temperature and neutralized with saturated sodium bicarbonate, the mixture was extracted with dichloromethane, obtained organic phases were combined, dried with anhydrous sodium sulfate, and filtered, and an obtained filtrate was concentrated to obtain an oily compound 11-2 (0.42 g). MS (ESI) 137 [M+H]⁺.

Step 3: the compound 11-2 (0.42 g, 3.09 mmol) was added to methanol (10 mL), sodium borohydride (352 mg, 9.26 mmol) was added in batches, and the reaction solution was stirred at the room temperature for 1 h. After the raw materials completely reacted, the reaction was quenched with a saturated ammonium chloride aqueous solution, the mixture was extracted with dichloromethane, an obtained organic phase was dried with anhydrous sodium sulfate and filtered, and an obtained filtrate was concentrated to obtain an oily compound 11-3 (270 mg). MS (ESI) 139 [M+H]⁺.

Steps 4 and 5: referring to steps 3 and 4 of Example 7 by replacing the compound 7-2 with the compound 11-3, a white solid product L-11 (33 mg, yield: 20%) was obtained. ¹H NMR (400 MHz, DMSO-d6) δ 8.93 (s, 1H), 8.70 (d, J=5.1 Hz, 1H), 7.97 (dd, J=7.8, 1.2 Hz, 1H), 7.85 (dd, J=7.7, 1.2 Hz, 1H), 7.47 (t, J=7.7 Hz, 1H), 7.06 (d, J=5.1 Hz, 1H), 5.82 (s, 2H), 2.83 (q, J=7.6 Hz, 2H), 2.66 (s, 3H), 1.20 (t, J=7.6 Hz, 3H). MS (ESI) 432 [M+H]⁺.

### Example 12: preparation of a compound L-12

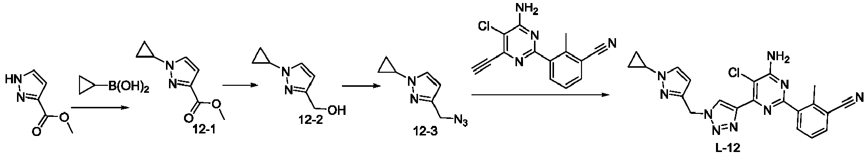

Step 1: methyl lH-pyrazole-3-carboxylate (2.52 g, 20 mmol), cyclopropylboronic acid (2.58 g, 30 mmol), copper acetate (3.98 g, 22 mmol), 2,2-bipyridine (3.43 g, 22 mmol), and sodium carbonate (4.24 g, 40 mmol) were added to 1,2-dichloroethane (150 mL), the reaction solution was heated to 70°C in oxygen and stirred for 3 h, sodium methoxide (1.62 g, 30 mmol) was added to the reaction solution, and the reaction solution was heated to reflux for 2 h and stirred at the room temperature for 16 h. Then, the reaction solution was diluted with dichloromethane and washed twice with water, a combined organic phase was dried with anhydrous sodium sulfate and filtered, and an obtained filtrate was concentrated and subjected to silica-gel column chromatography (EA/PE=0 to 20%) to obtain an oily product 12-1 (1.8 g). MS (ESI) 167 [M+H]⁺.

Steps 2 to 4: referring to steps 2 to 4 of Example 7 by replacing the compound 7-1 with the compound 12-1, a white solid product L-12 (34 mg, yield: 21%) was obtained. ¹H NMR (400 MHz, DMSO-d6) δ 8.74 (s, 1H), 8.00-7.92 (m, 1H), 7.85 (dd, J=7.7, 1.2 Hz, 1H), 7.72 (d, J=2.3 Hz, 1H), 7.46 (t, J=7.8 Hz, 1H), 6.23 (d, J=2.3 Hz, 1H), 5.59 (s, 2H), 3.66 (ddd, J=11.2, 7.3, 3.9 Hz, 1H), 2.64 (s, 3H), 1.01-0.94 (m, 2H), 0.94-0.84 (m, 2H). MS (ESI) 432 [M+H]⁺.

### Example 13: preparation of a compound L-13

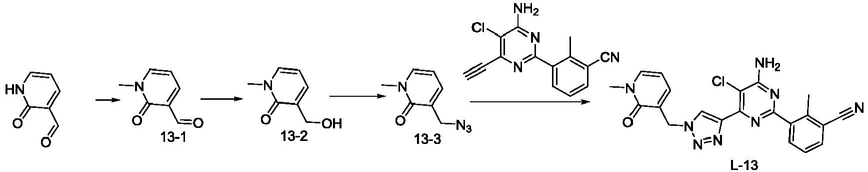

Step 1: referring to the preparation method of Example 10 by replacing the compound 10-1 with 2-oxo-1,2-dihydropyridine-3-acetaldehyde, a solid product 13-1 (2.1 g, yield: 77%) was obtained. MS (ESI) 138 [M+H]⁺.

Steps 2 and 3: referring to steps 2 and 3 of Example 7 by replacing the compound 7-1 with the compound 13-1, an oily compound 13-3 (199 mg) was obtained. MS (ESI) 165 [M+H]⁺.

Step 4: referring to step 5 of Example 1, a white solid product L-13 (16 mg, yield: 10%) was obtained. ¹H NMR (400 MHz, DMSO-d6) δ 8.73 (s, 1H), 7.99-7.93 (m, 1H), 7.85 (dd, J=7.7, 1.2 Hz, 1H), 7.72 (dd, J=6.8, 2.0 Hz, 1H), 7.47 (t, J=7.8 Hz, 1H), 7.40 (dd, J=6.9, 1.9 Hz, 1H), 6.23 (t, J=6.8 Hz, 1H), 5.45 (s, 2H), 3.42 (s, 3H), 2.64 (s, 3H). MS (ESI) 433 [M+H]⁺.

### Example 14: preparation of a compound L-14

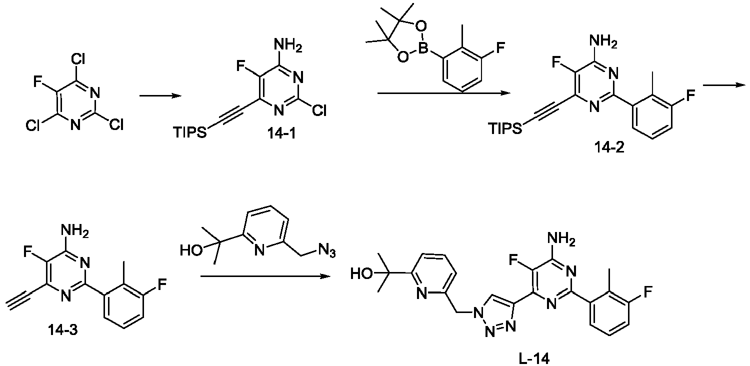

Referring to steps 3 to 5 of Example 1 by replacing the intermediate V1 with the intermediate V3, a pale-yellow solid product L-14 (65 mg) was obtained. ¹H NMR (400 MHz, DMSO-d6) δ 8.76 (s, 1H), 7.77 (t, J=7.8 Hz, 1H), 7.57 (d, J=7.8 Hz, 1H), 7.52-7.40 (m, 3H), 7.317.15 (m, 2H), 7.11 (d, J=7.5 Hz, 1H), 5.78 (s, 2H), 5.18 (s, 1H), 2.35 (s, 3H), 1.35 (s, 6H). MS (ESI) 438.1 [M+H]⁺.

### Example 15: preparation of a compound L-15

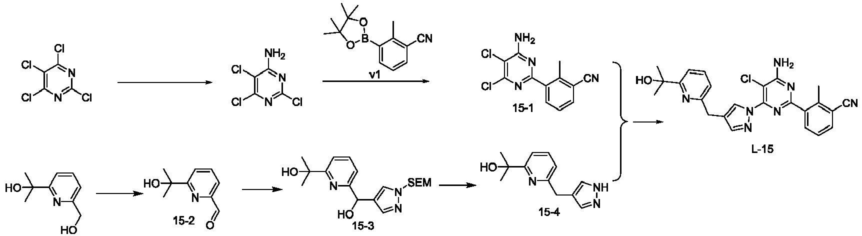

Steps 1-1 and 1-2: referring to steps 2 and 3 of Example 1 by replacing the compound 1-2 with perchloropyrimidine, a solid compound 15-1 (0.51 g, yield: 24%) was obtained. MS (ESI) 279 [M+H]⁺.

Step 2-1: 2-(6-(hydroxymethyl)pyridin-2-yl)propan-2-ol (550 mg, 3.27 mmol) was dissolved in dichloromethane (40 mL), the reaction solution was cooled to 0°C, DMP (2.1 g, 4.91 mmol) was added, and the reaction solution was stirred at the room temperature for 4 h until the reaction process monitored by TLC was completed. Then, the reaction solution was diluted with dichloromethane (200 mL) and washed with water (150 mL × 2), and an organic phase was separated, dried with anhydrous sodium sulfate, concentrated under reduced pressure, and subjected to silica-gel column chromatography (EA/PE=0 to 30%) to obtain a yellow oily compound 15-2 (320 mg, yield: 59.3%). MS (ESI) 166 [M+H]⁺.

Step 2-2: 4-iodo-1-(((2-(trimethylsilyl)ethoxy)methyl)-1H-pyrazole (1.2 g, 3.61 mmol) was dissolved in tetrahydrofuran (20 mL), n-butyllithium (2.7 mL, 5.43 mmol) was dropwise added at -70°C, and the reaction solution was stirred for 2 h. The compound 15-2 (300 mg, 1.81 mmol) was dissolved in tetrahydrofuran (2 mL) and added to the above solution at -70°C. The reaction solution was slowly heated to 0°C and stirred for 2 h until the reaction process monitored by TLC was completed. Then, a saturated ammonium chloride aqueous solution (5 mL) was added to the reaction solution, the mixture was extracted with ethyl acetate, obtained organic phases were combined, dried with anhydrous sodium sulfate, and filtered, and an obtained filtrate was concentrated under reduced pressure and subjected to silica-gel column chromatography (EA/PE=0 to 70%) to obtain a yellow oily compound 15-3 (80 mg, yield: 12%). MS (ESI) 364 [M+H]⁺.

Step 2-3: the compound 15-3 (80 mg, 0.22 mmol) was dissolved in a mixed solvent of triethylsilane (3 mL) and trifluoroacetic acid (1 mL), and the reaction solution was stirred at 80°C for 24 h until the reaction process monitored by TLC was completed. Then, the reaction solution was concentrated under reduced pressure, and an obtained residue was subjected to silica-gel column chromatography (EA/PE=0 to 70%) to obtain a yellow colloidal compound 15-4 (22 mg, yield: 46%). MS (ESI) 218 [M+H]⁺.

Step 3: the compound 15-1 (30 mg, 0.11 mmol) and the compound 15-4 (22 mg, 0.19 mmol) were dissolved in acetonitrile (6 mL), potassium carbonate (297 mg, 2.15 mmol) was added, and the reaction solution was stirred at 140°C under microwave conditions for 1 h. Then, the reaction solution was concentrated and separated and purified by preparative high-performance liquid chromatography to obtain a white solid product L-15 (8.94 mg, yield: 19%, purity: 95.95%). ¹H NMR (400 MHz, DMSO-d6) δ 8.33 (s, 1H), 7.97 (d, J=7.8 Hz, 1H), 7.87 (d, J=6.4 Hz, 1H), 7.78 (s, 1H), 7.65 (t, J=7.7 Hz, 1H), 7.49-7.43 (m, 2H), 7.10 (d, J=7.2 Hz, 1H), 5.17 (s, 1H), 3.96 (s, 2H), 2.64 (s, 3H), 1.40 (s, 6H). MS (ESI) 460.2 [M+H]⁺.

### Example 16: preparation of a compound L-16

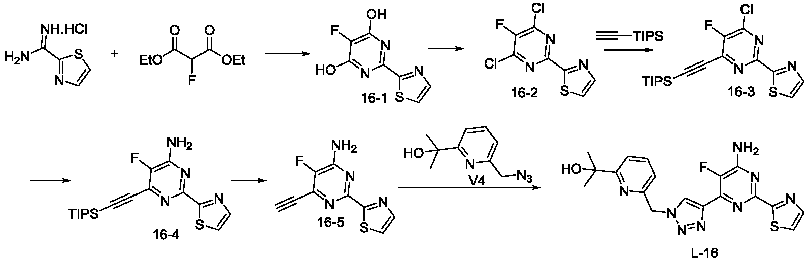

Steps 1 and 2: referring to steps 1 and 2 of Example 4, a solid compound 16-2 (2.07 g, purity: 100%, yield: 79%) was prepared from diethyl 2-fluoromalonate (9.80 g, 55.00 mmol) and 2-thiazolecarboxamidine hydrochloride (4.5 g, 27.50 mmol). MS (ESI) 250.0 [M+H]⁺.

Steps 3 to 6: referring to steps 1, 2, and 4 of Example 1 by replacing the compound 1-1 with the compound 16-2, a white solid product L-16 (32.58 mg, yield: 9%) was obtained. ¹H NMR (400 MHz, DMSO-d6) δ 8.79 (s, 1H), 7.94 (d, J=3.1 Hz, 1H), 7.85 (d, J=3.1 Hz, 1H), 7.77 (t, J=7.8 Hz, 1H), 7.67 (s, 2H), 7.58 (d, J=7.3 Hz, 1H), 7.11 (d, J=7.1 Hz, 1H), 5.81 (s, 2H), 5.18 (s, 1H), 1.36 (s, 6H). MS (ESI) 413.1 [M+H]⁺.

### Example 17: preparation of a compound L-17

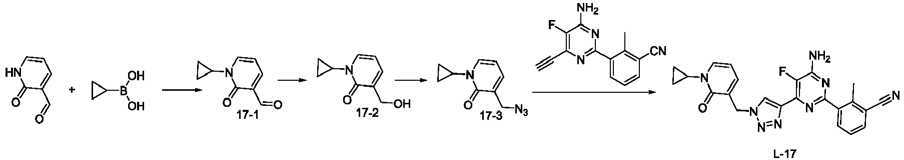

Step 1: referring to step 1 of Example 12 by replacing methyl lH-pyrazole-3-carboxylate with 2-oxo-1,2-dihydropyridine-3-carbaldehyde, a solid compound 17-1 (280 mg, yield: 17%) was obtained. MS (ESI) 164.1 [M+H]⁺.

Steps 2 to 4: referring to steps 2 to 4 of Example 7 by replacing the compound 7-1 with the compound 17-1 and replacing the intermediate V8 with the compound 1-5, a white solid product L-17 (26 mg, yield: 15%) was obtained. ¹H NMR (400 MHz, DMSO-d6) δ 8.65 (s, 1H), 7.91 (dd, J=7.8, 1.3 Hz, 1H), 7.82 (dd, J=7.7, 1.3 Hz, 1H), 7.56 (dd, J=6.9, 1.9 Hz, 1H), 7.51 (s, 2H), 7.45 (t, J=7.8 Hz, 1H), 7.34 (dd, J=6.9, 1.9 Hz, 1H), 6.19 (t, J=6.9 Hz, 1H), 5.45 (s, 2H), 3.37-3.34 (m, 1H), 2.61 (s, 3H), 1.00-0.92 (m, 2H), 0.84-0.75 (m, 2H). MS (ESI) 443.2 [M+H]⁺.

### Example 18: preparation of a compound L-18

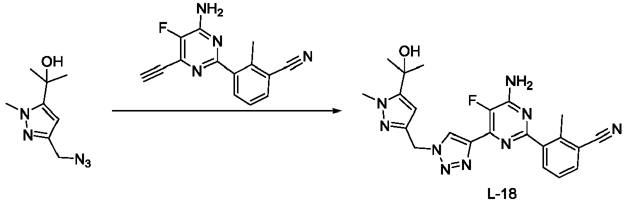

Referring to step 5 of Example 1 by replacing the intermediate V4 with 2-(3-(azidomethyl)-1-methyl-1H-pyrazol-5-yl)propan-2-ol, a white solid product L-18 (28.76 mg, yield: 23%) was obtained. ¹H NMR (400 MHz, cdcl3) δ 8.69 (s, 1H), 7.95 (d, J=7.0 Hz, 1H), 7.86 (d, J=7.7 Hz, 1H), 7.54 (s, 2H), 7.48 (t, J=7.7 Hz, 1H), 6.12 (s, 1H), 5.56 (s, 2H), 5.28 (s, 1H), 3.92 (s, 3H), 2.65 (s, 3H), 1.45 (s, 6H). MS (ESI) 448.2 [M+H]⁺.

### Example 19: preparation of a compound L-19

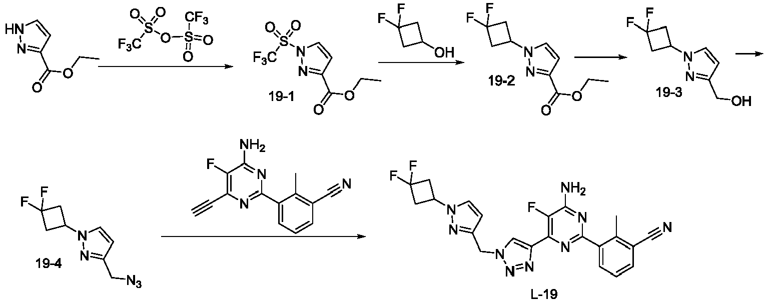

Step 1: ethyl 1*H*-pyrazole-3-carboxylate (1.4 g, 9.99 mmol) was dissolved in dichloromethane (50 mL), trifluoromethylsulfonyl triflate (4.23 g, 14.99 mmol) was added, and the reaction solution was stirred at the room temperature for 1 h. Then, the reaction solution was added to a saturated ammonium chloride aqueous solution, the mixture was extracted with dichloromethane, obtained organic phases were combined, dried with anhydrous sodium sulfate, and filtered, and an obtained filtrate was concentrated and separated by silica-gel column chromatography (EA/PE=0 to 50%) to obtain a white solid compound 18-1 (2.4 g, yield: 88%). MS (ESI) 273.0 [M+H]⁺.

Step 2: 3,3-difluorocyclobutanol (600 mg, 5.55 mmol) was dissolved in acetonitrile (10 mL), cesium carbonate (1.99 g, 6.11 mmol) was added, the reaction solution was cooled to 0°C, an acetonitrile solution (10 mL) of the compound 18-1 (1.38 g, 5.05 mmol) was dropwise added, and the reaction solution was stirred at 0°C for 1 h, heated to the room temperature, and stirred for 2 h. Then, the reaction solution was diluted with dichloromethane and filtered, and an obtained filtrate was concentrated and separated by silica-gel column chromatography (EA/PE=0 to 50%) to obtain a colorless oily compound 18-2 (712 mg, yield: 54%). MS (ESI) 231.1 [M+H]⁺.

Steps 3 to 5: referring to steps 2 to 4 of Example 7 by replacing the compound 7-1 with the compound 18-2 and replacing the intermediate V8 with the compound 1-5, a white solid product L-19 (36.59 mg, yield: 25%) was obtained. ¹H NMR (400 MHz, cdcl₃) δ 8.71 (s, 1H), 7.95 (d, J=7.0 Hz, 1H), 7.89-7.81 (m, 2H), 7.54 (s, 2H), 7.48 (t, J=7.8 Hz, 1H), 6.31 (d, J=2.3 Hz, 1H), 5.68 (s, 2H), 4.90 (dd, J=13.5, 6.9 Hz, 1H), 3.21-3.01 (m, 4H), 2.65 (s, 3H). MS (ESI) 466.2 [M+H]⁺.

### Example 20: preparation of a compound L-20

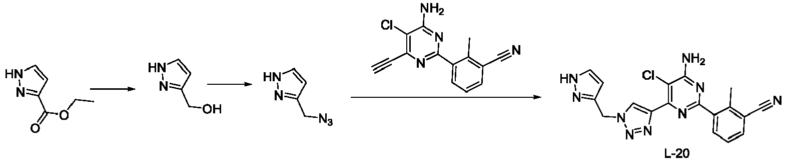

Referring to steps 2 to 4 of Example 7 by replacing the compound 7-1 with ethyl 1H-pyrazole-3-carboxylate, a white solid product L-20 (27.69 mg, yield: 24%) was obtained. ¹H NMR (400 MHz, DMSO-d6) δ 12.86 (s, 1H), 8.73 (s, 1H), 8.01-7.92 (m, 1H), 7.85 (dd, J=7.7, 1.1 Hz, 1H), 7.69 (s, 1H), 7.46 (t, J=7.8 Hz, 1H), 6.28 (d, J=1.9 Hz, 1H), 5.65 (s, 2H), 2.64 (s, 3H). MS (ESI) 392.1 [M+H]⁺.

### Example 21: preparation of a compound L-21

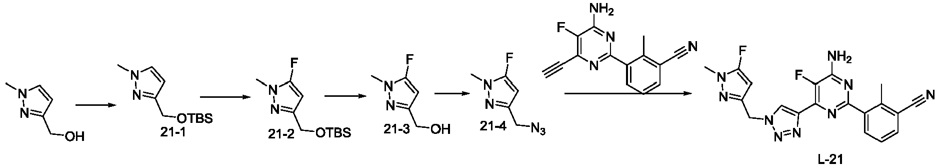

Step 1: (1-methylpyrazol-3-yl)methanol (1.12 g, 9.99 mmol) was dissolved in dichloromethane (60 mL), 1*H*-imidazole (1.36 g, 19.98 mmol), 4-dimethylaminopyridine (1.83 g, 14.98 mmol), and TBSCl (1.23 g, 14.98 mmol) were added, and the reaction solution was stirred at the room temperature for 2 h. After the reaction was completed, the reaction was quenched with an aqueous solution, the mixture was extracted with dichloromethane, an obtained organic phase was washed with a saturated salt water, dried with anhydrous sodium sulfate, and filtered, and an obtained filtrate was concentrated and separated by silica-gel column chromatography (EA/PE=0 to 30%) to obtain a colorless oily compound 21-1 (1.7 g, yield: 75%). MS (ESI) 227.2 [M+H]⁺.

Step 2: the compound 21-1 (1.5 g, 6.63 mmol) was dissolved in tetrahydrofuran (6 mL), the obtained solution was cooled to -78°C, n-butyllithium (2.5 M, 3.98 mL) was dropwise added, the reaction solution was stirred at -78°C for 1 h, a tetrahydrofuran solution (6 mL) of N-fluorobisbenzenesulfonamide (3.13 g, 9.94 mmol) was dropwise added, and after the tetrahydrofuran solution of N-fluorobisbenzenesulfonamide was added, the reaction solution was stirred at -78°C for 2 h, naturally heated to the room temperature, and stirred for 18 h. Then, the reaction was quenched with a saturated ammonium chloride aqueous solution, the mixture was extracted with ethyl acetate, an obtained organic phase was dried with anhydrous sodium sulfate and filtered, and an obtained filtrate was concentrated and separated by silica-gel column chromatography (EA/PE=0 to 20%) to obtain a reddish-brown oily compound 21-2 (267 mg, yield: 18%). MS (ESI) 245.2 [M+H]⁺.

Step 3: the compound 21-2 (240 mg, 982 mmol) was dissolved in methanol (20 mL), ammonium fluoride (1.09 g, 29 mmol) was added, and the reaction solution was stirred at 80°C for 1 h. After the reaction was completed, the reaction solution was cooled to the room temperature and filtered, the reaction was quenched with an aqueous solution, the mixture was extracted with ethyl acetate, an obtained organic phase was dried with anhydrous sodium sulfate and filtered, and an obtained filtrate was concentrated to obtain a brown oily compound 21-3 (210 mg). MS (ESI) 131.1 [M+H]⁺.

Steps 4 and 5: referring to steps 3 and 4 of Example 7 by replacing the compound 7-2 with the compound 21-3 and replacing the intermediate V8 with the compound 1-5, a white solid product L-21 (63.01 g, yield: 48.77%) was obtained. ¹H NMR (400 MHz, DMSO-d6) δ 8.68 (s, 1H), 7.91 (dd, J=7.8, 1.2 Hz, 1H), 7.83 (dd, J=7.7, 1.3 Hz, 1H), 7.52 (s, 2H), 7.45 (t, J=7.8 Hz, 1H), 6.02 (d, J=5.7 Hz, 1H), 5.53 (s, 2H), 3.63 (d, J=1.2 Hz, 3H), 2.62 (s, 3H). MS (ESI) 408.3 [M+H]⁺.

### Example 22: preparation of a compound L-22

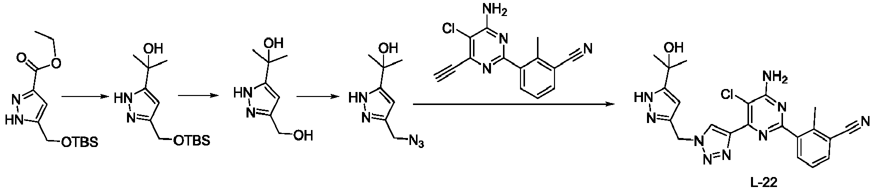

Referring to steps 3 to 6 of Example 10 by replacing the compound 10-2 with the compound 10-1, a white solid product L-22 (32.99 mg, yield: 25%) was obtained. ¹H NMR (400 MHz, DMSO-d6) δ 12.56 (s, 1H), 8.74 (s, 1H), 7.95 (dd, J=7.9, 1.2 Hz, 1H), 7.84 (dd, J=7.7, 1.3 Hz, 1H), 7.46 (t, J=7.8 Hz, 1H), 6.07 (s, 1H), 5.57 (s, 2H), 5.16 (s, 1H), 2.63 (s, 3H), 1.37 (s, 6H). MS (ESI) 450.1 [M+H]⁺.

### Example 23: preparation of a compound L-23

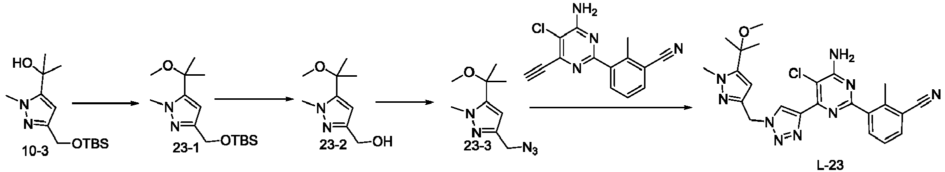

Step 1: the compound 10-3 (380 mg, 1.34 mmol) was dissolved in tetrahydrofuran (20 mL), sodium hydride (385 mg, 16.03 mmol) was added, the reaction solution was stirred for 10 min, iodomethane (1.90 g, 13.36 mmol) was added, and the reaction solution was stirred at the room temperature for 2 h. Then, the reaction solution was placed into an ice-water bath, sodium sulfate decahydrate was added in batches, after the sodium sulfate decahydrate was added, the reaction solution was stirred for 10 min and filtered, and an obtained filtrate was directly concentrated to obtain an oily compound 23-1 (371 mg, yield: 93%). MS (ESI) 299.2 [M+H]⁺.

Step 2: referring to step 3 of Example 21 by replacing the compound 21-2 with the compound 23-1, an oily compound 23-2 (310 mg) was obtained. MS (ESI) 185.1 [M+H]⁺.

Steps 3 and 4: referring to steps 3 and 4 of Example 7 by replacing the compound 7-2 with the compound 23-2 and replacing the intermediate V8 with the compound 1-5, a white solid product L-23 (89.26 mg, yield: 50%) was obtained. ¹H NMR (400 MHz, DMSO-d6) δ 8.74 (s, 1H), 7.95 (dd, J=7.8, 1.1 Hz, 1H), 7.84 (dd, J=7.7, 1.1 Hz, 1H), 7.45 (t, J=7.7 Hz, 1H), 6.18 (s, 1H), 5.55 (s, 2H), 3.83 (s, 3H), 2.91 (s, 3H), 2.63 (s, 3H), 1.43 (s, 6H). MS (ESI) 210.1 [M+H]⁺.

### Example 24: preparation of a compound L-24

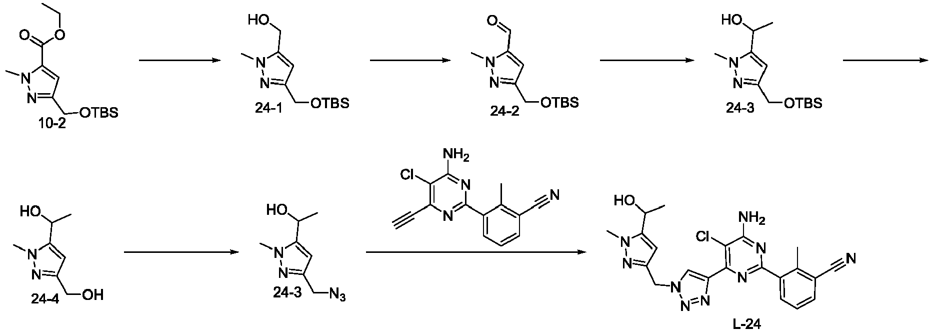

Step 1: referring to step 2 of Example 7 by replacing the compound 7-1 with the compound 10-2, an oily compound 24-1 (451 mg, yield: 75%) was obtained. MS (ESI) 257.2 [M+H]⁺.

Step 2: the compound 24-1 (451 mg, 1.76 mmol) was dissolved in tetrahydrofuran (20 mL), active manganese dioxide (3.06 g, 35.18 mmol) was added, and the reaction solution was stirred at 85°C for 24 h. After the reaction was completed, the reaction solution was cooled to the room temperature and filtered to remove undissolved materials, an obtained filter cake was washed with dichloromethane, and an obtained filtrate was spun under reduced pressure to dryness to obtain a pale-yellow oily compound 24-2 (345 mg, yield: 77%). MS (ESI) 255.2 [M+H]⁺.

Step 3: referring to step 3 of Example 10 by replacing the compound 10-2 with the compound 24-2, a pale-yellow oily compound 24-3 (230 mg) was obtained. MS (ESI) 271.2 [M+H]⁺.

Step 4: referring to step 3 of Example 21 by replacing the compound 21-2 with the compound 24-3, a pale-yellow oily compound 24-4 (214 mg) was obtained. MS (ESI) 157.1 [M+H]⁺.

Steps 5 and 6: referring to steps 3 and 4 of Example 7 by replacing the compound 7-2 with the compound 24-4, a white solid product L-24 (17.62 mg, yield: 25%) was obtained. ¹H NMR (400 MHz, DMSO-d6) δ 8.74 (s, 1H), 7.94 (d, J=7.7 Hz, 1H), 7.84 (d, J=7.6 Hz, 1H), 7.46 (t, J=7.8 Hz, 1H), 6.15 (s, 1H), 5.54 (s, 2H), 5.23 (d, J=5.6 Hz, 1H), 4.73 (p, J=6.4 Hz, 1H), 3.73 (s, 3H), 2.63 (s, 3H), 1.33 (d, J=6.5 Hz, 3H). MS (ESI) 450.2 [M+H]⁺.

### Example 25: preparation of a compound L-25

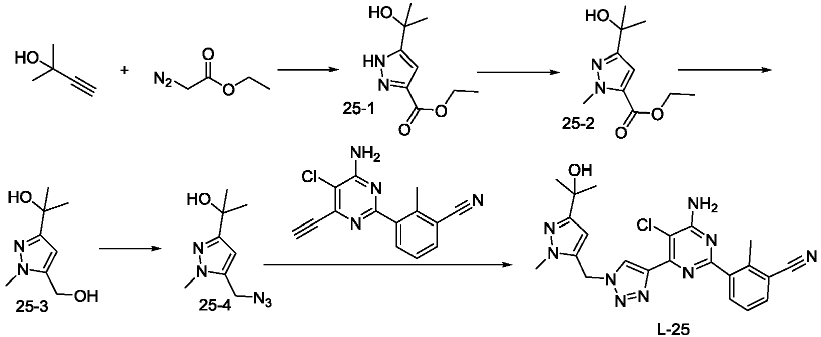

Referring to the preparation method of Example 7, a white solid product L-25 (25.99 mg, yield: 15%) was prepared from 2-methyl-3-butyn-2-ol (20 g, 238.10 mmol) and ethyl 2-diazoacetate (36 g, 313.04 mmol). ¹H NMR (400 MHz, DMSO-d6) δ 8.81 (s, 1H), 7.95 (dd, J=7.8, 1.2 Hz, 1H), 7.84 (d, J=7.7 Hz, 1H), 7.46 (t, J=7.8 Hz, 1H), 6.18 (s, 1H), 5.76 (s, 2H), 4.79 (s, 1H), 3.80 (s, 3H), 2.63 (s, 3H), 1.32 (s, 6H). MS (ESI) 464.2 [M+H]⁺.

### Example 26: preparation of a compound L-26

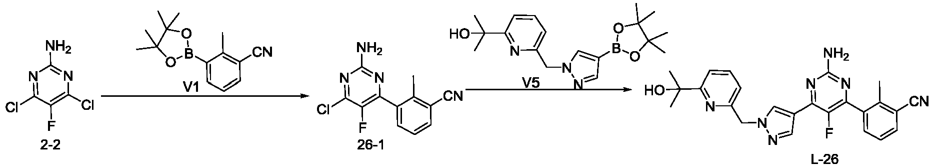

Step 1: referring to step 3 of Example 1 by replacing the compound 1-3 with the compound 2-2, a compound 26-1 (170 mg, yield: 52.3%) was obtained. MS (ESI) 263 [M+H]⁺.

Step 2: referring to step 3 of Example 1 by replacing the compound 1-3 with the compound 26-1 and replacing the intermediate V1 with the intermediate V5, a white solid L-26 (38.59 mg, yield: 17.4%) was obtained. MS (ESI) 444 [M+H]⁺. ¹H NMR (400 MHz, DMSO-d6) δ 8.47 (d, J=1.2 Hz, 1H), 8.03 (s, 1H), 7.90 (d, J=7.2 Hz, 1H), 7.71 (t, J=8.0 Hz, 2H), 7.53 (d, J=8.4 Hz, 1H), 7.50 (t, J=8.0 Hz, 1H), 6.86 (d, J=7.6 Hz, 1H), 6.67 (s, 2H), 5.49 (s, 2H), 5.19 (s, 1H), 2.39 (s, 3H), 1.36 (s, 6H).

### Example 27: preparation of a compound L-27

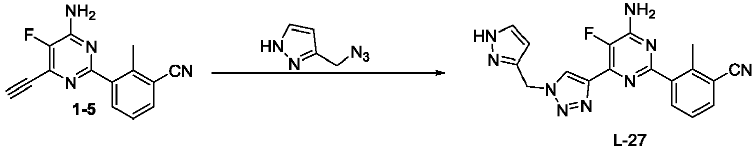

Referring to step 5 of Example 1 by replacing the intermediate V4 with 3-(azidomethyl)-1*H*-pyrazole, a white solid L-27 (12.65 mg, yield: 14%) was obtained. MS (ESI) 376 [M+H]⁺. ¹H NMR (400 MHz, DMSO-d6) δ 12.82 (s, 1H), 8.56 (s, 1H), 7.90 (dd, J=8.0, 4.0 Hz, 1H), 7.76 (dd, J=8.0, 4.0 Hz, 1H), 7.62 (s, 1H), 7.41 (t, J=8.0 Hz, 3H), 6.255 (d, J=4.0 Hz, 1H), 5.63 (s, 2H), 2.62 (s, 3H).

### Example 28: preparation of a compound L-28

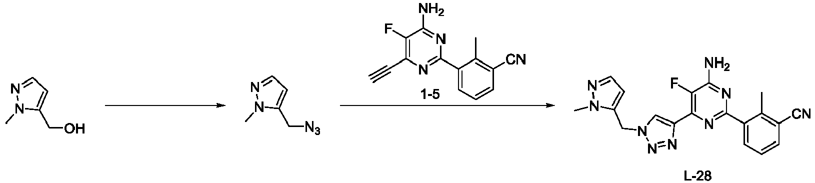

Referring to steps 3 and 4 of Example 7 by replacing the compound 7-2 with (1-methyl-pyrazol-5-yl)methanol and replacing the intermediate V8 with the compound 1-5, a white solid L-28 (4.22 mg, yield: 2.7%) was obtained. MS (ESI) 390 [M+H]⁺. ¹H NMR (400 MHz, DMSO-d6) δ 8.72 (s, 1H), 7.91 (dd, J=7.8, 1.3 Hz,1H), 7.83 (dd, J=7.8, 1.3 Hz, 1H), 7.53 (s, 2H), 7.45 (t, J=7.8 Hz, 1H), 7.34 (d, J=1.9 Hz, 1H), 6.28 (d, J=1.9 Hz, 1H), 5.81 (s, 2H), 3.85 (s, 3H), 2.61 (s, 3H).

### Example 29: preparation of a compound L-29

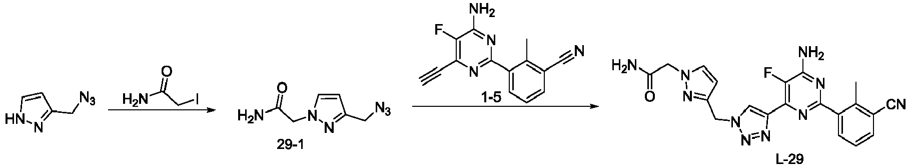

Step 1: referring to step 2 of Example 10, a compound 29-1 (160 mg, yield: 89%) was prepared from 3-azidomethylpyridine (170 mg, 1 mmol) and 2-iodoacetamide (278 mg, 1.5 mmol). MS (ESI) 181 [M+H]⁺.

Step 2: referring to step 5 of Example 1 by replacing the compound V4 with the compound 29-1, a white solid L-29 (10.79 mg, yield: 8.3%) was obtained. MS (ESI) 433 [M+H]⁺. ¹H NMR (400 MHz, DMSO-d6) δ 8.65 (s, 1H), 7.92 (dd, J=8.0, 1.2 Hz, 1H), 7.83 (dd, J=8.0, 1.2 Hz, 1H), 7.65 (d, J=2.3 Hz, 1H), 7.51 (s, 1H), 7.45 (t, J=7.7 Hz, 1H), 7.20 (s, 1H), 6.26 (d, J=2.0 Hz, 1H), 5.61 (s, 2H), 4.70 (s, 2H), 2.62 (s, 3H).

### Example 30: preparation of a compound L-30

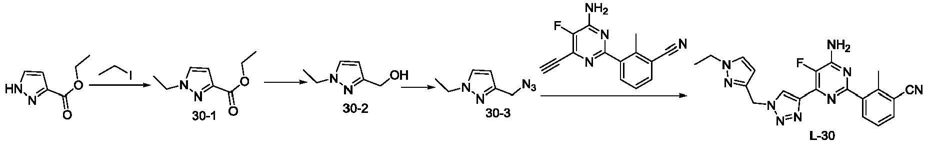

Step 1: referring to step 2 of Example 10, a compound 30-1 (2.2 g, yield: 74%) was prepared from ethyl 1H-pyrazole-3-carboxylate (2.8 g, 20 mmol) and iodoethane (6240 mg, 40 mmol). MS (ESI) 169 [M+H]⁺.

Steps 2 and 3: referring to steps 2 and 3 of Example 7 by replacing the compound 7-1 with the compound 30-1, a crude compound 30-3 (847 mg) was obtained and stored in tert-butanol (10 mL). MS (ESI) 152 [M+H]⁺.

Step 4: referring to step 5 of Example 1 by replacing the compound V4 with the compound 30-3, a white solid L-30 (33.29 mg, purity: 100%, yield: 27.5%) was obtained. MS (ESI) 404 [M+H]⁺. ¹H NMR (400 MHz, DMSO-d6) δ 8.64 (s, 1H), 7.92 (dd, J=7.8, 1.2 Hz, 1H), 7.83 (dd, J=7.7, 1.2 Hz, 1H), 7.68 (d, J=2.2 Hz, 1H), 7.51 (s, 2H), 7.45 (t, J=7.7 Hz, 1H), 6.24 (d, J=2.2 Hz, 1H), 5.61 (s, 2H), 4.07 (q, J=7.3 Hz, 2H), 2.62 (s, 3H), 1.32 (t, J=7.3 Hz, 3H).

### Example 31: preparation of a compound L-31

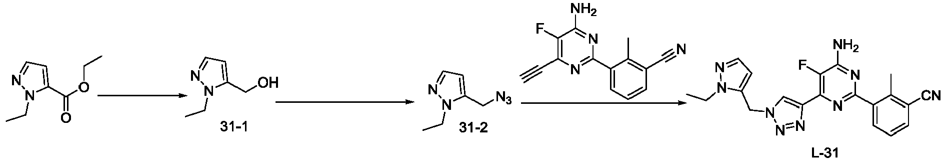

Steps 1 and 2: referring to steps 2 and 3 of Example 7 by replacing the compound 7-1 with ethyl 1-ethyl-1H-pyrazole-5-carboxylate (285 mg, 1.7 mmol), a crude compound 31-2 (538 mg) was obtained and stored in tert-butanol (10 mL). MS (ESI) 152 [M+H]⁺.

Step 3: referring to step 5 of Example 1 by replacing the compound V4 with the compound 31-2, a white solid L-31 (20.93 mg, yield: 17.3%) was obtained. MS (ESI) 404 [M+H]⁺. 1H NMR (400 MHz, DMSO-d6) δ 8.72 (s, 1H), 7.91 (dd, J=8.0, 1.2 Hz, 1H), 7.83 (dd, J=8.0, 1.2Hz, 1H), 7.53 (s, 2H), 7.45 (t, J=7.6 Hz, 1H), 7.39 (d, J=2.0 Hz, 1H), 6.29(d, J=2.0 Hz, 1H), 5.83 (s, 2H), 4.20 (q, J=7.2 Hz, 2H), 2.62 (s, 3H), 1.20 (t, J=7.6 Hz, 3H).

### Example 32: preparation of L-32

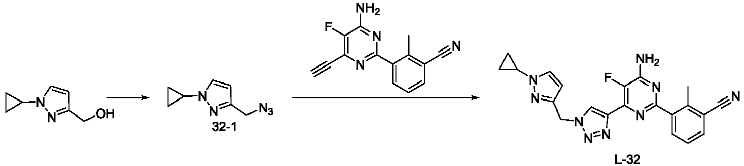

Step 1: referring to step 3 of Example 7 by replacing the compound 7-2 with (1-cyclopropyl-1*H*-pyrazol-3-yl)methanol (138 mg, 1 mmol), a crude compound 32-1 (660 mg) was obtained and stored in tert-butanol (10 mL). MS (ESI) 416 [M+H]⁺.

Step 2: referring to step 5 of Example 1 by replacing the compound V4 with the compound 32-1, a white solid L-32 (18.01 mg, yield: 14.5%) was obtained. MS (ESI) 416 [M+H]⁺. ¹H NMR (400 MHz, DMSO-d6) δ 8.65 (s, 1H), 7.92 (s, 1H), 7.82 (s, 1H), 7.72 (s, 1H), 7.48 (d, J=2.4 Hz, 3H), 6.23 (s, 1H), 5.59 (s, 2H), 3.66 (s, 1H), 2.62 (s, 3H), 0.97 (d, J=4.0 Hz, 2H), 0.91 (d, J=8.0 Hz, 2H).

### Example 33: preparation of a compound L-33

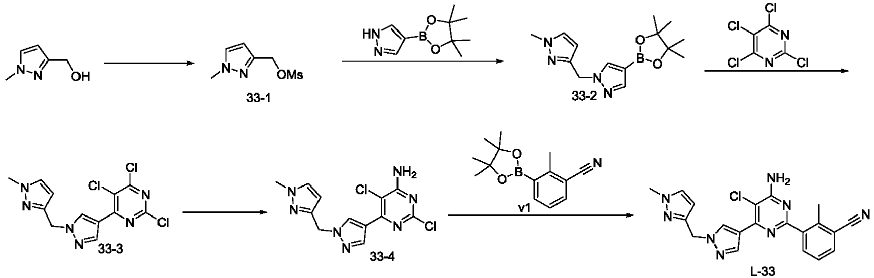

Steps 1 and 2: referring to the preparation method of the intermediate V5, a colorless liquid compound 33-2 (2.1 g, yield: 54%) was prepared from (1-methyl-1H-pyrazol-3-yl)methanol (2 g, 17.86 mmol). MS (ESI) 289 [M+H]⁺.

Step 3: referring to step 3 of Example 1, a yellow solid compound 33-3 (660 mg, 1.67 mmol, yield: 43.6%) was prepared from the compound 33-2 (1.38 g, 3.82 mmol) and 2,4,5,6-tetrachloropyrimidine (1.08 g, 4.96 mmol). MS (ESI) 345 [M+H]⁺.

Step 4: referring to step 2 of Example 1, a white solid compound 33-4 (300 mg) was prepared from the compound 33-3. MS (ESI) 325 [M+H]⁺.

Step 5: referring to step 3 of Example 1, a white solid L-33 (13.10 mg) was prepared from the compound 33-4 and the compound V1. MS (ESI) 405 [M+H]⁺. ¹H NMR (400 MHz, DMSO-d6) δ 8.56 (s, 1H), 8.16 (s, 1H), 7.96 (dd, J=7.8, 1.1 Hz, 1H), 7.84 (dd, J=7.7, 1.1 Hz, 1H), 7.59 (d, J=2.1 Hz, 1H), 7.46 (t, J=7.7 Hz, 2H), 7.39 (s, 1H), 6.15 (d, J=2.2 Hz, 1H), 5.31 (s, 2H), 3.77 (s, 3H), 2.64 (s, 3H).

### Example 34: preparation of a compound L-34

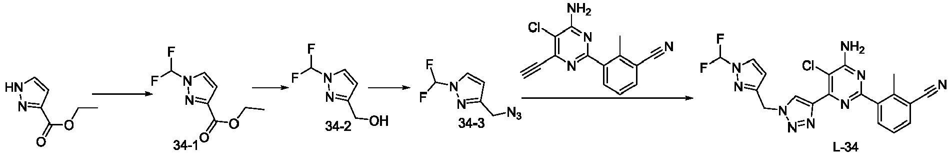

Step 1: ethyl 1H-pyrazole-3-carboxylate (1.4 g, 9.99 mmol) and sodium chlorodifluoroacetate (4.57 g, 29.97 mmol) were dissolved in a mixed solvent of DMF (20 mL) and acetonitrile (12 mL), cesium carbonate (9.76 mg, 29.97 mmol) was added, and the reaction solution was stirred at 100°C for 3 h. After the reaction was completed, the reaction solution was filtered, an obtained residue was washed with ethyl acetate, and an obtained filtrate was spun under reduced pressure to dryness and separated by column chromatography (20 g, ethyl acetate/petroleum ether=0 to 20%) to obtain a compound 34-1 (1.1 g, yield: 57.91%). MS (ESI) 191 [M+H]⁺.

Steps 2 and 3: referring to steps 2 and 3 of Example 7, a compound 34-3 (780 mg, purity: 20%) was prepared from the compound 34-1 and stored in tert-butanol (10 mL). MS (ESI) 174 [M+H]⁺.

Step 4: referring to step 5 of Example 1, a compound L-34 (34.73 mg, yield: 25.83%) was prepared from the compound 34-3 and the intermediate V8. MS (ESI) 442 [M+H]⁺. ¹H NMR (400 MHz, DMSO-d6) δ 8.86 (s, 1H), 8.20 (d, J=2.7 Hz, 1H), 7.96 (dd, J=7.9, 1.3 Hz, 1H), 7.85 (dd, J=7.8, 1.3 Hz, 1H), 7.76 (t, J=60 Hz, 1H), 7.47 (t, J=7.7 Hz, 1H), 6.53 (d, J=2.7 Hz, 1H), 5.74 (s, 2H), 2.64 (s, 3H).

### Example 35: preparation of a compound L-35

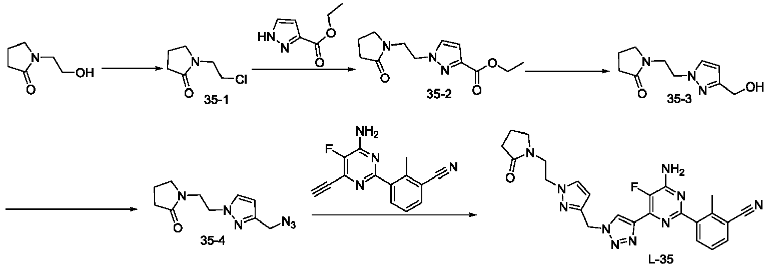

Step 1: 1-(2-hydroxyethyl)pyrrolidin-2-one (800 mg, 6.19 mmol) was dissolved in thionyl chloride (18 mL), and the reaction solution was stirred at 50°C for 1 h. Then, the reaction solution was extracted with ethyl acetate (15 mL × 3), a combined organic phase was washed with a saturated sodium carbonate aqueous solution, dried with anhydrous sodium sulfate, and spun under reduced pressure to remove the solvent so as to obtain a compound 35-1 (910 mg, yield: 99.53%). MS (ESI) 148 [M+H]⁺.

Step 2: the compound 35-1 (735 mg, 4.98 mmol) and ethyl 1H-pyrazole-3-carboxylate (1.05 g, 7.47 mmol) were dissolved in acetonitrile (20 mL), and the reaction solution was stirred at 80°C for 2 h. After the reaction was completed, the reaction solution was filtered, washed with ethyl acetate, spun under reduced pressure to remove the solvent, and separated by column chromatography (20 g, ethyl acetate/petroleum ether=0 to 60%) to obtain a yellow oily compound 35-2 (480 mg, yield: 38.36%). MS (ESI) 252 [M+H]⁺.

Steps 3 and 4: referring to steps 2 and 3 of Example 7, a crude compound 35-4 (460 mg) was prepared from the compound 35-2 and stored in a solvent tert-butanol (5 mL). MS (ESI) 235 [M+H]⁺.

Step 5: referring to step 5 of Example 1 by replacing the compound V4 with the compound 35-4, a pale-green solid L-35 (92.48 mg, yield: 63.84%) was obtained. MS (ESI) 486 [M+H]⁺. ¹H NMR (400 MHz, DMSO-d6) δ 8.76 (s, 1H), 7.92 (d, J=7.8 Hz, 1H), 7.83 (d, J=7.5 Hz, 1H), 7.54 (s, 2H), 7.45 (t, J=8.0 Hz, 1H), 7.42 (s, 1H), 6.30 (s, 1H), 5.82 (s, 2H), 4.32 (t, J=5.9 Hz, 2H), 3.47 (t, J=5.8 Hz, 2H), 3.02 (t, J=7.0 Hz, 2H), 2.62 (s,3H), 2.08 (t, J=8.1 Hz, 2H), 1.86-1.69 (m, 2H).

### Example 36: preparation of a compound L-36

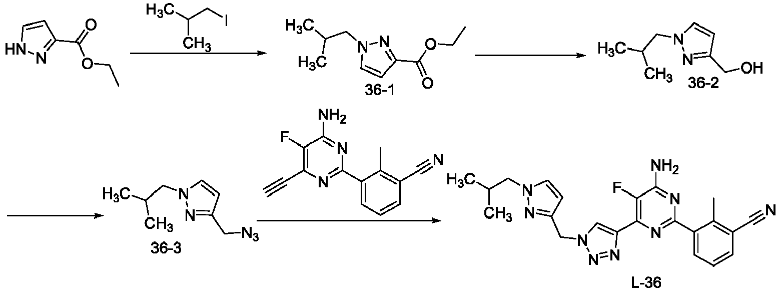

Step 1: referring to step 2 of Example 10, a white solid compound 36-1 (510 mg, yield: 52%) was prepared from ethyl 1*H*-pyrazole-3-carboxylate (700 mg, 5.00 mmol) and 1-iodo-2-methylpropane (1.19 g, 6.49 mmol). MS (ESI) 197 [M+H]⁺.

Steps 2 and 3: referring to steps 2 and 3 of Example 7, a compound 36-3 (840 mg, purity: 20%) was prepared from the compound 36-1 and stored in a solvent tert-butanol (5 mL). MS (ESI) 180 [M+H]⁺.

Step 4: referring to step 5 of Example 1 by replacing the compound V4 with the compound 36-3, a white solid L-36 (71.83 mg, yield: 54.87%) was obtained. MS (ESI) 432 [M+H]⁺. ¹H NMR (400 MHz, DMSO-d6) δ 8.60 (s, 1H), 7.92 (dd, J=7.6, 1.2 Hz, 1H), 7.92 (dd, J=7.6, 0.8 Hz, 1H), 7.66 (d, J=2.2 Hz, 1H), 7.52 (s, 2H), 7.45 (t, J=7.8 Hz, 1H), 6.25 (d, J=2.2 Hz, 1H), 5.61 (s, 2H), 3.85 (d, J=7.2 Hz, 2H), 3.31 (s, 3H), 2.62 (s, 3H), 2.09-1.99 (m, 6.8 Hz, 1H), 0.78 (d, J=6.7 Hz, 6H).

### Example 37: preparation of a compound L-37

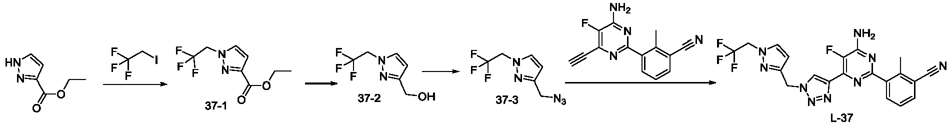

Step 1: referring to step 2 of Example 10, a white solid compound 37-1 (650 mg, yield: 27.33%) was prepared from ethyl 1H-pyrazole-3-carboxylate (1.4 g, 10.06 mmol) and 1,1,1-trifluoro-2-iodo-ethane (4.22 g, 20.12 mmol). MS (ESI) 223 [M+H]⁺.

Steps 2 and 3: referring to steps 2 and 3 of Example 7, a compound 37-3 (800 mg) was prepared from the compound 37-1 and stored in tert-butanol (4 mL). MS (ESI) 206 [M+H]⁺.

Steps 4: referring to step 5 of Example 1 by replacing the compound V4 with the compound 37-3, a compound L-37 (24.70 mg, yield: 18.12%) was obtained. MS (ESI) 458 [M+H]⁺. ¹H NMR (400 MHz, DMSO-d6) δ 8.67 (s, 1H), 7.92 (dd, J=7.8, 1.2 Hz, 1H), 7.83 (dd, J=7.7, 1.3 Hz, 1H), 7.81 (d, J=2.3 Hz, 1H), 7.52 (s, 2H), 7.45 (t, J=7.8 Hz, 1H), 6.38 (d, J=2.4 Hz, 1H), 5.67 (s, 2H), 5.10 (q, J=9.1 Hz, 2H), 2.62 (s, 3H).

### Example 38: preparation of a compound L-38

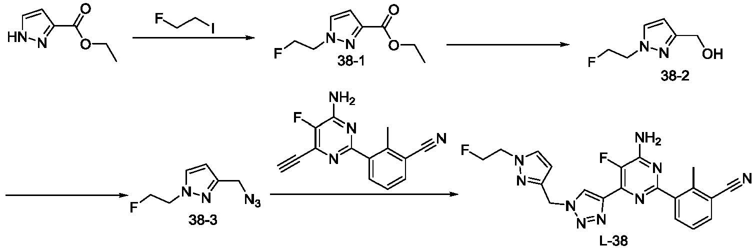

Step 1: referring to step 2 of Example 10, a colorless liquid compound 38-1 (310 mg, yield: 82.74%) was prepared from ethyl 1H-pyrazole-3-carboxylate (282.03 mg, 2.01 mmol) and 1-fluoro-2-iodoethane (525.12 mg, 3.02 mmol). MS (ESI) 187 [M+H]⁺.

Steps 2 and 3: referring to steps 2 and 3 of Example 7, a crude compound 38-3 (536 mg, purity: 22%) was prepared from the compound 38-1 and stored in tert-butanol (5 mL). MS (ESI) 170 [M+H]⁺.

Step 4: referring to step 5 of Example 1 by replacing the compound V4 with the compound 38-3, a white compound L-38 (45.55 mg, yield: 44.63%) was obtained. MS (ESI) 422 [M+H]⁺. ¹H NMR (400 MHz, DMSO-d6) δ 8.66 (s, 1H), 7.92 (d, J=7.4 Hz, 1H), 7.83 (d, J=6.4 Hz, 1H), 7.72 (s, 1H), 7.52 (s, 2H), 7.45 (t, J=7.3 Hz, 1H), 6.29 (s, 1H), 5.63 (s, 2H), 4.71 (d, J=46.8 Hz, 2H), 4.38 (d, J=28.0 Hz, 2H), 2.62 (s, 3H).

### Example 39: preparation of a compound L-39

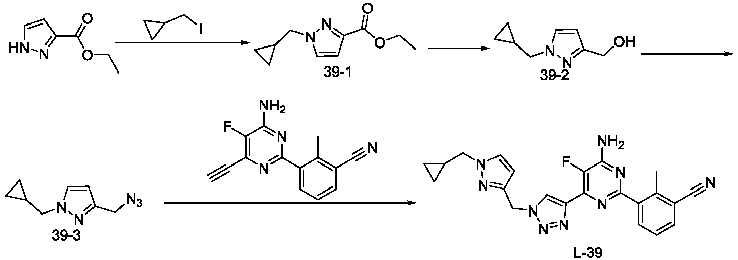

Step 1: referring to step 2 of Example 10, a colorless liquid compound 39-1 (270 mg, yield: 86.98%) was prepared from ethyl 1H-pyrazole-3-carboxylate (215 mg, 1.53 mmol) and iodomethylcyclopropane (280 mg, 1.54 mmol). MS (ESI) 195 [M+H]⁺.

Steps 2 and 3: referring to steps 2 and 3 of Example 7, a crude compound 39-3 (690 mg, purity: 18%) was prepared from the compound 39-1 and stored in tert-butanol (6 mL). MS (ESI) 178 [M+H]⁺.

Step 4: referring to step 5 of Example 1 by replacing the compound V4 with the compound 39-3, a pale-green solid L-39 (48.12 mg, yield: 37.14%) was obtained. MS (ESI) 430 [M+H]⁺. ¹H NMR (400 MHz, DMSO-d6) δ 8.65 (s, 1H), 7.91(d, J=4.5 Hz, 1H), 7.83(d, J=5.5 Hz, 1H), 7.71 (s, 1H), 7.52 (s, 2H), 7.45 (s, 1H), 6.25 (s, 1H), 5.62 (s, 2H), 3.90 (d, J=4.6 Hz, 2H), 2.61 (s, 3H), 1.17 (s, 1H), 0.47 (s, 2H), 0.30 (s, 2H).

### Example 40: preparation of a compound L-40

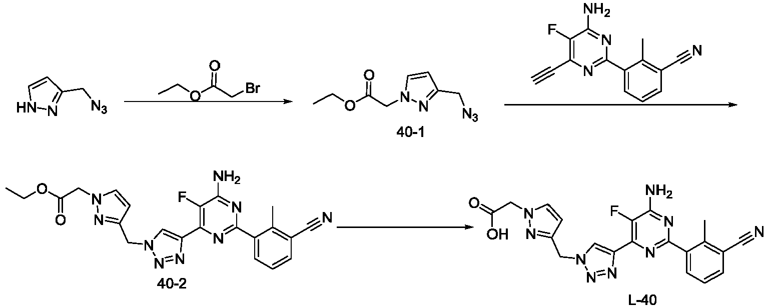

Step 1: referring to step 2 of Example 10, a pale-yellow liquid compound 40-1 (160 mg, yield: 74.39%) was prepared from 3-(azidomethyl)-1H-pyrazole (100 mg, 812.24 µmol) and ethyl 2-bromoacetate (203.47 mg, 1.22 mmol). MS (ESI) 210 [M+H]⁺.

Step 2: referring to step 5 of Example 1 by replacing the compound V4 with the compound 40-1, a yellow solid compound 40-2 (170 mg, yield: 45.28%) was obtained. MS (ESI) 462 [M+H]⁺.

Step 3: the compound 40-2 (150 mg, 325.06 µmol) was dissolved in methane (5 mL), a lithium hydroxide aqueous solution (0.4 mmol, 0.2 M, 2 mL) was dropwise added, and the reaction solution was stirred at the room temperature for 20 min. After the reaction was completed, the reaction solution was spun under reduced pressure to remove the solvent and purified by preparative liquid chromatography to obtain a compound L-40 (30.23 mg, yield: 21.46%). MS (ESI) 434 [M+H]⁺. ¹H NMR (400 MHz, DMSO-d6) δ 8.65 (s, 1H), 7.91 (dd, J=7.8, 1.1 Hz, 1H), 7.82 (dd, J=7.7, 1.1 Hz, 1H), 7.67 (s, 1H), 7.51 (s, 2H), 7.45 (t, J=7.8 Hz, 1H), 6.28 (s, 1H), 5.61 (s, 2H), 4.87 (s, 2H), 2.61 (s, 3H).

### Example 41: preparation of a compound L-41

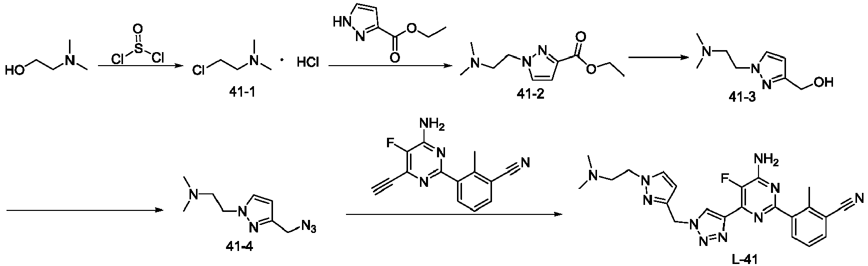

Step 1: 2-(dimethylamino)ethanol (180 mg, 2.02 mmol) was dissolved in dichloromethane (2 mL), the reaction solution was cooled to 0°C, and thionyl chloride (1 M, 2.63 mL) was dropwise added, and the reaction solution was stirred at the room temperature overnight. Then, the reaction solution was spun under reduced pressure to remove the solvent so as to obtain a white solid compound 41-1 (275 mg, yield: 94.54%).

Step 2: the compound 41-1 (275 mg, 2.56 mmol) and ethyl 1H-pyrazole-3-carboxylate (300 mg, 2.14 mmol) were dissolved in DMF (8 mL), cesium carbonate (1.67 g, 5.11 mmol) was added, and the reaction solution was stirred at 110°C for 5 h. Then, the reaction solution was filtered, an obtained residue was washed with dichloromethane, and obtained organic phases were combined, spun under reduced pressure to remove the solvent, and separated by column chromatography (12 g, ethyl acetate/petroleum ether=0 to 50%) to obtain a pale-yellow solid compound 41-2 (260 mg, yield: 31.29%). MS (ESI) 212 [M+H]⁺.

Steps 3 and 4: referring to steps 2 and 3 of Example 7, a yellow liquid compound 41-4 (570 mg) was prepared from the compound 41-2 and stored in tert-butanol (6 mL). MS (ESI) 195 [M+H]⁺.

Step 5: referring to step 5 of Example 1 by replacing the compound V4 with the compound 41-4, a pale-green solid L-41 (25.76 mg, yield: 18.05%) was obtained. MS (ESI) 447 [M+H]⁺. ¹H NMR (400 MHz, CD₃OD) δ 8.50 (s, 1H), 7.93 (s, 1H), 7.71 (s, 1H), 7.64 (s, 1H), 7.41 (s, 1H), 6.33 (s, 1H), 5.67 (s, 2H), 4.25 (s, 2H), 2.77 (s, 2H), 2.66 (s, 3H), 2.23 (s, 6H).

### Example 42: preparation of a compound L-42

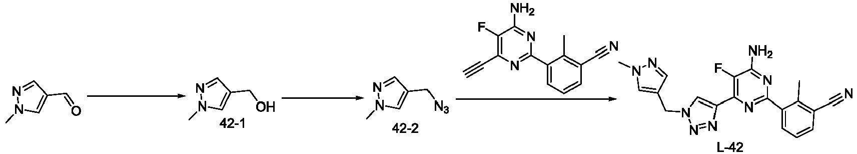

Step 1: referring to step 3 of Example 11, a colorless liquid compound 42-1 (610 mg, yield: 56.91%) was prepared from 1-methylpyrazole-4-carbaldehyde (1 g, 9.08 mmol). MS (ESI) 113 [M+H]⁺.

Step 2: referring to step 3 of Example 7, a crude compound 42-2 (447 mg) was prepared from the compound 42-1 and stored in tert-butanol (5 mL). MS (ESI) 138 [M+H]⁺.

Step 3: referring to step 5 of Example 1 by replacing the compound V4 with the compound 42-2, a compound L-42 (52.83 mg, yield: 31.33%) was obtained. MS (ESI) 419 [M+H]⁺. ¹H NMR (400 MHz, DMSO-d6) δ 8.64 (s, 1H), 7.90 (dd, J=7.8, 1.1 Hz, 1H), 7.83 (dd, J=7.7, 1.1 Hz, 1H), 7.78 (s, 1H), 7.51 (s, 2H), 7.49 (s, 1H), 7.45 (t, J=7.8 Hz, 1H), 5.50 (s, 3H), 2.61 (s, 3H).

### Example 43: preparation of a compound L-43

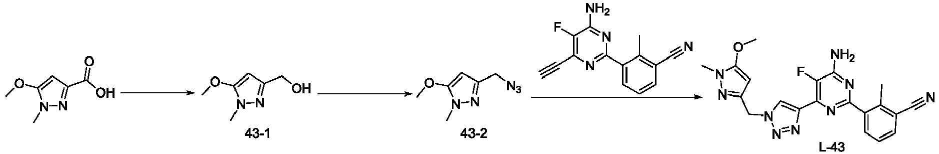

Steps 1 and 2: referring to steps 2 and 3 of Example 7, a crude compound 43-2 (800 mg) was prepared from 5-methoxy-1-methyl-pyrazole-3-carboxylic acid (260 mg, 1.67 mmol) and stored in tert-butanol (4 mL). MS (ESI) 168 [M+H]⁺.

Step 3: referring to step 5 of Example 1 by replacing the compound V4 with the compound 43-2, a compound L-43 (17.4 mg, yield: 9.21%) was obtained. MS (ESI) 449 [M+H]⁺. ¹H NMR (400 MHz, DMSO-d6) δ 8.65 (s, 1H), 7.92 (d, J=7.7 Hz, 1H), 7.84 (d, J=7.7 Hz, 1H), 7.52 (s, 2H), 7.45 (t, J=7.5 Hz, 1H), 5.69 (s, 1H), 5.49 (s, 2H), 3.79 (s, 3H), 3.48 (s, 3H), 2.62 (s, 3H).

### Example 44: preparation of a compound L-44

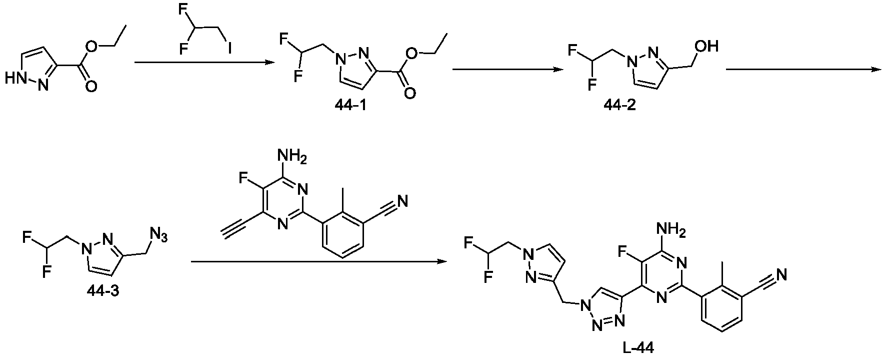

Step 1: referring to step 2 of Example 10, a colorless liquid compound 44-1 (340 mg, yield: 55.56%) was prepared from ethyl 1H-pyrazole-3-carboxylate (420 mg, 3.00 mmol) and 1,1-difluoro-2-iodoethane (862.90 mg, 4.50 mmol). MS (ESI) 205 [M+H]⁺.

Steps 2 and 3: referring to steps 2 and 3 of Example 7, a crude compound 44-3 (790 mg) was prepared from the compound 44-1 and stored in tert-butanol (8 mL). MS (ESI) 188 [M+H]⁺.

Step 4: referring to step 5 of Example 1 by replacing the compound V4 with the compound 44-3, a compound L-44 (33.51 mg, yield: 19.07%) was obtained. MS (ESI) 440 [M+H]⁺. ¹H NMR (400 MHz, DMSO-d6) δ 8.66 (s, 1H), 7.92 (d, J=7.3 Hz, 1H), 7.83 (d, J=7.3 Hz, 1H), 7.75 (s, 1H), 7.52 (s, 2H), 7.45 (t, J=7.2 Hz, 1H), 6.49-6.08 (m, 2H), 5.65 (s, 2H), 4.58 (td, J=15.3, 2.6 Hz, 2H), 2.62 (s, 3H).

### Example 45: preparation of a compound L-45

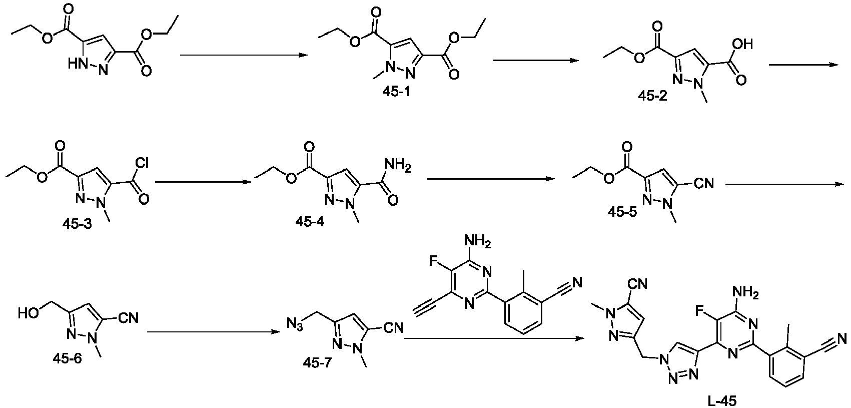

Step 1: referring to step 2 of Example 10, a white solid compound 45-1 (2.36 g, yield: 95.66%) was prepared from diethyl 1H-pyrazole-3,5-dicarboxylate (2120 mg, 9.99 mmol) and iodomethane (2.28 g, 16.06 mmol, 1 mL). MS (ESI) 227 [M+H]⁺.

Step 2: referring to step 3 of Example 40, a white solid compound 45-2 (2 g, yield: 96.74%) was prepared from the compound 45-1. MS (ESI) 199 [M+H]⁺.

Step 3: referring to step 1 of Example 35, a pale-yellow liquid compound 45-3 (2.3 g, yield: 99.95%) was prepared from the compound 45-2. MS (ESI) 199 [M+H]⁺.

Step 4: referring to step 2 of Example 1, a white solid compound 45-4 (980 mg, yield: 46.81%) was prepared from the compound 45-3. MS (ESI) 198 [M+H]⁺.

Step 5: the compound 45-4 (400 mg, 2.03 mmol) was dissolved in dichloromethane (8 mL), triethylamine (362.75 mg, 3.58 mmol, 0.5 mL) was added at the room temperature, the reaction solution was stirred for 15 min, trifluoroacetic anhydride (1.51 g, 7.19 mmol, 1 mL) was then dropwise added, and the reaction solution was stirred at the room temperature for 1 h. Then, the reaction solution was extracted with dichloromethane (20 mL × 3), a combined organic phase was washed with a saturated salt water (20 mL), dried with anhydrous sodium sulfate, and concentrated under reduced pressure to remove the solvent so as to obtain a pale-yellow liquid compound 45-5 (360 mg, yield: 99.05%). MS (ESI) 180 [M+H]⁺.

Steps 6 and 7: referring to steps 2 and 3 of Example 7, a crude compound 45-7 (847 mg) was prepared from the compound 45-5 and stored in tert-butanol (8 mL). MS (ESI) 163 [M+H]⁺.

Step 8: referring to step 5 of Example 1 by replacing the compound V4 with the compound 45-7, a white solid compound L-45 (19.5 mg, yield: 11.75%) was obtained. MS (ESI) 415 [M+H]⁺. ¹H NMR (400 MHz, DMSO-d6) δ 8.73 (s, 1H), 7.91 (dd, J=7.8, 1.2 Hz, 1H), 7.84 (dd, J=7.7, 1.2 Hz, 1H), 7.54 (s, 2H), 7.46 (t, J=7.8 Hz, 1H), 7.18 (s, 1H), 5.70 (s, 2H), 3.95 (s, 3H), 2.62 (s, 3H).

### Example 46: preparation of a compound L-46

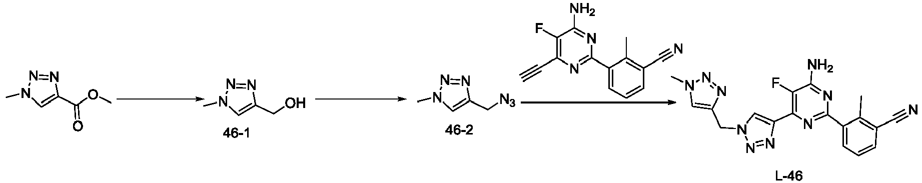

Steps 1 and 2: referring to steps 2 and 3 of Example 7, a brown oily liquid crude compound 46-2 (816 mg) was prepared from methyl 1-methyltriazole-4-carboxylate (250 mg, 1.77 mmol) and stored in tert-butanol (8 mL). MS (ESI) 139 [M+H]⁺.

Step 3: referring to step 5 of Example 1 by replacing the compound V4 with the compound 46-2, a white solid L-46 (10.03 mg, yield: 6.38%) was obtained. MS (ESI) 391 [M+H]⁺. ¹H NMR (400 MHz, DMSO-d6) δ 8.70 (s, 1H), 8.14 (s, 1H), 7.91 (dd, J=7.8, 1.0 Hz, 1H), 7.83 (dd, J=7.6, 0.8 Hz, 1H), 7.53 (s, 2H), 7.45 (t, J=7.8 Hz, 1H), 5.76 (s, 2H), 4.00 (s, 3H), 2.62 (s, 3H).

### Example 47: preparation of a compound L-47

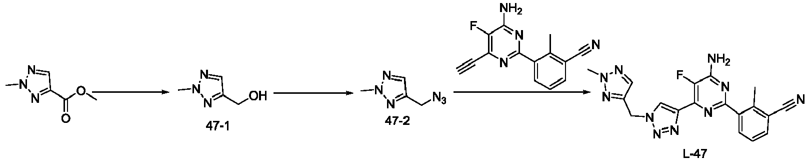

Steps 1 and 2: referring to steps 2 and 3 of Example 7, a brown oily liquid crude compound 47-2 (747 mg) was prepared from methyl 2-methyltriazole-4-carboxylate (250 mg, 1.77 mmol) and stored in tert-butanol (8 mL). MS (ESI) 139 [M+H]⁺.

Step 3: referring to step 5 of Example 1 by replacing the compound V4 with the compound 47-2, a white solid L-47 (10.0 mg, yield: 6.38%) was obtained. MS (ESI) 391 [M+H]⁺. 1H NMR (400 MHz, DMSO-d6) δ 8.72 (s, 1H), 7.91 (dd, J=7.8, 1.3 Hz, 1H), 7.82 (dd, J=7.7, 1.1 Hz, 1H), 7.79 (s, 1H), 7.52 (s, 2H), 7.44 (t, J=7.8 Hz, 1H), 5.76 (s, 2H), 4.08 (s, 3H), 2.61 (s, 3H).

### Example 48: preparation of a compound L-48

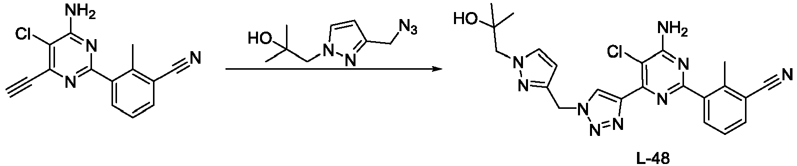

Referring to step 5 of Example 1, a compound L-48 (74.9 mg, yield: 43.60%) was prepared from 1-[3-(azidomethyl)pyrazol-1-yl]-2-methyl-propan-2-ol (70 mg, 358.57 µmol) and the intermediate V8 (95 mg, 353.55 µmol). MS (ESI) 465 [M+H]⁺. ¹H NMR (400 MHz, DMSO-d6) δ 8.66 (s, 1H), 7.94 (d, J=6.5 Hz, 1H), 7.83 (d, J=7.3 Hz, 1H), 7.60 (s, 2H), 7.45 (t, J=8.0 Hz, 1H), 6.26 (s, 1H), 5.60 (s, 2H), 4.62 (s, 1H), 3.95 (s, 2H), 2.62 (s, 3H), 0.99 (s, 6H).

### Example 49: preparation of a compound L-49

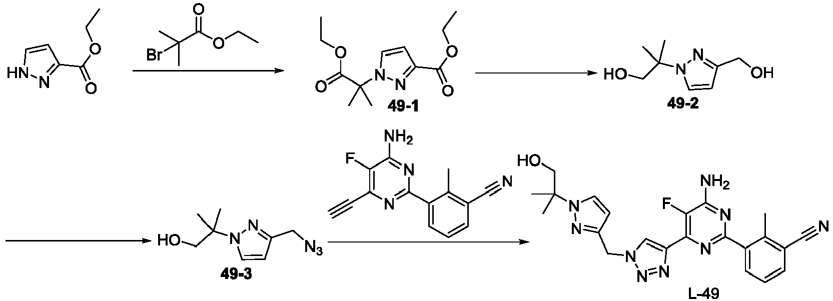

Steps 1 to 3: referring to steps 1 to 3 of Example 7, a compound 49-3 (3.1 g) was prepared from ethyl 1H-pyrazole-3-carboxylate (1.4 g, 9.99 mmol) and ethyl 2-bromo-2-methylpropanoate (2.92 g, 14.99 mmol) and stored in tert-butanol (10 mL). MS (ESI) 196 [M+H]⁺.

Step 4: referring to step 5 of Example 1 by replacing the compound V4 with the compound 49-3, a white solid L-49 (7.77 mg, yield: 1.74%) was obtained. MS (ESI) 448 [M+H]⁺. ¹H NMR (400 MHz, DMSO-d6) δ 8.63 (s, 1H), 7.92 (dd, J=7.8, 1.2 Hz, 1H), 7.82 (d, J=7.7 Hz, 1H), 7.71 (d, J=2.3 Hz, 1H), 7.50 (s, 2H), 7.44 (t, J=7.8 Hz, 1H), 6.19 (d, J=2.3 Hz, 1H), 5.61 (s, 2H), 4.92 (t, J=5.4 Hz, 1H), 3.51 (d, J=5.2 Hz, 2H), 2.62 (s, 3H), 1.40 (s, 6H).

### Example 50: preparation of a compound L-50

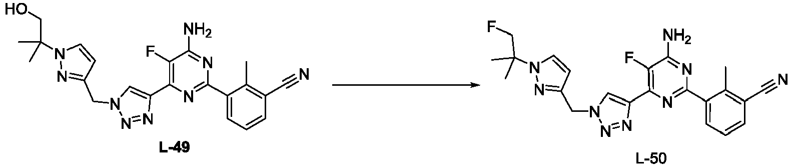

The compound L-49 (50 mg, 111.74 µmol) was dissolved in dichloromethane (3 mL), the reaction solution was cooled to 0°C, and bis(2-methoxyethyl)aminosulfur trifluoride (24.7 mg, 111.74 µmol) was dropwise added, and the reaction solution was stirred at 0°C, gradually heated to the room temperature, and stirred for 3 h. Then, a lithium hydroxide aqueous solution was added to the reaction solution to quench the reaction, the mixture was extracted with dichloromethane (10 mL × 3), and a combined organic phase was washed with a saturated salt water (15 mL), dried with anhydrous sodium sulfate, spun under reduced pressure to remove the solvent, and purified by preparative liquid chromatography to obtain a compound L-50 (1.1 mg). MS (ESI) 450 [M+H]⁺. ¹H NMR (400 MHz, DMSO-d6) δ 8.70 (s, 1H), 7.90 (dd, J=7.8, 1.1 Hz, 1H), 7.82 (dd, J=7.7, 1.2 Hz, 1H), 7.52 (s, 2H), 7.46-7.42 (m, 2H), 6.23 (d, J=1.8 Hz, 1H), 5.78 (s, 2H), 4.46 (d, J=21.1 Hz, 2H), 2.61 (s, 3H), 1.28 (d, J=21.8 Hz, 6H).

### Example 51: preparation of a compound L-51

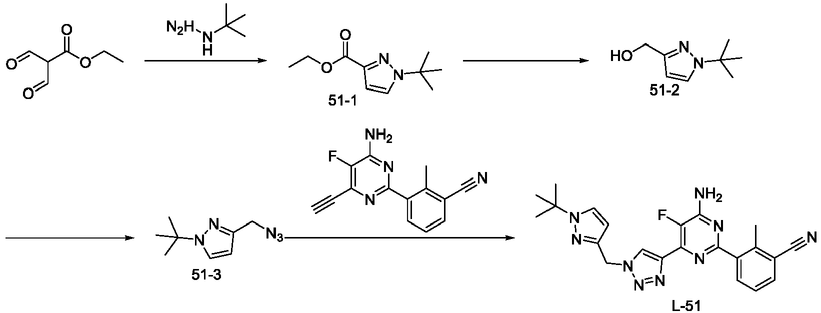

Step 1: ethyl 2-formyl-3-oxopropanoate (720 mg, 5.00 mmol) and tert-butylhydrazine hydrochloride (625 mg, 5.00 mol) were dissolved in ethanol (20 mL), and triethylamine (757 mg, 7.5 mmol) was added. The reaction solution was stirred at the room temperature overnight. Then, the reaction solution was subjected to rotary evaporation under reduced pressure to remove the ethanol and extracted with ethyl acetate (20 mL × 3), a combined organic phase was washed with a saturated salt water (20 mL), dried with anhydrous sodium sulfate, and spun under reduced pressure to remove the solvent so as to obtain a crude product, and the crude product was separated by column chromatography (12 g, ethyl acetate/petroleum ether=0% to 10%) to obtain a yellow oily liquid compound 51-1 (500 mg, yield: 51.28%). MS (ESI) 197 [M+H]⁺.

Steps 2 and 3: referring to steps 2 and 3 of Example 7, a compound 51-3 (684 mg) was prepared from the compound 51-1 and stored in tert-butanol (5 mL). MS (ESI) 189 [M+H]⁺.

Step 4: referring to step 5 of Example 1 by replacing the compound V4 with the compound 51-3, a yellow solid L-51 (34.5 mg, yield: 27%) was obtained. MS (ESI) 432 [M+H]⁺. ¹H NMR (400 MHz, DMSO-d6) δ 8.65 (s, 1H), 7.93 (s, 1H), 7.90 (d, J=7.6 Hz, 1H), 7.82 (d, J=7.4 Hz, 1H), 7.52 (s, 1H), 7.50 (s, 1H),7.44 (t, J=8.0 Hz, 1H), 5.50 (s, 2H), 2.60 (s, 3H), 1.45 (s, 9H).

### Example 52: preparation of a compound L-52

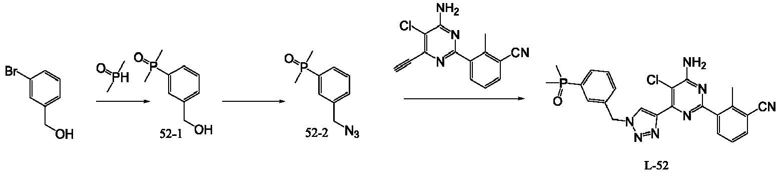

Step 1: (3-bromophenyl)methanol (190 mg, 1 mmol), dimethylphosphine oxide (90 mg, 1.1 mmol), and cesium carbonate (650 mg, 2 mmol) were placed into a microwave tube, and Xantphos (24 mg, 0.04 mmol) and Pd₂dba₃ (18 mg, 0.02 mmol) were added under the protection of nitrogen gas. After the air was replaced, the reaction solution was heated to 150°C in a microwave reactor and reacted for 30 min. After the reaction was completed, the reaction solution was filtered, an obtained solid was washed, an obtained filtrate was spun under reduced pressure to dryness, and an obtained crude product was purified by column chromatography (EA/PE=80%) to obtain a compound 52-1 (86 mg). MS (ESI) 185.1 [M+H]⁺.

Step 2: referring to step 3 of Example 7, a compound 52-2 was prepared from the compound 52-1. MS (ESI) 210.1 [M+H]⁺.

Step 3: referring to step 5 of Example 1, a yellow solid product L-52 was prepared from the compound 52-2 and the intermediate V8. MS (ESI) 478.1 [M+H]⁺. ¹H NMR (400 MHz, DMSO-d6) δ 8.95 (s, 1H), 7.99-7.97 (m, 1H), 7.90-7.85 (m, 2H), 7.75-7.71 (m, 2H), 7.54-7.46 (m, 4H), 5.77 (s, 2H), 2.66 (s, 3H), 1.64 (d, J=12Hz, 6H).

### Example 53: preparation of a compound L-53

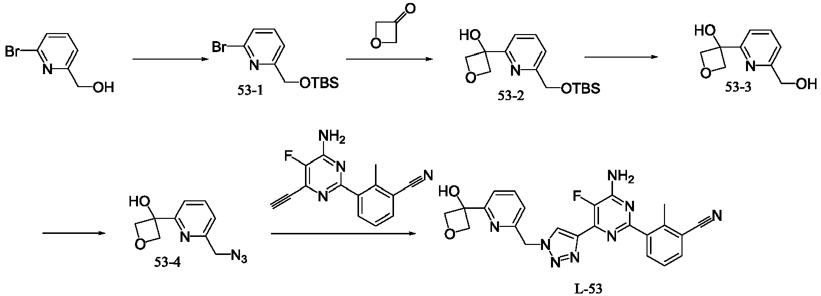

Step 1: referring to step 1 of Example 21, a yellow solid compound 53-1 (2.3 g) was prepared from (6-bromo-pyridin-2-yl)methanol (2 g, 10.7 mmol). MS (ESI) 302.1 [M+H]⁺.

Step 2: the compound 53-1 (2 g, 10.7 mmol) was dissolved in tetrahydrofuran (30 mL), the reaction solution was cooled to -78°C, n-butyllithium (4.7 mL, 11.8 mmol, 2.5 M) was added under the protection of nitrogen gas, the reaction solution was stirred at this temperature for 30 min, a tetrahydrofuran (5 mL) solution in which 3-oxetanone (1.08 g, 15 mmol) was dissolved was slowly added, and the reaction solution was stirred at -78°C for 2 h. Then, the reaction was quenched with a saturated ammonium chloride solution, the mixture was extracted with ethyl acetate, and an obtained organic phase was dried with anhydrous sodium sulfate, spun under reduced pressure to remove the solvent, and purified by column chromatography to obtain a yellow solid compound 53-2 (1.1 g). MS (ESI) 296.2 [M+H]⁺.

Step 3: referring to step 4 of Example 1, a compound 53-3 was prepared from the compound 53-2. MS (ESI) 182.1 [M+H]⁺.

Step 4: referring to step 3 of Example 7, a compound 53-4 was prepared from the compound 53-3. MS (ESI) 207.1 [M+H]⁺.

Step 5: referring to step 5 of Example 1 by replacing the compound V4 with the compound 53-4, a white solid product L-53 (13 mg, 8.1%) was obtained. MS (ESI) 459.1 [M+H]⁺. ¹H NMR (400 MHz, DMSO-d6) δ 8.87 (s, 1H), 7.96 (d, J=8.0Hz, 1H), 7.88-7.85 (m, 2H), 7.56-7.48 (m, 3H), 7.24 (d, J=8.0Hz, 1H), 6.54 (s, 1H), 5.89 (s, 2H), 4.87-4.85 (m, 2H), 4.62-4.60 (m, 2H), 2.66 (s, 3H).

### Example 54: preparation of a compound L-54

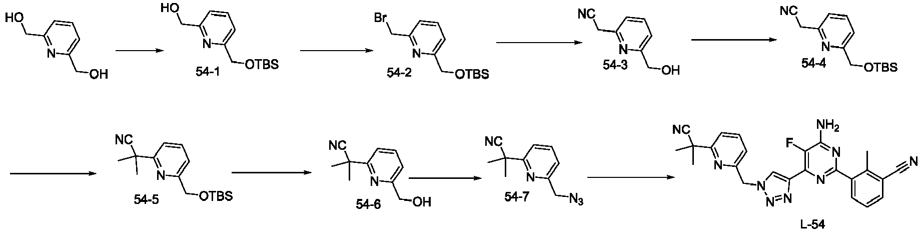

Step 1: 2,6-pyridinedimethanol (6 g, 43.2 mmol) was dissolved in tetrahydrofuran (80 mL), sodium hydride (1.75 g, 43.8 mmol, 60%) was slowly added under ice bath conditions, the reaction solution was stirred for 30 min, TBSCl (6.61 g, 43.8 mmol) was slowly added, and the reaction solution was stirred at the room temperature for 20 h. Then, the reaction solution was added to ice water, the mixture was extracted with dichloromethane (50 mL × 2), a combined organic phase was dried with anhydrous sodium sulfate, concentrated under reduced pressure to dryness, and purified by column chromatography to obtain a colorless oily liquid compound 54-1 (6.47 g, 59.2%). MS (ESI) 254.1 [M+H]⁺.

Step 2: the compound 54-1 (6.35 g, 25.1 mmol) and triphenylphosphine (7.89 g, 30.1 mmol) were dissolved in dichloromethane (80 mL), tetrabromomethane (10.83 g, 32.6 mmol) was slowly added under ice bath conditions, and the reaction solution was stirred at the room temperature for 20 h. Then, the reaction solution was spun under reduced pressure to remove the solvent and purified by column chromatography to obtain a yellow solid compound 54-2 (4.5 g, 57.0%). MS (ESI) 316.1 [M+H]⁺.

Step 3: the compound 54-2 (3.15 g, 10 mmol), trimethylsilyl cyanide (1.51 g, 15 mmol), and TBAF (15 mL, 15 mmol, 1 M) were dissolved in acetonitrile, and the reaction solution was heated to 80°C and stirred under the protection of nitrogen gas for 20 h. Then, the reaction solution was spun under reduced pressure to remove the solvent and purified by column chromatography (EA/PE=0 to 100%) to obtain a yellow oily compound 54-3 (1.4 g, 94.5%). MS (ESI) 149.1 [M+H]⁺.

Step 4: referring to step 1 of Example 21, a yellow solid compound 54-4 (2.01 g, 82.1%) was prepared from the compound 54-3. MS (ESI) 263.1 [M+H]⁺.

Step 5: referring to step 1 of Example 23, a compound 54-5 (2.5 g, 82.1%) was prepared from the compound 54-4. MS (ESI) 291.1 [M+H]⁺.

Step 6: referring to step 4 of Example 1, a compound 54-6 was prepared from the compound 54-5. MS (ESI) 177.1 [M+H]⁺.

Step 7: referring to step 3 of Example 7, a compound 54-7 was prepared from the compound 54-6. MS (ESI) 202.1 [M+H]⁺.

Step 8: referring to step 5 of Example 1 by replacing the compound V4 with the compound 54-7, a white solid product L-54 (15 mg, 10.3%) was obtained. MS (ESI) 454.2 [M+H]⁺. ¹H NMR (400 MHz, DMSO-d6) δ 8.84 (s, 1H), 7.98-7.85 (m, 3H), 7.59-7.46 (m, 4H), 7.28 (d, J=8.0Hz, 1H), 5.87 (s, 2H), 2.67 (s, 3H), 1.65 (s, 6H).

### Example 55: preparation of a compound L-55

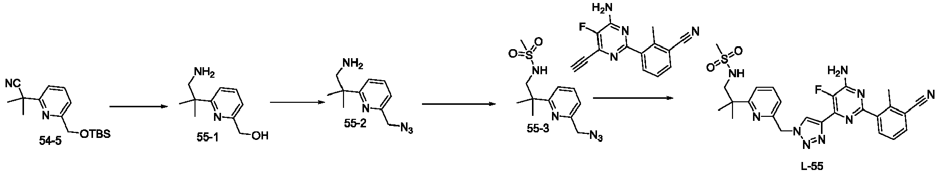

Steps 1 and 2: referring to steps 2 and 3 of Example 7, a compound 55-2 (190 mg, 41.7%) was prepared from the compound 54-5. MS (ESI) 206.1 [M+H]⁺.

Step 3: the compound 55-2 (190 mg, 0.93 mmol) and TEA (187 mg, 1.85 mmol) were dissolved in dichloromethane (10 mL), methylsulfonyl chloride (116 mg, 1.02 mmol) was slowly added under ice bath conditions, and the reaction solution was stirred at the room temperature for 4 h. Then, the reaction solution was spun under reduced pressure to remove the solvent and purified by column chromatography (EA/PE=0 to 90%) to obtain a yellow solid compound 55-3 (86 mg, 32.8%). MS (ESI) 283.1 [M+H]⁺.

Step 4: referring to step 5 of Example 1 by replacing the compound V4 with the compound 55-3, a white solid product L-55 (26 mg, 18.6%) was obtained. MS (ESI) 536.2 [M+H]⁺. ¹H NMR (400 MHz, DMSO-d6) δ 8.83 (s, 1H), 7.98-7.76 (m, 3H), 7.56-7.46 (m, 3H), 7.38 (d, J=8.0Hz, 1H), 7.15-7.13 (m, 1H), 7.69-7.67 (m, 1H), 5.83 (S, 2H), 3.14 (d, J=8.0Hz, 2H), 2.70 (s, 3H), 2.67 (s, 3H), 1.23 (s, 6H).

### Example 56: preparation of a compound L-56

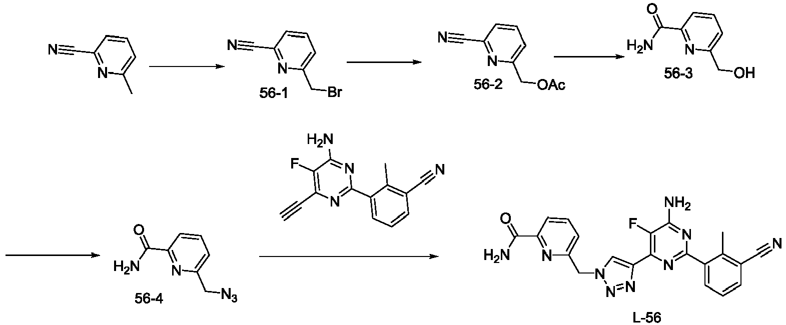

Step 1: 6-methylpyridine-2-carbonitrile (5 g, 42.3 mmol), NBS (7.53 g, 42.3 mmol), and benzoyl peroxide (512 mg, 2.1 mmol) were dissolved in carbon tetrachloride (100 mL), the reaction solution was heated to reflux and stirred for 20 h. Then, water was added to the reaction solution, the mixture was extracted with dichloromethane (50 mL × 2), a combined organic phase was dried with anhydrous sodium sulfate, concentrated under reduced pressure to dryness, and purified by column chromatography (EA/PE=0 to 40%) to obtain a colorless oily liquid compound 56-1 (3.5 g, 42.2%). MS (ESI) 197.1 [M+H]⁺.

Step 2: potassium acetate (5.97 g, 60.90 mmol) and the compound 56-1 (6 g, 30.45 mmol) were added to DMF (50 mL), and the reaction solution was stirred under ice bath conditions for 20 h. Then, ethyl acetate (200 mL) was added, and an obtained organic phase was washed three times with a saturated salt water, dried with anhydrous sodium sulfate, spun under reduced pressure to remove the solvent, and purified by column chromatography (EA/PE=0 to 90%) to obtain a yellow solid compound 56-2 (4.1 g, 76.4%). MS (ESI) 177.1 [M+H]⁺.

Step 3: hydrogen peroxide (0.5 mL) was mixed with a 3 N sodium hydroxide solution (3 mL), the compound 56-2 (500 mg, 2.84 mmol) and methanol (15 mL) were added, and the reaction solution was stirred at the room temperature for 1 h. Then, the reaction solution was neutralized with 1 N diluted hydrochloric acid, and the mixture was spun under reduced pressure to remove the solvent and purified by column chromatography (MeOH/dichloromethane=0 to 20%) to obtain a yellow solid compound 56-3 (300 mg, 69.5%). MS (ESI) 153.1 [M+H]⁺.

Step 4: referring to step 3 of Example 7, a compound 56-4 was prepared from the compound 56-3. MS (ESI) 178.1 [M+H]⁺.

Step 5: referring to step 5 of Example 1 by replacing the compound V4 with the compound 56-4, a white solid product L-56 (38 mg, 29.8%) was obtained. MS (ESI) 430.2 [M+H]⁺. ¹H NMR (400 MHz, DMSO-d6) δ 8.98 (s, 1H), 8.05-7.85 (m, 5H), 7.73 (s, 1H), 7.57-7.45 (s, 4H), 5.89 (s, 2H), 2.66 (s, 3H).

### Example 57: preparation of a compound L-57

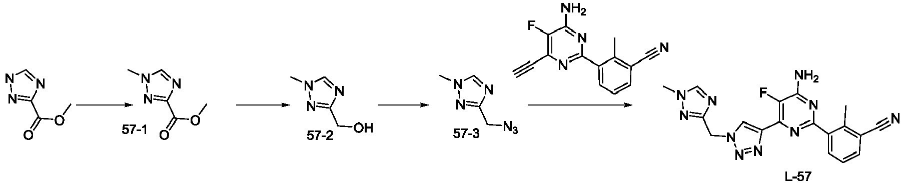

Step 1: referring to step 1 of Example 23, a colorless oily liquid compound 57-1 was prepared from methyl 1H-1,2,4-triazole-3-carboxylate (2 g, 15.74 mmol). MS (ESI) 142.1 [M+H]⁺.

Steps 2 and 3: referring to steps 2 and 3 of Example 7, a white solid compound 57-3 (80 mg, 13.1%) was prepared from the compound 57-1. MS (ESI) 139.1 [M+H]⁺.

Step 4: referring to step 5 of Example 1 by replacing the compound V4 with the compound 57-3, a pale-green solid product L-57 (16 mg, 17.5%) was obtained. MS (ESI) 391.2 [M+H]⁺. ¹H NMR (400 MHz, DMSO-d6) δ 8.82 (s, 1H), 7.97-7.86 (m, 3H), 7.58-7.45 (m, 3H), 6.00 (s, 2H), 3.96 (s, 3H), 2.66 (s, 3H).

### Example 58: preparation of a compound L-58

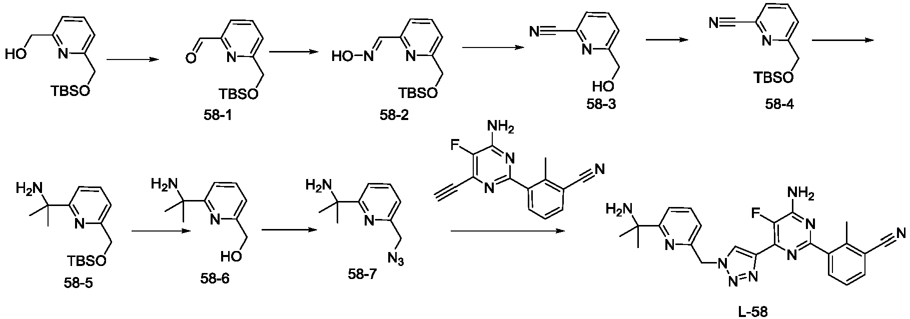

Step 1: referring to step 2 of Example 24, a colorless oily liquid compound 58-1 (2.1 g, 52.9%) was prepared from [6-[[tert-butyl(dimethyl)silyl]oxymethyl]-2-pyridyl]methanol (4 g, 15.78 mmol). MS (ESI) 252.1 [M+H]⁺.

Step 2: the compound 58-1 (1 g, 3.98 mmol) and potassium carbonate (1.10 g, 7.96 mmol) were mixed in MeOH (15 mL), hydroxylamine hydrochloride (304.06 mg, 4.38 mmol) was added, and the reaction solution was stirred at 20°C for 20 h. Then, the reaction solution was spun to remove the methanol, a saturated salt water was added, the mixture was extracted with EA (50 mL × 2), and a combined organic phase was dried with anhydrous sodium sulfate and spun under reduced pressure to remove the solvent so as to obtain a compound 58-2. MS (ESI) 267.1 [M+H]⁺.

Step 3: the compound 58-2 (1 g, 3.75 mmol) and potassium carbonate (1.04 g, 7.51 mmol) were mixed with DMF (10 mL), acetic anhydride (766.41 mg, 7.51 mmol) was added, and the reaction solution was stirred at 50°C for 20 h. Then, saturated salt water was added to the reaction solution, the mixture was extracted with dichloromethane (50 mL × 2), and a combined organic phase was dried with anhydrous sodium sulfate and spun under reduced pressure to remove the solvent so as to obtain a compound 58-3. MS (ESI) 135.1 [M+H]⁺.

Step 4: referring to step 1 of Example 21, a colorless oily liquid compound 58-4 (0.6 g, 54%) was prepared from the compound 58-3. MS (ESI) 249.1 [M+H]⁺.

Step 5: referring to step 3 of Example 10, a colorless oily liquid compound 58-5 (524 mg, 74.9%) was prepared from the compound 58-4. MS (ESI) 281.1 [M+H]⁺.

Step 6: referring to step 4 of Example 1, a compound 58-6 was prepared from the compound 58-5. MS (ESI) 167.1 [M+H]⁺.

Step 7: referring to step 3 of Example 7, a compound 58-7 (355 mg, 88.2%) was prepared from the compound 58-6. MS (ESI) 192.1 [M+H]⁺.

Step 8: referring to step 5 of Example 1 by replacing the compound V4 with the compound 58-7, a gray solid product L-58 (8.9 mg, 5.3%) was obtained. MS (ESI) 444.1 [M+H]⁺. ¹H NMR (400 MHz, DMSO-d6) δ 8.79 (s, 1H), 7.93 (d, J=8.0Hz, 1H), 7.84-7.73 (m, 2H), 7.51-7.45 (m, 4H), 7.09 (d, J=8.0Hz, 1H), 5.77 (s, 2H), 2.63 (s, 3H), 1.29 (s, 6H).

### Example 59: preparation of a compound L-59

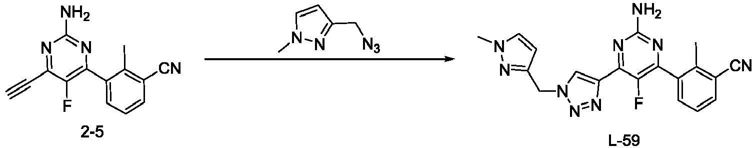

Referring to step 6 of Example 2 by replacing the compound V4 with 3-(azidomethyl)-1-methyl-1*H*-pyrazole, a white solid L-59 (1.56 mg, purity: 98.83%, yield: 2.03%) was obtained. ¹H NMR (400 MHz, DMSO-d6) δ 8.56 (d, J=2.1 Hz, 1H), 7.91 (d, J=6.8 Hz, 1H), 7.71 (d, J=7.5 Hz, 1H), 7.63 (d, J=2.1 Hz, 1H), 7.50 (t, J=7.7 Hz, 1H), 6.86 (s, 2H), 6.24 (d, J=2.1 Hz, 1H), 5.61 (s, 2H), 3.77 (s, 3H), 2.39 (s, 3H). MS (ESI) 390 [M+H]⁺.

### Example 60: preparation of a compound L-60

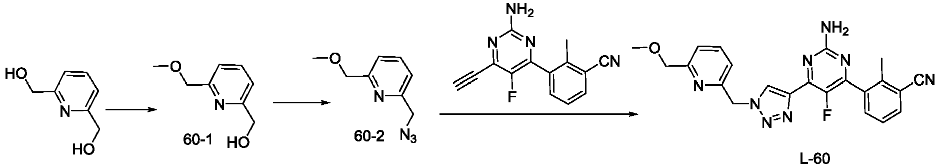

Step 1: referring to step 1 of Example 23, a compound 60-1 (100 mg, yield: 6.06%) was prepared from pyridine-2,6-diyldimethanol (1.5 g, 10.78 mmol). MS (ESI) 154 [M+H]⁺.

Step 2: referring to step 3 of Example 7, a compound 60-2 (117 mg, yield: 100%) was prepared from the compound 60-1. MS (ESI) 179 [M+H]⁺.

Step 3: referring to step 5 of Example 1 by replacing the compound V4 with the compound 60-2, a white solid L-60 (12.00 mg, purity: 98.93%, yield: 14.07%) was obtained. ¹H NMR (400 MHz, DMSO-d6) δ 8.72 (d, J=1.7 Hz, 1H), 7.92 (d, J=6.7 Hz, 1H), 7.81 (t, J=7.8 Hz, 1H), 7.72 (d, J=7.4 Hz, 1H), 7.51 (t, J=7.7 Hz, 1H), 7.34 (d, J=7.8 Hz, 1H), 7.17 (d, J=7.7 Hz, 1H), 6.87 (s, 2H), 5.79 (s, 2H), 4.42 (s, 2H), 3.30 (s, 3H), 2.40 (s, 3H). MS (ESI) 431 [M+H]⁺.

### Example 61: preparation of a compound L-61

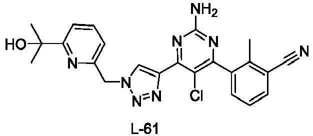

Referring to the preparation method of Example 2, a white solid L-61 (1.33 mg, yield: 3.33%) was obtained. ¹H NMR (400 MHz, DMSO-d6) δ 8.84 (s, 1H), 7.88 (dd, J=7.7, 1.1 Hz, 1H), 7.76 (t, J=7.8 Hz, 1H), 7.61 (d, J=6.7 Hz, 1H), 7.57 (d, J=7.4 Hz, 1H), 7.49 (t, J=7.7 Hz, 1H), 7.22-7.01 (m, 3H), 5.78 (s, 2H), 5.17 (s, 1H), 2.30 (s, 3H), 1.33 (s, 6H). MS (ESI) 461 [M+H]⁺.

### Example 62: preparation of a compound L-62

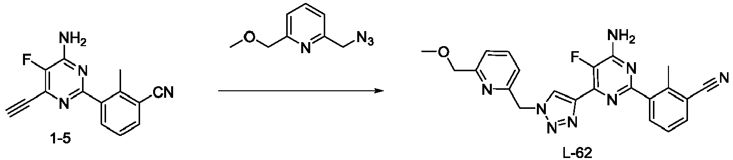

Referring to step 5 of Example 1 by replacing the compound V4 with 2-(azidomethyl)-6-(methoxymethyl)pyridine, a white solid product L-62 (15.45 mg, purity: 98.52%, yield: 12.94%) was obtained. ¹H NMR (400 MHz, DMSO-d6) δ 8.80 (s, 1H), 7.91 (s, 1H), 7.82 (t, J=7.7 Hz, 2H), 7.55 (s, 2H), 7.45 (s, 1H), 7.35 (d, J=7.7 Hz, 1H), 7.17 (d, J=7.9 Hz, 1H), 5.78 (s, 2H), 4.43 (s, 2H), 3.30 (s, 3H), 2.62 (s, 3H). MS (ESI) 431 [M+H]⁺.

### Example 63: preparation of a compound L-63

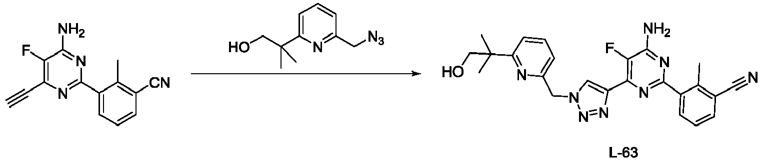

Referring to step 5 of Example 1 by replacing the compound V4 with 2-(6-(azidomethyl)pyridin-2-yl)-2-methylpropan-1-ol, a white solid L-63 (1.52 mg, purity: 97.42%, yield: 1.20%) was obtained. ¹H NMR (400 MHz, DMSO-d6) δ 8.78 (s, 1H), 7.93 (d, J=7.8 Hz, 1H), 7.83 (dd, J=7.8, 1.3 Hz, 1H), 7.71 (t, J=7.8 Hz, 1H), 7.53 (s, 2H), 7.45 (t, J=7.8 Hz, 1H), 7.33 (d, J=7.7 Hz, 1H), 7.07 (d, J=7.4 Hz, 1H), 5.77 (s, 2H), 4.54 (t, J=5.6 Hz, 1H), 3.45 (d, J=5.6 Hz, 2H), 2.64 (s, 3H), 1.18 (d, J=17.3 Hz, 6H). MS (ESI) 459 [M+H]⁺.

### Example 64: preparation of a compound L-64

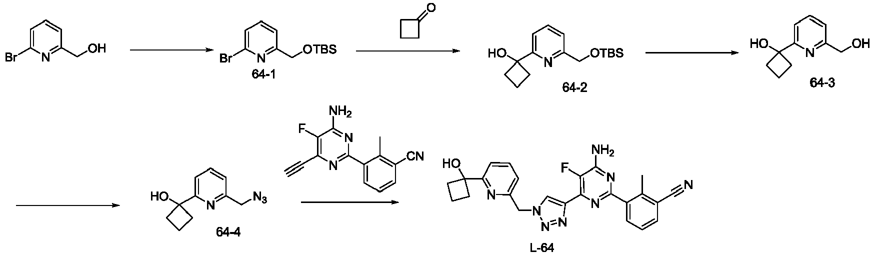

Steps 1 and 2: referring to steps 1 and 2 of Example 21, a compound 64-2 (2.0 g, yield: 100%) was prepared from (6-bromopyridin-2-yl)methanol (3.0 g, 15.96 mmol). MS (ESI) 294 [M+H]⁺.

Step 3: referring to step 4 of Example 1, a compound 64-3 (50 mg, purity: 95%, yield: 100%) was prepared from the compound 64-2. MS (ESI) 180 [M+H]⁺.

Step 4: referring to step 3 of Example 7, a compound 64-4 (380 mg, purity: 100%, yield: 27%) was prepared from the compound 64-3. MS (ESI) 205 [M+H]⁺.

Step 5: referring to step 5 of Example 1 by replacing the compound V4 with the compound 64-4, a white solid L-64 (7.41 mg, purity: 99.39%, yield: 9.0%) was obtained. ¹H NMR (400 MHz, DMSO-d6) δ 8.81 (s, 1H), 7.92 (dd, J=7.8, 1.2 Hz, 1H), 7.83 (dd, J=7.8, 1.2 Hz, 1H), 7.76 (t, J=7.8 Hz, 1H), 7.53 (s, 2H), 7.50-7.37 (m, 2H), 7.17 (d, J=7.2 Hz, 1H), 5.82 (s, 2H), 5.71 (s, 1H), 2.62 (s, 3H), 2.39 (ddd, J=12.0, 8.9, 4.9 Hz, 2H), 2.20-2.03 (m, 2H), 1.84-1.56 (m, 2H). MS (ESI) 457 [M+H]⁺.

### Example 65: preparation of a compound L-65

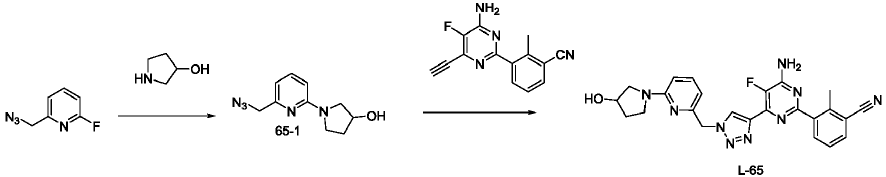

Step 1: referring to step 2 of Example 10, a colorless oily compound 65-1 (80 mg, purity: 76.09%, yield: 55%) was prepared from 2-(azidomethyl)-6-fluoropyridine (100 mg, 0.657 mmol) and pyrrolidin-3-ol (286 mg, 3.29 mmol). MS (ESI) 193 [M+H]⁺.

Step 2: referring to step 5 of Example 1 by replacing the compound V4 with the compound 65-1, a white solid L-65 (15.98 mg, purity: 98.49%, yield: 9%) was obtained. MS (ESI) 472.2 [M+H]⁺.

### Example 66: preparation of a compound L-66

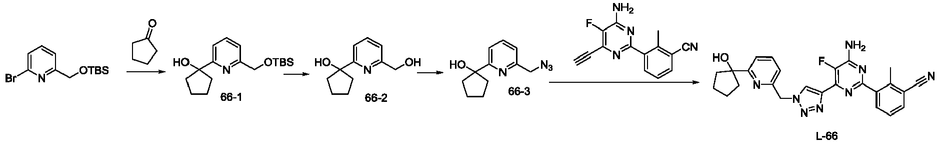

Steps 1 and 2: referring to steps 2 and 3 of Example 21, a colorless oily compound 66-2 (250 mg, purity: 90%, yield: 100%) was prepared from 2-bromo-6-(((tert-butyldimethylsilyl)oxy)methyl)pyridine (700 mg, 2.32 mmol) and cyclopentanone (234 mg, 2.78 mmol). MS (ESI) 194 [M+H]⁺.

Step 3: referring to step 3 of Example 7, a compound 66-3 (220 mg, purity: 94.69%, yield: 93%) was prepared from the compound 66-2. MS (ESI) 219 [M+H]⁺.

Step 4: referring to step 5 of Example 1 by replacing the compound V4 with the compound 66-3, a white solid L-66 (48.72 mg, purity: 98.69%, yield: 26%) was obtained. ¹H NMR (400 MHz, DMSO-d6) δ 8.77 (s, 1H), 7.92 (d, J=7.5 Hz, 1H), 7.83 (d, J=7.6 Hz, 1H), 7.75 (t, J=7.8 Hz, 1H), 7.59 (d, J=7.9 Hz, 1H), 7.53 (s, 2H), 7.45 (t, J=8.0 Hz, 1H), 7.11 (d, J=7.6 Hz, 1H), 5.77 (s, 2H), 5.03 (s, 1H), 2.63 (s, 3H), 2.01-1.94 (m, 2H), 1.78-1.73 (m, 2H), 1.71-1.51 (m, 4H). MS (ESI) 471.2 [M+H]⁺.

### Example 67: preparation of a compound L-67

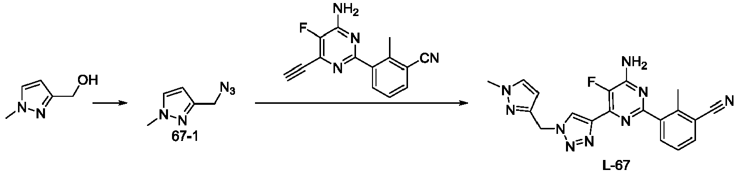

Step 1: referring to step 3 of Example 7, a compound 67-1 (1.0 g, purity: 100%, yield: 82%) was prepared from (1-methyl-1H-pyrazol-3-yl)methanol (1.0 g, 8.92 mmol). MS (ESI) 138 [M+H]⁺.

Step 2: referring to step 5 of Example 1 by replacing the compound V4 with the compound 67-1, a white solid L-67 (41.21 mg, purity: 99.12%, yield: 26%) was obtained. ¹H NMR (400 MHz, DMSO-d6) δ 8.64 (s, 1H), 7.91 (d, J=7.1 Hz, 1H), 7.83 (d, J=7.3 Hz, 1H), 7.63 (s, 1H), 7.52 (s, 2H), 7.45 (t, J=7.6 Hz, 1H), 6.25 (s, 1H), 5.59 (s, 2H), 3.77 (s, 3H), 2.61 (s, 3H). MS (ESI) 390.2 [M+H]⁺.

### Example 68: preparation of a compound L-68

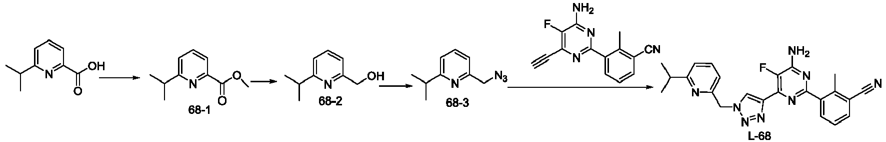

Step 1: 6-isopropylpicolinic acid (1.0 g, 6.05 mmol) was dissolved in methanol (20 mL), the reaction solution was cooled under ice bath conditions with the protection of argon gas, SOCl₂ (2 mL) was dropwise added, and the reaction solution was heated to 90°C and stirred overnight. Then, the reaction solution was concentrated under reduced pressure to dryness, a saturated sodium bicarbonate solution was added, the mixture was extracted with ethyl acetate (50 mL × 2), and a combined organic phase was dried with anhydrous sodium sulfate and concentrated under reduced pressure to dryness to obtain a colorless oily compound 68-1 (1.0 g, purity: 91.52%, yield: 93%). MS (ESI) 180 [M+H]⁺.

Steps 2 and 3: referring to steps 2 and 3 of Example 7, a compound 68-3 (500 mg, purity: 100%, yield: 61%) was prepared from the compound 68-1. MS (ESI) 176 [M+H]⁺.

Step 4: referring to step 5 of Example 1 by replacing the compound V4 with the compound 68-3, a yellow solid compound L-68 (50.72 mg, purity: 95.48%, yield: 30%) was obtained. ¹H NMR (400 MHz, DMSO-d6) δ 8.79 (s, 1H), 7.93 (d, J=7.3 Hz, 1H), 7.83 (d, J=7.5 Hz, 1H), 7.70 (t, J=7.7 Hz, 1H), 7.53 (s, 2H), 7.45 (t, J=7.6 Hz, 1H), 7.22 (d, J=7.8 Hz, 1H), 7.04 (d, J=7.8 Hz, 1H), 5.76 (s, 2H), 2.95 (dt, J=13.7, 6.8 Hz, 1H), 2.63 (s, 3H), 1.16 (d, J=6.9 Hz, 6H). MS (ESI) 429.2 [M+H]⁺.

### Example 69: preparation of a compound L-69

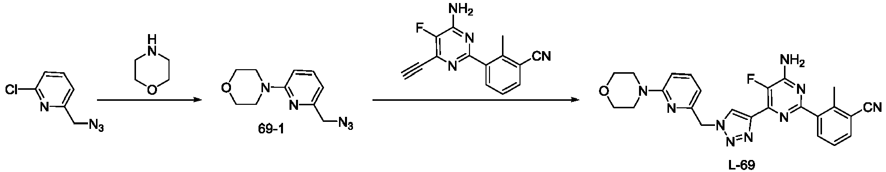

Step 1: a compound 2-(azidomethyl)-6-chloropyridine (300 mg, 1.78 mmol) and morphine (2 mL) were heated to 130°C under microwave conditions and reacted for 5 h. Then, the above reaction solution was added to water, the mixture was extracted with ethyl acetate (50 mL × 2), and a combined organic phase was dried with anhydrous sodium sulfate and concentrated under reduced pressure to dryness to obtain a colorless oily compound 69-1 (380 mg, purity: 79.95%, yield: 97%). MS (ESI) 220 [M+H]⁺.

Step 2: referring to step 5 of Example 1 by replacing the compound V4 with the compound 69-1, a white solid L-69 (18.27 mg, purity: 100%, yield: 9%) was obtained. ¹H NMR (400 MHz, DMSO-d6) δ 8.75 (s, 1H), 7.94 (dd, J=7.8, 1.2 Hz, 1H), 7.84 (dd, J=7.7, 1.3 Hz, 1H), 7.62-7.50 (m, 3H), 7.46 (t, J=7.7 Hz, 1H), 6.75 (d, J=8.6 Hz, 1H), 6.55 (d, J=7.3 Hz, 1H), 5.61 (s, 2H), 3.72-3.54 (m, 4H), 3.45-3.32 (m, 4H), 2.64 (s, 3H). MS (ESI) 472 [M+H]⁺.

### Example 70: preparation of a compound L-70

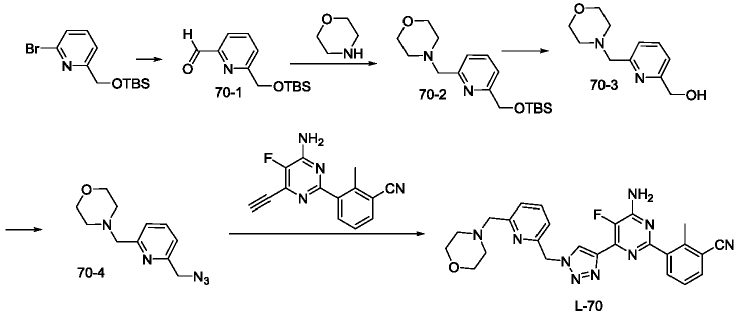

Step 1: referring to step 2 of Example 21, a compound 70-1 (650 mg, purity: 77.94%, yield: 78%) was prepared from 2-bromo-6-(((tert-butyldimethylsilyl)oxy)methyl)pyridine (1.0 g, 3.31 mmol). MS (ESI) 252 [M+H]⁺.

Step 2: the compound 70-1 (300 mg, 1.19 mmol) and morpholine (312 mg, 3.58 mmol) were added to dichloromethane (15 mL), sodium borohydride acetate (759 mg, 3.58 mmol) was added while the reaction solution was stirred at the room temperature, and the reaction solution was stirred at the room temperature overnight. Then, the reaction solution was added to water, the mixture was extracted with dichloromethane (50 mL × 2), and a combined organic phase was dried with anhydrous sodium sulfate, concentrated under reduced pressure to dryness, and separated and purified by silica-gel column chromatography to obtain a compound 70-2 (350 mg, purity: 70.73%, yield: 91%). MS (ESI) 323.2 [M+H]⁺.

Step 3: the compound 70-2 (350 mg, 1.09 mmol) was dissolved in ethanol (5 mL), a 4.0 M dioxane hydrochloride solution (5 mL) was added while the reaction solution was stirred at the room temperature, and the reaction solution was stirred at the room temperature for 2 h. Then, the reaction solution was concentrated under reduced pressure to dryness to obtain a compound 70-3 (266 mg, purity: 90%, yield: 100%). MS (ESI) 209 [M+H]⁺.

Step 4: referring to step 3 of Example 7, a colorless oily compound 70-4 (350 mg, purity: 93.52%, yield: 89%) was prepared from the compound 70-3. MS (ESI) 234 [M+H]⁺.

Step 5: referring to step 5 of Example 1 by replacing the compound V4 with the compound 70-4, a white solid L-70 (18.69 mg, purity: 98.04%, yield: 10%) was obtained. ¹H NMR (400 MHz, DMSO-d6) δ 8.79 (s, 1H), 7.92 (d, J=7.9 Hz, 1H), 7.87-7.71 (m, 2H), 7.54 (s, 2H), 7.45 (t, J=7.9 Hz, 1H), 7.38 (d, J=7.7 Hz, 1H), 7.14 (d, J=7.6 Hz, 1H), 5.79 (s, 2H), 3.54 (s, 6H), 2.62 (s, 3H), 2.35 (s, 4H). MS (ESI) 486 [M+H]⁺.

### Example 71: preparation of a compound L-71

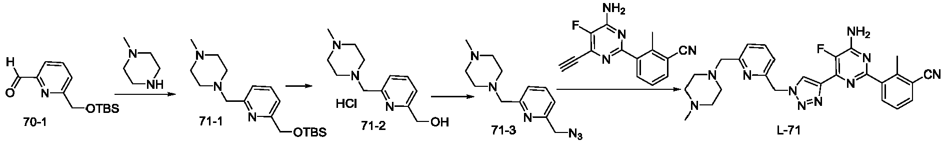

Steps 1 and 2: referring to steps 2 and 3 of Example 70, a compound 71-2 (350 mg, purity: 90%, yield: 100%) was prepared from the compound 70-1 (300 mg, 1.19 mmol) and 1-methylpiperazine (359 mg, 3.58 mmol). MS (ESI) 222 [M+H]⁺.

Step 3: referring to step 3 of Example 7, a colorless oily compound 71-3 (350 mg, purity: 94.24%, yield: 90%) was prepared from the compound 71-2. MS (ESI) 247 [M+H]⁺.

Step 5: referring to step 5 of Example 1 by replacing the compound V4 with the compound 71-3, a white solid L-71 (21.37 mg, purity: 95.93%, yield: 11%) was obtained. ¹H NMR (400 MHz, DMSO-d6) δ 8.76 (s, 1H), 7.92 (dd, J=7.9, 1.2 Hz, 1H), 7.83 (dd, J=7.7, 1.2 Hz, 1H), 7.76 (t, J=7.7 Hz, 1H), 7.53 (s, 2H), 7.45 (t, J=7.7 Hz, 1H), 7.34 (d, J=7.7 Hz, 1H), 7.12 (d, J=7.7 Hz, 1H), 5.76 (s, 2H), 3.50 (s, 2H), 2.63 (s, 3H), 2.42-2.12 (m, 8H), 2.07 (s, 3H). MS (ESI) 499 [M+H]⁺.

### Example 72: preparation of a compound L-72

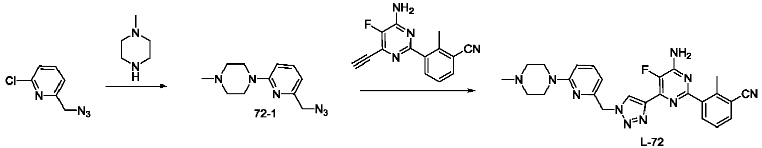

Step 1: referring to step 1 of Example 69, a colorless oily compound 72-1 (390 mg, purity: 94.65%, yield: 94%) was prepared from 2-(azidomethyl)-6-chloropyridine (300 mg, 1.78 mmol) and 1-methylpiperazine (2 mL). MS (ESI) 233 [M+H]⁺.

Step 2: referring to step 5 of Example 1 by replacing the compound V4 with the compound 72-1, a white solid L-72 (18.69 mg, purity: 100%, yield: 9%) was obtained. ¹H NMR (400 MHz, DMSO-d6) δ 8.74 (s, 1H), 7.98-7.89 (m, 1H), 7.83 (dd, J=7.7, 1.2 Hz, 1H), 7.48 (ddd, J=17.3, 13.5, 5.8 Hz, 4H), 6.73 (d, J=8.6 Hz, 1H), 6.50 (d, J=7.2 Hz, 1H), 5.59 (s, 2H), 3.47-3.36 (m, 4H), 2.63 (s, 3H), 2.33-2.21 (m, 4H), 2.13 (s, 3H). MS (ESI) 485 [M+H]⁺.

### Example 73: preparation of a compound L-73

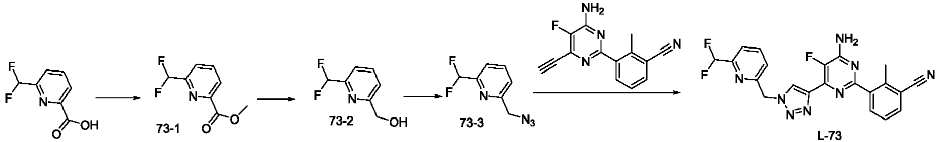

Step 1: 6-(difluoromethyl)picolinic acid (250 mg, 1.44 mmol) was dissolved in methanol (15 mL), the reaction solution was cooled under ice bath conditions with the protection of argon gas, thionyl chloride (1 mL) was dropwise added, and the reaction solution was heated to the room temperature and stirred overnight. Then, the reaction solution was directly concentrated under reduced pressure to dryness, a saturated sodium bicarbonate solution was added, the mixture was extracted with ethyl acetate (50 mL × 2), and a combined organic phase was dried with anhydrous sodium sulfate and concentrated under reduced pressure to dryness to obtain a compound 73-1 (270 mg, purity: 100%, yield: 100%). MS (ESI) 188 [M+H]⁺.

Steps 2 and 3: referring to steps 2 and 3 of Example 7, a compound 73-3 (180 mg, purity: 89.3%, yield: 74%) was prepared from the compound 73-1. MS (ESI) 185 [M+H]⁺.

Step 4: referring to step 5 of Example 1 by replacing the compound V4 with the compound 73-3, a white solid L-73 (30.24 mg, purity: 99.26%, yield: 19%) was obtained. ¹H NMR (400 MHz, DMSO-d6) δ 8.83 (s, 1H), 8.20-7.72 (m, 3H), 7.73-7.17 (m, 5H), 6.89 (t, J=55.2 Hz, 1H), 5.87 (s, 2H), 2.62 (s, 3H). MS (ESI) 437 [M+H]⁺.

### Example 74: preparation of a compound L-74

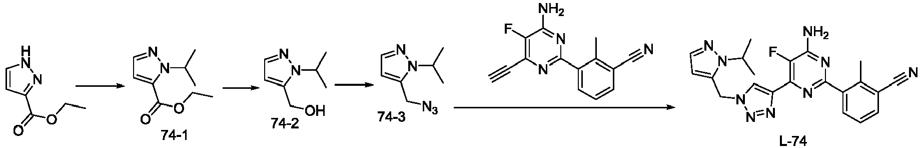

Step 1: referring to step 2 of Example 10, a compound 74-1 (1.35 g, purity: 100%, yield: 41%) was prepared from ethyl 1*H*-pyrazole-3-carboxylate (2.5 g, 17.98 mmol) and 2-iodopropane (3.1 g, 17.98 mmol). MS (ESI) 183 [M+H]⁺.

Steps 2 and 3: referring to steps 2 and 3 of Example 7, a compound 74-3 (200 mg, purity: 90%, yield: 59%) was prepared from the compound 74-1. MS (ESI) 166 [M+H]⁺.

Step 4: referring to step 5 of Example 1 by replacing the compound V4 with the compound 74-3, a yellow solid L-74 (50.72 mg, purity: 100%, yield: 8%) was obtained. ¹H NMR (400 MHz, DMSO-d6) δ 8.71 (s, 1H), 8.05-7.68 (m, 2H), 7.67-7.09 (m, 4H), 6.28 (s, 1H), 5.84 (s, 2H), 4.78 (dt, J=13.1, 6.5 Hz, 1H), 2.62 (s, 3H), 1.27 (d, J=6.5 Hz, 6H). MS (ESI) 418 [M+H]⁺.

### Example 75: preparation of a compound L-75

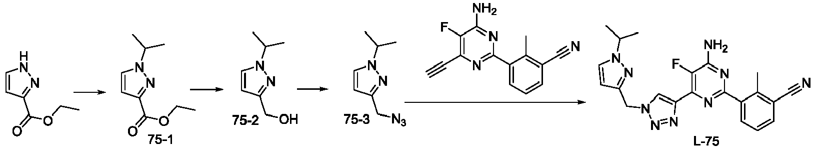

Step 1: referring to step 2 of Example 10, a compound 75-1 (1.2 g, purity: 100%, yield: 37%) was prepared from ethyl 1*H*-pyrazole-3-carboxylate (2.5 g, 17.98 mmol) and 2-iodopropane (3.1 g, 17.98 mmol). MS (ESI) 183 [M+H]⁺.

Steps 2 and 3: referring to steps 2 and 3 of Example 7, a compound 75-3 (600 mg, purity: 70%, yield: 51%) was prepared from the compound 75-1. MS (ESI) 166 [M+H]⁺.

Step 4: referring to step 5 of Example 1 by replacing the compound V4 with the compound 75-3, a yellow solid L-75 (30.34 mg, purity: 97.82%, yield: 18%) was obtained. ¹H NMR (400 MHz, DMSO-d6) δ 8.67 (s, 1H), 7.86 (d, J=36.6 Hz, 2H), 7.72 (d, J=2.1 Hz, 1H), 7.64-7.29 (m, 3H), 6.24 (s, 1H), 5.62 (s, 2H), 4.45 (dt, J=13.3, 6.6 Hz, 1H), 2.75-2.51 (m, 3H), 1.36 (d, J=6.7 Hz, 6H). MS (ESI) 418 [M+H]⁺.

### Example 76: preparation of a compound L-76

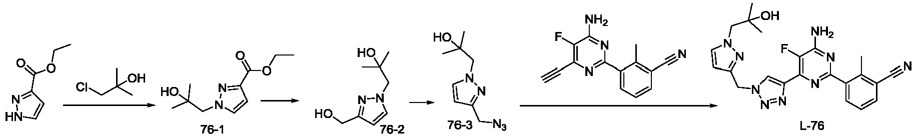

Step 1: referring to step 2 of Example 10, a compound 76-1 (1.4 g, purity: 100%, yield: 71%) was prepared from ethyl 1*H*-pyrazole-3-carboxylate (1.3 g, 9.28 mmol) and 1-chloro-2-methylpropan-2-ol (1.3 g, 12.06 mmol). MS (ESI) 213 [M+H]⁺.

Steps 2 and 3: referring to steps 2 and 3 of Example 7, a compound 76-3 (250 mg, purity: 73.52%, yield: 31%) was prepared from the compound 76-1. MS (ESI) 196 [M+H]⁺.

Step 4: referring to step 5 of Example 1 by replacing the compound V4 with the compound 76-3, a white solid L-76 (9.58 mg, purity: 98.67%, yield: 5%) was obtained. ¹H NMR (400 MHz, DMSO-d6) δ 8.59 (s, 1H), 7.92 (d, J=7.7 Hz, 1H), 7.83 (d, J=7.5 Hz, 1H), 7.61 (d, J=1.6 Hz, 1H), 7.50 (s, 2H), 7.45 (t, J=7.6 Hz, 1H), 6.26 (s, 1H), 5.61 (s, 2H), 4.63 (s, 1H), 3.96 (s, 2H), 2.62 (s, 3H), 1.00 (s, 6H). MS (ESI) 448 [M+H]⁺.

### Example 77: preparation of a compound L-77

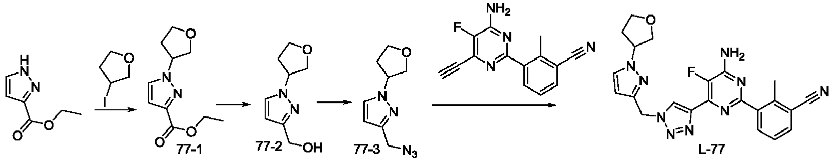

Step 1: referring to step 2 of Example 10, a compound 77-1 (0.41 g, purity: 87.79%, yield: 38%) was prepared from ethyl 1H-pyrazole-3-carboxylate (0.71 g, 5.07 mmol) and 3-iodotetrahydrofuran (1.0 g, 5.07 mmol). MS (ESI) 211 [M+H]⁺.

Steps 2 and 3: referring to steps 2 and 3 of Example 7, a compound 77-3 (300 mg, purity: 100%, yield: 81%) was prepared from the compound 77-1. MS (ESI) 166 [M+H]⁺.

Step 4: referring to step 5 of Example 1 by replacing the compound V4 with the compound 77-3, a white solid L-77 (46.16 mg, purity: 100%, yield: 26%) was obtained. ¹H NMR (400 MHz, DMSO-d6) δ 8.66 (s, 1H), 7.92 (d, J=7.1 Hz, 1H), 7.83 (d, J=7.0 Hz, 1H), 7.74 (s, 1H), 7.52 (s, 2H), 7.45 (t, J=7.1 Hz, 1H), 6.27 (s, 1H), 5.62 (s, 2H), 4.97 (s, 1H), 4.01-3.87 (m, 2H), 3.87-3.66 (m, 2H), 2.62 (s, 2H), 2.41-2.26 (m, 1H), 2.26-2.08 (m, 1H). MS (ESI) 446 [M+H]+.

### Example 78: preparation of a compound L-78

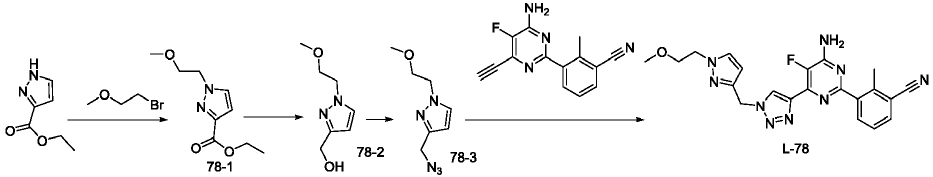

Step 1: referring to step 1 of Example 23, a colorless oily compound 78-1 (0.8 g, yield: 32.43%, purity: 88.82%) was prepared from ethyl 1H-pyrazole-3-carboxylate (1.5 g, 11.89 mmol) and 1-bromo-2-methoxyethane (1.98 g, 14.27 mmol).

Steps 2 and 3: referring to steps 2 and 3 of Example 7, a colorless oily compound 78-3 (600 mg, yield: 81.89%, purity: 95%) was prepared from the compound 78-1.

Step 4: referring to step 5 of Example 1 by replacing the compound V4 with the compound 78-3, a green solid L-78 (72.59 mg, yield: 52.29%, purity: 99.02%) was obtained. ¹H NMR (400 MHz, DMSO-d6) δ 8.64 (s, 1H), 7.87 (dd, J=33.8, 8.0 Hz, 2H), 7.66 (s, 1H), 7.47 (dd, J=23.1, 15.7 Hz, 3H), 6.24 (s, 1H), 5.61 (s, 2H), 4.20 (s, 2H), 3.62 (s, 2H), 3.16 (s, 3H), 2.62 (s, 3H). MS (ESI) 434 [M+H]⁺.

### Example 79: preparation of a compound L-79

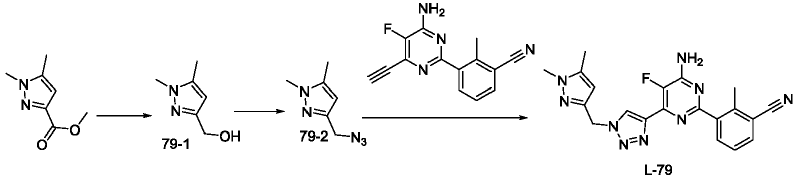

Steps 1 and 2: referring to steps 2 and 3 of Example 7, a yellow solid 79-2 (700 mg, yield: 83.45%) was prepared from methyl 1,5-dimethyl-1H-pyrazole-3-carboxylate (1.0 g, 6.49 mmol). MS (ESI) 152 [M+H]⁺.

Step 3: referring to step 5 of Example 1 by replacing the compound V4 with the compound 79-2, a white solid L-79 (26.66 mg, yield: 16.08%, purity: 96.47%) was obtained. ¹H NMR (400 MHz, DMSO-d6) δ 8.62 (s, 1H), 7.91 (d, J=7.9 Hz, 1H), 7.83 (d, J=7.6 Hz, 1H), 7.52 (s, 2H), 7.45 (t, J=7.8 Hz, 1H), 6.03 (s, 1H), 5.52 (s, 2H), 3.64 (s, 3H), 2.62 (s, 3H), 2.17 (s, 3H). MS (ESI) 404 [M+H]⁺.

### Example 80: preparation of a compound L-80

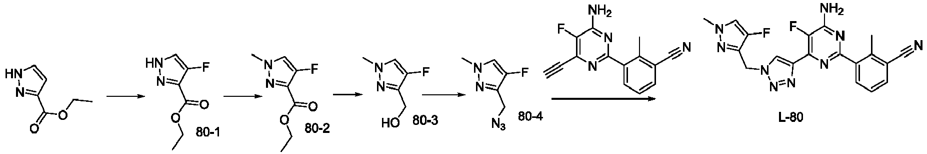

Step 1: referring to step 2 of Example 21, a compound 80-1 (0.55 g, yield: 29.57%, purity: 60.67%) was prepared from ethyl 1*H*-pyrazole-3-carboxylate (1 g, 7.14 mmol). MS (ESI) 159 [M+H]⁺.

Step 2: referring to step 1 of Example 23, a compound 80-2 (400 mg, yield: 47.61%, purity: 71.27%) was prepared from the compound 80-1 (550 mg, 3.48 mmol) and iodomethane (987.37 mg, 6.96 mmol). MS (ESI) 173 [M+H]⁺.

Steps 3 and 4: referring to steps 2 and 3 of Example 7, a compound 80-4 (100 mg, yield: 36.22%, purity: 95%) was prepared from the compound 80-2. MS (ESI) 156 [M+H]⁺.

Step 5: referring to step 5 of Example 1 by replacing the compound V4 with the compound 80-4, a white compound L-80 (34.57 mg, yield: 38.42%, purity: 98.73%) was obtained. ¹H NMR (400 MHz, DMSO-d6) δ 8.63 (s, 1H), 7.87 (d, J=29.0 Hz, 3H), 7.48 (d, J=28.2 Hz, 3H), 5.64 (s, 2H), 3.72 (s, 3H), 2.61 (s, 3H). MS (ESI) 408 [M+H]⁺.

### Example 81: preparation of a compound L-81

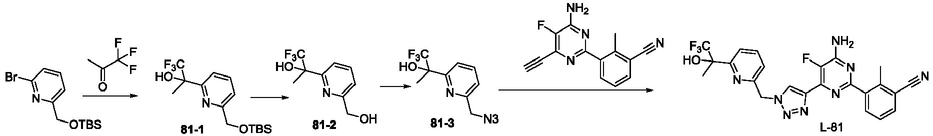

Step 1: referring to step 2 of Example 53, a colorless oily compound 81-1 (160 mg, yield: 27.32%, purity: 94.73%) was prepared from 2-bromo-6-(((tert-butyldimethylsilyl)oxy)methyl)pyridine (500 mg, 1.65 mmol) and 1,1,1-trifluoroacetone (278.01 mg, 2.48 mmol). MS (ESI) 336 [M+H]⁺.

Step 2: referring to step 3 of Example 70, a yellow oily compound 81-2 (100 mg, yield: 90.18%, purity: 95.14%) was prepared from the compound 81-1. MS (ESI) 222 [M+H]⁺.

Step 3: referring to step 3 of Example 7, a colorless oily compound 81-3 (80 mg, yield: 54.03%, purity: 97.72%) was prepared from the compound 81-2. MS (ESI) 247 [M+H]⁺.

Step 4: referring to step 5 of Example 1 by replacing the compound V4 with the compound 81-3, a white solid L-81 (42.86 mg, yield: 83.86%, purity: 98.40%) was obtained. ¹H NMR (400 MHz, DMSO-d6) δ 8.79 (s, 1H), 8.03-7.78 (m, 3H), 7.71 (d, J=7.5 Hz, 1H), 7.53 (s, 2H), 7.45 (t, J=7.9 Hz, 1H), 7.32 (d, J=7.4 Hz, 1H), 6.71 (s, 1H), 5.84 (s, 2H), 2.63 (s, 3H), 1.60 (s, 3H). MS (ESI) 499 [M+H]⁺.

### Example 82: preparation of a compound L-82

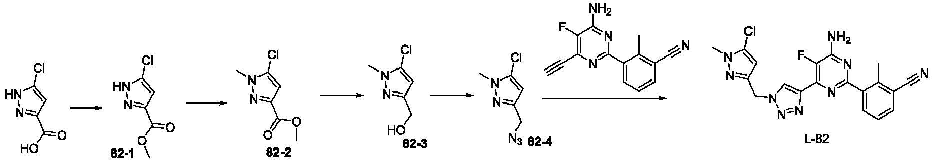

Step 1: referring to step 1 of Example 73, a white solid 82-1 (273 mg, yield: 99.66%, purity: 100%) was prepared from 5-chloro-1H-pyrazole-3-carboxylic acid (250 mg, 1.71 mmol). MS (ESI) 161 [M+H]⁺.

Step 2: referring to step 1 of Example 23, a colorless oily compound 82-2 (297 mg, yield: 81.49%, purity: 81.75%) was prepared from the compound 82-1. MS (ESI) 175 [M+H]⁺.

Steps 3 and 4: referring to steps 2 and 3 of Example 7, a colorless oily compound 82-4 (130 mg, yield: 55.52%) was prepared from the compound 82-2. MS (ESI) 172 [M+H]⁺.

Step 5: referring to step 5 of Example 1 by replacing the compound V4 with the compound 72-4, a white solid L-82 (48.13 mg, yield: 27.44%, purity: 95.79%) was obtained. ¹H NMR (400 MHz, DMSO-d6) δ 8.70 (s, 1H), 7.87 (d, J=35.4 Hz, 2H), 7.49 (d, J=25.7 Hz, 3H), 6.46 (s, 1H), 5.60 (s, 2H), 3.74 (s, 3H), 2.63 (s, 3H). MS (ESI) 424 [M+H]⁺.

### Example 83: preparation of a compound L-83

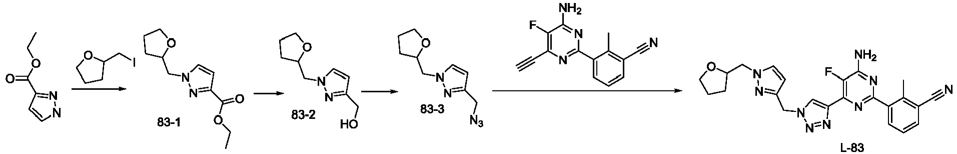

Step 1: referring to step 2 of Example 10, a compound 83-1 (550 mg, yield: 56.79%, purity: 95.02%) was prepared from ethyl 1*H*-pyrazole-3-carboxylate (552 mg, 4.38 mmol) and 2-(iodomethyl)tetrahydrofuran (1.06 g, 8.75 mmol). MS (ESI) 225 [M+H]⁺.

Steps 2 and 3: referring to steps 2 and 3 of Example 7, a compound 83-3 (250 mg, yield: 62.81%, purity: 100%) was prepared from the compound 83-1. MS (ESI) 208 [M+H]⁺.

Step 4: referring to step 5 of Example 1 by replacing the compound V4 with the compound 83-3, a white solid L-83 (14.23 mg, yield: 12.93%, purity: 99.29%) was obtained. ¹H NMR (400 MHz, DMSO-d6) δ 8.62 (s, 1H), 7.92 (d, J=7.5 Hz, 1H), 7.83 (d, J=7.3 Hz, 1H), 7.64 (d, J=2.2 Hz, 1H), 7.52 (s, 2H), 7.45 (t, J=7.8 Hz, 1H), 6.25 (d, J=2.2 Hz, 1H), 5.61 (s, 2H), 4.19-3.96 (m, 3H), 3.67 (dd, J=13.9, 7.5 Hz, 1H), 3.56 (dd, J=14.2, 7.6 Hz, 1H), 1.94-1.79 (m, 1H), 1.77-1.59 (m, 2H), 1.59-1.46 (m, 1H). MS (ESI) 460 [M+H]⁺.

### Example 84: preparation of a compound L-84

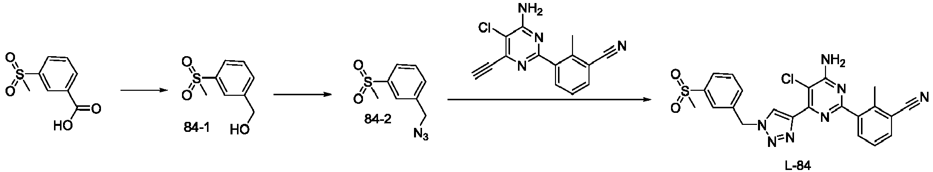

Steps 1 and 2: referring to steps 2 and 3 of Example 7, a crude compound 84-3 (256 mg) was prepared from 3-(methylsulfonyl)benzoic acid (300 mg, 1.50 mmol).

Step 3: referring to step 5 of Example 1 by replacing the compound V4 with the compound 84-3., an off-white solid L-84 (1.74 mg, purity: 99.33%, yield: 2.0%) was obtained MS (ESI) 480.0 [M+H]⁺. ¹H NMR (400 MHz, DMSO-d6) δ 8.98 (s, 1H), 7.98 (d, J=7.5 Hz, 2H), 7.92 (d, J=7.1 Hz, 1H), 7.87 (d, J=6.4 Hz, 1H), 7.69 (d, J=7.4 Hz, 2H), 7.49 (s, 1H), 5.84 (s, 2H), 3.22 (s, 2H), 2.67 (s, 3H), 1.24 (s, 3H).

### Example 85: preparation of a compound L-85

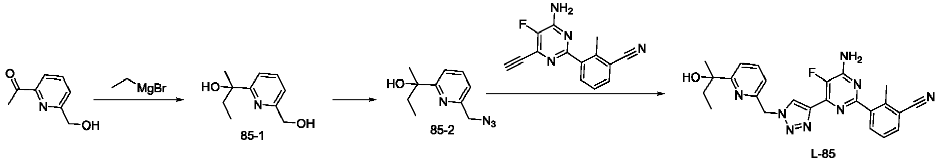

Step 1: 1-(6-(hydroxymethyl)pyridin-2-yl)ethan-1-one (500 mg, 3.31 mmol) was dissolved in anhydrous tetrahydrofuran (10 mL), ethylmagnesium bromide (3.3 mL, 6.60 mmol) was added under ice bath conditions, and the reaction solution was stirred at the room temperature for 3 h. Then, saturated ammonium chloride (2 mL) was dropwise added to the reaction solution at 0°C to quench the reaction, the mixture was stirred at the room temperature for 10 min until a solid was precipitated out and filtered, and an obtained filtrate was concentrated under reduced pressure to dryness and purified by column chromatography (ethyl acetate/petroleum ether=50 to 80%) to obtain a colorless oily compound 85-1 (212 mg, purity: 70.42%, yield: 35.4%). MS (ESI) 182.1 [M+H]⁺.

Step 2: referring to step 3 of Example 7, a crude compound 85-2 (264 mg) was prepared from the compound 85-1.

Step 3: referring to step 5 of Example 1 by replacing the compound V4 with the compound 85-2, a white solid L-85 (10.35 mg, purity: 96.97%, yield: 5.7%) was obtained. MS (ESI) 459.1 [M+H]⁺. ¹H NMR (400 MHz, DMSO-d6) δ 8.79 (s, 1H), 7.95 (d, J=1.2 Hz, 1H), 7.90-7.83 (m, 1H), 7.79 (t, J=7.8 Hz, 1H), 7.64-7.53 (m, 3H), 7.48 (s, 1H), 7.14 (d, J=7.0 Hz, 1H), 5.81 (s, 2H), 5.04 (s, 1H), 2.66 (s, 3H), 1.70 (dd, J=31.4, 7.4 Hz, 2H), 1.35 (s, 3H), 0.59 (s, 3H).

### Example 86: preparation of a compound L-86

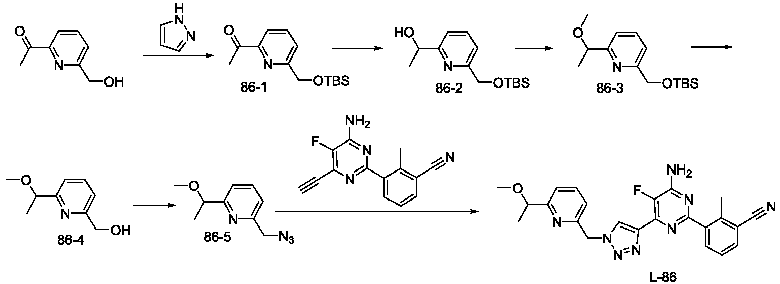

Step 1: referring to step 1 of Example 21, a colorless oily compound 86-1 (680 mg, purity: 91.74%, yield: 77.7%) was prepared from 1-(6-(hydroxymethyl)pyridin-2-yl)ethan-1-one (500 mg, 3.31 mmol). MS (ESI) 266.1 [M+H]⁺.

Step 2: referring to step 3 of Example 11, a colorless oily compound 86-2 (567 mg, purity: 89.12%, yield: 82.8%) was prepared from the compound 86-1. MS (ESI) 268.1 [M+H]⁺.

Step 3: referring to step 1 of Example 23, a compound 86-3 (518 mg) was prepared from the compound 86-2 (567 mg, 2.12 mmol) and iodomethane (602 mg, 4.24 mmol). MS (ESI) 282.1 [M+H]⁺.

Step 4: referring to step 4 of Example 1, a colorless oily compound 86-4 (288 mg, purity: 98.79%, yield: 95.2%) was prepared from the compound 86-3. MS (ESI) 168.1 [M+H]⁺.

Step 5: referring to step 3 of Example 7, a compound 86-5 (302 mg) was prepared from the compound 86-4.

Step 6: referring to step 5 of Example 1 by replacing the compound V4 with the compound 86-5, a white solid L-86 (7.31 mg, purity: 100%, yield: 4.1%) was obtained. MS (ESI) 445.2 [M+H]⁺. ¹H NMR (400 MHz, DMSO-d6) δ 8.79 (s, 1H), 7.92 (s, 1H), 7.82 (dd, J=12.8, 5.0 Hz, 2H), 7.53 (s, 2H), 7.45 (s, 1H), 7.34 (d, J=7.6 Hz, 1H), 7.13 (d, J=7.6 Hz, 1H), 5.79 (s, 2H), 4.31 (d, J=6.5 Hz, 1H), 3.16 (s, 3H), 2.63 (s, 3H), 1.29 (d, J=6.5 Hz, 3H).

### Example 87: preparation of a compound L-87

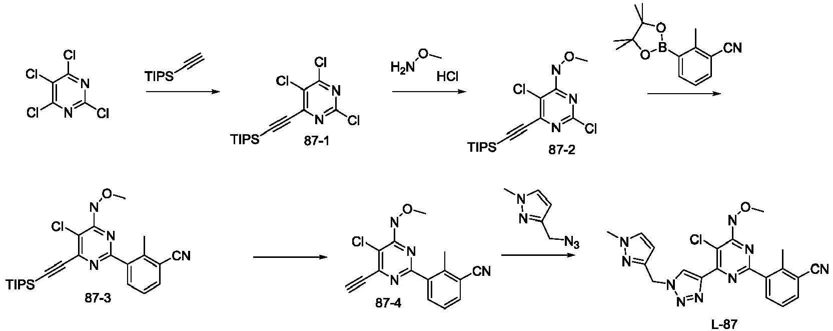

Step 1: referring to step 1 of Example 1 by replacing 2,4,6-trichloro-5-fluoropyrimidine with 2,4,5,6-tetrachloropyrimidine, a pale-yellow liquid 87-1 (2.78 g, purity: 81.12%, yield: 67.53%) was obtained. MS (ESI) 363.0 [M+H]⁺.

Step 2: the compound 87-1 (2.70 g, 7.43 mmol) and methoxyamine hydrochloride (1.24 g, 14.85 mmol) were added to anhydrous tetrahydrofuran (25 mL), triethylamine (2.25 g, 22.27 mmol) was added at the room temperature, and the reaction solution was stirred at the room temperature overnight. Then, the reaction solution was evaporated under reduced pressure to dryness and extracted with EA (30 mL), an obtained organic phase was dried with anhydrous sodium sulfate and evaporated under reduced pressure to dryness to obtain a compound 87-2 (1.66 g). MS (ESI) 374.1 [M+H]⁺.

Steps 3 to 5: referring to steps 3 to 5 of Example 1 by replacing the compound 1-3 with the compound 87-2 and replacing the intermediate V4 with 3-(azidomethyl)-1-methyl-1H-pyrazole, a pale-yellow solid product L-87 (27.56 mg, purity: 99.91%, yield: 22.12%) was obtained. MS (ESI) 436.1 [M+H]⁺. ¹H NMR (400 MHz, DMSO-d6) δ 10.99 (s, 1H), 8.79 (s, 1H), 8.07 (s, 1H), 7.91 (d, J=7.7 Hz, 1H), 7.66 (d, J=2.0 Hz, 1H), 7.53 (t, J=7.8 Hz, 1H), 6.28 (d, J=2.1 Hz, 1H), 5.63 (s, 2H), 3.79 (d, J=5.6 Hz, 6H), 2.71 (s, 3H).

### Example 88: preparation of a compound L-88

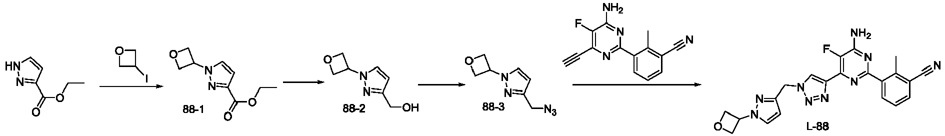

Step 1: referring to step 2 of Example 10, a compound 88-2 was prepared from ethyl 1*H*-pyrazole-3-carboxylate (400 mg, 2.85 mmol) and 3-iodooxetane (630 mg, 3.43 mmol). MS (ESI) 197.1 [M+H]⁺.

Steps 2 and 3: referring to steps 2 and 3 of Example 7, a compound 88-3 (146 mg) was prepared from the compound 88-2. MS (ESI) 180.1 [M+H]⁺.

Step 4: referring to step 5 of Example 1 by replacing the compound V4 with the compound 88-3, an off-white solid compound L-88 (10.86 mg, purity: 96.43%, yield: 12.25%) was obtained. MS (ESI) 432.1 [M+H]⁺. ¹H NMR (400 MHz, DMSO-d6) δ 8.73 (s, 1H), 7.91 (d, J=23.9 Hz, 3H), 7.52 (d, J=28.4 Hz, 3H), 6.35 (s, 1H), 5.71 (s, 2H), 5.56 (s, 1H), 5.04-4.70 (m, 4H), 2.65 (s, 3H).

### Example 89: preparation of a compound L-89

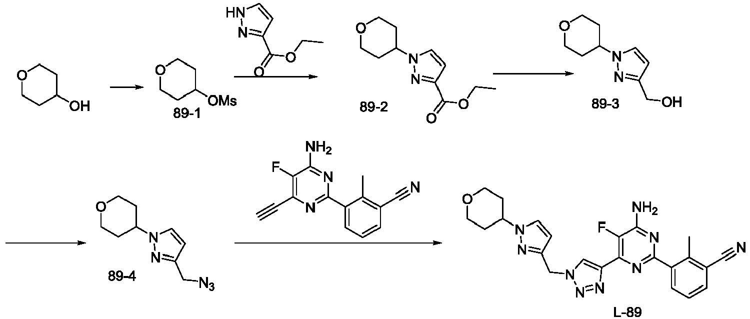

Steps 1 and 2: referring to step 1 of the preparation method of the intermediate V5, a compound 89-2 (197 mg, purity: 84.8%, a total yield of the two reactions: 20.88%) was prepared from tetrahydropyran-4-ol (1 g, 9.79 mmol). MS (ESI) 225.1 [M+H]⁺.

Steps 3 and 4: referring to steps 2 and 3 of Example 7, a compound 89-4 (162 mg) was prepared from the compound 89-2. MS (ESI) 208.1 [M+H]⁺.

Step 5: referring to step 5 of Example 1 by replacing the compound V4 with the compound 89-4, an off-white solid L-89 (11.10 mg, purity: 98.27%, yield: 11.26%) was obtained. MS (ESI) 460.1 [M+H]⁺. ¹H NMR (400 MHz, DMSO-d6) δ 8.68 (s, 1H), 7.95 (d, J=7.8 Hz, 1H), 7.90-7.83 (m, 1H), 7.79 (d, J=2.3 Hz, 1H), 7.55 (s, 2H), 7.48 (t, J=7.7 Hz, 1H), 6.29 (d, J=2.3 Hz, 1H), 5.65 (s, 2H), 4.45-4.32 (m, 1H), 4.01-3.86 (m, 2H), 3.43 (td, J=11.4, 3.3 Hz, 2H), 2.65 (s, 3H), 2.05-1.79 (m, 4H).

### Example 90: preparation of a compound L-90

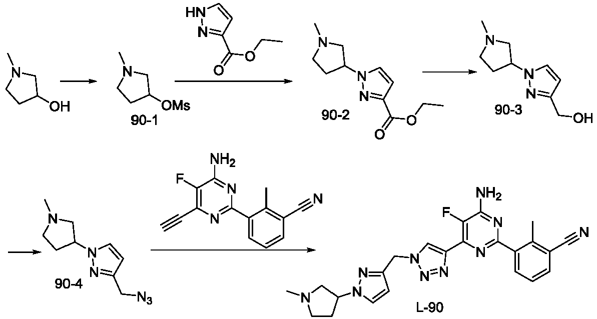

Steps 1 and 2: referring to step 1 of the preparation method of the intermediate V5, a compound 90-2 (542 mg, purity: 93.82%, yield: 53.96%) was prepared from 1-methylpyrrolidin-3-ol (1 g, 9.89 mmol). MS (ESI) 224.1 [M+H]⁺.

Steps 3 and 4: referring to steps 2 and 3 of Example 7, a compound 90-4 (261 mg) was prepared from the compound 90-2. MS (ESI) 207.1 [M+H]⁺.

Step 5: referring to step 5 of Example 1 by replacing the compound V4 with the compound 90-4, a white solid L-90 (2.09 mg, purity: 99.64%, a total yield of the three reactions: 1.54%) was obtained. MS (ESI) 459.2 [M+H]⁺. 1H NMR (400 MHz, DMSO-d6) δ 8.67 (s, 1H), 7.95 (d, J=7.7 Hz, 1H), 7.86 (dd, J=7.8, 1.2 Hz, 1H), 7.76 (d, J=2.3 Hz, 1H), 7.55 (s, 2H), 7.49 (d, J=7.8 Hz, 1H), 6.27 (d, J=2.3 Hz, 1H), 5.64 (s, 2H), 4.85 (s, 1H), 2.75 (dd, J=16.5, 6.6 Hz, 3H), 2.65 (s, 3H), 2.45 (d, J=6.4 Hz, 1H), 2.32 (d, J=9.4 Hz, 1H), 2.26 (s, 3H), 2.03 (d, J=6.2 Hz, 1H).

### Example 91: preparation of a compound L-91

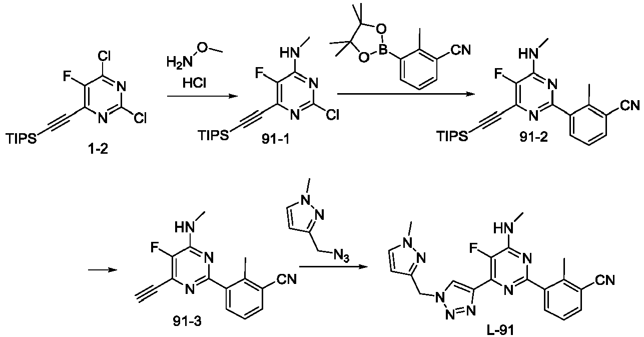

Step 1: referring to step 2 of Example 87, a compound 91-1 was prepared from the compound 1-2. MS (ESI) 342.1 [M+H]⁺.

Steps 2 to 4: referring to steps 3 to 5 of Example 1 by replacing the compound 1-3 with the compound 91-1 and replacing the intermediate V4 with 3-(azidomethyl)-1-methyl-1H-pyrazole, an off-white solid product L-91 (48.07 mg, purity: 99.22%, yield: 36.71%) was obtained. MS (ESI) 404.1 [M+H]⁺. ¹H NMR (400 MHz, DMSO-d6) δ 8.68 (s, 1H), 8.05 (d, J=7.0 Hz, 1H), 7.99-7.81 (m, 2H), 7.66 (s, 1H), 7.50 (s, 1H), 6.28 (s, 1H), 5.63 (s, 2H), 3.80 (s, 3H), 2.95 (d, J=4.2 Hz, 3H), 2.70 (s, 3H).

### Example 92: preparation of a compound L-92

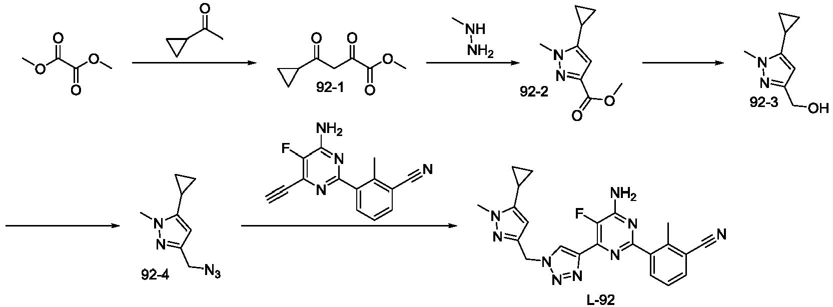

Step 1: potassium tert-butoxide (3.20 g, 28.53 mmol) was dissolved in an anhydrous tetrahydrofuran solution (10 mL), the reaction solution was slowly dropwise added to a mixed solution of 1-cyclopropanone (2 g, 23.78 mmol), dimethyl oxalate (3.09 g, 26.15 mmol), and toluene (20 mL) under ice bath conditions, and the reaction solution was slowly heated to the room temperature and stirred at the room temperature for 18 h. Then, HCl (1 mol/L, 15 mL) was added to the reaction solution under ice bath conditions, the mixture was stirred for 10 min and extracted three times with ethyl acetate (25 mL × 3), and a combined organic phase was dried with anhydrous sodium sulfate and spun under reduced pressure to dryness to obtain a compound 92-1 (3.18 g). MS (ESI) 171.1 [M+H]⁺.

Step 2: referring to step 1 of Example 51, a compound 92-2 was prepared from the compound 92-1. MS (ESI) 181.1 [M+H]⁺.

Steps 3 and 4: referring to steps 2 and 3 of Example 7, a compound 92-4 (243 mg) was prepared from the compound 92-2. MS (ESI) 178.1 [M+H]⁺.

Step 5: referring to step 5 of Example 1 by replacing the compound V4 with the compound 92-4, an off-white solid L-92 (16.91 mg, purity: 96.80%) was obtained. MS (ESI) 430.1 [M+H]⁺. ¹H NMR (400 MHz, DMSO-d6) δ 8.62 (s, 1H), 7.93 (d, J=7.6 Hz, 1H), 7.84 (d, J=6.7 Hz, 1H), 7.48 (dd, J=19.1, 11.3 Hz, 3H), 5.89 (s, 1H), 5.51 (s, 2H), 3.77 (s, 3H), 2.63 (s, 3H), 1.82 (s, 1H), 0.90 (dd, J=8.3, 2.2 Hz, 2H), 0.56 (dd, J=5.0, 2.2 Hz, 2H).

### Example 93: preparation of a compound L-93

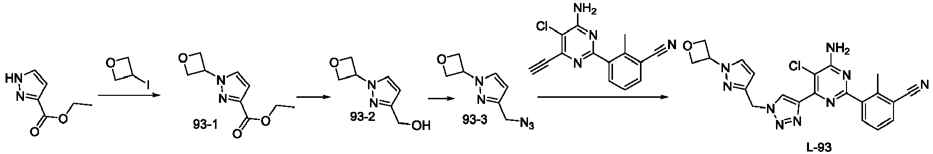

Step 1: referring to step 2 of Example 10, a colorless oily compound 93-1 (348 mg, purity: 100%, yield: 31.07%) was prepared from ethyl 1*H*-pyrazole-3-carboxylate (800 mg, 5.71 mmol) and 3-iodooxetane (1.37 g, 7.42 mmol). MS (ESI) 197.1 [M+H]⁺.

Steps 2 and 3: referring to steps 2 and 3 of Example 7, a compound 93-3 (295 mg) was prepared from the compound 93-1. MS (ESI) 180.1 [M+H]⁺.

Step 4: referring to step 5 of Example 1, a green solid product L-93 (6.02 mg, purity: 96.38%) was prepared from the compound 93-3 and the intermediate V8. MS (ESI) 448.1 [M+H]⁺. ¹H NMR (400 MHz, DMSO-d6) δ 8.81 (s, 1H), 7.99 (d, J=7.9 Hz, 1H), 7.92-7.84 (m, 2H), 7.55-7.45 (m, 2H), 6.35 (d, J=2.2 Hz, 1H), 5.70 (s, 2H), 5.55 (d, J=6.5 Hz, 2H), 4.87 (dt, J=16.0, 6.7 Hz, 4H), 2.67 (s, 3H).

### Example 94: preparation of a compound L-94

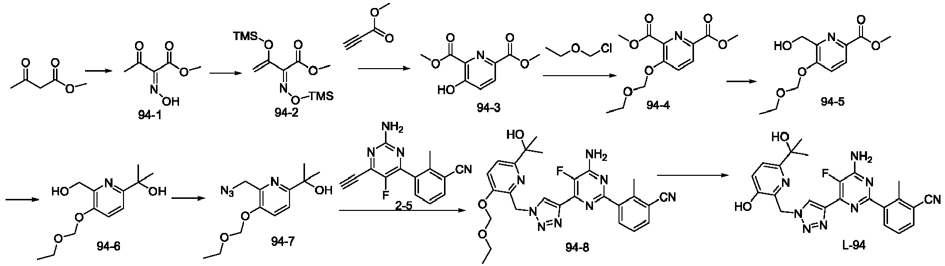

Step 1: methyl acetoacetate (31.4 g, 270.42 mmol) was dissolved in AcOH (34 mL), the reaction solution was cooled to 0°C, a solution of NaNO₂ (20.52 g, 297.46 mmol) in water (40 mL) was dropwise added to the reaction solution, after the solution of NaNO₂ was added, the reaction solution was naturally heated to the room temperature and stirred for 0.5 h, water (60 mL) was added, and the reaction solution was stirred for 16 h. After the reaction was completed, water (200 mL) was added to the reaction solution, the mixture was extracted with ethyl acetate, an obtained organic phase was washed with saturated sodium bicarbonate, washed with a saturated sodium chloride aqueous solution, dried with anhydrous sodium sulfate, and filtered, and an obtained filtrate was concentrated and purified by column chromatography (120 g, EA/PE=0 to 25%) to obtain a colorless oily compound 94-1 (27.6 g, yield: 70%). MS (ESI) 146.1 [M+H]⁺.

Step 2: the compound 94-1 (13.8 g, 95.10 mmol) and KI (8.68 g, 52.30 mmol) were dissolved in ACN (150 mL), TEA (20.21 g, 199.71 mmol) was added, the reaction solution was cooled to 0°C, TMSCl (21.70 g, 199.71 mmol) was dropwise added, and after TMSCl was added, the reaction solution was stirred at the room temperature for 18 h. Then, the reaction solution was filtered and concentrated, an obtained residue was dissolved in methyl tert-butyl ether, and the solution was filtered and concentrated to obtain a black oily compound 94-2 (27.53 g).

Step 3: the compound 94-2 (20.66 g, 71.37 mmol) and methyl propiolate (4 g, 47.58 mmol) were stirred at 150°C for 18 h, and the mixture was cooled to the room temperature and purified by column chromatography (120 g, EA/PE=0 to 30%) to obtain a pale-yellow solid compound 94-3 (3.2 g, yield: 32%). MS (ESI) 212.1 [M+H]⁺.

Step 4: the compound 94-3 (1 g, 4.74 mmol) and TEA (1.44 g, 14.21 mmol) were dissolved in DCM (35 mL), a DCM (2 mL) solution of (chloromethoxy)ethane (582.00 mg, 6.16 mmol) was dropwise added, and the reaction solution was stirred at the room temperature for 2 h. After the reaction was completed, the reaction solution was added to water, an organic phase was separated out and extracted with dichloromethane, obtained organic phases were combined, dried with anhydrous sodium sulfate, and filtered, and an obtained filtrate was concentrated to obtain a pale-yellow oily compound 94-4 (1.28 g). MS (ESI) 270.1 [M+H]⁺.

Step 5: referring to step 3 of Example 11, a pale-yellow oily compound 94-5 (421 mg, yield: 37%) was prepared from the compound 94-4 (1.28 g, 4.75 mmol). ¹H NMR (400 MHz, cdcl₃) δ 8.04 (1 H, d, J 8.6), 7.50 (1 H, d, J 8.5), 5.34 (2 H, s), 4.78 (2 H, s), 3.96 (3 H, s), 3.72 (2 H, q, J 7.1), 1.20 (3 H, t, J 7.1). MS (ESI) 242.1 [M+H]⁺.

Step 6: referring to step 3 of Example 10, a pale-yellow oily compound 94-6 (212 mg, yield: 54%) was prepared from the compound 94-5 (390.00 mg, 1.62 mmol). MS (ESI) 242.2 [M+H]⁺.

Step 7: referring to step 3 of Example 7, a pale-yellow oily compound 94-7 (324 mg) was prepared from the compound 94-6 (212 mg, 878.64 µmol). MS (ESI) 267.2 [M+H]⁺.

Step 8: referring to step 5 of Example 1 by replacing the compound V4 with the compound 94-7, an off-white solid compound 94-8 (201 mg, yield: 49%) was obtained. MS (ESI) 519.3 [M+H]⁺.

Step 9: the compound 94-8 (201 mg, 387.63 µmol) was dissolved in MeOH (15 mL), HCl (1 M, 5 mL) was added, and the reaction solution was stirred at 30°C for 10 h. Then, the reaction solution was spun under reduced pressure to dryness and separated by preparative high-performance liquid chromatography (HPLC) to obtain a white solid L-94 (60.12 mg, yield: 33%). MS (ESI) 461.2 [M+H]⁺. ¹H NMR (400 MHz, DMSO-d6) δ 10.17 (1 H, s), 8.62 (1 H, s), 7.94 (1 H, d, J 7.8), 7.84 (1 H, dd, J 7.7, 1.1), 7.52 (2 H, s), 7.46 (1 H, t, J 7.8), 7.42 (1 H, d, J 8.5), 7.21 (1 H, d, J 8.5), 5.71 (2 H, s), 4.98 (1 H, s), 2.64 (3 H, s), 1.27 (6 H, s).

### Example 96: preparation of a compound L-96

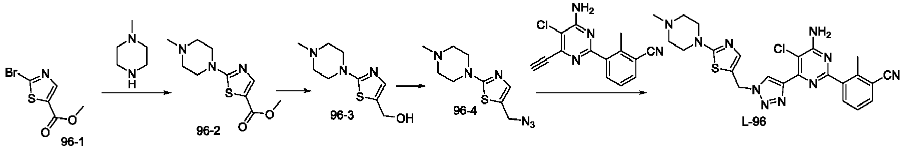

Referring to the preparation method of Example 7, a compound L-96 was prepared from a compound 96-1 and 1-methylpiperazine. MS (ESI) 507.1 [M+H]⁺. ¹H NMR (400 MHz, DMSO-d6) δ 8.79 (s, 1H), 7.98 (d, J=8 Hz, 1H), 7.88 (d, J=8 Hz, 1H), 7.47-7.51 (m, 1H), 7.31 (s, 1H), 5.76 (s, 2H), 3.34-3.37 (m, 4H), 2.67 (s, 3H), 2.36-2.38 (m, 4H), 2.19 (s, 3H).

### Example 97: preparation of a compound L-97

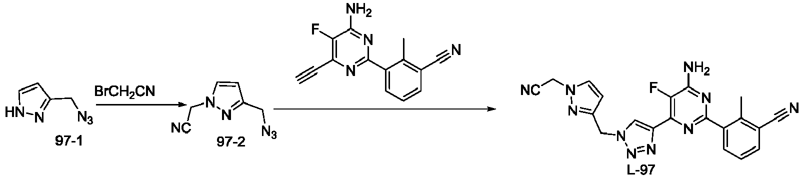

Step 1: referring to step 1 of Example 7, a compound 97-2 was prepared from a compound 97-1 and bromoacetonitrile.

Step 2: referring to step 5 of Example 1 by replacing the compound V4 with the compound 97-2, a compound L-97 was obtained. ¹H NMR (400 MHz, DMSO-d6) δ 8.71 (s, 1H), 7.92 (d, J=7.8 Hz, 1H), 7.83 (d, J=7.2 Hz, 1H), 7.53 (s, 3H), 7.45 (t, J=7.8 Hz, 1H), 6.36 (d, J=2.0 Hz, 1H), 5.67 (s, 2H), 5.45 (s, 2H), 2.62 (s, 3H). MS (ESI) 415.2 [M+H]⁺.

### Example 98: preparation of a compound L-98

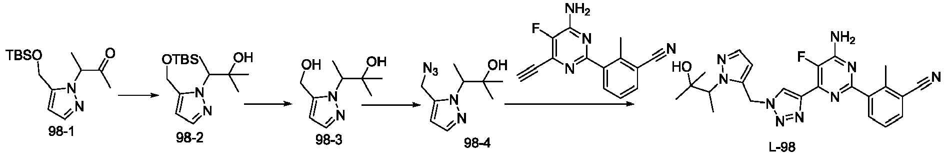

Step 1: referring to step 1 of Example 85, a compound 98-2 was prepared from a compound 98-1.

Step 2: referring to step 3 of Example 70, a compound 98-3 was prepared from the compound 98-2.

Step 3: referring to step 3 of Example 7, a compound 98-4 was prepared from the compound 98-3.

Step 4: referring to step 5 of Example 1 by replacing the compound V4 with the compound 98-4, a compound L-98 was obtained. ¹H NMR (400 MHz, DMSO-d6) δ 8.69 (s, 1H), 7.91 (d, J=6.8Hz, 1H), 7.82 (d, J=6.6 Hz, 1H), 7.52 (s, 2H), 7.47-7.36 (m, 2H), 6.23 (d, J=1.8 Hz, 1H), 5.86 (dd, J=38.4, 15.6 Hz, 2H), 5.72 (s, 1H), 4.64 (s, 1H), 4.48 (q, J=7.2 Hz, 1H), 2.61 (s, 3H), 1.31 (d, J=6.8 Hz, 3H), 1.04 (s, 3H), 0.95 (s, 3H). MS (ESI) 462.2 [M+H]⁺.

### Example 99: preparation of a compound L-99

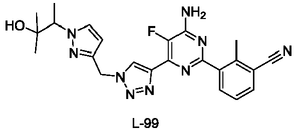

Referring to the preparation method of Example 98 by replacing the intermediate V6 with the intermediate V7, a compound L-99 was obtained. ¹H NMR (400 MHz, DMSO-d6) δ 8.57 (s, 1H), 7.91 (d, J=7.6 Hz, 1H), 7.82 (d, J=7.8 Hz, 1H), 7.64 (d, J=2.2 Hz, 1H), 7.59-7.33 (m, 3H), 6.23 (d, J=1.8 Hz, 1H), 5.61 (s, 2H), 4.56 (s, 1H), 4.16 (q, J=7.0 Hz, 1H), 2.61 (s, 3H), 1.37 (d, J=7.0 Hz, 3H), 0.96 (s, 3H), 0.92 (s, 3H). MS (ESI) 462.2 [M+H]⁺.

### Test 1: inhibitory activities to the A_{2A} receptor and the A_{2B} receptor

CHO-K1/ADORA2A/Gα15 (GenScript, M00246) and CHO-K1/ADORA2B/Gα15 (GenScript, M00329) cells were cultured in a Ham's F-12 (Gibco, 31765092) medium containing 10% FBS, Zeocin (200 ug/mL), and Hygromycin B (100 ug/mL), or containing 10% FBS, G418 (400 ug/mL), and Hygromycin B (100 ug/mL) under culture conditions specified in the corresponding instruction. Screening procedure was as follows:
1. The cell density was adjusted with a serum-free medium to 6×10⁵ cells/mL.
2. 5 uL of cell solution, 2.5 µL of NECA (Sigma, 119140-10MG), and 2.5 µL of compound solution were placed into each well of a 384-well plate (Greiner Bio-One, 784075) in sequence, the final concentration of NECA was 50 nM (CHO-K1/ADORA2A) or 10 nM (CHO-K1/ADORA2B), and the compound solution was subjected to 3-fold dilution from the final concentration of 3 uM.
3. The cells were placed still and cultured in an incubator at 37°C for 30 min.
4. 5 µL of cAMP-d₂ and 5 µL of cAMP-ab (Cisbio, 62AM4PEB) were added in sequence.
5. The 384-well plate was placed in dark at the room temperature for 1 h.
6. The plate was read (Victor X5, PerkinElmer), data were analyzed by XLfit nonlinear regression, and IC₅₀ values of the compounds were calculated.

**Table 1 Inhibitory activities of the compounds to the A_{2A} receptor and the A_{2B} receptor**

| Compound No. | A_{2A} receptor (IC₅₀/µM) | A_{2B} receptor (IC₅₀/µM) | Compound No. | A_{2A} receptor (IC₅₀/µM) | A_{2B} receptor (IC₅₀/µM) |
|---|---|---|---|---|---|
| L-1 | 0.001 | 0.003 | L-60 | 0.079 | 0.073 |
| L-2 | 0.062 | 0.029 | L-62 | 0.001 | 0.003 |
| L-3 | <0.001 | 0.004 | L-63 | 0.001 | 0.005 |
| L-4 | 0.001 | 0.006 | L-64 | 0.001 | 0.004 |
| L-5 | 0.004 | 0.006 | L-65 | 0.005 | 0.022 |
| L-7 | <0.001 | 0.007 | L-66 | 0.003 | 0.011 |
| L-8 | <0.001 | 0.003 | L-67 | 0.003 | 0.007 |
| L-9 | 0.001 | 0.001 | L-68 | 0.002 | 0.011 |
| L-10 | 0.004 | 0.001 | L-69 | 0.005 | 0.020 |
| L-11 | 0.001 | 0.014 | L-70 | 0.002 | 0.013 |
| L-12 | <0.001 | 0.004 | L-71 | 0.006 | 0.023 |
| L-13 | 0.001 | 0.013 | L-72 | 0.004 | 0.014 |
| L-14 | 0.017 | 0.014 | L-73 | 0.003 | 0.005 |
| L-17 | 0.001 | 0.007 | L-75 | <0.001 | 0.0005 |
| L-19 | 0.003 | 0.006 | L-76 | 0.005 | 0.007 |
| L-20 | 0.004 | 0.012 | L-77 | 0.001 | 0.003 |
| L-21 | 0.007 | 0.021 | L-78 | 0.003 | 0.003 |
| L-22 | 0.010 | 0.001 | L-79 | 0.019 | 0.025 |
| L-27 | 0.007 | 0.002 | L-80 | 0.015 | 0.013 |
| L-29 | 0.009 | 0.008 | L-81 | 0.004 | 0.023 |
| L-30 | <0.001 | 0.001 | L-83 | 0.003 | 0.007 |
| L-32 | <0.001 | 0.001 | L-84 | 0.004 | 0.024 |
| L-33 | 0.002 | 0.005 | L-85 | 0.002 | 0.004 |
| L-34 | 0.002 | 0.008 | L-86 | <0.001 | 0.001 |
| L-36 | 0.001 | 0.010 | L-88 | 0.002 | 0.008 |
| L-37 | 0.002 | 0.007 | L-89 | <0.001 | 0.005 |
| L-38 | 0.001 | 0.002 | L-90 | 0.005 | 0.029 |
| L-39 | 0.002 | 0.007 | L-92 | 0.011 | 0.005 |
| L-44 | 0.001 | 0.004 | L-93 | <0.001 | 0.004 |
| L-47 | 0.011 | 0.024 | L-97 | 0.006 | 0.005 |
| L-49 | 0.001 | 0.002 | | | |
| L-51 | 0.030 | 0.006 | | | |
| L-53 | 0.002 | 0.004 | | | |
| L-54 | 0.003 | 0.004 | | | |
| L-56 | 0.007 | 0.024 | | | |
| L-59 | 0.063 | 0.049 | | | |

It can be known from Table 1 that the compounds of the examples of the present disclosure have relatively high inhibitory activities to the A_{2A} receptor and the A_{2B} receptor.

### Test 2: inhibitory activities to the A_{2A} receptor and the A_{2B} receptor

CHO-K1/ADORA2A/Gα15 (GenScript, M00246) and CHO-K1/ADORA2B/Gα15 (GenScript, M00329) cells were cultured in a Ham's F-12 (Gibco, 31765092) medium containing 10% FBS, Zeocin (200 ug/mL), and Hygromycin B (100 ug/mL), or containing 10% FBS, G418 (400 ug/mL), and Hygromycin B (100 ug/mL) under culture conditions specified in the corresponding instruction. Screening procedure was as follows:
1. The cell density was adjusted with a serum-free medium to 6×10⁵ cells/mL.
2. 5 uL of cell solution, 2.5 µL of NECA (Sigma, 119140-10MG), and 2.5 µL of compound solution were placed into each well of a 384-well plate (Greiner Bio-One, 784075) in sequence, the final concentration of NECA was 1 uM (CHO-K1/ADORA2A) or 0.1 uM (CHO-K1/ADORA2B), and the compound solution was subjected to 3-fold dilution from the final concentration of 3 uM.
3. The cells were placed still and cultured in an incubator at 37°C for 30 min.
4. 5 µL of cAMP-d₂ and 5 µL of cAMP-ab (Cisbio, 62AM4PEB) were added in sequence.
5. The 384-well plate was placed in dark at the room temperature for 1 h.
6. The plate was read (Victor X5, PerkinElmer), data were analyzed by XLfit nonlinear regression, and IC₅₀ values of the compounds were calculated.

**Table 2 Inhibitory activities of the compounds to the A_{2A} receptor and the A_{2B} receptor**

| Compound No. | A_{2A} receptor (IC₅₀/µM) | A_{2B} receptor (IC₅₀/µM) | Compound No. | A_{2A} receptor (IC₅₀/µM) | A_{2B} receptor (IC₅₀/µM) |
|---|---|---|---|---|---|
| L-1 | 0.024 | 0.062 | L-44 | 0.022 | 0.064 |
| L-38 | 0.018 | 0.050 | L-89 | 0.011 | 0.068 |
| D1 | 0.065 | 0.075 | L-94 | 0.016 | 0.038 |

The compound D1 (CAS No.: 2239273-34-6, supplier: Suzhou Chukai PharmaTech Co., Ltd., batch No.: 2009010AHP07) has the following structure:

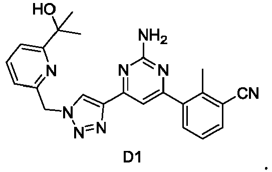

### Test 3: in-vivo pharmacokinetics test

Mice were respectively administrated with the compound of the present disclosure by intravenous injection or intragastric administration, and the concentration of the drug in the plasma at different time points was determined by LC/MS/MS to explore pharmacokinetic behavior of the compound of the present disclosure in the mouse so as to evaluate the pharmacokinetic characteristics of the compound.

### Experimental scheme

Experimental animals: healthy adult male ICR mice (weight: 25 to 40 g, 12 mice, mice in the intravenous injection group had free access to water and food, and mice in the intragastric administration group fasted overnight and had free access to water and food 4 h after administration), provided by Beijing Vital River Laboratory Animal Co., Ltd.

Administration method and dosage: before administration, animals that met the experimental requirements were selected, weighed, and marked. The ICR mice were subjected to tail vein administration (2 mg/kg, 5% DMSO, pH=4.5, 20% Captisol) or intragastric administration (10 mg/kg, 5% DMSO, pH=4.5, 20% Captisol).

Blood sample collection: before blood sample collection, each mouse was tied, and about 100 µL of blood was collected from each mouse subjected to administration at a preset blood collection time point through the jugular vein (for the mice subjected to intravenous administration: blood was collected from each mouse at a total of 9 time points, i.e., 0.083 h, 0.25 h, 0.5 h, 1 h, 2 h, 4 h, 6 h, 7.5 h, and 24 h after administration; and for the mice subjected to intragastric administration: blood was collected from each mouse at a total of 9 time points, i.e., 0.083 h, 0.25 h, 0.5 h, 1 h, 2 h, 4 h, 6 h, 7.5 h, and 24 h after administration). The blood was transferred to a test tube (1.5 mL) containing K2EDTA and centrifuged for 4 min (8,000 rpm, 4°C) to separate out plasma, and the whole process was completed within 15 min after blood collection. All samples were stored in a refrigerator at -20°C before sample analysis. The concentration of the drug was determined by LC/MS/MS.

Pharmacokinetic property parameters of the compounds of some examples of the present disclosure in the mice under the conditions of same dosage and intravenous administration are shown in Table 3:

**Table 3**

| Compound No. | Clearance rate CLz (mL/min/kg) | Area under the curve AUC₀₋ₜ (hr*ng/mL) | Compound No. | Clearance rate CLz (mL/min/kg) | Area under the curve AUC₀₋ₜ (hr*ng/mL) |
|---|---|---|---|---|---|
| L-1 | 14.7 | 2252 | L-32 | 13.3 | 2506 |
| L-3 | 9.12 | 3603 | L-38 | 12.7 | 2587 |
| L-9 | 6.97 | 4676 | L-67 | 14.2 | 2311 |
| L-10 | 12.1 | 2736 | D1 | 19.0 | 1646 |

Pharmacokinetic property parameters of the compounds of some examples of the present disclosure in the mice under the conditions of same dosage and intragastric administration are shown in Table 4:

**Table 4**

| Compound No. | Cmax (ng/mL) | Area under the curve AUClast (hr*ng/mL) | Compound No. | Cmax (ng/mL) | Area under the curve AUClast (hr*ng/mL) |
|---|---|---|---|---|---|
| L-1 | 8730 | 10446 | L-32 | 8983 | 14801 |
| L-3 | 15933 | 22213 | L-38 | 7210 | 13329 |
| L-9 | 6793 | 23798 | L-67 | 5717 | 17323 |
| L-10 | 12607 | 18029 | D1 | 5957 | 10291 |

### Test 4: in-vivo pharmacodynamics test of the compounds of the present disclosure

Experimental scheme: a mouse model of subcutaneously transplanted tumor of a mouse colon cancer cell line MC38(#22)-hpd-L1 was used, and orally administrated with the compound of the present disclosure, and the efficacy of the compound in the tumor-bearing mouse model of colon cancer MC38(#22)-hpd-L1 was tested.

Experimental materials: C57BL/6 mice (female); and mouse colon cancer cells MC38(#22)-hpd-L1 (Cell Bank, Shanghai JiaoTong University). The cells were subjected to monolayer culture in vitro, an RPMI-1640 medium containing 10% fetal bovine serum was used, and the cells were cultured in an incubator with 5% CO₂ at 37°C. The cells were sub-cultured by routine digestion with trypsin-EDTA. When the cells grew to exponential phase and the saturation was 80% to 90%, the cells were collected and counted.

Preparation of compounds: the compound was weighed and added to a solvent (40% captisol in acetic acid buffer (pH=3.8)) to form a sample (10 mg/mL). 75 µL of Tecentriq solution (60 mg/mL) was added to 8.925 mL of phosphate buffer solution (PBS) to form a solution (0.5 mg/mL).

Experimental operation: the cells were resuspended in a phosphate buffer solution according to a density of 5×10⁶ cells/mL. 0.1 mL of PBS (containing 5×10⁵ MC38(#22)-hpd-L1 cells) was subcutaneously inoculated into the right back of each mouse, and the mice were randomly grouped (10 mice/group) on the day of inoculation according to weight and administrated with the drug twice a day for continuous 22 days. The animals were weighed and the health was monitored daily throughout the experimental period. Tumor diameter was measured twice a week by using a vernier caliper.

A formula for calculating tumor volume: V=0.5×a×b², where, a and b represent a long diameter and a short diameter of tumor, respectively .

The tumor-inhibitory efficacy of the compound was evaluated by relative tumor proliferation rate T/C (%). Relative tumor proliferation rate T/C (%)=Vt/Vc×100% (Vt: average tumor volume of the treatment group; and Vc: average tumor volume of the negative control group). Values of Vt and Vc of the same day were taken.

**Table 5 Relative tumor proliferation rates of the compounds of the present disclosure**

| Compound No. | Dosage (mg/kg) | Tumor volume (mm³) | T/C (%) |
|---|---|---|---|
| Solvent group | / | 1191 | / |
| L-1 | 10 | 899 | 75.5 |
| | 30 | 405 | 34.0 |
| | 100 | 286 | 24.0 |
| D1 | 100 | 902 | 75.8 |

All documents mentioned herein are incorporated by reference in the present disclosure as if each document was individually incorporated by reference. In addition, it should be understood that after reading the above teaching content of the present disclosure, those skilled in the art can make various changes or modifications to the present disclosure, and these equivalent forms also fall within the scope defined by the appended claims of the present disclosure.

## Claims

1. A compound represented by Formula (I), or a pharmaceutically acceptable salt, stereoisomer, solvate, or prodrug thereof: 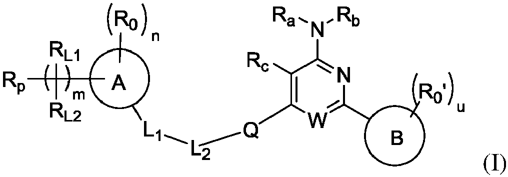 wherein
the ring A is five- to ten-membered heteroaryl, phenyl, or pyridonyl;
the ring B is phenyl or five- to ten-membered heteroaryl;
Q is five- or six-membered heteroaryl, phenyl, C₃₋₆ cycloalkyl, four- to eight-membered heterocycloalkyl, six- to twelve-membered fused heterocycloalkyl, seven- to eleven-membered benzoheterocycloalkyl, seven- to eleven-membered heteroaryl-fused heterocycloalkyl, seven- to eleven-membered spirocyclyl, or seven- to eleven-membered heterospirocyclyl, wherein the five- or six-membered heteroaryl, the phenyl, the C₃₋₆ cycloalkyl, the four- to eight-membered heterocycloalkyl, the six- to twelve-membered fused heterocycloalkyl, the seven- to eleven-membered benzoheterocycloalkyl, the seven- to eleven-membered heteroaryl-fused heterocycloalkyl, the seven- to eleven-membered spirocyclyl, and the seven- to eleven-membered heterospirocyclyl are unsubstituted or substituted with one, two, or three substituents each independently selected from the group consisting of deuterium, halogen, cyano, hydroxyl, carboxyl, oxo, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, NRₐ₀R_{b0}, -C(O)(C₁₋₃ alkyl), -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
L₁ and L₂ are each independently a bond, NR₁', CR₂'R₃', O, S, or C(O), provided that L₁ and L₂ are not both O or S;
R₁', R₂', and R₃' are each independently hydrogen, deuterium, cyano, hydroxyl, halogen, preferably fluorine or chlorine, or C₁₋₆ alkyl; or R₁' is linked to R₂' to form a three- to eight-membered heterocycloalkyl ring, or R₂' is linked to R₃' to form a three- to eight-membered heterocycloalkyl ring, wherein the three- to eight-membered heterocycloalkyl ring is unsubstituted or substituted with one, two, or three substituents each independently selected from the group consisting of deuterium, halogen, cyano, hydroxyl, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
W is N or CR_{w};
R_{w} is cyano, hydroxyl, halogen, halogenated C₁₋₈ alkyl, halogenated C₁₋₈ alkoxyl, or C₁₋₈ alkoxyl;
R_{c}, Rₐ, and R_{b} are defined as follows:
(i) R_{c} is fluorine, chlorine, cyano, C₁₋₃ alkoxyl, or halogenated C₁₋₃ alkoxyl;
Rₐ and R_{b} are each independently hydrogen, deuterium, C₁₋₈ alkyl, C₁₋₈ alkoxyl, -C(O)(C₁₋₈ alkyl), -(CH₂)ₜ(C₃₋₈ cycloalkyl), -(CH₂)ₜ-(three- to eight-membered 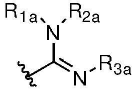 heterocycloalkyl), or a structure represented by Formula (a): Formula (a) , wherein the C₁₋₈ alkyl, the -C(O)(C₁₋₈ alkyl), and the -(CH₂)ₜ-(three- to eight-membered heterocycloalkyl) are unsubstituted or substituted with one, two, or three substituents each independently selected from the group consisting of deuterium, halogen, cyano, hydroxyl, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl; or
Rₐ and R_{b} together with the nitrogen atom linked thereto form a five- or six-membered saturated or partially unsaturated heteromonocyclic ring, wherein the five- or six-membered saturated or partially unsaturated heteromonocyclic ring is unsubstituted or substituted with one, two, or three substituents each independently selected from the group consisting of deuterium, halogen, cyano, hydroxyl, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl,
wherein R₁ₐ is hydrogen or C₁₋₃ alkyl; and R₂ₐ and R₃ₐ are each independently hydrogen or C₁₋₃ alkyl, or R₂ₐ is linked to R₃ₐ to form a five- to eight-membered heterocycloalkenyl ring or a five- or six-membered heteroaryl ring, wherein the five- to eight-membered heterocycloalkenyl ring contains two, three, or four nitrogen atoms and zero, one, or two oxygen atoms; the five- or six-membered heteroaryl ring contains two, three, or four nitrogen atoms and zero or one oxygen atom; and the five- to eight-membered heterocycloalkenyl ring and the five- or six-membered heteroaryl ring are unsubstituted or substituted with one, two, or three substituents each independently selected from the group consisting of halogen, cyano, hydroxyl, C₁₋₃ alkyl, C₁₋₃ alkoxyl, halogenated C₁₋₃ alkyl, and halogenated C₁₋₃ alkoxyl; or
(ii) R_{c} is linked to Rₐ to form a fused five- or six-membered saturated or partially unsaturated heteromonocyclic ring, or a fused five- or six-membered heteroaryl ring, wherein the fused five- or six-membered saturated or partially unsaturated heteromonocyclic ring and the fused five- or six-membered heteroaryl ring are unsubstituted or substituted with one, two, or three substituents each independently selected from the group consisting of deuterium, halogen, cyano, hydroxyl, oxo, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, -SO₂(C₁₋₃ alkyl), -S(O)(C₁₋₃ alkyl), -C(O)NRₐ₀R_{b0}, -C(O)O(C₁₋₃ alkyl), C₃₋₆ cycloalkyl, C₃₋₆ cycloalkyloxy, three- to six-membered heterocycloalkyl, phenyl, and five- or six-membered heteroaryl;
R_{b} is hydrogen, deuterium, C₁₋₈ alkyl, -C(O)(C₁₋₈ alkyl), -(CH₂)ₜ(C₃₋₈ cycloalkyl), -(CH₂)ₜ-(three- to eight-membered heterocycloalkyl), or a structure represented by Formula (a), wherein the C₁₋₈ alkyl, the -C(O)(C₁₋₈ alkyl), and the -(CH₂)ₜ-(three- to eight-membered heterocycloalkyl) are unsubstituted or substituted with one, two, or three substituents each independently selected from the group consisting of deuterium, halogen, cyano, hydroxyl, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
t is 0, 1, 2, or 3;
R_{L1} and R_{L2} are each independently hydrogen, hydroxyl, halogen, C₁₋₃ alkyl, C₁₋₃ alkoxyl, or halogenated C₁₋₃ alkyl; or R_{L1} and R_{L2} together with the carbon atom linked thereto form a three- to seven-membered saturated or partially unsaturated monocyclic ring or a three- to seven-membered saturated or partially unsaturated heteromonocyclic ring, wherein the three-to seven-membered saturated or partially unsaturated monocyclic ring and the three- to seven-membered saturated or partially unsaturated heteromonocyclic ring are unsubstituted or substituted with one, two, or three substituents each independently selected from the group consisting of deuterium, halogen, cyano, hydroxyl, carboxyl, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
m is 0, 1, 2, or 3;
Rₚ is hydrogen, hydroxyl, carboxyl, cyano, C₁₋₈ alkyl, halogenated C₁₋₈ alkyl, halogenated C₁₋₈ alkoxyl, C₁₋₈ alkoxyl, C₃₋₈ cycloalkyl, -SO₂(C₁₋₈ alkyl), -SO₂NRₐ₀R_{b0}, -(PO)(C₁₋₃ alkyl)₂, -NHSO₂(C₁₋₃ alkyl), -C(O)NRₐ₀R_{b0}, -C(O)NRₐ₁R_{b1}, three- to six-membered heterocycloalkyl, -X-(CRₚ₁Rₚ₂)_{q}-(three- to six-membered heterocycloalkyl), eight- to ten-membered heterocycloalkyl, seven- to eleven-membered heterospirocyclyl, five- or six-membered heteroaryl, eight- to ten-membered heteroaryl, NRₐ₁R_{b1}, or NRₐ'R_{b}', wherein the C₃₋₈ cycloalkyl, the three- to six-membered heterocycloalkyl, the eight- to ten-membered heterocycloalkyl, the seven- to eleven-membered heterospirocyclyl, the five- or six-membered heteroaryl, and the eight- to ten-membered heteroaryl are unsubstituted or substituted with one, two, or three substituents each independently selected from the group consisting of deuterium, halogen, cyano, hydroxyl, carboxyl, oxo, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, NRₐ₀R_{b0}, -C(O)(C₁₋₃ alkyl), -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
X is O or NRₚ₃;
Rₚ₁ and Rₚ₂ are each independently hydrogen, hydroxyl, halogen, or C₁₋₃ alkyl;
Rₚ₃ is hydrogen or C₁₋₃ alkyl;
q is 0, 1, 2, or 3;
Rₐ' and R_{b}' together with the nitrogen atom linked thereto form a four- to eight-membered saturated heteromonocyclic ring, an eight- to ten-membered saturated heterobicyclic ring, or a seven- to eleven-membered heterospiro ring, wherein the four- to eight-membered saturated heteromonocyclic ring, the eight- to ten-membered saturated heterobicyclic ring, and the seven- to eleven-membered heterospiro ring are unsubstituted or substituted with one, two, or three substituents each independently selected from the group consisting of halogen, cyano, hydroxyl, carboxyl, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, -(CH₂)ₜ₁-NRₐ₀R_{b0}, -(CH₂)ₜ₁-SO₂(C₁₋₃ alkyl), -(CH₂)ₜ₁-S(O)(C₁₋₃ alkyl), -(CH₂)ₜ₁-C(O)NRₐ₀R_{b0}, -(CH₂)ₜ₁-C(O)O(C₁₋₃ alkyl), -(CH₂)ₜ₁-OC(O)(C₁₋₃ alkyl), -(CH₂)ₜ₁-(C₃₋₆ cycloalkyl), C₃₋₆ cycloalkyloxy, and -(CH₂)ₜ₁-(three- to six-membered heterocycloalkyl);
t1 is independently selected from 0, 1, 2, or 3;
(R₀)ₙ refers to n substituents R₀ replacing n hydrogen atoms on the ring A, where n is 0, 1, 2, or 3, wherein the n substituents R₀ are identical or different from each other, the n substituents R₀ being each independently halogen, cyano, hydroxyl, carboxyl, C₁₋₃ alkyl, hydroxyl-substituted C₁₋₃ alkyl, C₁₋₃ alkoxyl-substituted C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, -SO₂(C₁₋₃ alkyl), -SO₂NRₐ₀R_{b0}, -N(Rₐ₀)SO₂(C₁₋₃ alkyl), -P(O)Rₐ₀R_{b0}, -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, or three- to six-membered heterocycloalkyl;
(R₀')ᵤ refers to u substituents R₀' replacing u hydrogen atoms on the ring B, where u is 0, 1, 2, 3, 4, or 5, wherein the u substituents R₀' are identical or different from each other, the u substituents R₀' being each independently hydrogen, cyano, acetyl, hydroxyl, carboxyl, halogen, NRₐ₀R_{b0}, C₁₋₈ alkyl, Ci-s alkoxyl, C₃₋₈ cycloalkyl, three- to six-membered heterocycloalkyl, five- or six-membered heteroaryl, or C₆₋₁₀ aryl, wherein the C₁₋₈ alkyl, the C₁₋₈ alkoxyl, the C₃₋₈ cycloalkyl, the three- to six-membered heterocycloalkyl, the five- or six-membered heteroaryl, and the C₆₋₁₀ aryl are unsubstituted or substituted with one, two, or three substituents each independently selected from the group consisting of deuterium, halogen, cyano, hydroxyl, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, C₃₋₆ cycloalkyl, C₃₋₆ cycloalkyloxy, three- to six-membered heterocycloalkyl, phenyl, and five- or six-membered heteroaryl;
Rₐ₀ and R_{b0} are each independently hydrogen or C₁₋₃ alkyl; or Rₐ₀ and R_{b0} together with the nitrogen atom linked thereto form a four- to six-membered saturated heteromonocyclic ring, wherein the four- to six-membered saturated heteromonocyclic ring is optionally substituted with one, two, or three substituents each independently selected from the group consisting of deuterium, halogen, cyano, hydroxyl, carboxyl, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl;
Rₐ₁ and R_{b1} are each independently hydrogen, C₁₋₃ alkyl, C₃₋₆ cycloalkyl, three- to six-membered heterocycloalkyl, or -(CR^{a}R^{b})ₛ-R^{c}, wherein the three- to six-membered heterocycloalkyl is unsubstituted or substituted with one, two, or three substituents each independently selected from the group consisting of deuterium, halogen, cyano, hydroxyl, carboxyl, oxo, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, -SO₂(C₁₋₃ alkyl), -S(O)(C₁₋₃ alkyl), -C(O)NRₐ₀R_{b0}, -C(O)(C₁₋₃ alkyl), -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
R^{a} and R^{b} are each independently hydrogen, hydroxyl, halogen, C₁₋₃ alkyl, C₁₋₃ alkoxyl, or halogenated C₁₋₃ alkyl; or R^{a} and R^{b} together with the carbon atom linked thereto form a three- to seven-membered saturated or partially unsaturated monocyclic ring or a three- to seven-membered saturated or partially unsaturated heteromonocyclic ring, wherein the three-to seven-membered saturated or partially unsaturated monocyclic ring and the three- to seven-membered saturated or partially unsaturated heteromonocyclic ring are unsubstituted or substituted with one, two, or three substituents each independently selected from the group consisting of deuterium, halogen, cyano, hydroxyl, carboxyl, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, three- to six-membered heterocycloalkyl, phenyl, and five- or six-membered heteroaryl;
R^{c} is hydrogen, hydroxyl, carboxyl, C₁₋₈ alkyl, halogenated C₁₋₈ alkyl, halogenated C₁₋₈ alkoxyl, C₁₋₈ alkoxyl, or C₃₋₈ cycloalkyl; and
s is 1, 2, or 3.

2. The compound, or the pharmaceutically acceptable salt, stereoisomer, solvate, or prodrug thereof according to claim 1, wherein the ring A is phenyl, pyridyl, pyrazolyl, pyrimidinyl, 1,2,3- triazolyl, 1,2,4- triazolyl, or pyridinonyl.

3. The compound, or the pharmaceutically acceptable salt, stereoisomer, solvate, or prodrug thereof according to claim 1, wherein R_{c} is fluorine, chlorine, cyano, hydroxyl, methyl, ethyl, n-propyl, isopropyl, methoxyl, ethoxyl, n-propoxyl, or isopropoxyl.

4. The compound, or the pharmaceutically acceptable salt, stereoisomer, solvate, or prodrug thereof according to claim 1, wherein Q is five- or six-membered heteroaryl, phenyl, C₃₋₆ cycloalkyl, four- to six-membered heterocycloalkyl, eight- to ten-membered fused heterocycloalkyl, eight- to ten-membered benzoheterocycloalkyl, eight- to ten-membered heteroaryl-fused heterocycloalkyl, seven- to elven-membered spirocyclyl, or seven- to eleven-membered heterospirocyclyl.

5. The compound, or the pharmaceutically acceptable salt, stereoisomer, solvate, or prodrug thereof according to claim 1, wherein Rₐ and R_{b} are each independently hydrogen, deuterium, C₁₋₃ alkyl, -C(O)(C₁₋₃ alkyl), -(CH₂)ₜ(C₃₋₆ cycloalkyl), -(CH₂)ₜ-(four- to six-membered heterocycloalkyl), or a structure represented by Formula (a); or Rₐ and R_{b}, together with the nitrogen atom linked thereto form a five- or six-membered saturated heteromonocyclic ring, wherein the C₁₋₃ alkyl, the -C(O)(C₁₋₃ alkyl), the -(CH₂)ₜ-(four- to six-membered heterocycloalkyl), and the five- or six-membered saturated heteromonocyclic ring are unsubstituted or substituted with one, two, or three substituents each independently selected from the group consisting of deuterium, halogen, cyano, hydroxyl, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, -SO₂(C₁₋₃ alkyl), -S(O)(C₁₋₃ alkyl), -C(O)NRₐ₀R_{b0}, -C(O)O(C₁₋₃ alkyl), -OC(O)(C₁₋₃ alkyl), C₃₋₆ cycloalkyl, C₃₋₆ cycloalkyloxy, and four- to six-membered heterocycloalkyl, wherein the four-to six-membered heterocycloalkyl is selected from the group consisting of azetidinyl, oxetanyl, tetrahydrofuranyl, tetrahydrothienyl, tetrahydropyrrolyl, oxazolidinyl, dioxolanyl, piperidinyl, piperazinyl, morpholinyl, dioxanyl, thiomorpholinyl, thiomorpholine-1,1-dioxide, and tetrahydropyranyl; the C₃₋₆ cycloalkyl is selected from the group consisting of cyclopropyl, cyclobutyl, cyclopentyl, and cyclohexyl; and the five- or six-membered saturated heteromonocyclic ring is selected from the group consisting of a tetrahydrofuran ring, a tetrahydrothiophene ring, a tetrahydropyrrole ring, a piperidine ring, oxazolidine, a piperazine ring, dioxolane, dioxane, a morpholine ring, a thiomorpholine ring, and a tetrahydropyran ring.

6. The compound, or the pharmaceutically acceptable salt, stereoisomer, solvate, or prodrug thereof according to claim 1, wherein W is N.

7. The compound, or the pharmaceutically acceptable salt, stereoisomer, solvate, or prodrug thereof according to claim 1, wherein Q is

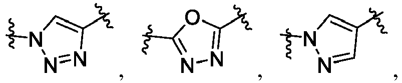

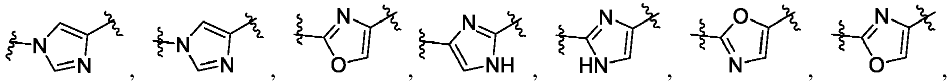

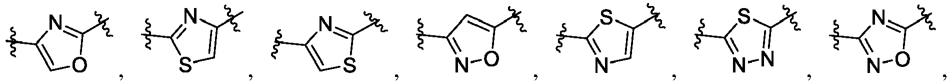

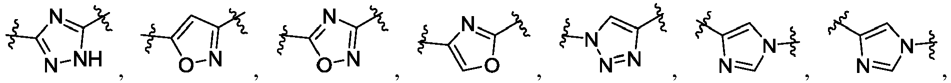

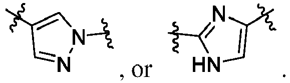

8. The compound, or the pharmaceutically acceptable salt, stereoisomer, solvate, or prodrug thereof according to claim 1, wherein L₁ is a bond; and L₂ is CR₂'R₃'.

9. The compound, or the pharmaceutically acceptable salt, stereoisomer, solvate, or prodrug thereof according to claim 1, wherein:
the ring B is phenyl;
(R₀')ᵤ refers to u substituents R₀' replacing u hydrogen atoms on the ring B, where u is 1 or 2, wherein the u substituents R₀' are identical or different frome each other, the u substituents R₀' being each independently hydrogen, cyano, halogen, or Ci-s alkyl.

10. The compound, or the pharmaceutically acceptable salt, stereoisomer, solvate, or prodrug thereof according to claim 1, wherein:
R_{L1} and R_{L2} are each independently hydrogen, hydroxyl, halogen, C₁₋₃ alkyl, C₁₋₃ alkoxyl, or halogenated C₁₋₃ alkyl;
m is 0, 1 or 2;
Rₚ is hydrogen, hydroxyl, carboxyl, cyano, C₁₋₆ alkyl, halogenated C₁₋₆ alkyl, C₁₋₆ alkoxyl, C₃₋₆ cycloalkyl, -SO₂(C₁₋₆ alkyl), -(PO)(C₁₋₃ alkyl)₂, -NHSO₂(C₁₋₃ alkyl), -C(O)NRₐ₀R_{b0}, three- to six-membered heterocycloalkyl, or NRₐ₁R_{b1}, wherein the C₃₋₆ cycloalkyl and the three- to six-membered heterocycloalkyl are unsubstituted or substituted with one or two substituents each independently selected from halogen, hydroxyl, or C₁₋₃ alkyl;
Rₐ₀ and R_{b0} are each independently hydrogen or C₁₋₃ alkyl; and
Rₐ₁ and R_{b1} are each independently hydrogen or C₁₋₃ alkyl.

11. The compound, or the pharmaceutically acceptable salt, stereoisomer, solvate, or prodrug thereof according to claim 1, wherein (R₀)ₙ refers to n substituents R₀ replacing n hydrogen atoms on the ring A, where n is 0 or 1, wherein R₀ is halogen, hydroxyl, C₁₋₃ alkyl, C₁₋₃ alkoxyl, hydroxyl-substituted C₁₋₃ alkyl, C₁₋₃ alkoxyl-substituted C₁₋₃ alkyl, or C₃₋₆ cycloalkyl.

12. The compound, or the pharmaceutically acceptable salt, stereoisomer, solvate, or prodrug thereof according to claim 1, wherein the compound has a structure represented by Formula (II) or Formula (III): 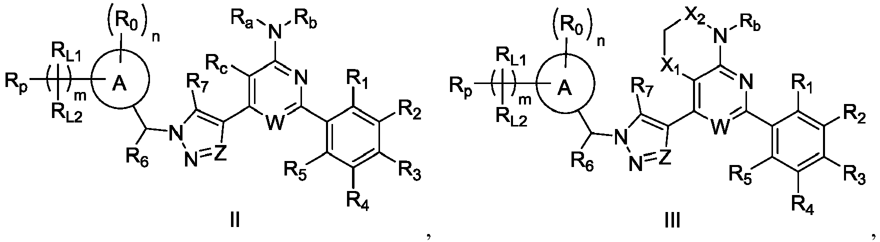 where:
R_{c}, Rₐ, and R_{b} in Formula (II) are as defined in (i); and R_{b} in Formula (III) is as defined in (ii);
X₁ is O, NR₁₁, or CR₁₂R₁₃;
X₂ is C(O) or CR₂₁R₂₂;
R₁₁ is hydrogen or C₁₋₃ alkyl;
R₁₂, R₁₃, R₂₁, and R₂₂ are each independently hydrogen, halogen, C₁₋₃ alkyl, or C₁₋₃ alkoxyl;
Z is N or CRz; and Rz is hydrogen, deuterium, or C₁₋₃ alkyl;
R₁, R₂, R₃, R₄, and R₅ are each independently hydrogen, cyano, acetyl, hydroxyl, carboxyl, halogen, NRₐ₀R_{b0}, C₁₋₈ alkyl, C₁₋₈ alkoxyl, C₃₋₈ cycloalkyl, three- to six-membered heterocycloalkyl, five- or six-membered heteroaryl, or C₆₋₁₀ aryl, wherein the C₁₋₈ alkyl, the C₁₋₈ alkoxyl, the C₃₋₈ cycloalkyl, the three- to six-membered heterocycloalkyl, the five- or six-membered heteroaryl, and the C₆₋₁₀ aryl are unsubstituted or substituted with one, two, or three substituents each independently selected from the group consisting of deuterium, halogen, cyano, hydroxyl, C₁₋₃ alkyl, C₁₋₃ alkoxyl, C₂₋₄ alkenyl, C₂₋₄ alkynyl, halogenated C₁₋₃ alkyl, halogenated C₁₋₃ alkoxyl, C₃₋₆ cycloalkyl, C₃₋₆ cycloalkyloxy, three- to six-membered heterocycloalkyl, phenyl, and five- or six-membered heteroaryl; and
R₆ and R₇ are each independently hydrogen, deuterium, cyano, hydroxyl, halogen, or C₁₋₆ alkyl.

13. The compound, or the pharmaceutically acceptable salt, stereoisomer, solvate, or prodrug thereof according to claim 1, wherein

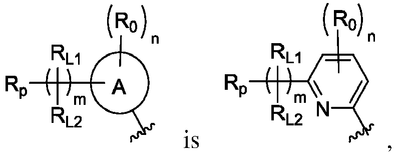

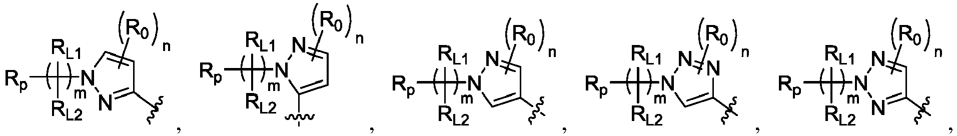

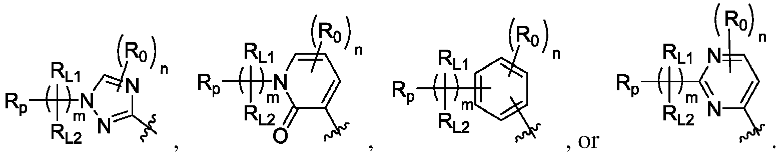

14. The compound, or the pharmaceutically acceptable salt, stereoisomer, solvate, or prodrug thereof according to claim 1, wherein the compound represented by Formula (I) is any one of the following compounds: 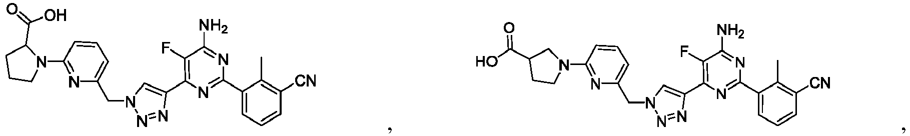 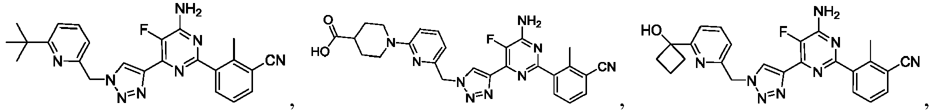 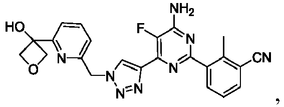 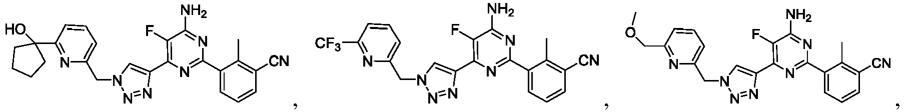 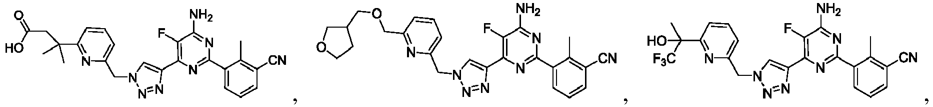 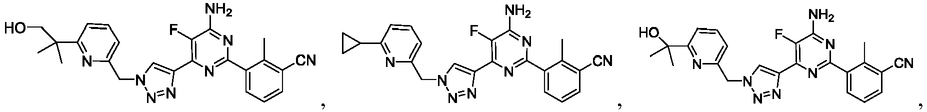 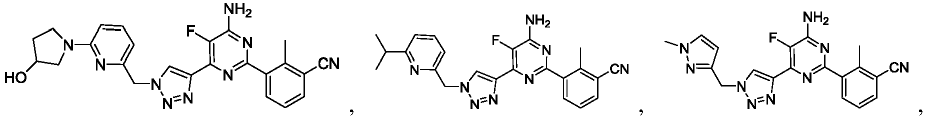 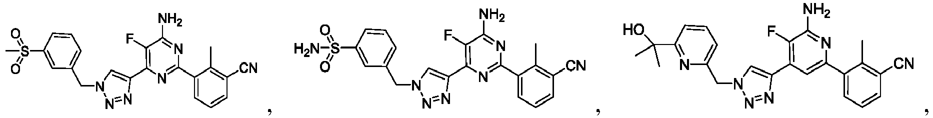 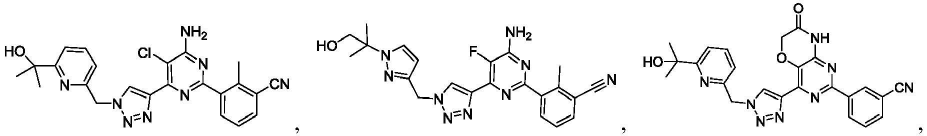 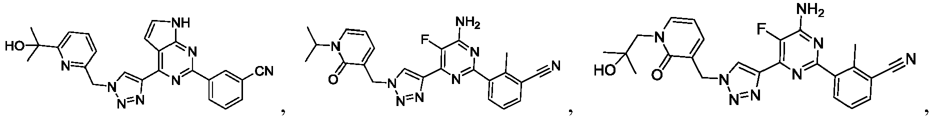 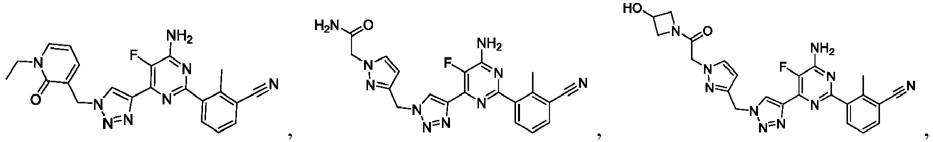 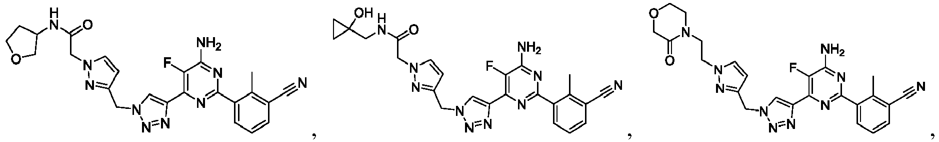 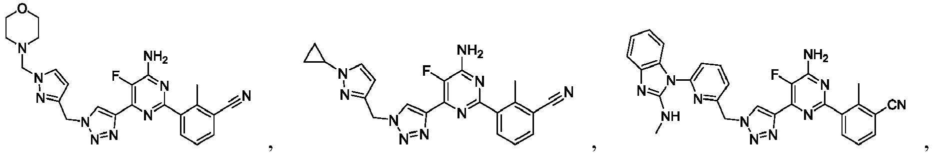 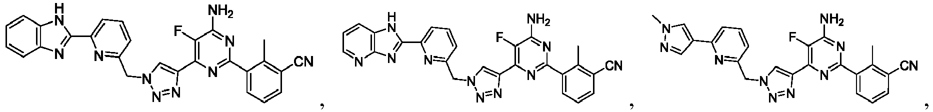 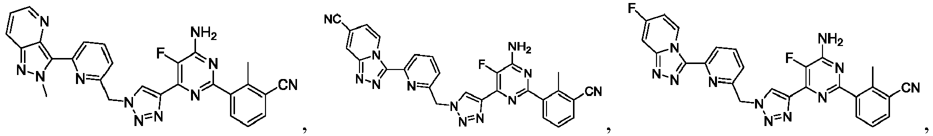 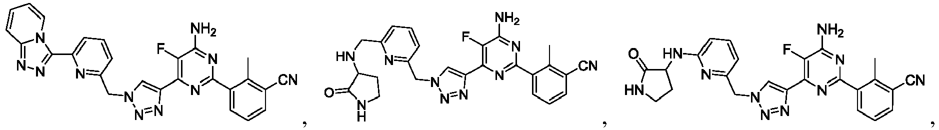 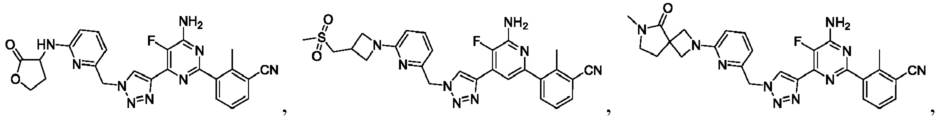 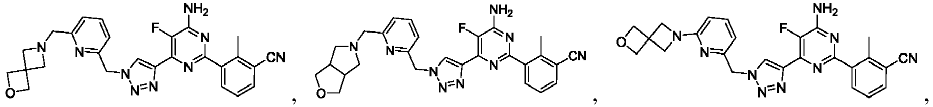 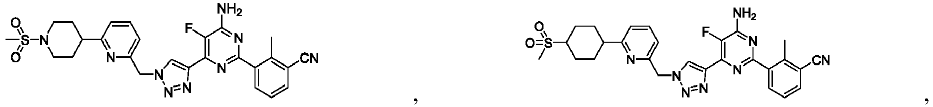 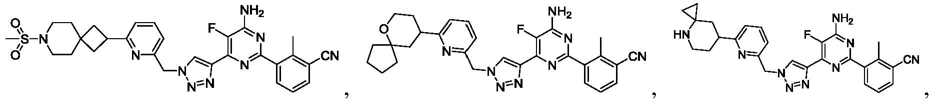 and

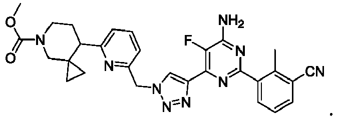

15. The compound, or the pharmaceutically acceptable salt, stereoisomer, solvate, or prodrug thereof according to claim 1, wherein the compound represented by Formula (I) is any one of the following compounds: 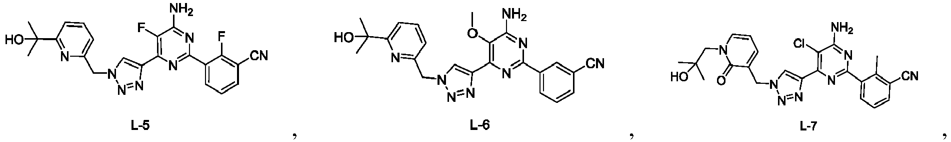 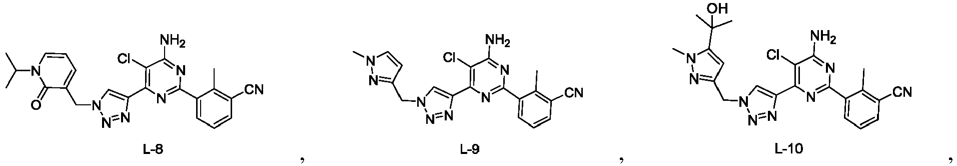 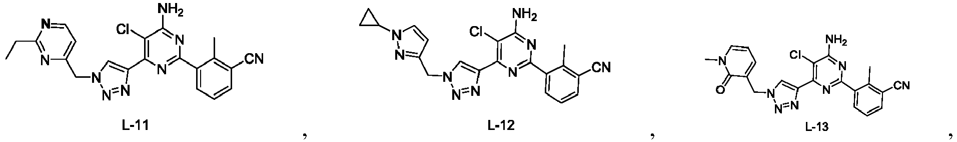 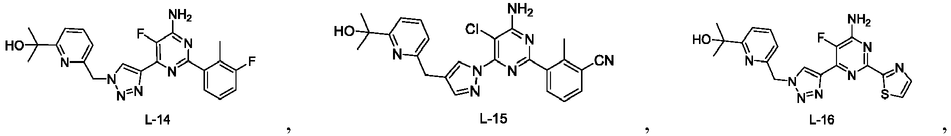 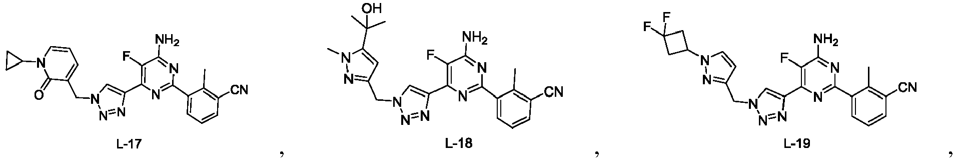 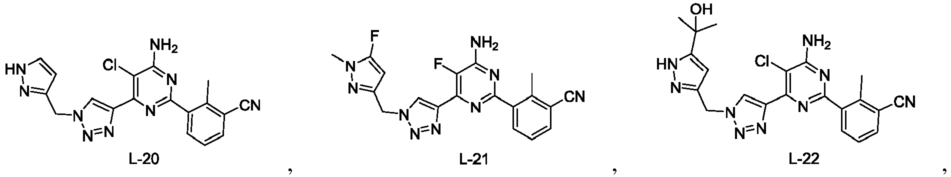 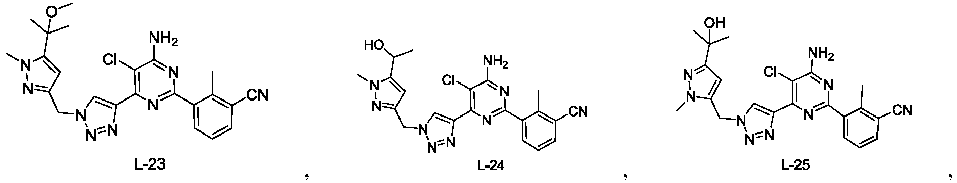 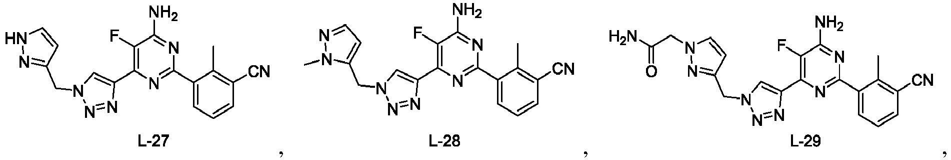 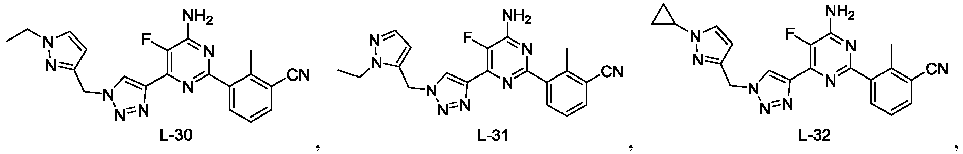 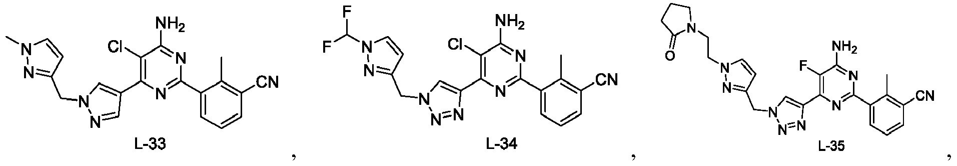 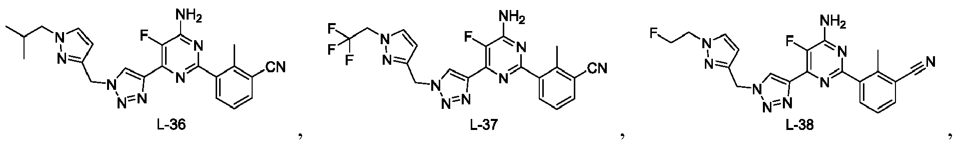 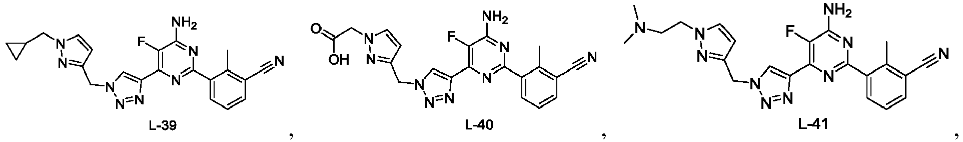 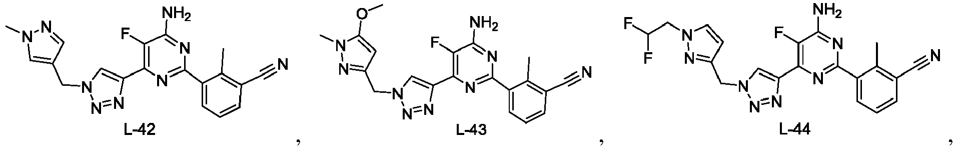 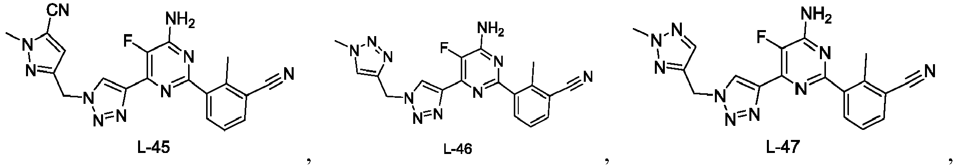 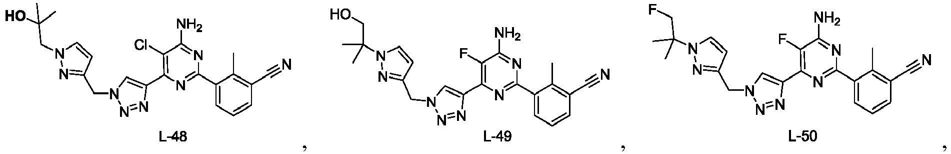 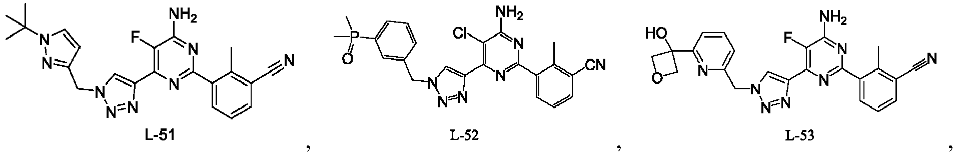 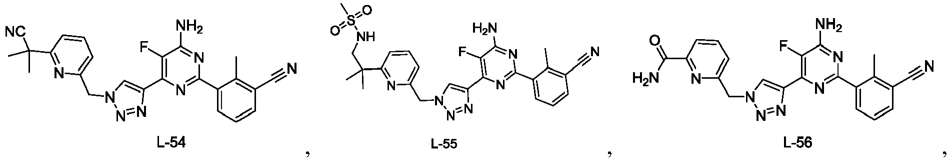 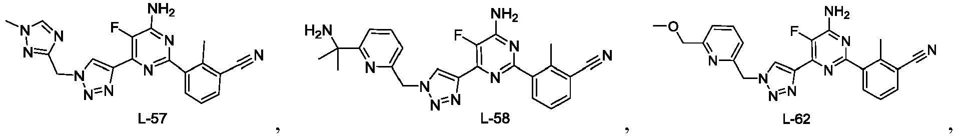 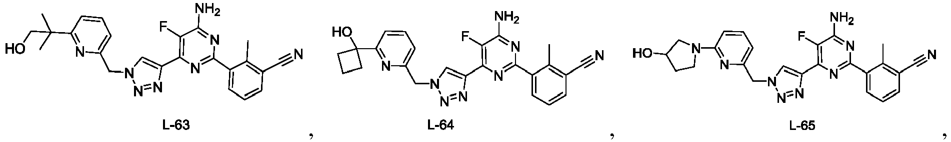 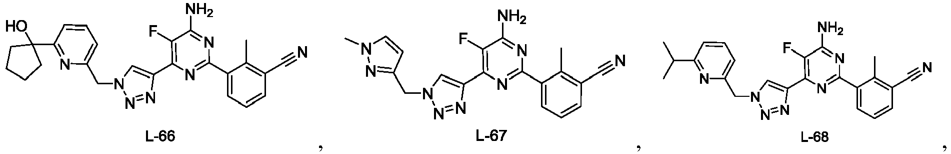 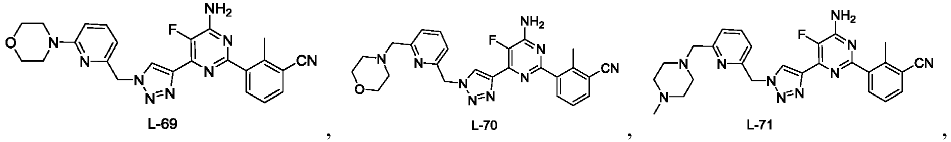 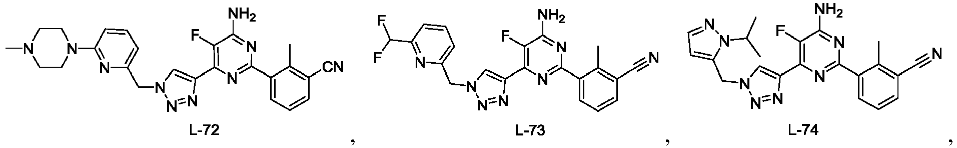 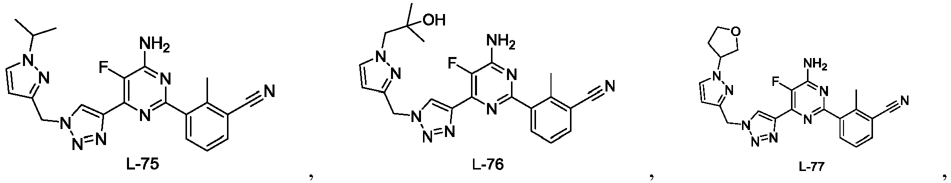 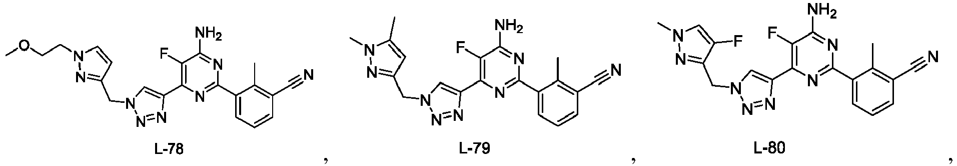 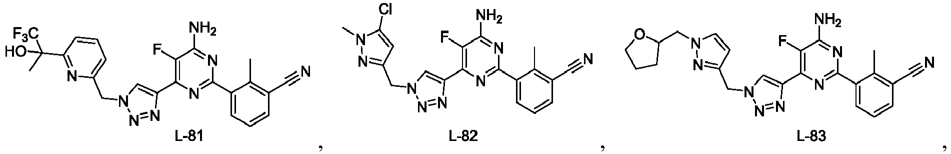 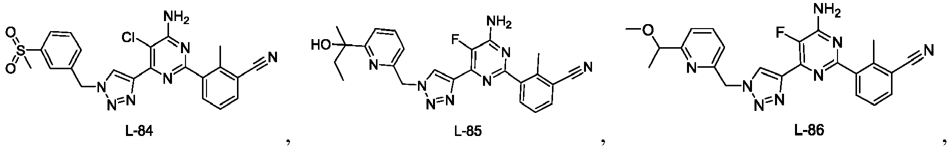 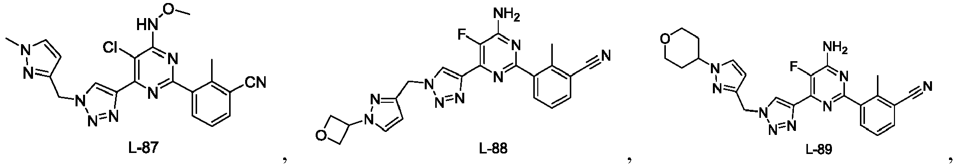 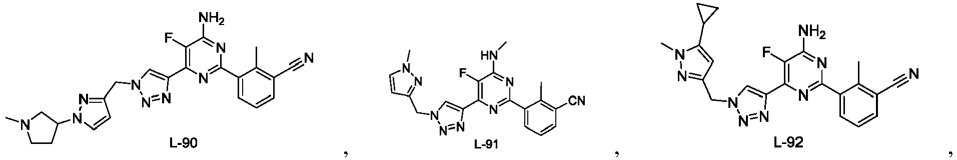 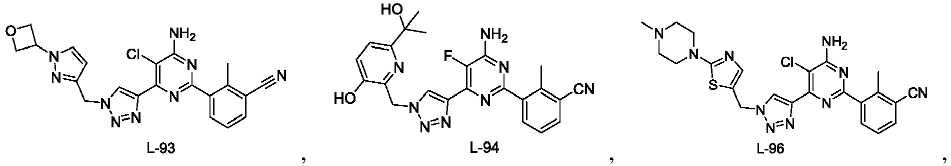 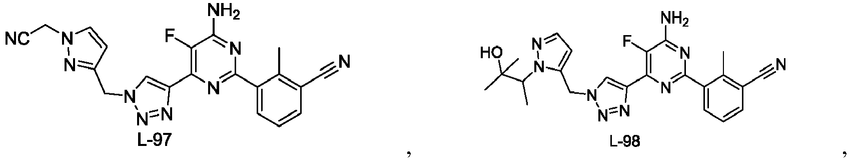 and

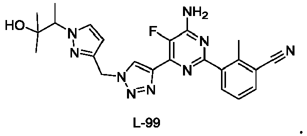

16. A pharmaceutical composition, comprising:
the compound, or the pharmaceutically acceptable salt, stereoisomer, solvate, or prodrug thereof according to any one of claims 1 to 15; and
a pharmaceutically acceptable carrier.

17. Use of the compound, or the pharmaceutically acceptable salt, stereoisomer, solvate, or prodrug thereof according to any one of claims 1 to 15, or the pharmaceutical composition according to claim 16 in preparation of a drug for treating an adenosine A_{2A} receptor and/or adenosine A_{2B} receptor-mediated disease.

18. The use according to claim 17, wherein the adenosine A_{2A} receptor and/or adenosine A_{2B} receptor-mediated disease is cancer or an immune-associated disease.

19. The use according claim 18, wherein:
the cancer is selected from the group consisting of prostate cancer, colon cancer, rectal cancer, pancreatic cancer, cervical cancer, stomach cancer, endometrial cancer, brain cancer, liver cancer, bladder cancer, ovarian cancer, testicular cancer, head cancer, neck cancer, melanoma, basal carcinoma, cancer of inner layer of mesothelium, leukemia, esophageal cancer, breast cancer, muscle cancer, connective tissue tumor, small cell lung cancer, non-small cell lung cancer, adrenal cancer, thyroid cancer, kidney cancer, and bone cancer; or
the cancer is selected from the group consisting of malignant gliomas, mesothelioma, renal cell carcinoma, stomach cancer, sarcoma, choriocarcinoma, skin basal cell carcinoma, and testicular seminoma.

20. The use according to claim 18, wherein the immune-associated disease is selected from the group consisting of rheumatoid arthritis, kidney failure, lupus, asthma, psoriasis, colitis, pancreatitis, allergies, fibrosis, anemic fibromyalgia, Alzheimer's disease, congestive heart failure, stroke, aortic valve stenosis, arteriosclerosis, osteoporosis, Parkinson's disease, infection, Crohn's disease, ulcerative colitis, allergic contact dermatitis and other eczema, systemic sclerosis, and multiple sclerosis.
